# EUROPÄISCHE PATENTSCHRIFT

(11) **EP 1 066 258 B1**
(45) Veröffentlichungstag und Bekanntmachung des Hinweises auf die Patenterteilung: **14.12.2005**
(21) Anmeldenummer: 99915653.2
(22) Anmeldetag: 18.03.1999
(51) Int. Cl.: C07D 209/96, A01N 43/08, A01N 43/10, A01N 43/12, A01N 43/36, A01N 43/42, C07D 207/40, C07D 491/10, C07D 309/28, C07D 307/24, C07D 307/94, C07D 333/50, C07C 49/733, C07C 49/88, C07C 49/747

(54) **ARYLPHENYLSUBSTITUIERTE CYCLISCHE KETOENOLE**
ARYL PHENYL SUBSTITUTED CYCLIC KETOENOLS
CETOENOLS CYCLIQUES A SUBSTITUTION ARYLPHENYLE

(30) Priorität: 26.03.1998 DE 19813354
(43) Veröffentlichungstag der Anmeldung: 10.01.2001
(73) Patentinhaber: Bayer CropScience AG, 40789 Monheim (DE)
(72) Erfinder: LIEB, Folker, D-51375 Leverkusen (DE); FISCHER, Reiner, D-40789 Monheim (DE); GRAFF, Alan, D-51061 Köln (DE); SCHNEIDER, Udo, D-51373 Leverkusen (DE); BRETSCHNEIDER, Thomas, D-53797 Lohmar (DE); ERDELEN, Christoph, D-42799 Leichlingen (DE); ANDERSCH, Wolfram, D-51469 Bergisch Gladbach (DE); DREWES, Mark, Wilhelm, D-40764 Langenfeld (DE); DOLLINGER, Markus, Overland Park, KS 66213 (US); WETCHOLOWSKY, Ingo, CEP-13 280 000 Vinhedo, SP (BR); MYERS, Randy, Allen, D-40489 Düsseldorf (DE)
(86) Internationale Anmeldenummer: PCT/EP1999/001787
(87) Internationale Veröffentlichungsnummer: WO 1999/048869

(56) Entgegenhaltungen:
- EP-A- 0 442 077
- EP-A- 0 521 334
- WO-A-96/20196
- WO-A-96/25395
- WO-A-97/14667
- WO-A-97/36868
- DE-A- 4 431 730
- DE-A- 19 543 864
- DE-A- 19 649 665
- US-A- 4 613 617
- US-A- 5 719 310

## Beschreibung

Die vorliegende Erfindung betrifft neue arylphenylsubstituierte cyclische Ketoenole, mehrere Verfahren zu ihrer Herstellung und ihre Verwendung als Schädlingsbekämpfungsmittel und Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-415 211) sowie substituierte monocyclische 3-Aryl-pyrrolidin-2,4-dion-Derivate (EP-A-377 893 und EP-442 077).

Weiterhin bekannt sind polycyclische 3-Arylpyrrolidin-2,4-dion-Derivate (EP-442 073) sowie 1H-Arylpyrrolidin-dion-Derivate (EP-456 063, EP-521 334, EP-596 298, EP-613 884, EP-613 885, WO 94/01 997, WO 95/26 954, WO 95/20 572, EP-A-0 668 267, WO 96/25 395, WO 96/35 664, WO 97/01 535, WO 97/02 243 und WO 97/36 868, DE-19 716 591).

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A-0 647 637, WO 95/26 345, WO 96/20196, WO 96/25 395, WO 96/35 664, WO 97/01 535, WO 97/02 243 und WO 97/36 868, DE 19 716 591 bekannt. Auch 3-Aryl-Δ³-dihydrothiphen-on-Derivate sind bekannt (WO 95/26 345, 96/25 395, WO 97/01 535, WO 97/02 243, WO 97/36 868, DE 19716591).

Aus der Literatur sind ferner bestimmte 3H-Pyrazol-3-on-Derivate, wie beispielsweise 1,2-Diethyl-1,2-dihydro-5-hydroxy-4-phenyl-3H-pyrazol-3-on oder {[5-Oxo-1,2-diphenyl-4-(p-sulfophenyl)-3-pyrazolin-3-yl]-oxy}-dinatriumsalz oder p-(3-Hydroxy-5-oxo-1,2-diphenyl-3-pyrazolin-4-yl)-benzolsulfonsäure bekannt (vgl. J. Heterocycl. Chem., 25(5), 1301-1305, 1988 oder J. Heterocycl. Chem., 25(5), 1307-1310, 1988 oder Zh. Obshch. Khim., 34(7), 2397-2402, 1964). Eine biologische Wirkung dieser Verbindungen wird aber nicht beschrieben.

Weiterhin ist bekannt, daß das Trinatriumsalz der 4,4',4"-(5-Hydroxy-3-oxo-1H-pyrazol-1,2,4(3H)-triyl)-tris-benzolsulfonsäure pharmakologische Eigenschaften besitzt (vgl. Farmakol. Toksikol. (Moscow), 38(2), 180-186, 1976). Seine Verwendung im Pflanzenschutz ist aber nicht bekannt.

Außerdem sind in EP-508 126 und in WO 92/16 510, WO 96/21 652 4-Arylpyrazolidin-3,5-dion-Derivate mit herbiziden, akariziden und insektiziden Eigenschaften beschrieben. Zudem wurden 4-Arylpyrazolidine bekannt, von denen fungizide Eigenschaften beschrieben wurden (WO 96/36 229, WO 96/36 615, WO 96/36 616, WO 96/36 633).

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137, WO 96/25395, WO 96/35664, WO 97/01 535, WO 97/02243, WO 97/16 436, WO 97/19 941 und WO 97/36 868, DE-19 716 591 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785, WO 96/02 539, WO 96/35 664, WO 97/01 535, WO 97/02 243, WO 97/02 243, WO 97/36 868 beschrieben.

Es ist bekannt, daß bestimmte substituierte 2-Arylcyclopentandione herbizide und akarizide Eigenschaften besitzen (vgl. z.B. US-4 283 348; 4 338 122; 4 436 666; 4 526 723; 4 551 547; 4 632 698; WO 96/01 798; WO 96/03 366 sowie WO 97/14 667). Außerdem sind ähnlich substituierte Verbindungen bekannt; 3-Hydroxy-5,5-dimethyl-2-phenylcyclopent-2-en-1-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66, (1973), 584 und der Offenlegungsschrift DE-2 361 084 bekannt, mit Angabe von herbiziden und akariziden Wirkungen.

Es ist bekannt, daß bestimmte substituierte 2-Arylcyclohexandione herbizide und akarizide Eigenschaften besitzen (US-4 175 135, 4 209 432, 4 256 657, 4 256 658, 4 256 659, 4 257 858, 4 283 348, 4 303 669, 4 351 666, 4 409 153, 4 436 666, 4 526 723, 4 613 617, 4 659 372, DE-2 813 341, sowie Wheeler, T.N., J. Org. Chem. 44, 4906 (1979)).

Die Wirksamkeit und Wirkungsbreite dieser Verbindungen ist jedoch insbesondere bei niedrigen Aufwandmengen und Konzentrationen nicht immer völlig zufriedenstellend. Weiterhin ist die Pflanzenverträglichkeit dieser Verbindungen nicht immer ausreichend.

Es wurden nun neue Verbindungen der Formel (I) 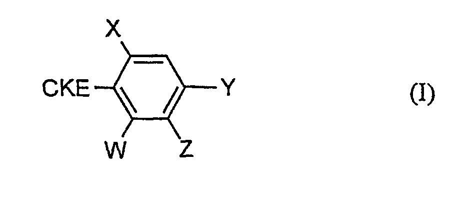 in welcher
- X: für Halogen, C₁-C₆-Alkyl, C₁-C₆-Halogenalkyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkoxy, C₃-C₆-Halogenalkenyloxy, Nitro, Cyano oder jeweils gegebenenfalls durch Halogen, C₁-C₆-Alkyl, C₁-C₆-Alkoxy, C₁-C₄-Halogenalkyl; C₁-C₄-Halogenalkoxy, Nitro oder Cyano substituiertes Phenyl, Phenoxy, Phenylthio, Benzyloxy oder Benzylthio,
- Z: für einen der Reste 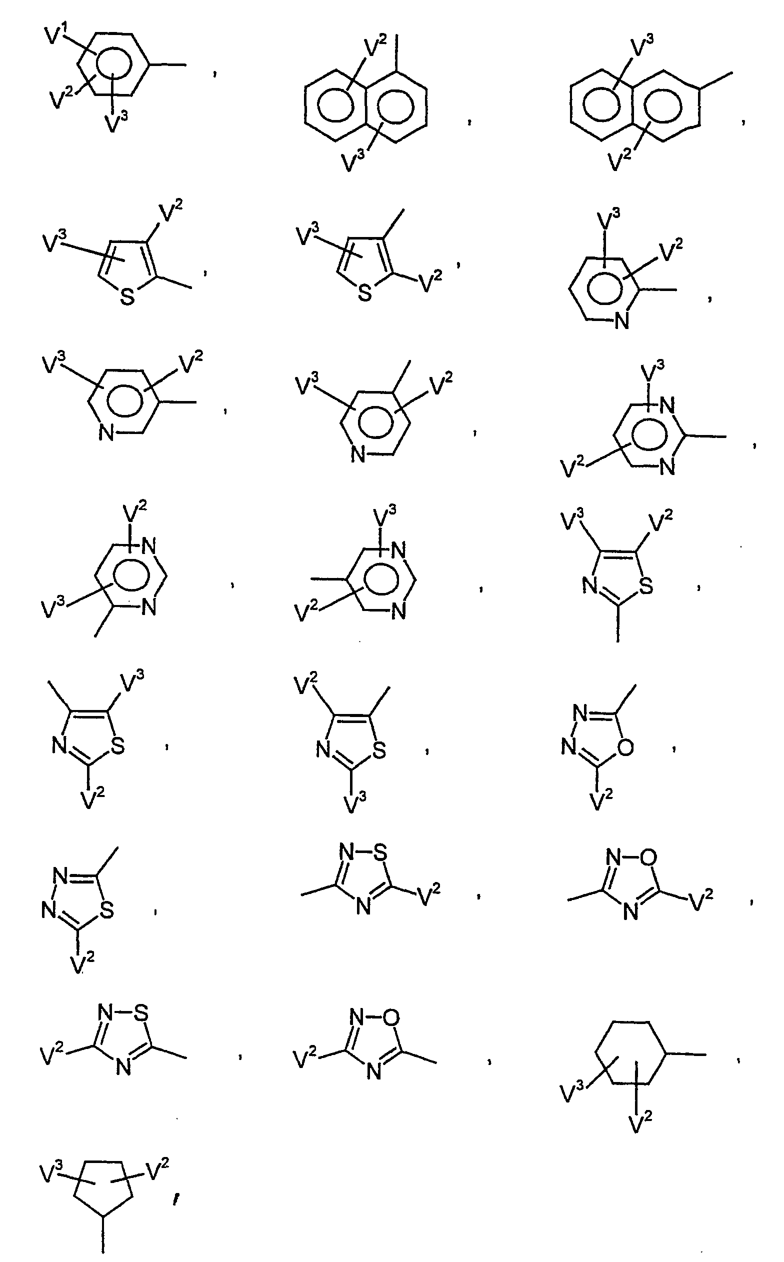
- V¹: für Wasserstoff, Halogen, C₁-C₁₂-Alkyl, C₁-C₆-Alkoxy, C₁-C₆-Alkylthio, C₁-C₆-Alkylsulfinyl, C₁-C₆-Alkylsulfonyl, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Akoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio,
- V² und V³: unabhängig voneinander für Wasserstoff, Halogen, C₁-C₆-Alkyl, C₁-C₆-Alkoxy, C₁-C₄-Halogenalkyl oder C₁-C₄-Halogenalkoxy,
- W und Y: unabhängig voneinander für Wasserstoff, Halogen, C₁-C₆-Alkyl, C₁-C₆-Halogenalkyl, C₁-C₆-Alkoxy, C₁-C₆-Halogenalkoxy, Nitro oder Cyano,
- CKE: für eine der Gruppen 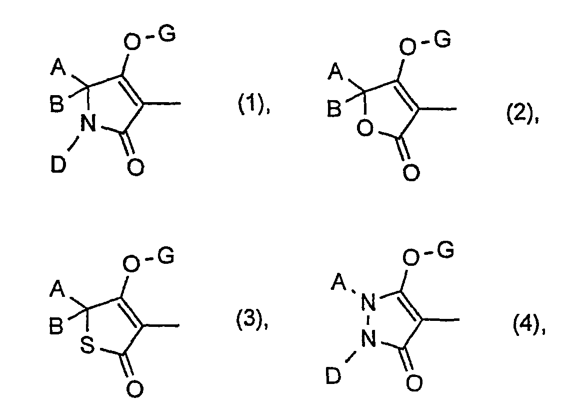 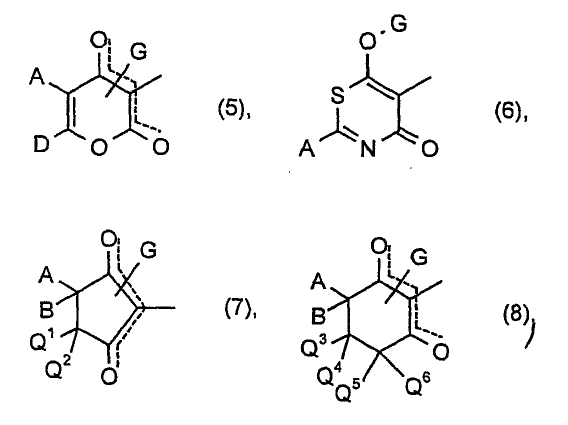
- A: für Wasserstoff oder für jeweils gegebenenfalls durch Halogen substituiertes C₁-C₁₂-Alkyl, C₃-C₈-Alkenyl, C₁-C₁₀-Alkoxy-C₁-C₈-alkyl, Poly-C₁-C₈-alkoxy-C₁-C₈-alkyl, C₁-C₁₀-Alkylthio-C₁-C₆-alkyl, gegebenenfalls durch Halogen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes C₆- oder C₁₀-Aryl (Phenyl oder Naphthyl), Hetaryl mit 5 oder 6 Ringatomen (beispielsweise Furanyl, Pyridyl, Imidazolyl, Triazolyl, Pyrazolyl, Pyrimidyl, Thiazolyl oder Thienyl) oder C₆- oder C₁₀-Aryl-C₁-C₆-alkyl (Phenyl-C₁-C₆-alkyl oder Naphthyl-C₁-C₆-alkyl),
- B: für Wasserstoff, C₁-C₁₂-Alkyl oder C₁-C₈-Alkoxy-C₁-C₆-alkyl oder
- A, B: und das Kohlenstoffatom an das sie gebunden sind, für gesättigtes C₃-C₁₀-Cycloalkyl oder ungesättigtes C₅-C₁₀-Cycloalkyl, worin gegebenenfalls ein Ringglied durch Sauerstoff oder Schwefel ersetzt ist und welche gegebenenfalls einfach oder zweifach durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
- A, B: und das Kohlenstoffatom, an das sie gebunden sind, für C₃-C₆-Cycloalkyl, welches durch eine gegebenenfalls ein oder zwei nicht direkt benachbarte Sauerstoff- und/oder Schwefelatome enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind, für C₃-C₈-Cycloalkyl oder C₅-C₈-Cycloalkenyl, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
- D: für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl, C₁-C₁₀-Alkylthio-C₂-C₈-alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 oder 6 Ringatomen (beispielsweise Furanyl, Imidazolyl, Pyridyl, Thiazolyl, Pyrazolyl, Pyrimidyl, Pyrrolyl, Thienyl oder Triazolyl), Phenyl-C₁-C₆-alkyl oder Hetaryl-C₁-C₆-alkyl mit 5 oder 6 Ringatomen (beispielsweise Furanyl-, Imidazolyl-, Pyridyl-, Thiazolyl-, Pyrazolyl-, Pyrimidyl-, Pyrrolyl-, Thienyl- oder Triazolyl-C₁-C₆-alkyl) oder
- A und D: für jeweils gegebenenfalls substituiertes C₃-C₆-Alkandiyl oder C₃-C₆-Alkendiyl, in welchen gegebenenfalls eine Methylengruppe durch Sauerstoff oder Schwefel ersetzt ist und wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppe, C₃-C₆-Alkendiylgruppe oder eine Butadienylgruppe, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Ring mit 5 oder 6 Ringatomen bilden (im Fall der Verbindung der Formel (I-1) stehen A und D dann gemeinsam mit den Atomen, an die sie gebunden sind beispielsweise für die weiter unten genannten Gruppen AD-1 bis AD-10), der Sauerstoff oder Schwefel enthalten kann, oder worin gegebenenfalls eine der folgenden Gruppen 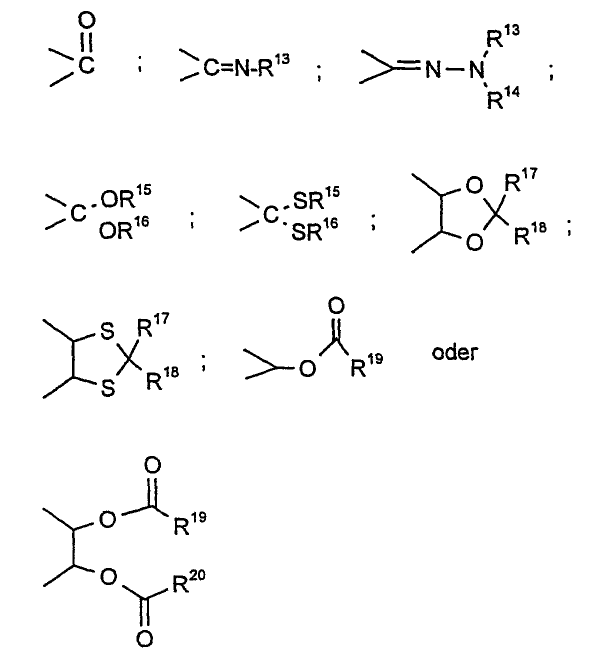
enthalten ist, oder
- A und Q¹: für jeweils gegebenenfalls einfach oder zweifach, gleich oder verschieden durch Halogen, Hydroxy, durch jeweils gegebenenfalls einfach bis dreifach, gleich oder verschieden durch Halogen substituiertes C₁-C₁₀-Alkyl, C₁-C₆-Alkoxy, C₁-C₆-Alkylthio, C₃-C₇-Cycloalkyl oder durch jeweils gegebenenfalls einfach bis dreifach, gleich oder verschieden durch Halogen, C₁-C₆-Alkyl oder C₁-C₆-Alkoxy substituiertes Benzyloxy oder Phenyl substituiertes C₃-C₆-Alkandiyl oder C₄-C₆-Alkendiyl, welches außerdem gegebenenfalls eine der nachstehenden Gruppen 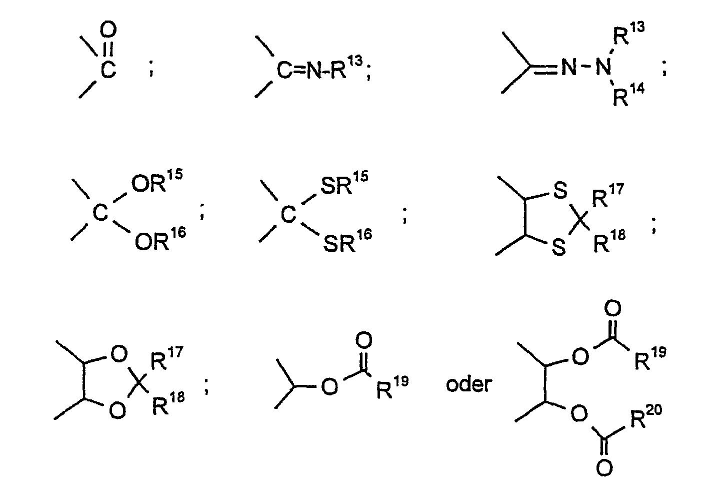 enthält oder durch eine C₁-C₂-Alkandiylgruppe oder durch ein Sauerstoffatom überbrückt ist oder
- Q¹: für Wasserstoff oder C₁-C₄-Alkyl,
- Q², Q⁴, Q⁵ und Q⁶: unabhängig voneinander für Wasserstoff oder C₁-C₄-Alkyl,
- Q³: für Wasserstoff, C₁-C₆-Alkyl, C₁-C₆-Alkoxy-C₁-C₂-akyl, C₁-C₆-Alkylthio-C₁-C₂-alkyl, gegebenenfalls durch C₁-C₄-Alkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl oder
- Q³ und Q⁴: gemeinsam mit dem Kohlenstoffatom, an das sie gebunden sind, für einen gegebenenfalls durch C₁-C₄-Alkyl, C₁-C₄-Alkoxy oder C₁-C₂-Halogenalkyl substituierten C₃-C₇-Ring, in welchem gegebenenfalls ein Ringglied durch Sauerstoff oder Schwefel ersetzt ist,
- G: für Wasserstoff (a) oder für eine der Gruppen 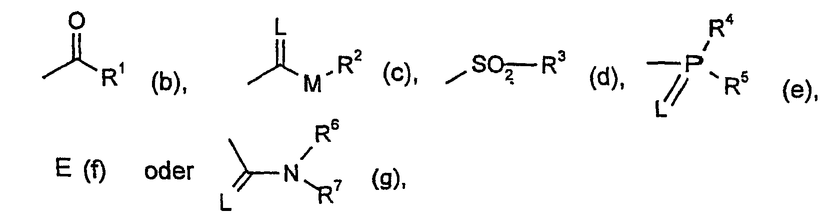 insbesondere für (a), (b) oder (c),
in welchen
E für ein Metallion oder ein Ammoniumion,
L für Sauerstoff oder Schwefel und
M für Sauerstoff oder Schwefel,
- R¹: für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere (bevorzugt nicht mehr als zwei) nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl (beispielsweise Pyrazolyl, Thiazolyl, Pyridyl, Pyrimidyl, Furanyl oder Thienyl),
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl (beispielsweise Pyridyloxy-C₁-C₆-alkyl, Pyrimidyloxy-C₁-C₆-alkyl oder Thiazolyloxy-C₁-C₆-alkyl),
- R²: für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl,
- R³: für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
- R⁴ und R⁵: unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Akoxy, C₁-C₄-Halogenakoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio,
- R⁶ und R⁷: unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist,
- R¹³: für Wasserstoff, für jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl oder Phenyl-C₁-C₄-alkoxy,
- R¹⁴: für Wasserstoff oder C₁-C₈-Alkyl oder
- R¹³ und R¹⁴: gemeinsam für C₄-C₆-Alkandiyl,
- R¹⁵ und R¹⁶: gleich oder verschieden und für C₁-C₆-Alkyl oder
- R¹⁵ und R¹⁶: gemeinsam für einen C₂-C₄-Alkandiylrest, der gegebenenfalls durch C₁-C₆-Alkyl, C₁-C₆-Halogenalkyl oder durch gegebenenfalls durch Halogen, C₁-C₆-Alkyl, C₁-C₄-Halogenalkyl, C₁-C₆-Alkoxy, C₁-C₄-Halogenalkoxy, Nitro oder Cyano substituiertes Phenyl substituiert ist,
- R¹⁷ und R¹⁸: unabhängig voneinander für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl oder
- R¹⁷ und R¹⁸: gemeinsam mit dem Kohlenstoffatom, an das sie gebunden sind, für eine Carbonylgruppe oder für gegebenenfalls durch Halogen, C₁-C₄-Alkyl oder C₁-C₄-Alkoxy substituiertes C₅-C₇-Cycloalkyl, in dem gegebenenfalls eine Methylengruppe durch Sauerstoff oder Schwefel ersetzt ist,
- R¹⁹ und R²⁰: unabhängig voneinander für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino,
stehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (8) der Gruppe CKE ergeben sich folgende hauptsächliche Strukturen (I-1) bis (I-8): 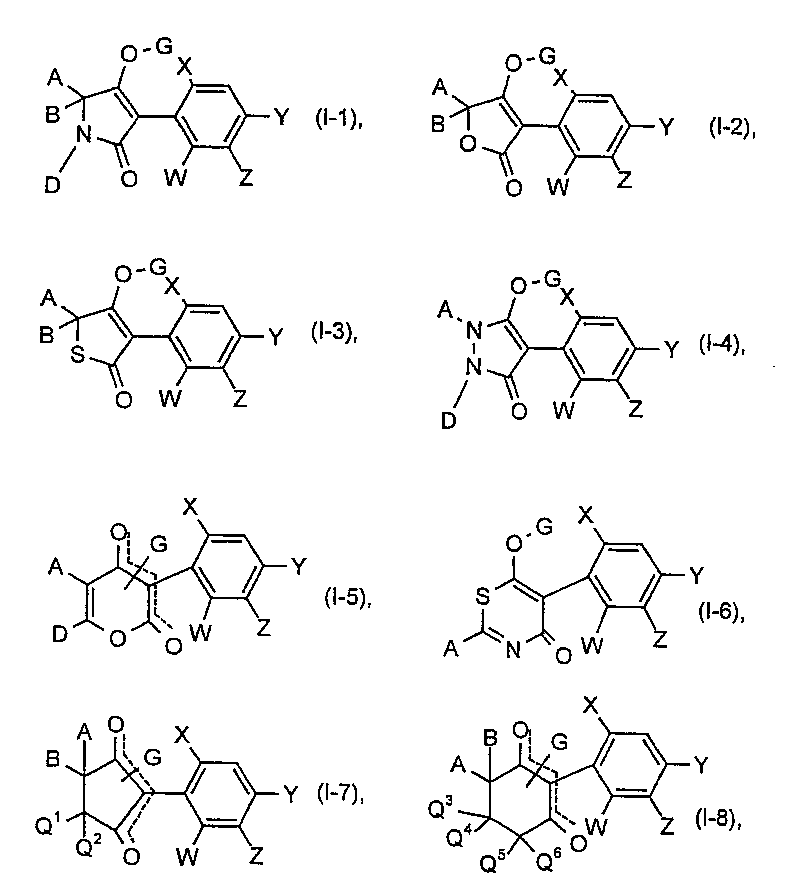 worin

A, B, D, G, Q¹, Q², Q³, Q⁴, Q⁵, Q⁶, W, X, Y und Z die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn CKE für die Gruppe (1) steht, 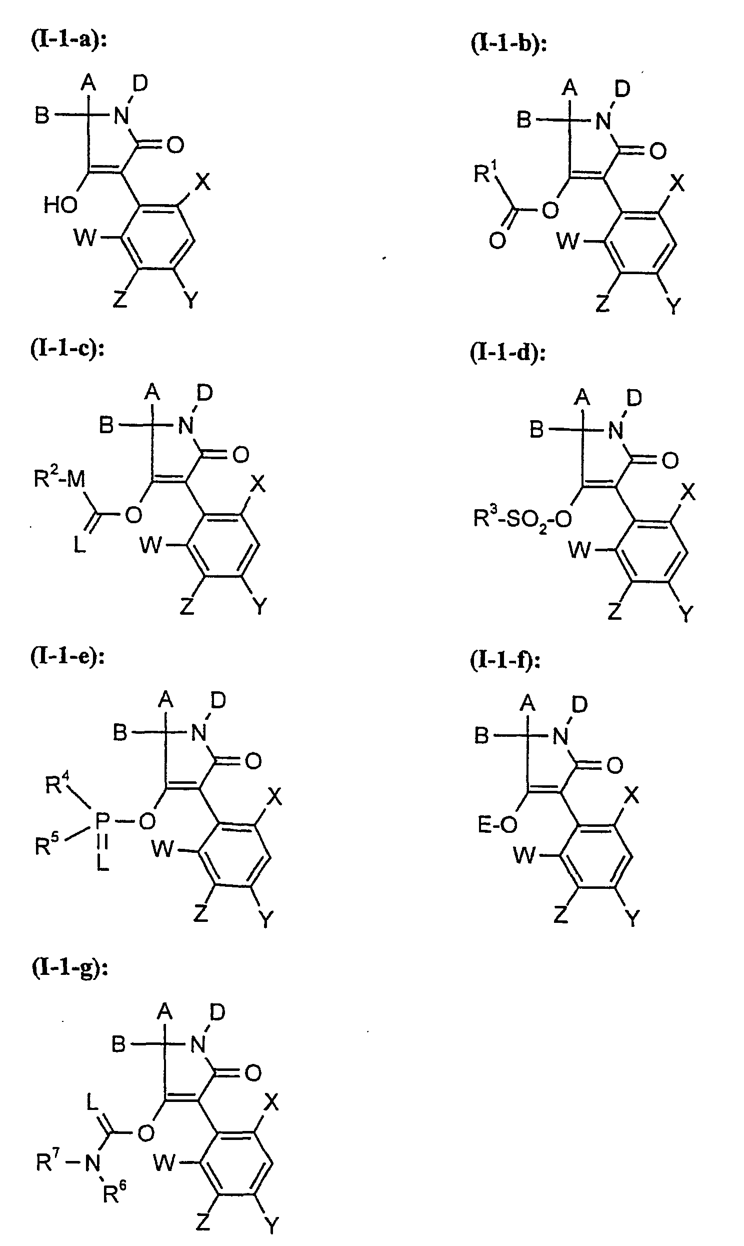 worin
A, B, D, E, L, M, W, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn CKE für die Gruppe (2) steht, 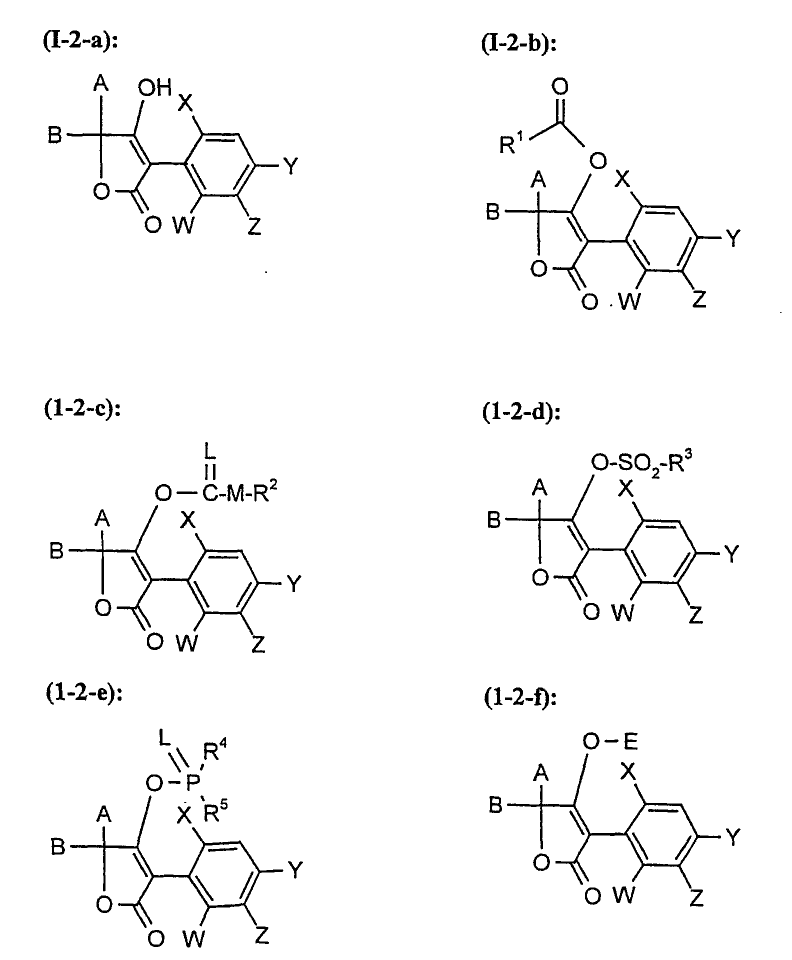 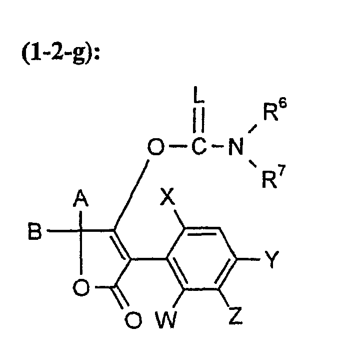 worin
A, B, E, L, M, W, X, Y, Z, R¹, R², R³, R⁴, R⁵, R⁶ und R⁷ die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-3-a) bis (I-3-g), wenn CKE für die Gruppe (3) steht, 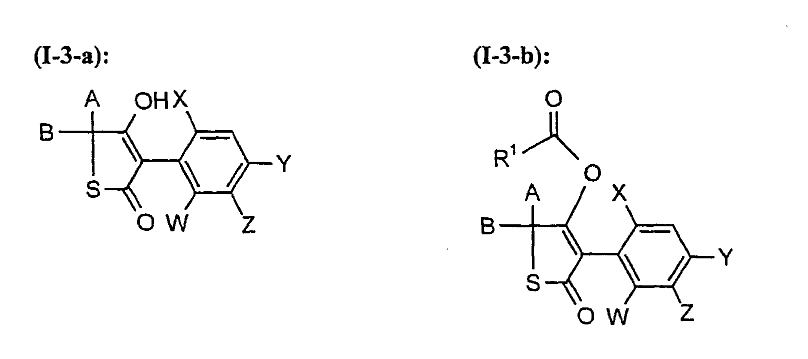 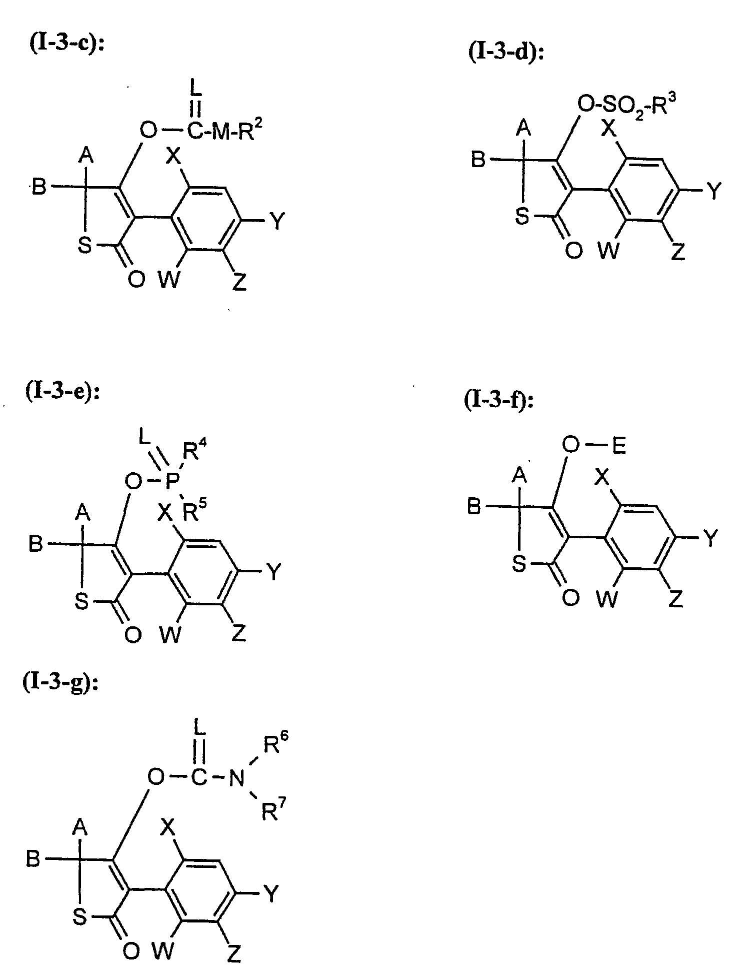 worin
A, B, E, L, M, W, X, Y, Z, R¹, R², R³, R⁴, R⁵, R⁶ und R⁷ die oben angegebenen Bedeutung besitzen.

Unter Einbeziehung der verschiedenen Bedeutungen (a), (b), (c), (d), (e), (f) und (g) der Gruppe G ergeben sich folgende hauptsächliche Strukturen (I-4-a) bis (I-4-g), wenn CKE für die Gruppe (4) steht, 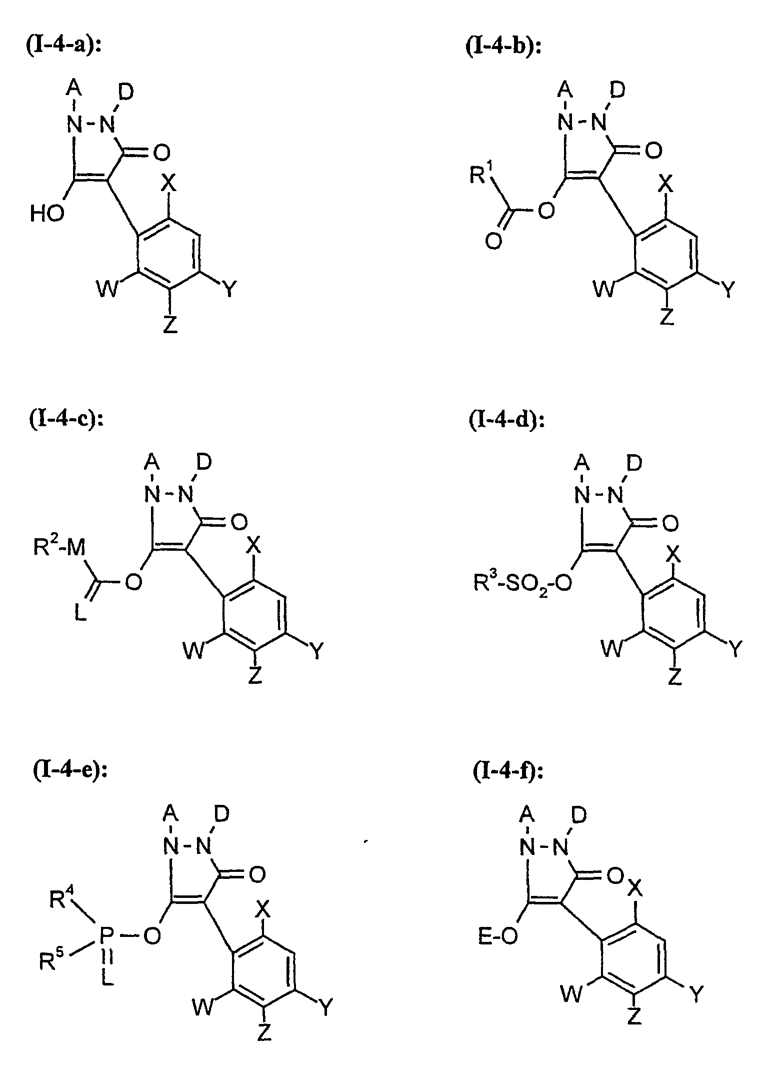 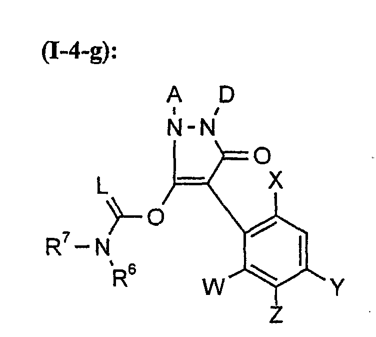 worin
A, D, E, L, M, W, X, Y, Z, R¹, R², R³, R⁴, R⁵, R⁶ und R⁷ die oben angegebenen Bedeutungen besitzen.

Die Verbindungen der Formel (1-5) können in Abhängigkeit von der Stellung des Substituenten G in den zwei isomeren Formen der Formeln (I-5-A) und (I-5-B) vorliegen, 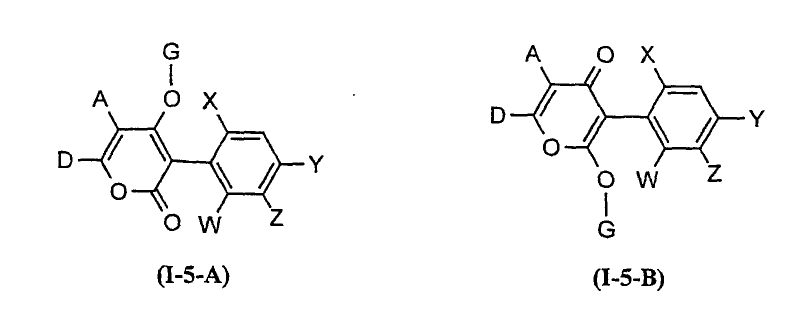 was durch die gestrichelte Linie in der Formel (1-5) zum Ausdruck gebracht werden soll.

Die Verbindungen der Formeln (I-5-A) und (1-5-B) können sowohl als Gemische als auch in Form ihrer reinen Isomeren vorliegen. Gemische der Verbindungen der Formeln (1-5-A) und (I-5-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-5-a) bis (I-5-g), wenn CKE für die Gruppe (5) steht, 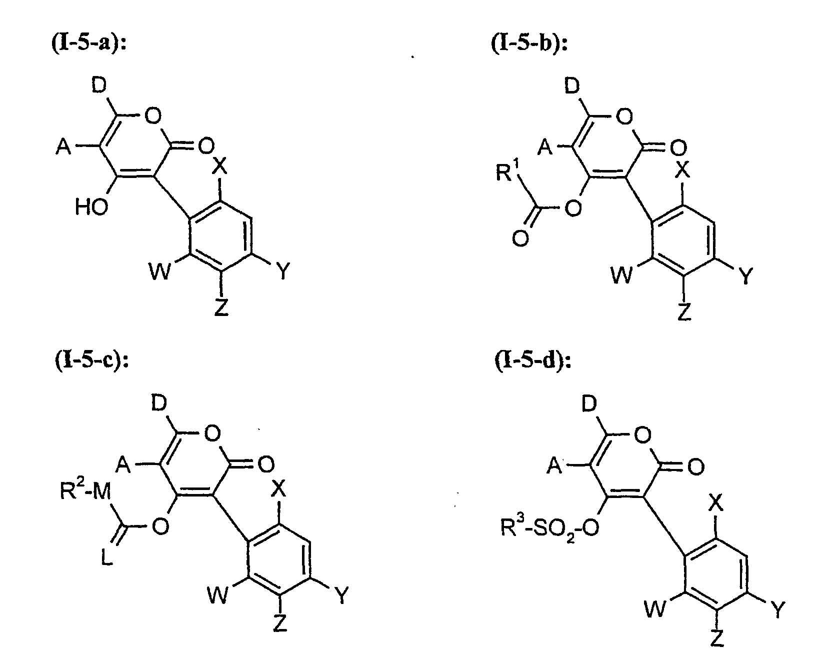 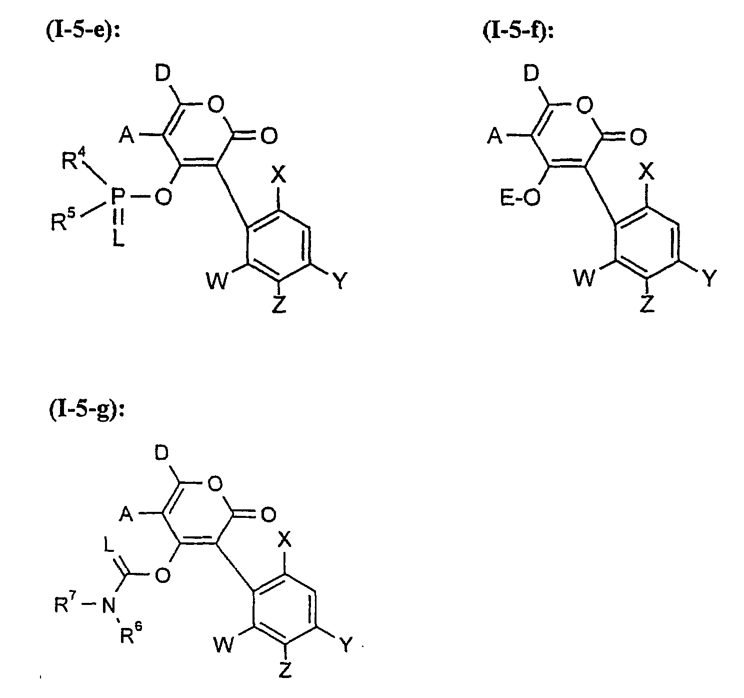 worin
A, D, E, L, M, W, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-6-a) bis (I-6-g), wenn CKE für die Gruppe (6) steht, 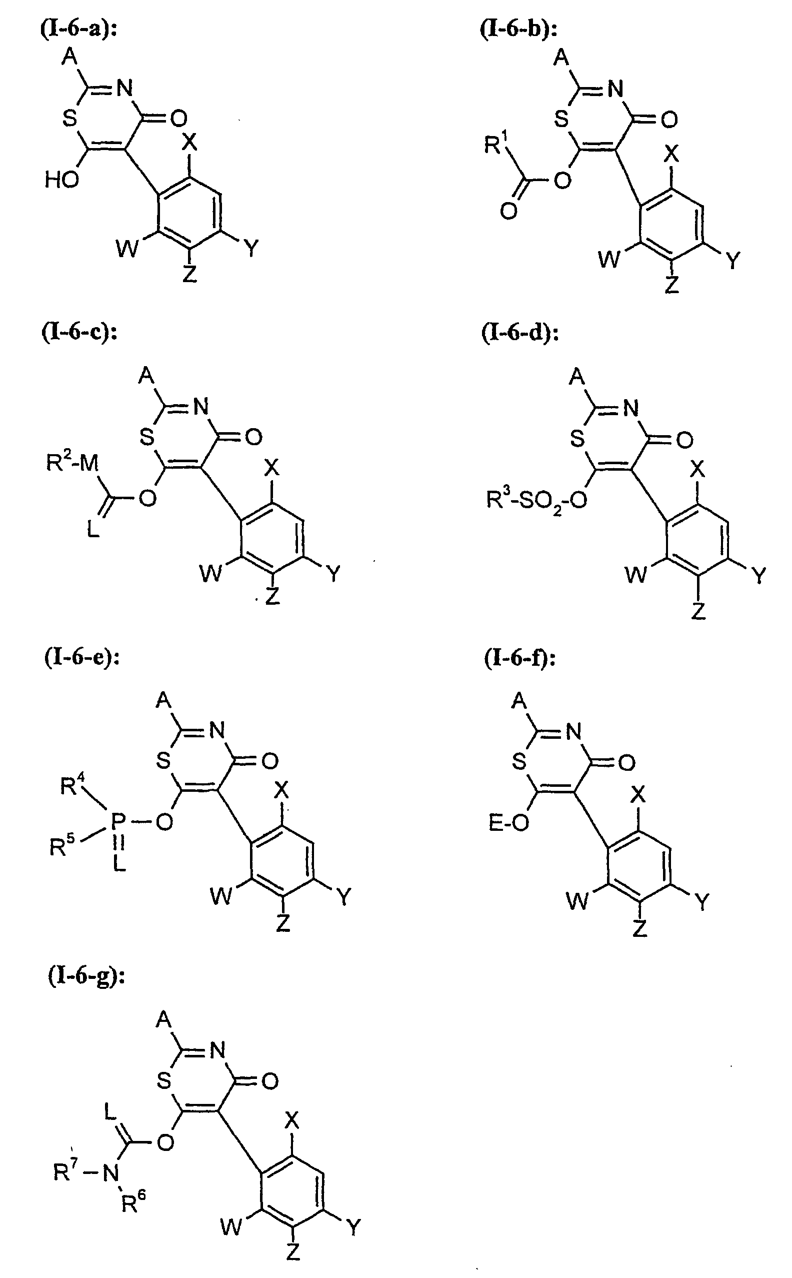 worin
A, E, L, M, W, X, Y, Z, R¹, R², R³, R⁴, R⁵, R⁶ und R⁷ die oben angegebenen Bedeutungen besitzen.

Die Verbindungen der Formel (I-7) können in Abhängigkeit von der Stellung des Substituenten G in den zwei isomeren Formen der Formeln (I-7-A) und (I-7-B) vorliegen, 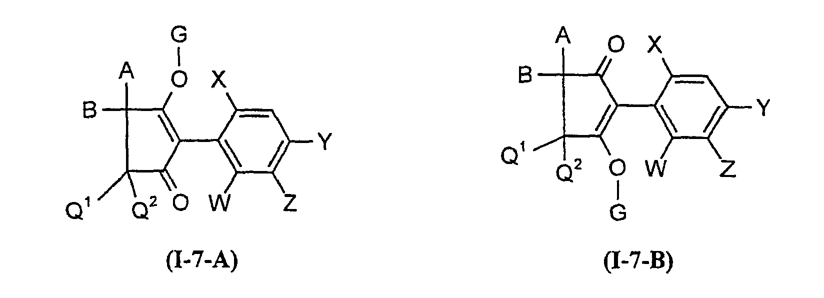 was durch die gestrichelte Linie in der Formel (I) zum Ausdruck gebracht werden soll.

Die Verbindungen der Formeln (I-7-A) und (I-7-B) können sowohl als Gemische als auch in Form ihrer reinen Isomeren vorliegen. Gemische der Verbindungen der Formeln (I-7-A) und (I-7-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächlichen Strukturen (I-7-a) bis (I-7-g): 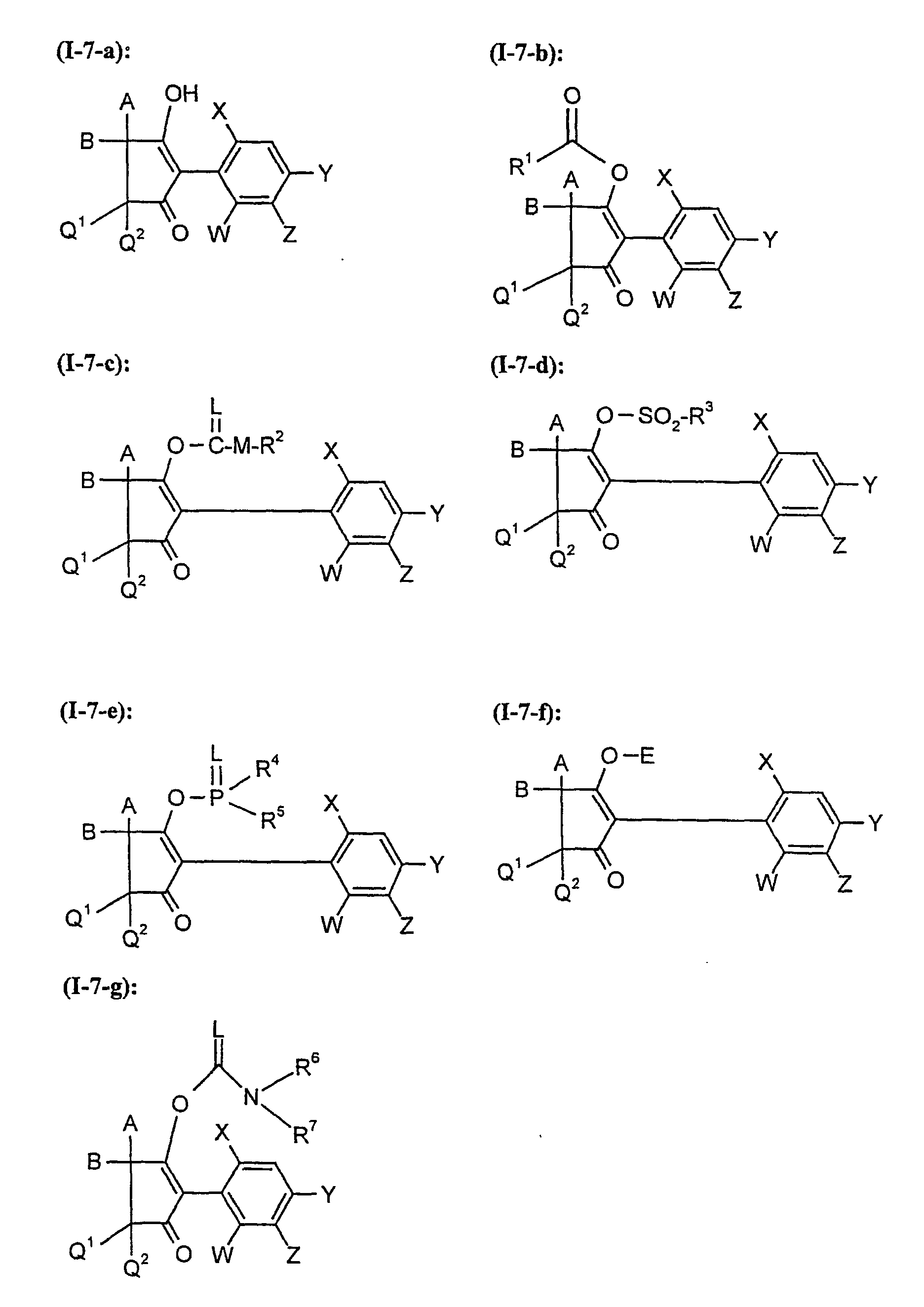 worin
A, B, Q¹, Q², E, L, M, W, X, Y, Z, R¹, R², R³, R⁴, R⁵, R⁶ und R⁷ die oben angegebenen Bedeutungen besitzen.

Die Verbindungen der Formel (I-8) können in Abhängigkeit von der Stellung des Substituenten G in den zwei isomeren Formen der Formeln (I-8-A) bzw. (I-8-B) vorliegen, was durch die gestrichelte Linie in der Formel (I-8) zum Ausdruck gebracht werden soll:

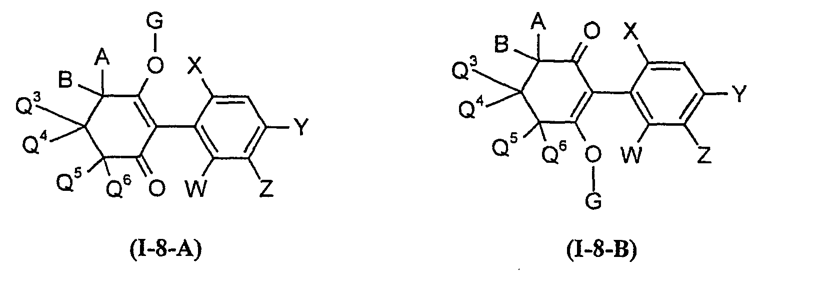

Die Verbindungen der Formeln (I-8-A) bzw. (I-8-B) können sowohl als Gemische als auch in Form ihrer reinen Isomeren vorliegen. Gemische der Verbindungen der Formeln (I-8-A) und (I-8-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt ein, daß die betreffende Verbindung gegebenenfalls als Isomerengemisch oder in der jeweils anderen isomeren Form vorliegen kann.

Unter Einbeziehung der verschiedenen Bedeutungen (a), (b), (c), (d), (e), (f) und (g) der Gruppe G ergeben sich folgende hauptsächlichen Strukturen (I-8-a) bis (I-8-g): 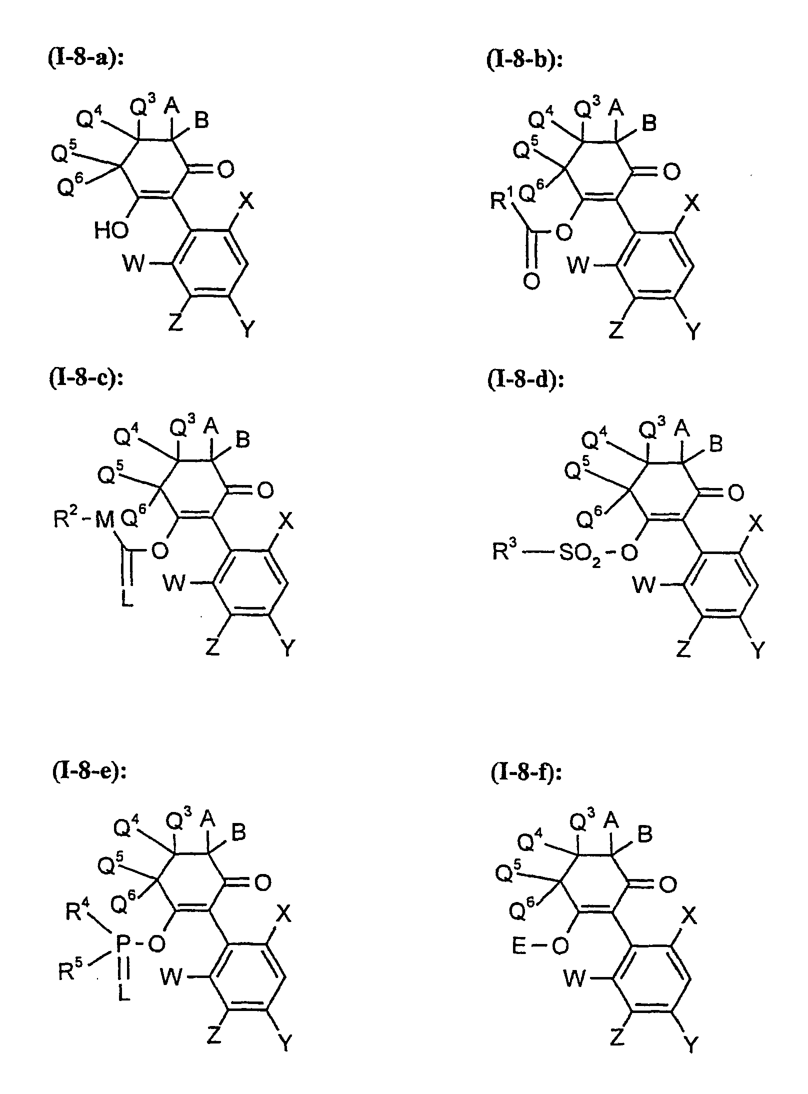 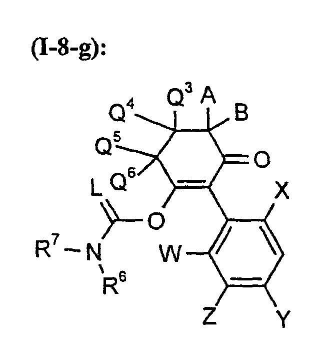 worin
A, B, E, L, M, Q³, Q⁴, Q⁵, Q⁶, W, X, Y, Z, R¹, R², R³, R⁴, R⁵, R⁶ und R⁷ die oben angegebenen Bedeutungen besitzen.

Weiterhin wurde gefunden, daß man die neuen Verbindungen der Formel (I) nach einem der im folgenden beschriebenen Verfahren erhält:
(A) Man erhält substituierte 3-Phenylpyrrolidin-2,4-dione bzw. deren Enole der Formel (I-1-a) 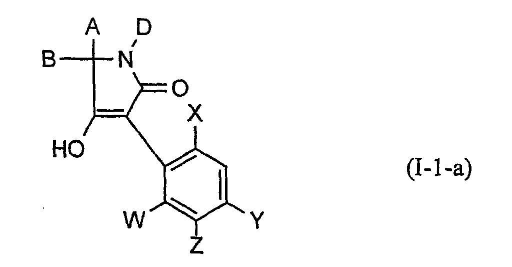 in welcher
   A, B, D, W, X, Y und Z die oben angegebenen Bedeutungen haben,
   wenn man
   N-Acylaminosäureester der Formel (II) 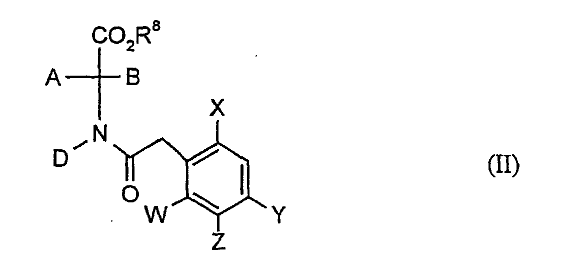 in welcher
   A, B, D, W, X, Y und Z die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, daß man substituierte 3-Phenyl-4-hydroxy-Δ³-dihydrofuranon-Derivate der Formel (I-2-a) 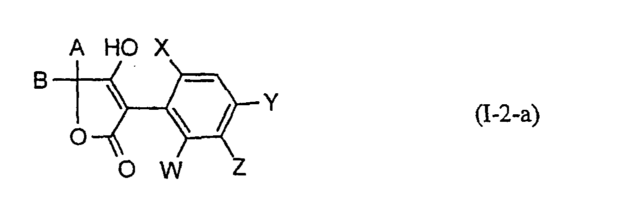 in welcher
   A, B, W, X, Y und Z die oben angegebenen Bedeutungen haben,
   erhält, wenn man
   Carbonsäureester der Formel (III) 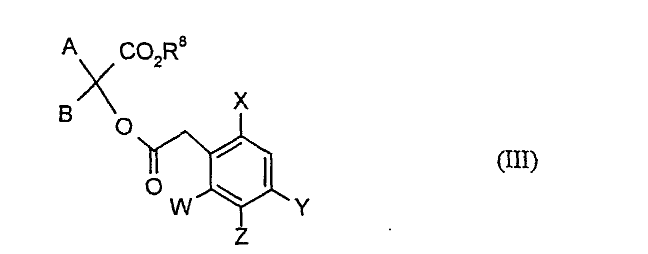 in welcher
   A, B, W, X, Y, Z und R⁸ die oben angegebenen Bedeutungen haben,
   in Gegenwart eines Verdünnungsmittels und in Gegenwart einer Base intramolekular kondensiert.
(C) Weiterhin wurde gefunden, daß man substituierte 3-Phenyl-4-hydroxy-Δ³-dihydrothiophenon-Derivate der Formel (I-3-a) 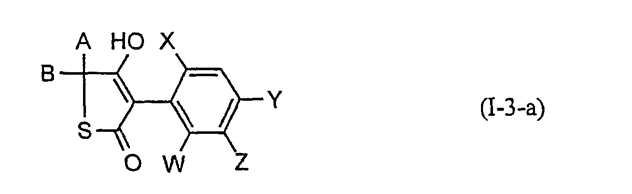 in welcher
   A, B, W, X, Y und Z die oben angegebenen Bedeutungen haben,
   erhält, wenn man
   ß-Ketocarbonsäureester der Formel (IV) 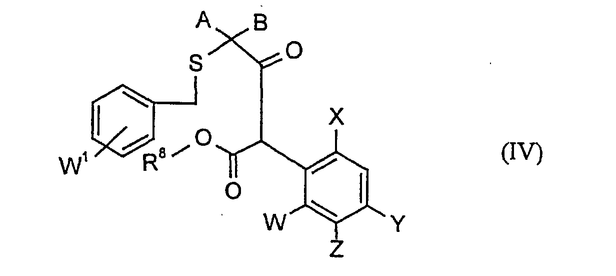 in welcher
   A, B, W, X, Y, Z und R⁸ die oben angegebenen Bedeutungen haben und
   - W¹: für Wasserstoff, Halogen, Alkyl (bevorzugt C₁-C₆-Alkyl) oder Alkoxy (bevorzugt C₁-C₈-Alkoxy) steht,
   gegebenenfalls in Gegenwart eines Verdünnungsmittels und in Gegenwart einer Säure intramolekular cyclisiert.
(D) Weiterhin erhält man substituierte 3-Hydroxy-4-phenyl-5-oxo-pyrazoline der Formel (I-4-a) 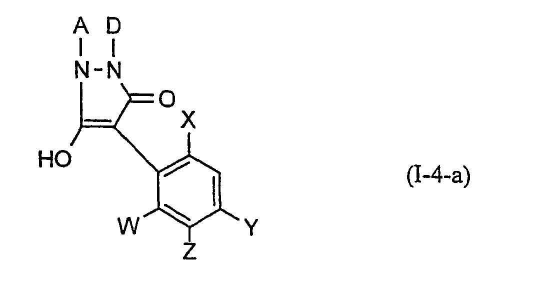 in welcher
   A, D, W, X, Y und Z die oben angegebenen Bedeutungen haben,
   wenn man
(α) Halogencarbonylketene der Formel (V) 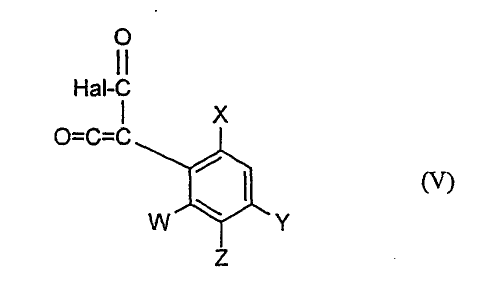 in welcher
   W, X, Y und Z die oben angegebenen Bedeutungen haben
   und
   - Hal: für Halogen (insbesondere Chlor oder Brom) steht,
   oder
(β) Malonsäurederivate der Formel (VI) 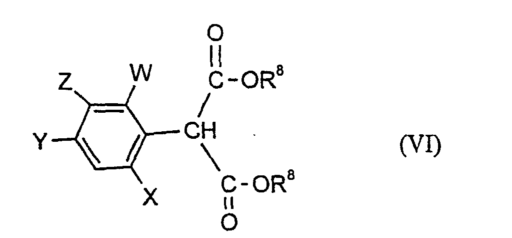 in welcher
   R⁸, W, X, Y und Z die oben angegebenen Bedeutungen haben,
   mit Hydrazinen der Formel (VII)

   A-NH-NH-D (VII)

   in welcher
   - A und D: die oben angegebenen Bedeutungen haben,
   gegebenenfalls in Gegenwart eines Verdünnungsmittels und gegebenenfalls in Gegenwart einer Base umsetzt.
(E) Weiterhin wurde gefunden, daß man die neuen substituierten 3-Phenylpyron-Derivate der Formel (I-5-a) 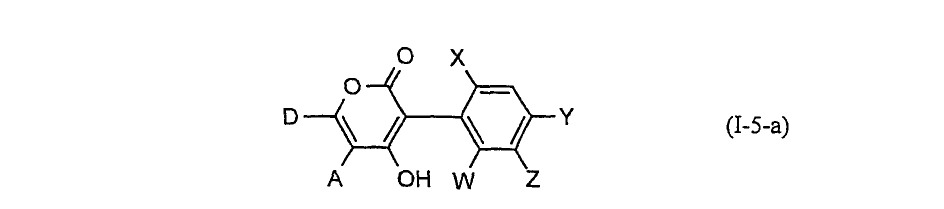 in welcher
   A, D, W, X, Y und Z die oben angegebenen Bedeutungen haben,
   erhält, wenn man
   Carbonylverbindungen der Formel (VIII) 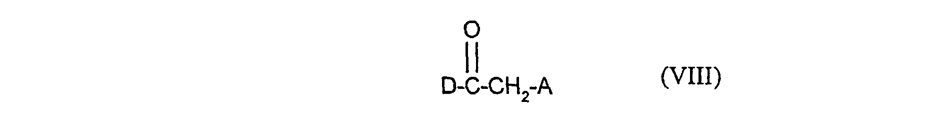 in welcher
   - A und D: die oben angegebenen Bedeutungen haben,
   oder deren Silylenolether der Formel (VIIIa) 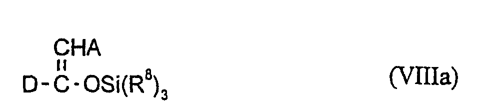 in welcher
   A, D und R⁸ die oben angegebene Bedeutung haben,
   mit Ketensäurehalogeniden der Formel (V) 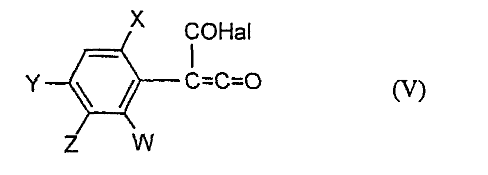 in welcher
   W, X, Y und Z die oben angegebenen Bedeutungen haben und
   - Hal: für Halogen (vorzugsweise für Chlor oder Brom) steht,
   gegebenenfalls in Gegenwart eines Verdünnungsmittels und gegebenenfalls in Gegenwart eines Säureakzeptors umsetzt.
   Weiterhin wurde gefunden,
(F) daß man die neuen substituierten Phenyl-1,3-thiazin-Derivate der Formel (I-6-a) 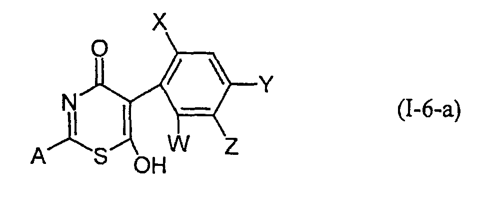 in welcher
   A, W, X, Y und Z die oben angegebene Bedeutung haben,
   erhält, wenn man Thioamide der Formel (IX) 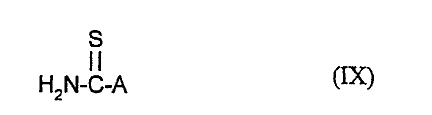 in welcher
   - A: die oben angegebene Bedeutung hat,
   mit Ketensäurehalogeniden der Formel (V) 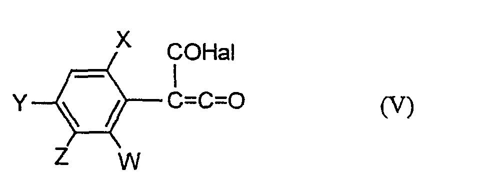 in welcher
   Hal, W, X, Y und Z die oben angegebenen Bedeutungen haben,
   gegebenenfalls in Gegenwart eines Verdünnungsmittels und gegebenenfalls in Gegenwart eines Säureakzeptors umsetzt.
   Weiterhin wurde gefunden,
(G) daß man Verbindungen der Formel (I-7-a) 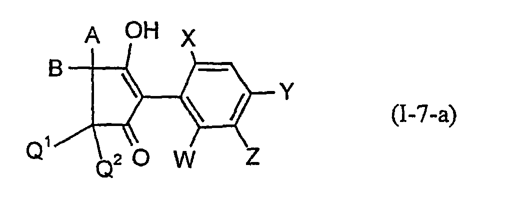 in welcher
   A, B, Q¹, Q², W, X, Y und Z die oben angegebene Bedeutung haben,
   erhält, wenn man
   Ketocarbonsäureester der Formel (X) 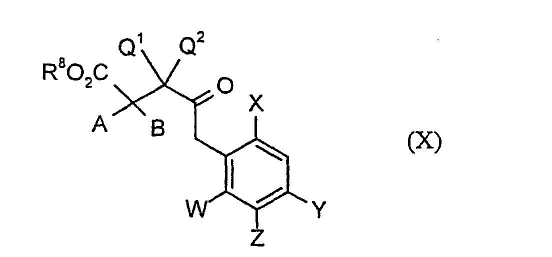 in welcher
   A, B, Q¹, Q², W, X, Y und Z die oben angegebene Bedeutung haben, und
   - R⁸: für Alkyl (insbesondere C₁-C₈-Alkyl) steht,
   gegebenenfalls in Gegenwart eines Verdünnungsmittels und in Gegenwart einer Base intramolekular cyclisiert.
   Außerdem wurde gefunden,
(H) daß man Verbindungen der Formel (I-8-a) 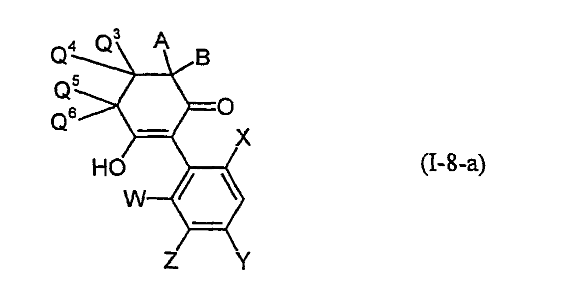 in welcher
   A, B, Q³, Q⁴, Q⁵, Q⁶, W, X, Y und Z die oben angegebene Bedeutung haben,
   erhält, wenn man
   6-Aryl-5-keto-hexansäureester der Formel (XI) 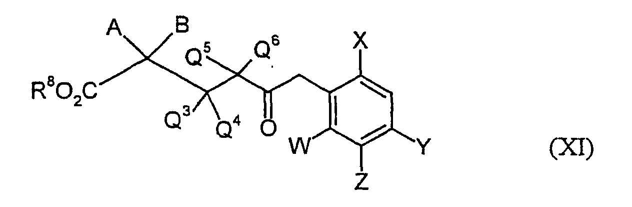 in welcher
   A, B, Q³, Q⁴, Q⁵, Q⁶, W, X, Y und Z die oben angegebene Bedeutung haben
   und
   - R⁸: für Alkyl (bevorzugt C₁-C₆-Alkyl) steht,
   in Gegenwart eines Verdünnungsmittels und in Gegenwart einer Base intramolekular kondensiert;
(I) daß man Verbindungen der oben gezeigten Formeln (I-1-a) bis (I-8-a), in welchen A, B, D, Q¹, Q², Q³, Q⁴, Q⁵, Q⁶, W, X, Y und Z die oben angegebene Bedeutung haben, erhält, wenn man Verbindungen der Formel (I-1'-a) bis (I-8'-a), 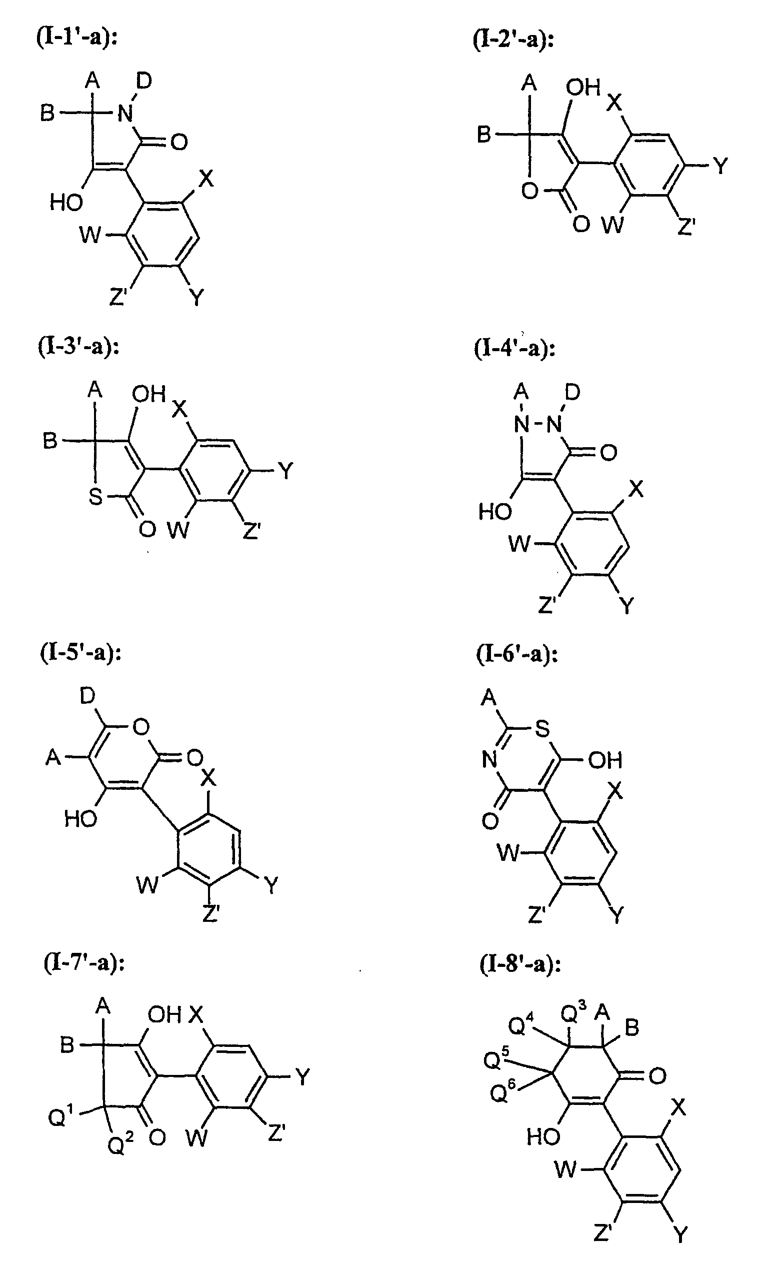 in welchen
   A, B, D, Q¹, Q², Q³, Q⁴, Q⁵, Q⁶, W, X und Y die oben angegebene Bedeutung haben und
   - Z': für Chlor, Brom, Jod, bevorzugt für Brom steht,
   mit Boronsäuren der Formel (XII) 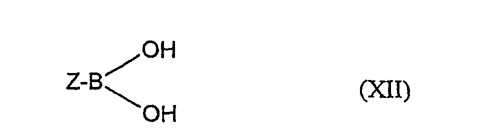 in welcher
   - Z: die oben angegebene Bedeutung hat,
   in Gegenwart eines Lösungsmittels, einer Base und eines Katalysators umsetzt, wobei als Katalysator insbesondere Palladiumkomplexe in Frage kommen.
   Außerdem wurde gefunden
(J) daß man die Verbindungen der oben gezeigten Formeln (I-1-b) bis (I-8-b), in welchen A, B, D, Q¹, Q², Q³, Q⁴, Q⁵, Q⁶, R¹, W, X, Y und Z die oben angebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
(α) mit Säurehalogeniden der Formel (XIII) 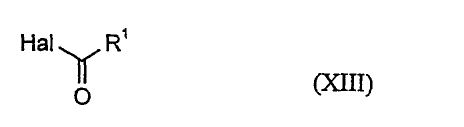 in welcher
   - R¹: die oben angegebene Bedeutung hat und
   - Hal: für Halogen (insbesondere Chlor oder Brom) steht
   oder
(β) mit Carbonsäureanhydriden der Formel (XIV)

   R¹-CO-O-CO-R¹ (XIV)

   in welcher
   - R¹: die oben angegebene Bedeutung hat,
   gegebenenfalls in Gegenwart eines Verdünnungsmittels und gegebenenfalls in Gegenwart eines Säurebindemittels umsetzt;
(K) daß man die Verbindungen der oben gezeigten Formeln (I-1-c) bis (I-8-c), in welchen A, B, D, Q¹, Q², Q³, Q⁴, Q⁵, Q⁶, R², M, W, X, Y und Z die oben angegebenen Bedeutungen haben und L für Sauerstoff steht, erhält, wenn man Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
   mit Chlorameisensäureestern oder Chlorameisensäurethioestern der Formel (XV)

   R²-M-CO-Cl (XV)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(L) daß man Verbindungen der oben gezeigten Formeln (I-1-c) bis (I-8-c), in welchen A, B, D, Q¹, Q², Q³, Q⁴, Q⁵, Q⁶, R², M, W, X, Y und Z die oben angegebenen Bedeutungen haben und L für Schwefel steht, erhält, wenn man Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
   mit Chlormonothioameisensäureestern oder Chlordithioameisensäureestern der Formel (XVI) 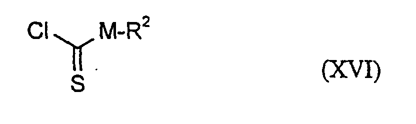 in welcher
   - M und R²: die oben angegebenen Bedeutungen haben,
   gegebenenfalls in Gegenwart eines Verdünnungsmittels und gegebenenfalls in Gegenwart eines Säurebindemittels umsetzt
   und
(M) daß man Verbindungen der oben gezeigten Formeln (I-1-d) bis (I-8-d), in welchen A, B, D, Q¹, Q², Q³, Q⁴, Q⁵, Q⁶, R³, W, X, Y und Z die oben angegebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
   mit Sulfonsäurechloriden der Formel (XVII)

   R³-SO₂-Cl (XVII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(N) daß man Verbindungen der oben gezeigten Formeln (I-1-e) bis (I-8-e), in welchen A, B, D, L, Q¹, Q², Q³, Q⁴, Q⁵, Q⁶, R⁴, R⁵, W, X, Y und Z die oben angegebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
   mit Phosphorverbindungen der Formel (XVIII) 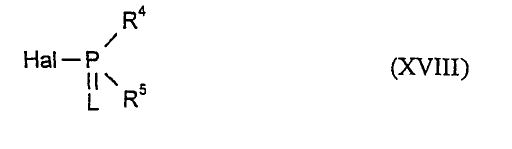 in welcher
   L, R⁴ und R⁵ die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(L) daß man Verbindungen der oben gezeigten Formeln (I-1-f) bis (I-8-f), in welchen A, B, D, E, Q¹, Q², Q³, Q⁴, Q⁵, Q⁶, W, X, Y und Z die oben angegebenen Bedeutungen haben, erhält, wenn man Verbindungen der Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
   mit Metallverbindungen oder Aminen der Formeln (XIX) oder (XX)

   Me(OR¹⁰)ₜ (XIX)

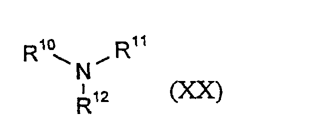

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(P) daß man Verbindungen der oben gezeigten Formeln (I-1-g) bis (I-8-g), in welchen A, B, D, L, Q¹, Q², Q³, Q⁴, Q⁵, Q⁶, R⁶, R⁷, W, X, Y und Z die oben angegebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
(α) mit Isocyanaten oder Isothiocyanaten der Formel (XXI)

   R⁶-N=C=L (XXI)

   in welcher
   - R⁶ und L: die oben angegebenen Bedeutungen haben,
   gegebenenfalls in Gegenwart eines Verdünnungsmittels und gegebenenfalls in Gegenwart eines Katalysators umsetzt oder
(β) mit Carbamidsäurechloriden oder Thiocarbamidsäurechloriden der Formel (XXII) 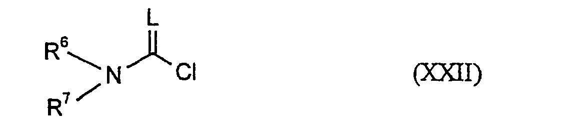 in welcher
   - L, R⁶ und R⁷: die oben angegebenen Bedeutungen haben,
   gegebenenfalls in Gegenwart eines Verdünnungsmittels und gegebenenfalls in Gegenwart eines Säurebindemittels, umsetzt.

Weiterhin wurde gefunden, daß die neuen Verbindungen der Formel (I) eine sehr gute Wirksamkeit als Schädlingsbekämpfungsmittel, vorzugsweise als Insektizide, Akarizide und als Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert.

In den genannten Restedefinitionen steht Halogen für Fluor, Chlor, Brom und Iod, insbesondere für Fluor, Chlor und Brom.
- X: steht besonders bevorzugt für Fluor, Chlor, Brom, C₁-C₄-Alkyl, C₁-C₄-Alkoxy, C₃-C₄-Alkenyloxy, C₁-C₄-Halogenalkyl, C₁-C₄-Halogenalkoxy, C₃-C₄-Halogenalkenyloxy, Nitro oder Cyano.
- Z: steht besonders bevorzugt für einen der Reste 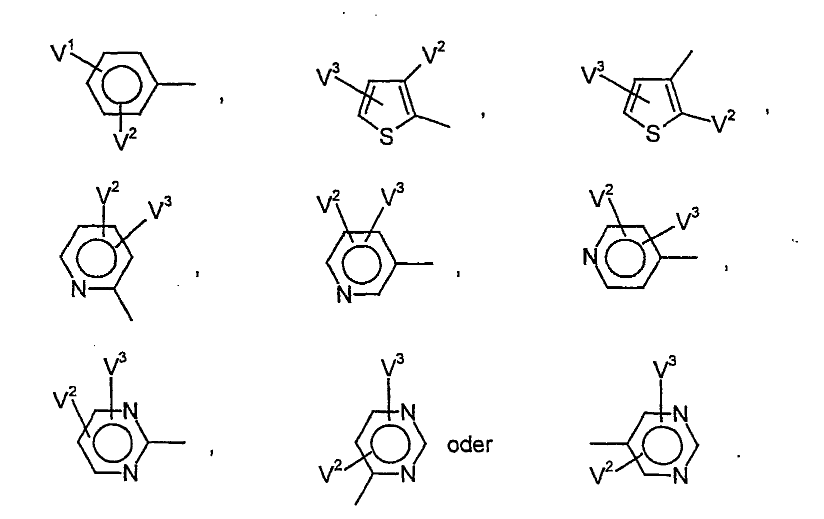
- V¹: steht besonders bevorzugt für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro,Cyano oder jeweils gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Phenoxy-C₁-C₂-alkyl, Phenyl-C₁-C₂-alkoxy, Phenylthio-C₁-C₂-alkyl oder Phenyl-C₁-C₂-alkylthio.
- V² und V³: stehen besonders bevorzugt unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy.
- W und Y: stehen besonders bevorzugt unabhängig voneinander für Wasserstoff, Fluor, Chlor, Brom, C₁-C₄-Alkyl, C₁-C₄-Halogenalkyl, C₁-C₄-Alkoxy oder C₁-C₄-Halogenalkoxy.
- CKE: steht besonders bevorzugt für eine der Gruppen 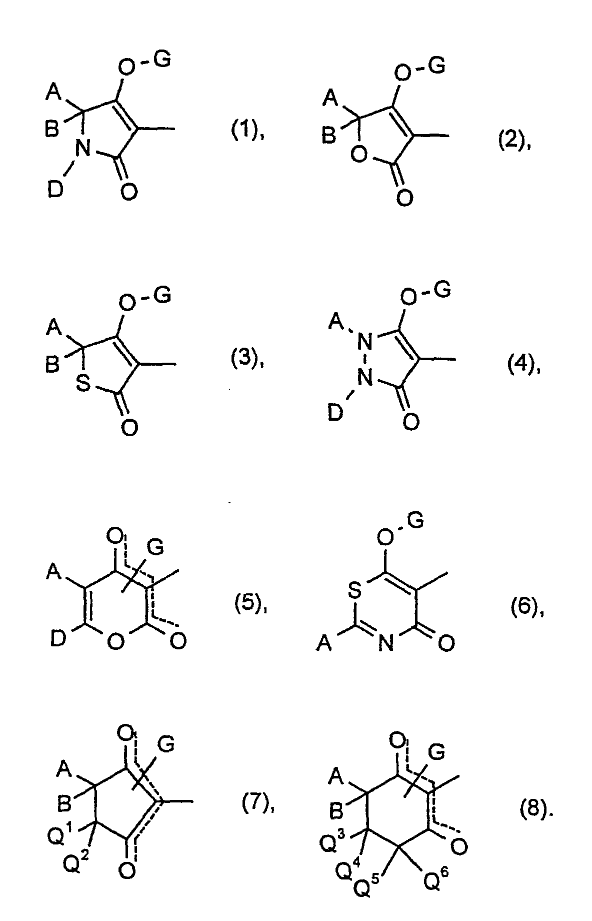
- A: steht besonders bevorzugt für Wasserstoff oder für jeweils gegebenenfalls durch Fluor oder Chlor substituiertes C₁-C₁₀-Alkyl, C₁-C₈-Alkoxy-C₁-C₆-alkyl, gegebenenfalls durch Fluor, Chlor, C₁-C₄-Alkyl oder C₁-C₄-Alkoxy substituiertes C₃-C₇-Cycloalkyl, in welchem gegebenenfalls ein Ringglied durch Sauerstoff oder Schwefel ersetzt ist oder (jedoch nicht im Fall der Verbindungen der Formeln (I-5), (I-7) und (I-8)) jeweils gegebenenfalls durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl, Furanyl, Pyridyl, Imidazolyl, Triazolyl, Pyrazolyl, Pyrimidyl, Thiazolyl, Thienyl oder Phenyl-C₁-C₄-alkyl.
- B: steht besonders bevorzugt für Wasserstoff oder C₁-C₆-Alkyl oder
- A, B: und das Kohlenstoffatom an das sie gebunden sind, stehen besonders bevorzugt für gesättigtes oder ungesättigtes C₅-C₇-Cycloalkyl, worin gegebenenfalls ein Ringglied durch Sauerstoff oder Schwefel ersetzt ist und welches gegebenenfalls einfach durch C₁-C₆-Alkyl, C₅-C₈-Cycloalkyl, C₁-C₃-Halogenalkyl, C₁-C₆-Alkoxy, Fluor, Chlor oder Phenyl substituiert ist oder
- A, B: und das Kohlenstoffatom, an das sie gebunden sind, stehen besonders bevorzugt für C₅-C₆-Cycloalkyl, welches durch eine gegebenenfalls ein oder zwei nicht direkt benachbarte Sauerstoff- oder Schwefelatome enthaltende Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet oder
- A, B: und das Kohlenstoffatom, an das sie gebunden sind, stehen besonders bevorzugt für C₃-C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₅-Alkyl, C₁-C₅-Alkoxy, Fluor, Chlor oder Brom substituiertes C₂-C₄-Alkandiyl oder C₂-C₄-Alkendiyl, in welchem gegebenenfalls eine Methylengruppe durch Sauerstoff oder Schwefel ersetzt ist oder für Butadiendiyl stehen.
- D: steht besonders bevorzugt für Wasserstoff, für jeweils gegebenenfalls durch Fluor oder Chlor substituiertes C₁-C₁₀-Alkyl, C₃-C₆-Alkenyl, C₁-C₈-Alkoxy-C₂-C₆-alkyl oder C₁-C₈-Alkylthio-C₂-C₆-alkyl, für gegebenenfalls durch Fluor, Chlor, C₁-C₄-Alkyl, C₁-C₄-Alkoxy oder C₁-C₂-Halogenalkyl substituiertes C₃-C₇-Cycloalkyl, in welchem gegebenenfalls eine Methylengruppe durch Sauerstoff oder Schwefel ersetzt ist oder (jedoch nicht im Fall der Verbindungen der Formeln (I-1) und (I-4)) für jeweils gegebenenfalls durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl, Furanyl, Imidazolyl, Pyridyl, Thiazolyl, Pyrazolyl, Pyrimidyl, Pyrrolyl, Thienyl, Triazolyl oder Phenyl-C₁-C₄-alkyl oder
- A und D: stehen gemeinsam besonders bevorzugt für gegebenenfalls substituiertes C₃-C₅-Alkandiyl, in welchem eine Methylengruppe durch eine Carbonylgruppe, Sauerstoff oder Schwefel ersetzt sein kann, wobei als Substituenten Hydroxy, C₁-C₆-Alkyl oder C₁-C₄-Alkoxy in Frage kommen oder
- A und D: stehen (im Fall der Verbindungen der Formel (I-1)) gemeinsam mit den Atomen, an die sie gebunden sind, für eine der Gruppen AD-1 bis AD-10: 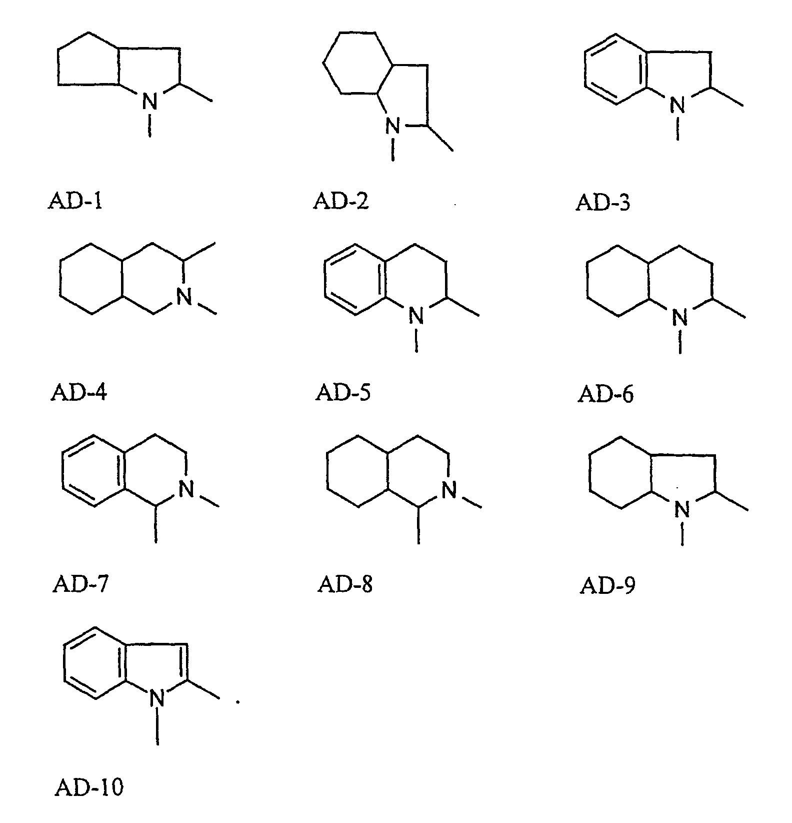 oder
- A und Q¹: stehen gemeinsam besonders bevorzugt für jeweils gegebenenfalls einfach oder zweifach, gleich oder verschieden durch Fluor, Chlor, Hydroxy, durch jeweils gegebenenfalls einfach bis dreifach durch Fluor substituiertes C₁-C₈-Alkyl oder C₁-C₄-Alkoxy substituiertes C₃-C₄-Alkandiyl oder C₃-C₄-Alkendiyl oder
- Q¹: steht besonders bevorzugt für Wasserstoff.
- Q²: steht besonders bevorzugt für Wasserstoff.
- Q⁴, Q⁵ und Q⁶: stehen besonders bevorzugt unabhängig voneinander für Wasserstoff oder C₁-C₃-Alkyl.
- Q³: steht besonders bevorzugt für Wasserstoff, C₁-C₄-Alkyl, C₁-C₄-Alkoxy-C₁-C₂-alkyl, C₁-C₄-Alkylthio-C₁-C₂-alkyl oder gegebenenfalls durch Methyl oder Methoxy substituiertes C₃-C₆-Cycloalkyl, worin gegebenenfalls eine Methylengruppe durch Sauerstoff oder Schwefel ersetzt ist oder
- Q³ und Q⁴: stehen besonders bevorzugt gemeinsam mit dem Kohlenstoff, an das sie gebunden sind, für einen gegebenenfalls durch C₁-C₄-Alkyl oder C₁-C₄-Alkoxy substituierten gesättigten C₅-C₆-Ring, in welchem gegebenenfalls ein Ringglied durch Sauerstoff oder Schwefel ersetzt ist.
- G: steht besonders bevorzugt für Wasserstoff (a) oder für eine der Gruppen 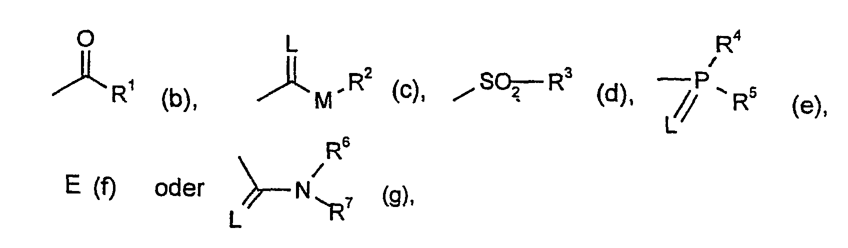 insbesondere für (a), (b) oder (c),
in welchen
E für ein Metallion oder ein Ammoniumion steht,
L für Sauerstoff oder Schwefel steht und
M für Sauerstoff oder Schwefel steht.
- R¹: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl, Poly-C₁-C₆-alkoxy-C₁-C₆-alkyl oder gegebenenfalls durch Fluor, Chlor, C₁-C₅-Alkyl oder C₁-C₅-Alkoxy substituiertes C₃-C₇-Cycloalkyl, in welchem gegebenenfalls ein oder zwei nicht direkt benachbarte Ringglieder durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₃-alkyl oder
für jeweils gegebenenfalls durch Fluor, Chlor, Brom, Amino oder C₁-C₄-Alkyl substituiertes Pyridyloxy-C₁-C₃-alkyl, Pyrimidyloxy-C₁-C₃-alkyl oder Thiazolyloxy-C₁-C₃-alkyl.
- R²: steht besonders bevorzugt für jeweils gegebenenfalls durch Fluor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl.
- R³: steht besonders bevorzugt für gegebenenfalls durch Fluor substituiertes C₁-C₆-Alkyl oder für jeweils gegebenenfalls durch Fluor, Chlor, Brom, C₁-C₄-Alkyl, C₁-C₄-Alkoxy, C₁-C₃-Halogenalkyl, C₁-C₃-Halogenalkoxy, Cyano oder Nitro substituiertes Phenyl oder Benzyl.
- R⁴ und R⁵: stehen besonders bevorzugt unabhängig voneinander für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
- R⁶ und R⁷: stehen unabhängig voneinander besonders bevorzugt für Wasserstoff, C₁-C₆-Alkyl, C₃-C₆-Cycloalkyl, C₁-C₆-Alkoxy, C₃-C₆-Alkenyl, C₁-C₆-Alkoxy-C₁-C₆-alkyl, für gegebenenfalls durch Fluor, Chlor, Brom, C₁-C₃-Halogenalkyl, C₁-C₄-Alkyl oder C₁-C₄-Alkoxy substituiertes Phenyl, für gegebenenfalls durch Fluor, Chlor, Brom, C₁-C₄-Alkyl, C₁-C₃-Halogenalkyl oder C₁-C₄-Alkoxy substituiertes Benzyl, oder zusammen für einen gegebenenfalls durch Methyl oder Ethyl substituierten C₄-C₅-Alkylenrest, in welchem gegebenenfalls eine Methylengruppe durch Sauerstoff oder Schwefel ersetzt ist.

In den als besonders bevorzugt genannten Restedefinitionen steht Halogen für Fluor, Chlor, Brom und Iod, insbesondere für Fluor, Chlor und Brom.
- X: steht ganz besonders bevorzugt für Fluor, Chlor, Methyl, Ethyl, Propyl, iso-Propyl, Methoxy, Ethoxy, Propoxy, iso-Propoxy, Trifluormethyl, Difluormethoxy, Trifluormethoxy, Nitro oder Cyano.
- Z: steht ganz besonders bevorzugt für einen der Reste 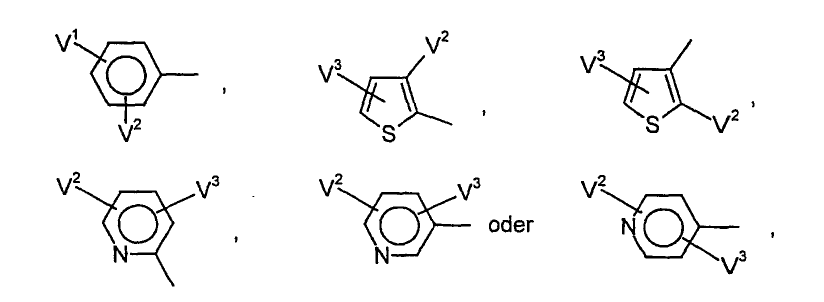 insbesondere für 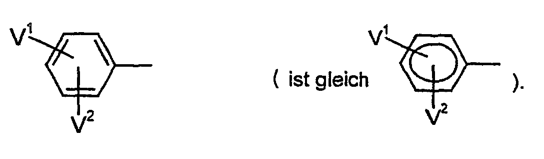
- V¹: steht ganz besonders bevorzugt für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Nitro, Cyano oder gegebenenfalls einfach durch Fluor, Chlor, Methyl, Methoxy, Trifluormethyl oder Trifluormethoxy substituiertes Phenyl.
- V² und V³: stehen ganz besonders bevorzugt unabhängig voneinander für Wasserstoff, Fluor, Chlor, Methyl, Ethyl, n-Propyl, iso-Propyl, Methoxy, Ethoxy, Trifluormethyl oder Trifluormethoxy.
- W und Y: stehen ganz besonders bevorzugt unabhängig voneinander für Wasserstoff, Fluor, Chlor, Methyl, Ethyl, n-Propyl, Methoxy, Ethoxy oder Propoxy.
- CKE: steht ganz besonders bevorzugt für eine der Gruppen 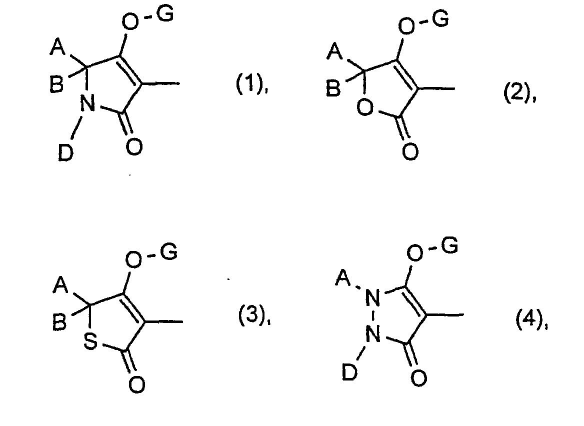 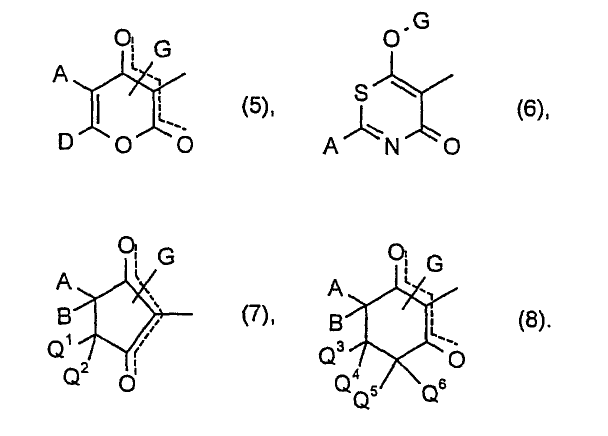
- A: steht ganz besonders bevorzugt für Wasserstoff oder für jeweils gegebenenfalls durch Fluor substituiertes C₁-C₈-Alkyl oder C₁-C₆-Alkoxy-C₁-C₄-alkyl, gegebenenfalls durch Fluor, Methyl, Ethyl oder Methoxy substituiertes C₃-C₆-Cycloalkyl, in welchem gegebenenfalls ein Ringglied durch Sauerstoff oder Schwefel ersetzt ist oder (jedoch nicht im Fall der Verbindungen der Formeln (I-5), (I-7) und (I-8)) für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl oder Benzyl.
- B: steht ganz besonders bevorzugt für Wasserstoff oder C₁-C₄-Alkyl oder
- A, B: und das Kohlenstoffatom an das sie gebunden sind, stehen ganz besonders bevorzugt für gesättigtes C₅-C₆-Cycloalkyl, in welchem gegebenenfalls ein Ringglied durch Sauerstoff oder Schwefel ersetzt ist und welches gegebenenfalls einfach durch Methyl, Ethyl, Propyl, Isopropyl, Butyl, iso-Butyl, sec.-Butyl, tert.-Butyl, Trifluormethyl, Methoxy, Ethoxy, Propoxy, iso-Propoxy, Butoxy, iso-Butoxy, sek.-Butoxy, tert.-Butoxy, Fluor oder Chlor substituiert ist oder
- A, B: und das Kohlenstoffatom, an das sie gebunden sind, stehen ganz besonders bevorzugt für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder für Butadiendiyl stehen.
- D: steht ganz besonders bevorzugt für Wasserstoff, für jeweils gegebenenfalls durch Fluor oder Chlor substituiertes C₁-C₈-Alkyl, C₃-C₄-Alkenyl, C₁-C₆-Alkoxy-C₂-C₄-alkyl, C₁-C₄-Alkylthio-C₂-C₄-alkyl oder C₃-C₆-Cycloalkyl, in welchem gegebenenfalls eine Methylengruppe durch Sauerstoff oder Schwefel ersetzt ist oder (jedoch nicht im Fall der Verbindungen der Formeln (I-1) und (I-4)) für jeweils gegebenenfalls durch Fluor, Chlor, Methyl, Ethyl, n-Propyl, iso-Propyl, Methoxy, Ethoxy, Trifluormethyl oder Trifluormethoxy substituiertes Phenyl, Furanyl, Pyridyl, Thienyl oder Benzyl,
oder
- A und D: stehen gemeinsam ganz besonders bevorzugt für gegebenenfalls substituiertes C₃-C₄-Alkandiyl, worin gegebenenfalls ein Kohlenstoffatom durch Schwefel ersetzt ist und welches gegebenenfalls durch Hydroxy, Methyl, Ethyl, Methoxy oder Ethoxy substituiert ist oder
- A und D: stehen (im Fall der Verbindungen der Formel (I-1)) gemeinsam mit den Atomen, an die sie gebunden sind, für eine der folgenden Gruppen AD: 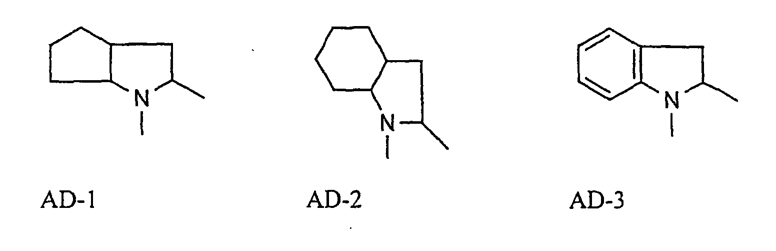 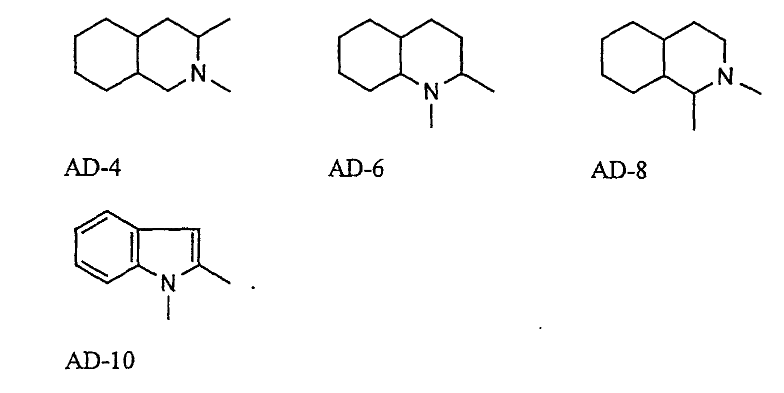
- A und Q¹: stehen gemeinsam ganz besonders bevorzugt für gegebenenfalls einfach oder zweifach durch Fluor, Hydroxy, Methyl oder Methoxy substituiertes C₃-C₄-Alkandiyl oder Butendiyl oder
- Q¹: steht ganz besonders bevorzugt für Wasserstoff.
- Q²: steht ganz besonders bevorzugt für Wasserstoff.
- Q⁴, Q⁵ und Q⁶: stehen ganz besonders bevorzugt unabhängig voneinander für Wasserstoff, Methyl oder Ethyl.
- Q³: steht ganz besonders bevorzugt für Wasserstoff, Methyl, Ethyl oder C₃-C₆-Cycloalkyl, worin gegebenenfalls eine Methylengruppe durch Sauerstoff oder Schwefel ersetzt ist oder
- Q³ und Q⁴: stehen ganz besonders bevorzugt gemeinsam mit dem Kohlenstoff, an den sie gebunden sind, für einen gegebenenfalls durch Methyl oder Methoxy substituierten gesättigten C₅-C₆-Ring, in welchem gegebenenfalls ein Ringglied durch Sauerstoff oder Schwefel ersetzt ist.
- G: steht ganz besonders bevorzugt für Wasserstoff (a) oder für eine der Gruppen 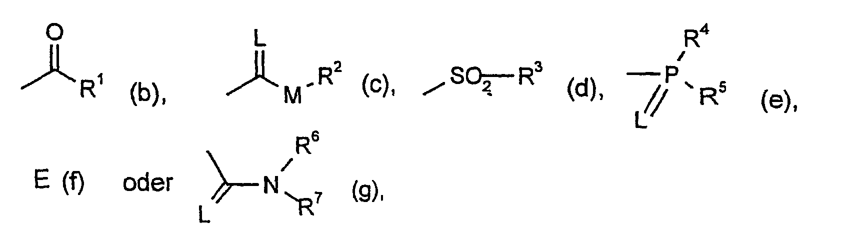 insbesondere für (a), (b) oder (c),
in welchen
E für ein Metallion oder ein Ammoniumion steht,
L für Sauerstoff oder Schwefel steht und
M für Sauerstoff oder Schwefel steht.
- R¹: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder gegebenenfalls durch Fluor, Chlor, Methyl, Ethyl, Propyl, i-Propyl, Butyl, i-Butyl, tert.-Butyl, Methoxy, Ethoxy, n-Propoxy oder iso-Propoxy substituiertes C₃-C₆-Cycloalkyl, in welchem gegebenenfalls ein oder zwei nicht direkt benachbarte Ringglieder durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl, Pyridyl, Pyrimidyl, Thiazolyl oder Pyrazolyl,
für gegebenenfalls durch Fluor, Chlor, Methyl oder Ethyl substituiertes Phenoxy-C₁-C₂-alkyl oder
für jeweils gegebenenfalls durch Fluor, Chlor, Amino, Methyl oder Ethyl substituiertes Pyridyloxy-C₁-C₂-alkyl, Pyrimidyloxy-C₁-C₂-alkyl oder Thiazolyloxy-C₁-C₂-alkyl.
- R²: steht ganz besonders bevorzugt für jeweils gegebenenfalls durch Fluor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl.
- R³: steht ganz besonders bevorzugt für gegebenenfalls durch Fluor substituiertes Methyl, Ethyl, n-Propyl, Isopropyl oder jeweils gegebenenfalls durch Fluor, Chlor, Brom, Methyl, tert.-Butyl, Methoxy, Trifluormethyl, Trifluormethoxy, Cyano oder Nitro substituiertes Phenyl oder Benzyl.
- R⁴ und R⁵: stehen ganz besonders bevorzugt unabhängig voneinander für C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio oder C₁-C₃-Alkyl substituiertes Phenyl, Phenoxy oder Phenylthio.
- R⁶ und R⁷: stehen unabhängig voneinander ganz besonders bevorzugt für WasserStoff, für C₁-C₄-Alkyl, C₃-C₆-Cycloalkyl, C₁-C₄-Alkoxy, C₃-C₄-Alkenyl, C₁-C₄-Alkoxy-C₁-C₄-alkyl, für gegebenenfalls durch Fluor, Chlor, Brom, Trifluormethyl, Methyl oder Methoxy substituiertes Phenyl, für gegebenenfalls durch Fluor, Chlor, Brom, Methyl, Trifluormethyl oder Methoxy substituiertes Benzyl, oder zusammen für einen C₅-C₆-Alkylenrest, in welchem gegebenenfalls eine Methylengruppe durch Sauerstoff oder Schwefel ersetzt ist.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

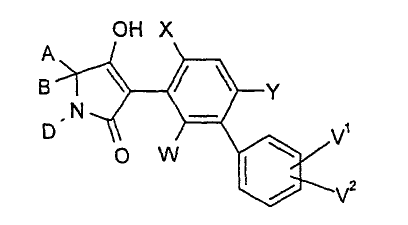

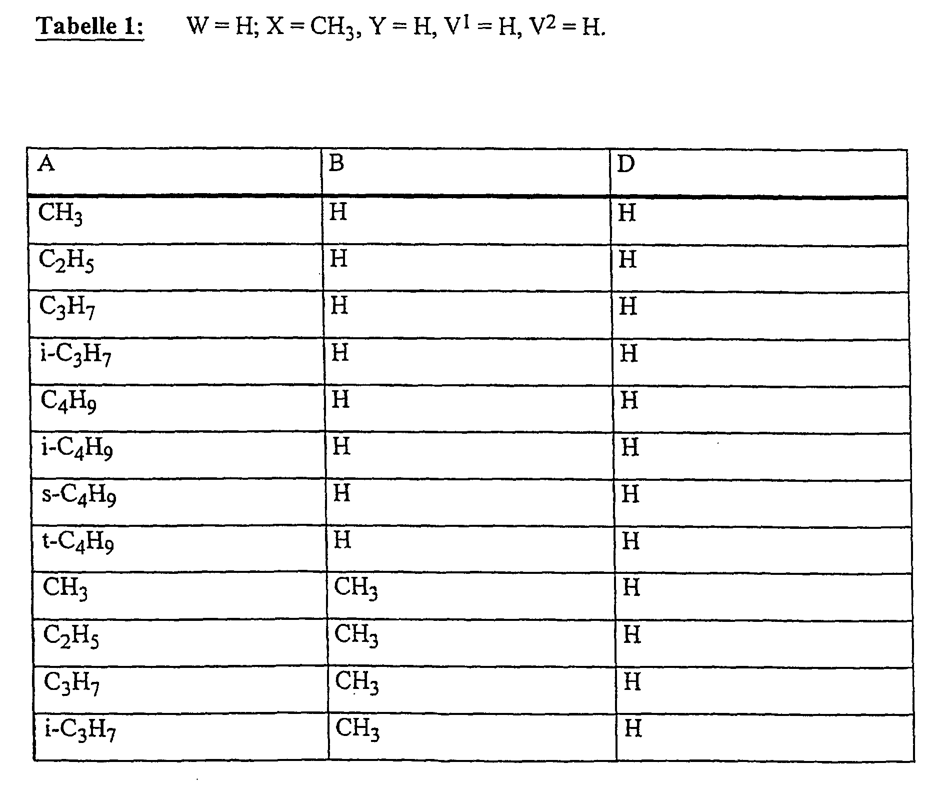

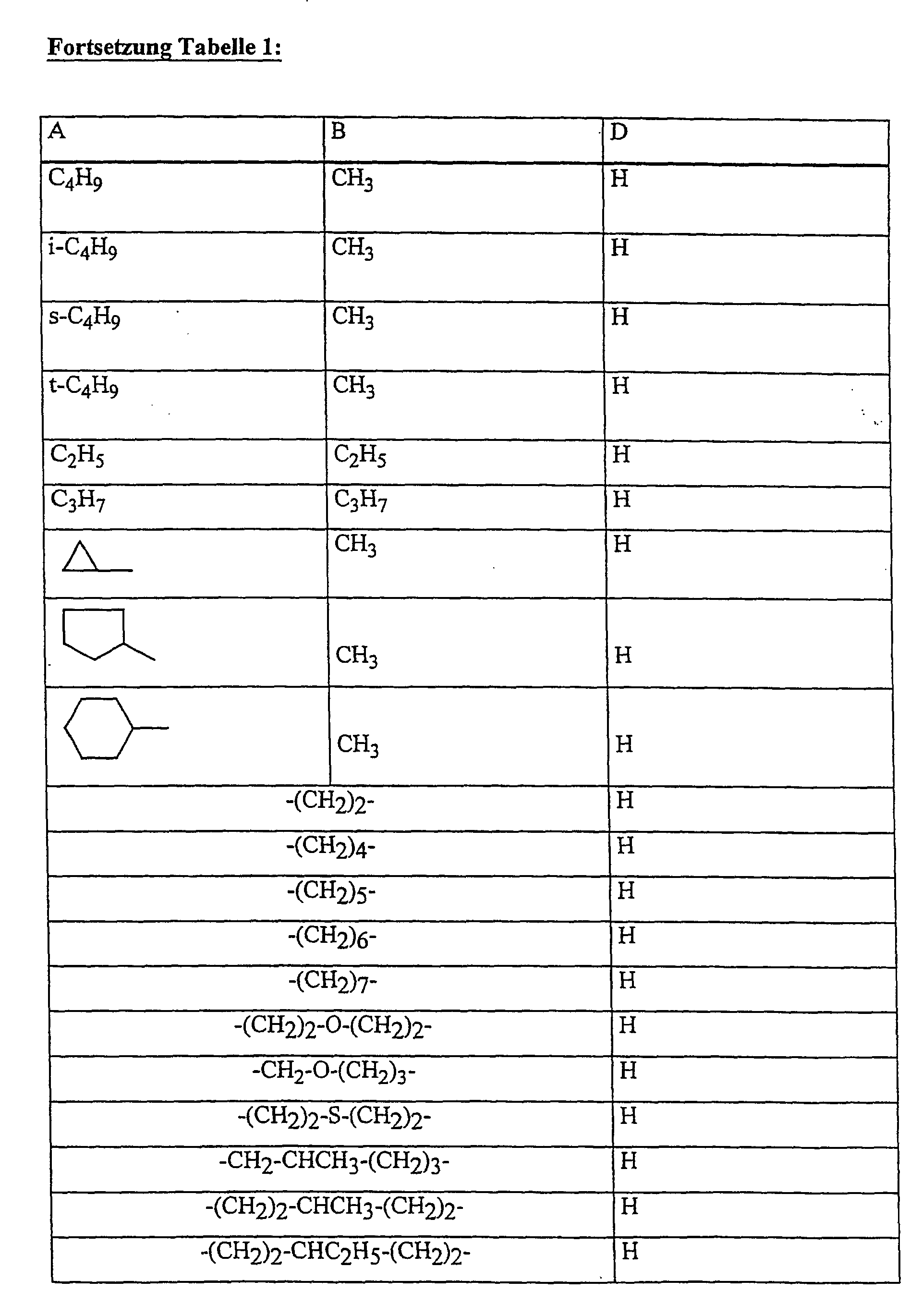

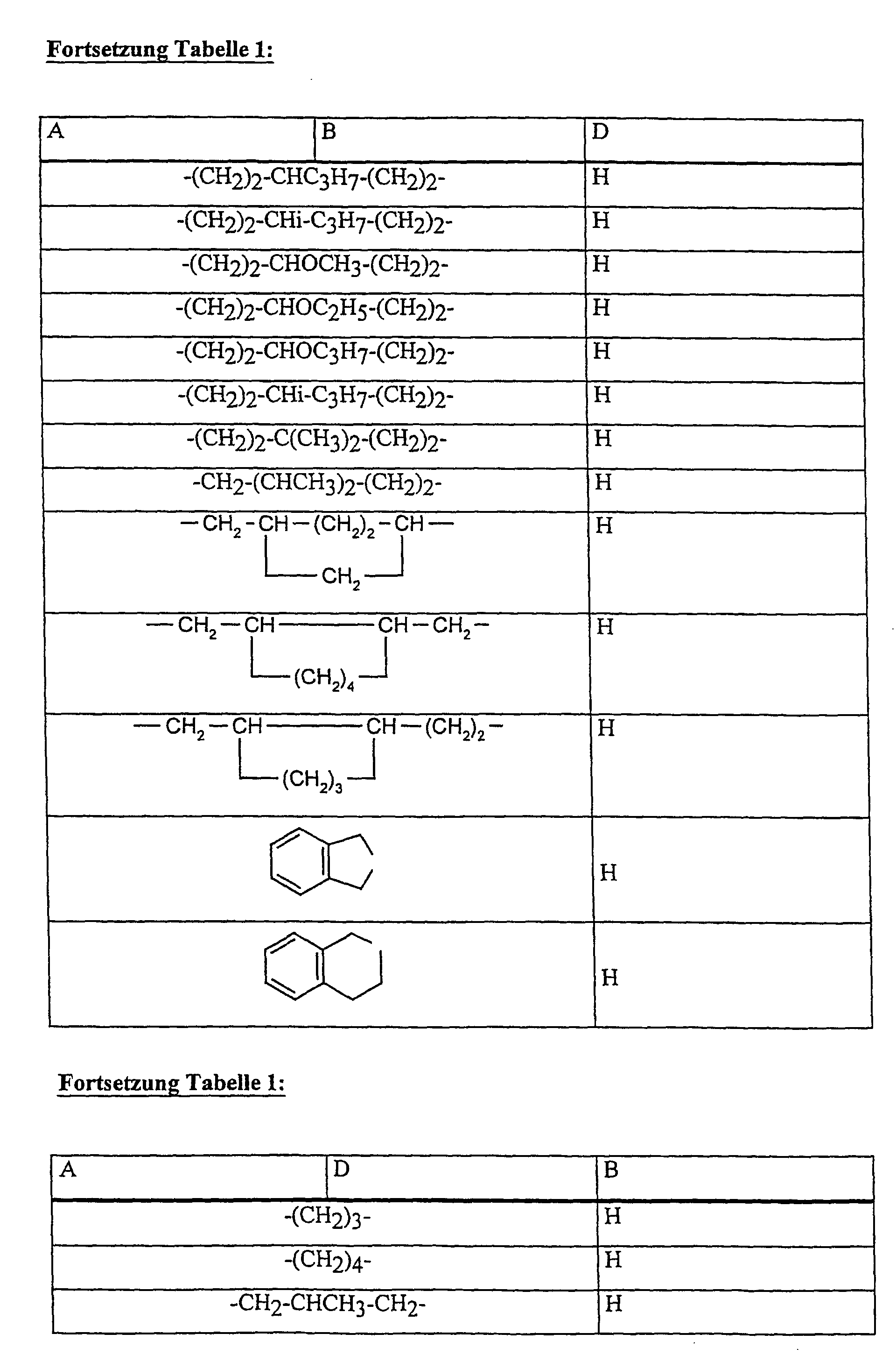

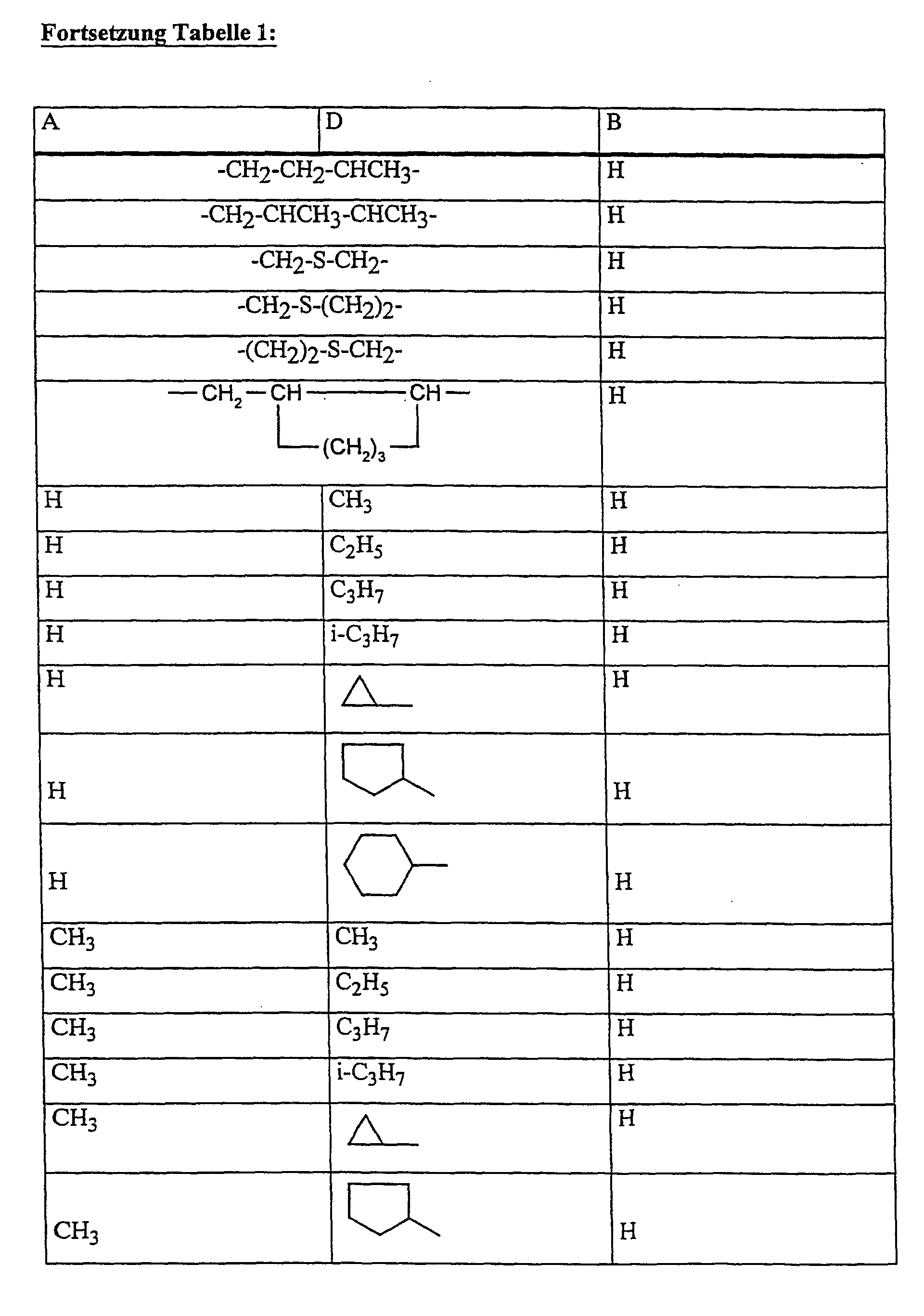

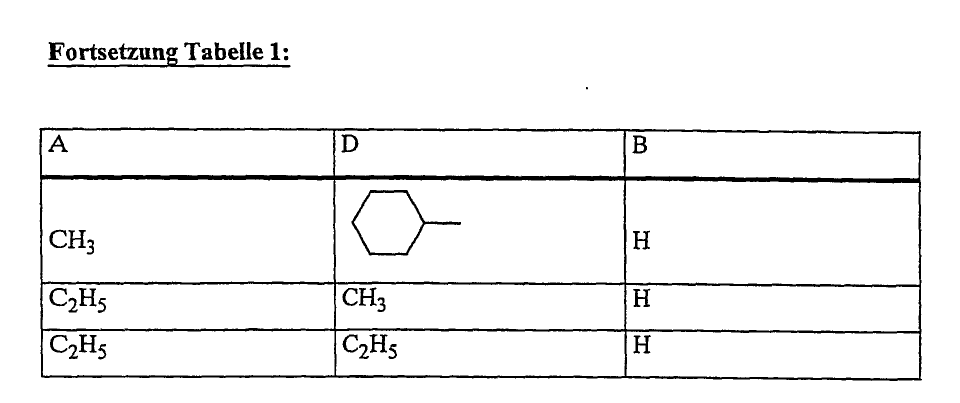

**Tabelle 2:** A, B und D wie in Tabelle 1 angegeben
   W = H; X = CH₃; Y = CH₃; V¹ = H; V² = H.
**Tabelle 3:** A, B und D wie in Tabelle 1 angegeben
   W = CH₃; X = CH₃; Y = CH₃; V¹ = H; V² = H.
**Tabelle 4:** A, B und D wie in Tabelle 1 angegeben
   W = H; X = CH₃; Y = H; V¹ = 4-Cl; V² = H.
**Tabelle 5:** A, B und D wie in Tabelle 1 angegeben
   W = H; X = CH₃; Y = CH₃; V¹ = 4-Cl; V² = H.
**Tabelle 6:** A, B und D wie in Tabelle 1 angegeben
   W = CH₃; X = CH₃; Y = CH₃; V¹ = 4-Cl; V² = H.
**Tabelle 7:** A, B und D wie in Tabelle 1 angegeben
   W = H; X = CH₃; Y = H; V¹= 3-Cl; V² = H.
**Tabelle 8:** A, B und D wie in Tabelle 1 angegeben
   W = H; X = CH₃; Y = CH₃; V¹ = 3-Cl; V² = H.
**Tabelle 9:** A, B und D wie in Tabelle 1 angegeben
   W = CH₃; X = CH₃; Y = CH₃; V¹ = 3-Cl; V² = H.
**Tabelle 10:** A, B und D wie in Tabelle 1 angegeben
   W = H; X = CH₃; Y = H; V¹ = 2-Cl; V² = 4-Cl.
**Tabelle 11:** A, B und D wie in Tabelle 1 angegeben
   W = H; X = CH₃; Y = CH₃; V¹ = 2-Cl; V² = 4-Cl.
**Tabelle 12:** A, B und D wie in Tabelle 1 angegeben
   W = CH₃; X = CH₃; Y = CH₃; V¹ = 2-Cl; V² = 4-Cl.
**Tabelle 13:** A, B und D wie in Tabelle 1 angegeben
   W = H; X = CH₃; Y = H; V¹ = 4-CF₃; V² = H.
**Tabelle 14:** A, B und D wie in Tabelle 1 angegeben
   W = H; X = CH₃; Y = CH₃; V¹ = 4-CF₃; V² = H.
**Tabelle 15:** A, B und D wie in Tabelle 1 angegeben
   W = CH₃; X = CH₃; Y = CH₃; V¹ = 4-CF₃; V² = H.
**Tabelle 16:** A, B und D wie in Tabelle 1 angegeben
   W = H; X = CH₃; Y = H; V ¹ = 4-CH₃; V² = H.
**Tabelle 17:** A, B und D wie in Tabelle 1 angegeben
   W = H; X = CH₃; Y = CH₃; V¹ = 4-CH₃; V² = H.
**Tabelle 18:** A, B und D wie in Tabelle 1 angegeben
   W = CH₃; X = CH₃; Y = CH₃; V¹ = 4-CH₃; V² = H.
**Tabelle 19:** A, B und D wie in Tabelle 1 angegeben
   W = H; X = CH₃; Y = H; V¹ = 4-OCH₃; V² = H.
**Tabelle 20:** A, B und D wie in Tabelle 1 angegeben
   W = H; X = CH₃; Y = CH₃; V¹ = 4-OCH₃; V² = H.
**Tabelle 21:** A, B und D wie in Tabelle 1 angegeben
   W = CH₃; X = CH₃; Y = CH₃; V¹ = 4-OCH₃; V² = H.

Im einzelnen seien außer den bei den Herstellungsbeispielen genannten Verbindungen die folgenden Verbindungen der Formel (I-2-a) genannt:

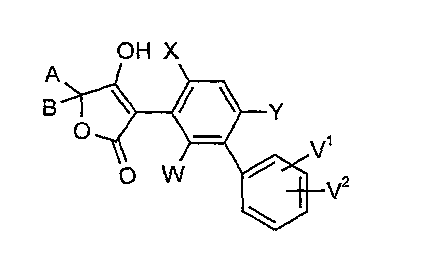

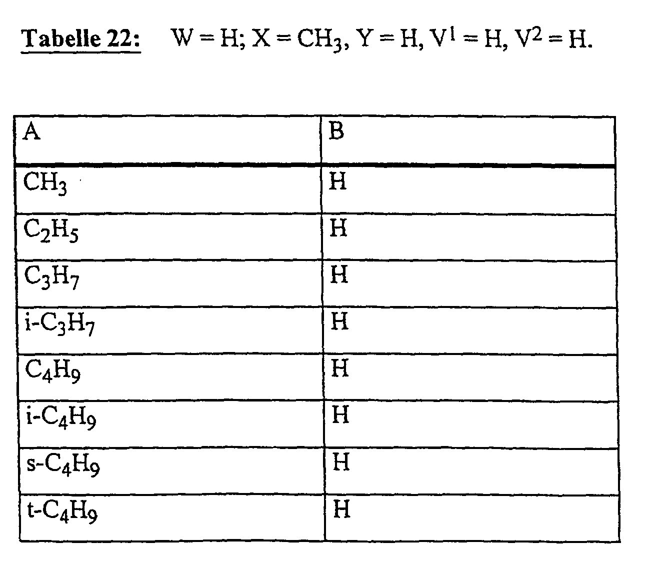

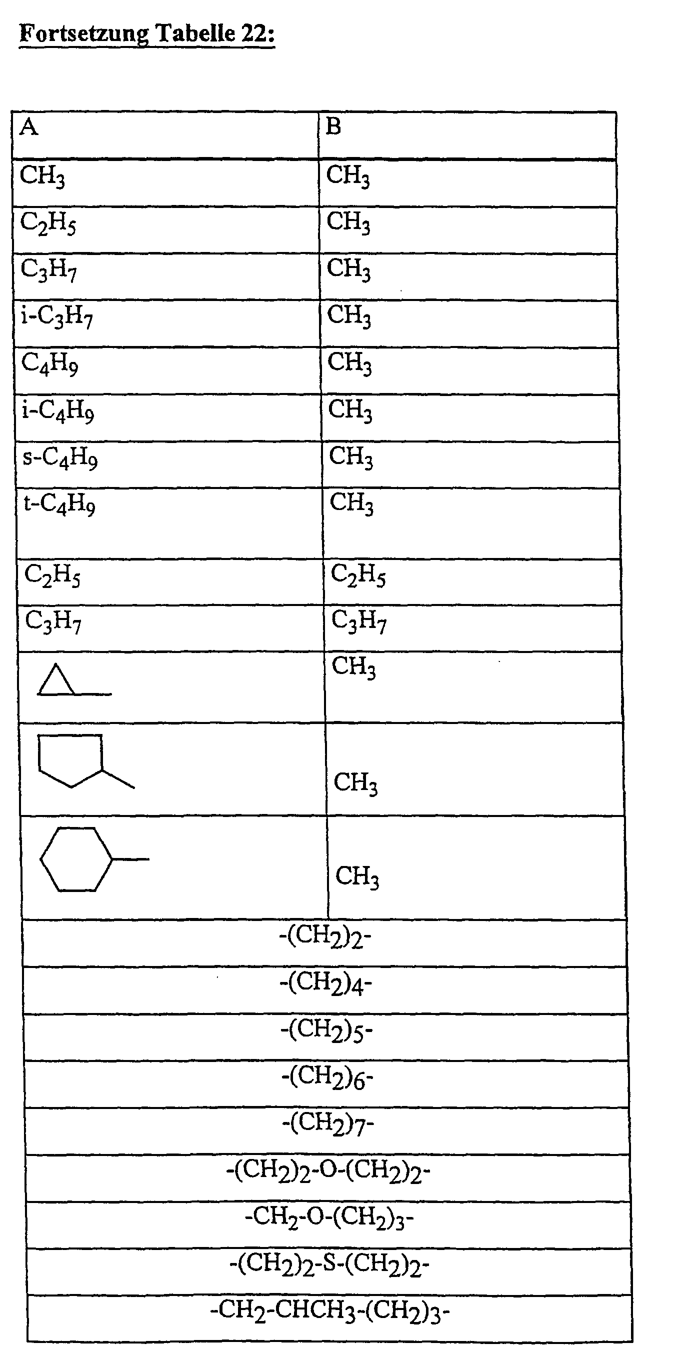

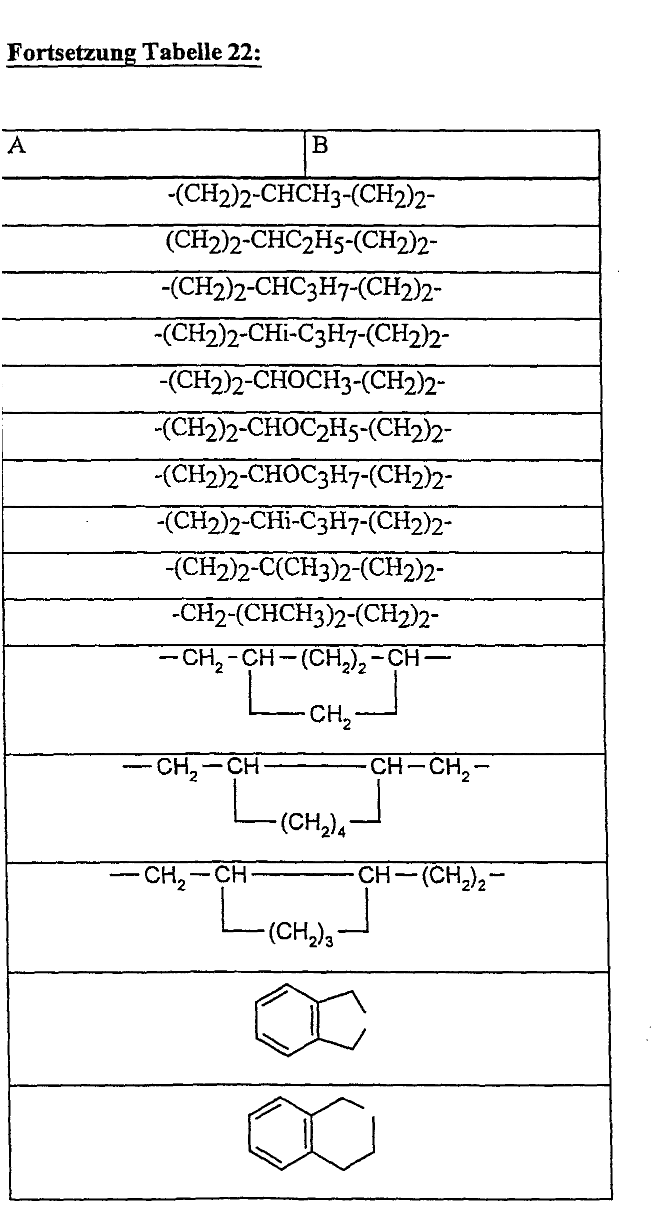

**Tabelle 23:** A und B wie in Tabelle 22 angegeben
   W = H; X = CH₃; Y = CH₃; V¹ = H; V² = H.
**Tabelle 24:** A und B wie in Tabelle 22 angegeben
   W = CH₃; X = CH₃; Y = CH₃; V¹ = H; V² =H.
**Tabelle 25:** A und B wie in Tabelle 22 angegeben
   W = H; X = CH₃; Y = H; V¹ = 4-Cl; V² = H.
**Tabelle 26:** A und B wie in Tabelle 22 angegeben
   W = H; X = CH₃; Y = CH₃; V¹ = 4-Cl; V² = H.
**Tabelle 27:** A und B wie in Tabelle 22 angegeben
   W = CH₃; X = CH₃; Y = CH₃; V¹ = 4-Cl; V² = H.
**Tabelle 28:** A und B wie in Tabelle 22 angegeben
   W = H; X = CH₃; Y = H; V¹ = 3-Cl; V² = H.
**Tabelle 29:** A und B wie in Tabelle 22 angegeben
   W = H; X = CH₃; Y = CH₃; V¹ = 3-Cl; V² = H.
**Tabelle 30:** A und B wie in Tabelle 22 angegeben
   W = CH₃; X = CH₃; Y = CH₃; V¹ = 3-Cl; V² = H.
**Tabelle 31:** A und B wie in Tabelle 22 angegeben
   W = H, X = CH₃; Y = H; V¹ = 4-CF₃; V² = H.
**Tabelle 32:** A und B wie in Tabelle 22 angegeben
   W = H; X = CH₃; Y = CH₃; V¹ = 4-CF₃; V² = H.
**Tabelle 33:** A und B wie in Tabelle 22 angegeben
   W = CH₃; X = CH₃; Y = CH₃; V¹ = 4-CF₃; V² = H.
**Tabelle 34:** A und B wie in Tabelle 22 angegeben
   W = H; X = CH₃; Y = H; V¹ = 2-Cl; V² = 4-Cl.
**Tabelle 35:** A und B wie in Tabelle 22 angegeben
   W = H; X = CH₃; Y = CH₃; V¹ = 2-Cl; V² = 4-Cl.
**Tabelle 36:** A und B wie in Tabelle 22 angegeben
   W = CH₃; X = CH₃; Z = CH₃; V¹ = 2-Cl; V¹ = 4-Cl.
**Tabelle 37:** A und B wie in Tabelle 22 angegeben
   W = H; X = CH₃; Y = H; V¹ = 4-CH₃; V² = H.
**Tabelle 38:** A und B wie in Tabelle 22 angegeben
   W = H; X = CH₃; Y = CH₃; V¹ = 4-CH₃; V² = H.
**Tabelle 39:** A und B wie in Tabelle 22 angegeben
   W = CH₃; X = CH₃; Y = CH₃; V¹ = 4-CH₃; V² = H.
**Tabelle 40:** A und B wie in Tabelle 22 angegeben
   W = H; X = CH₃; Y = H; V¹= 4-OCH₃; V² = H.
**Tabelle 41:** A und B wie in Tabelle 22 angegeben
   W = H; X = CH₃; Y = CH₃; V¹ = 4-OCH₃; V² = H.
**Tabelle 42:** A und B wie in Tabelle 22 angegeben
   W = CH₃; X = CH₃; Y = CH₃; V = 4-OCH₃; V² = H.

Verwendet man gemäß Verfahren (A) N-[(6-Methyl-3-phenyl)-phenylacetyl]-1-amino-cyclohexan-carbonsäureethylester als Ausgangsstoff, so kann der Verlauf des erfindungsgemäßen Verfahrens durch folgendes Reaktionsschema wiedergegeben werden:

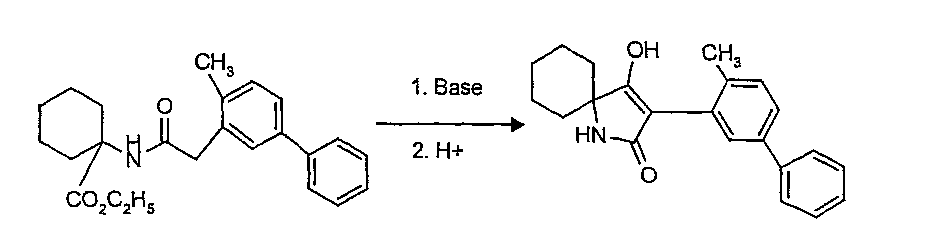

Verwendet man gemäß Verfahren (B) O-[(2-Chlor-5-(4-chlor)-phenyl)-phenylacetyl]-2-hydroxyisobuttersäureethylester, so kann der Verlauf des erfindungsgemäßen Verfahrens durch folgendes Reaktionsschema wiedergegeben werden:

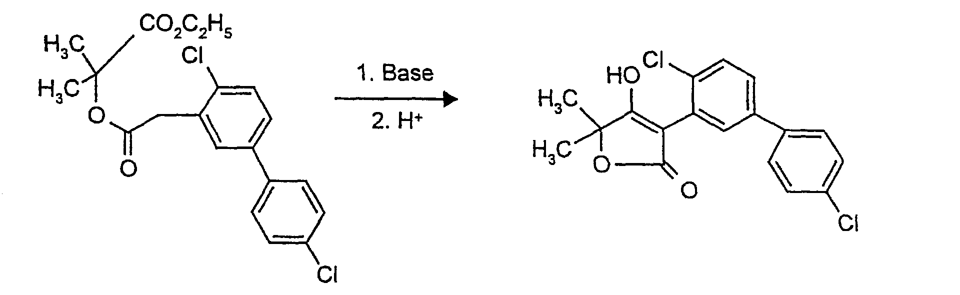

Verwendet man gemäß Verfahren (C) 2-[(2,6-Dimethyl-3-phenyl)-phenyl]-4-(4-methoxy)-benzylmercapto-4-methyl-3-oxo-valeriansäure-ethylester, so kann der Verlauf des erfindungsgemäßen Verfahrens durch folgendes Reaktionsschema wiedergegeben werden:

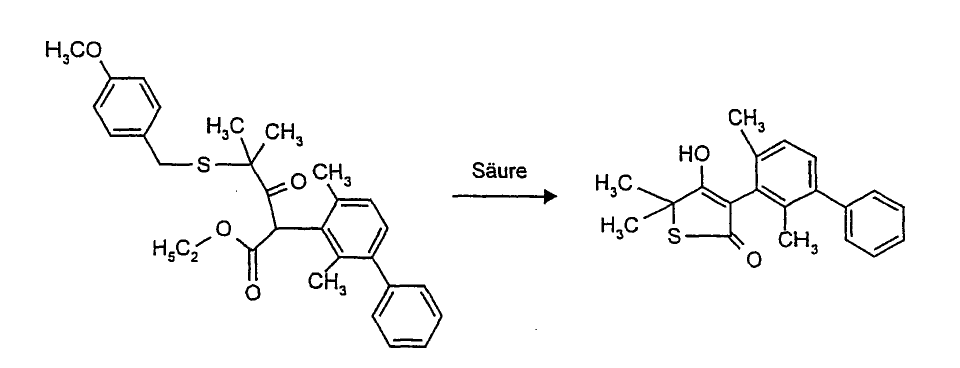

Verwendet man beispielsweise gemäß Verfahren (D-α) (Chlorcarbonyl)-3-[(6-methyl-3-(4-methyl)-phenyl))-phenyl]-keten und 1,2-Diazacyclopentan als Ausgangsverbindungen, so kann der Verlauf des erfindungsgemäßen Verfahrens durch folgendes Reaktionsschema wiedergegeben werden:

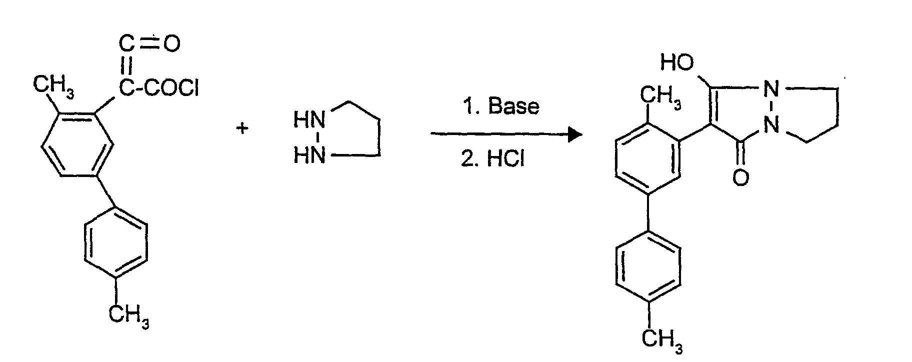

Verwendet man beispielsweise gemäß Verfahren (D-ß) 3-[6-Methyl-3-(3-chlor-phenyl)]-phenylmalonsäurediethylester und 1,2-Diazacyclopentan als Ausgangsverbindungen, so kann der Verlauf des erfindungsgemäßen Verfahrens durch das folgende Reaktionsschema wiedergegeben werden:

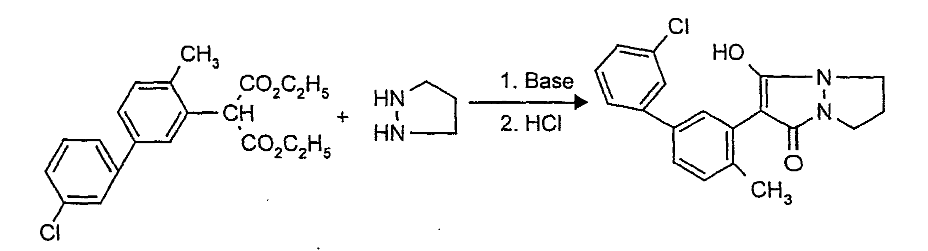

Verwendet man beispielsweise gemäß Verfahren (E) (Chlorcarbonyl)-2-[(2,6-dimethyl-3-(4-trifluormethoxy-phenyl))-phenyl]-keten und Aceton als Ausgangsverbindungen, so kann der Verlauf des erfindungsgemäßen Verfahrens durch das folgende Reaktionsschema wiedergegeben werden:

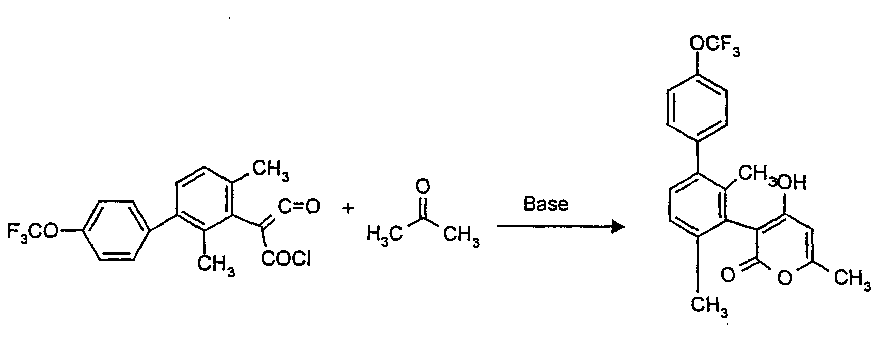

Verwendet man beispielsweise gemäß Verfahren (F) (Chlorcarbonyl)-2-[(2,4,6-Trimethyl-3-phenyl)-phenyl]-keten und Thiobenzamid als Ausgangsverbindungen, so kann der Verlauf des erfindungsgemäßen Verfahrens durch das folgende Reaktionsschema wiedergegeben werden:

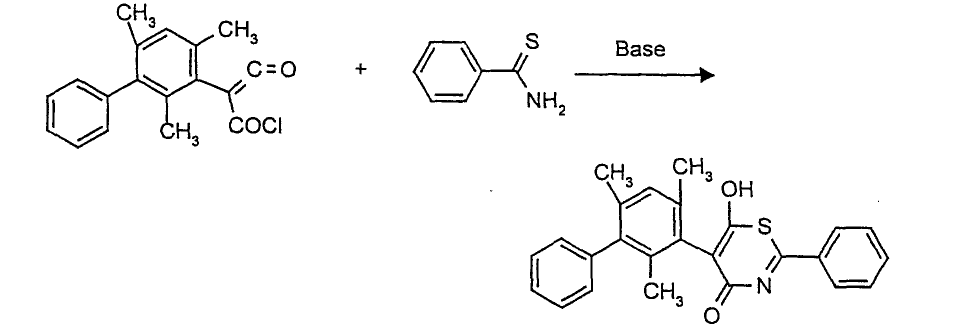

Verwendet man gemäß Verfahren (G) 5-[(6-Methyl-3-phenyl)-phenyl]-2,3-tetramethylen-4-oxo-valeriansäureethylester, so kann der Verlauf des erfindungsgemäßen Verfahrens durch folgendes Reaktionsschema wiedergegeben werden:

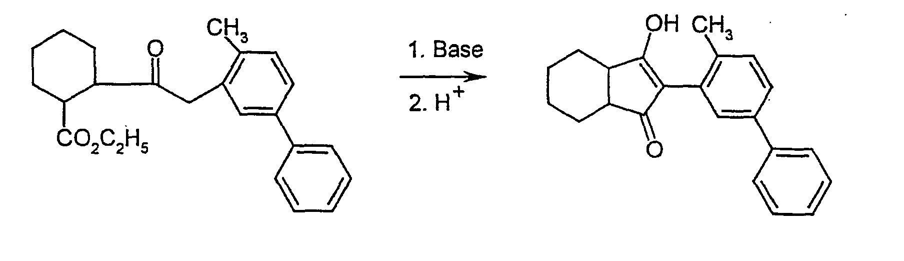

Verwendet man gemäß Verfahren (H) 5-[(2,4,6-Trimethyl-3-phenyl)-phenyl]-2,2-dimethyl-5-oxo-hexansäure-ethylester, so kann der Verlauf des erfindungsgemäßen Verfahrens durch folgendes Reaktionsschema wiedergegeben werden:

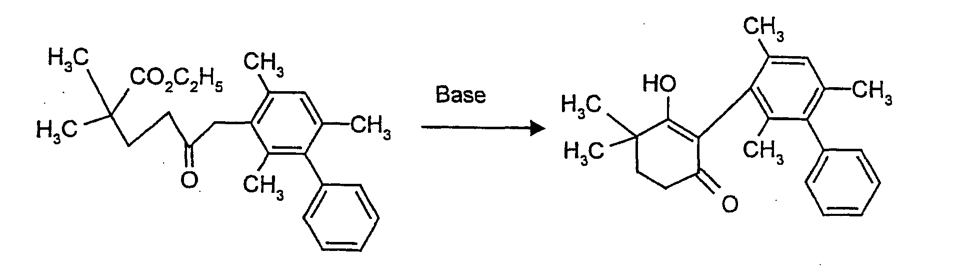

Verwendet man gemäß Verfahren (I) 3-[(2,6-Dimethyl-3-brom)-phenyl]-4,4-(pentamethylen)-pyrrolidin-2,4-dion und 4-Chlorphenylboronsäure als Ausgangsprodukte, so kann der Reaktionsverlauf durch folgendes Schema wiedergegeben werden:

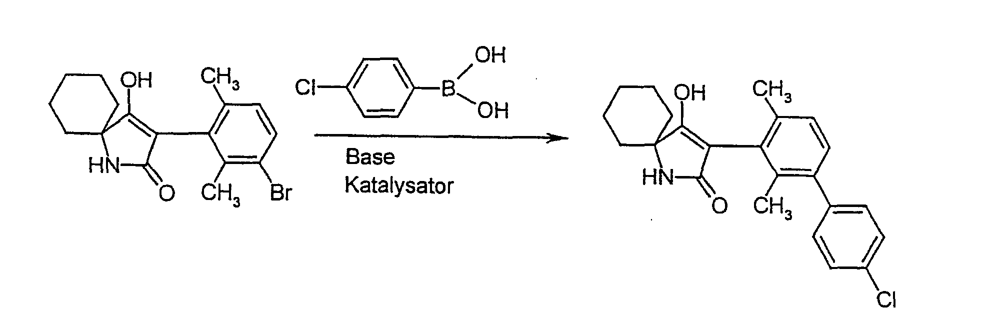

Verwendet man gemäß Verfahren (Jα) 3-[(2-Chlor-5-(3-chlor-phenyl))-phenyl]-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

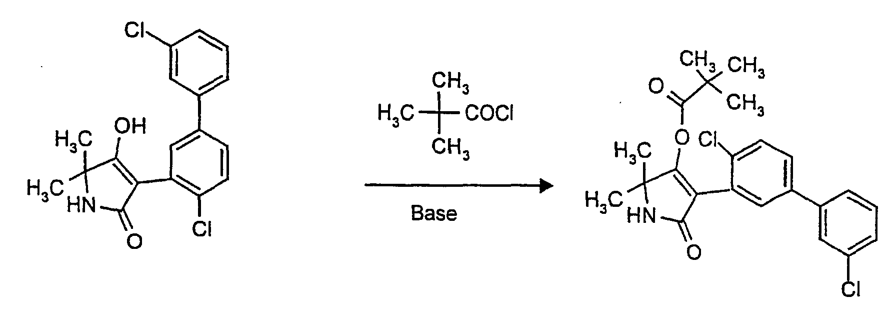

Verwendet man gemäß Verfahren (J) (Variante ß) 3-[(6-Methyl-3-(4-methoxy-phenyl))-phenyl]-4-hydroxy-5-phenyl-Δ³-dihydrokran-2-on und Acetanhydrid als Ausgangsverbindungen, so kann der Verlauf des erfindungsgemäßen Verfahrens durch folgendes Reaktionsschema wiedergegeben werden:

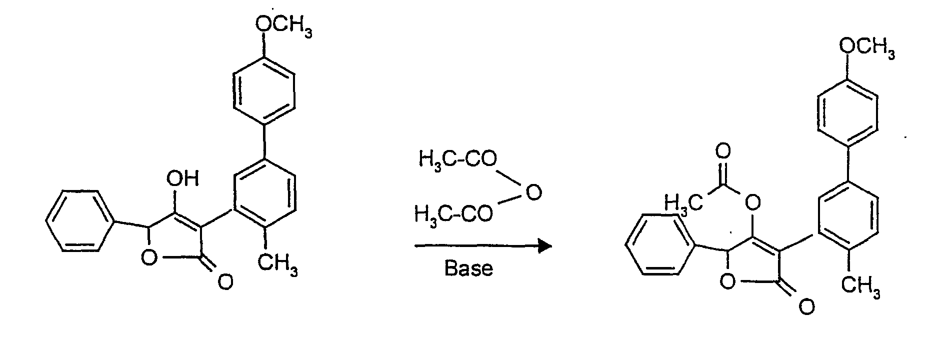

Verwendet man gemäß Verfahren (K) 8-[(2,6-Dimethyl-3-phenyl)-phenyl]-1,6-diazabicyclo-(4,3,0^{1,6})-nonan-7,9-dion und Chlorameisensäureethoxyethylester als Ausgangsverbindungen, so kann der Verlauf des erfindungsgemäßen Verfahrens durch folgendes Reaktionsschema wiedergegeben werden:

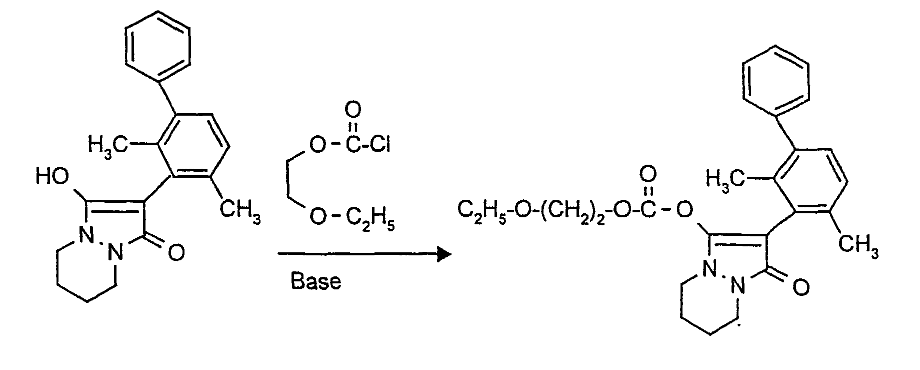

Verwendet man gemäß Verfahren (L), 3-[(2-Chlor-5-(4-fluor-phenyl))-phenyl]-4-hydroxy-5-methyl-6-(3-pyridyl)-pyron und Chlormonothioameisensäuremethylester als Ausgangsprodukte, so kann der Reaktionsverlauf folgendermaßen wiedergegeben werden:

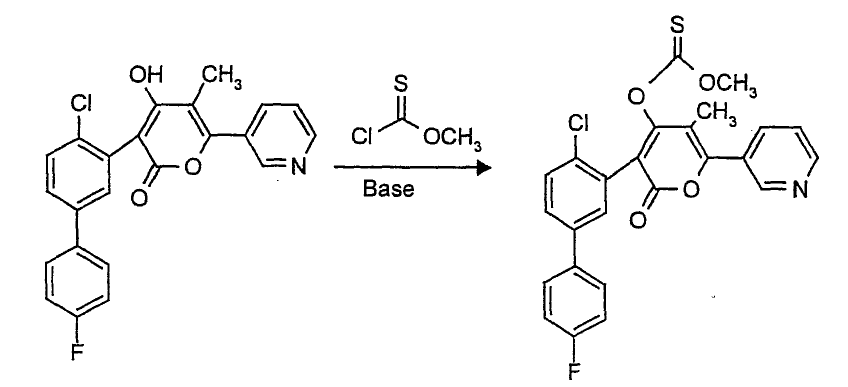

Verwendet man gemäß Verfahren (M) 2-[(2,4,6-Trimethyl-3-(4-methyl-phenyl))-phenyl]-5,5-pentamethylen-pyrrolidin-2,4-dion und Methansulfonsäurechlorid als Ausgangsprodukt, so kann der Reaktionsverlauf durch folgendes Reaktionsschema wiedergegeben werden:

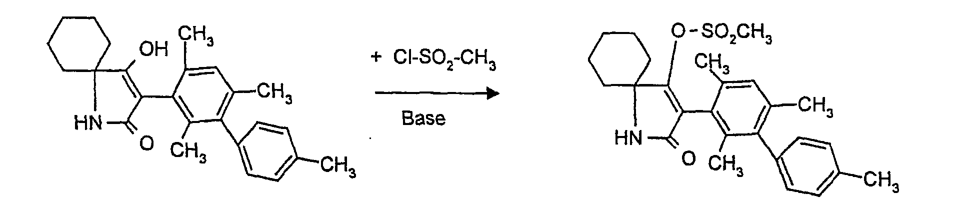

Verwendet man gemäß Verfahren (N) 2-[(6-Methyl-3-phenyl)-phenyl]-4-hydroxy-5,5-dimethyl-Δ³-dihydrofuran-2-on und Methanthio-phosphonsäurechlorid-(2,2,2-trifluorethylester) als Ausgangsprodukte, so kann der Reaktionsverlauf durch folgendes Reaktionsschema wiedergegeben werden:

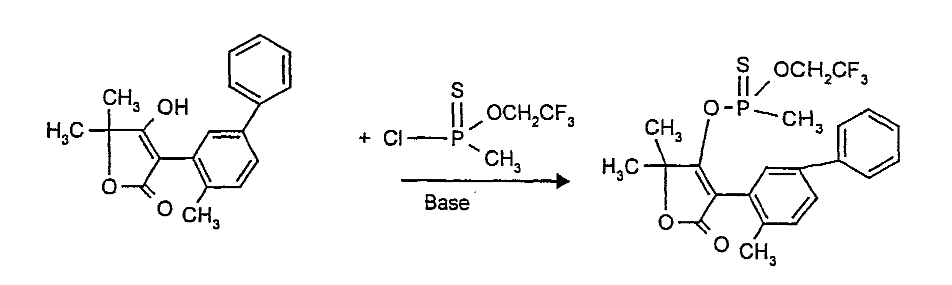

Verwendet man gemäß Verfahren (O) 3-[(2-Trifluormethyl-5-(4-trifluormethyl-phenyl))-phenyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

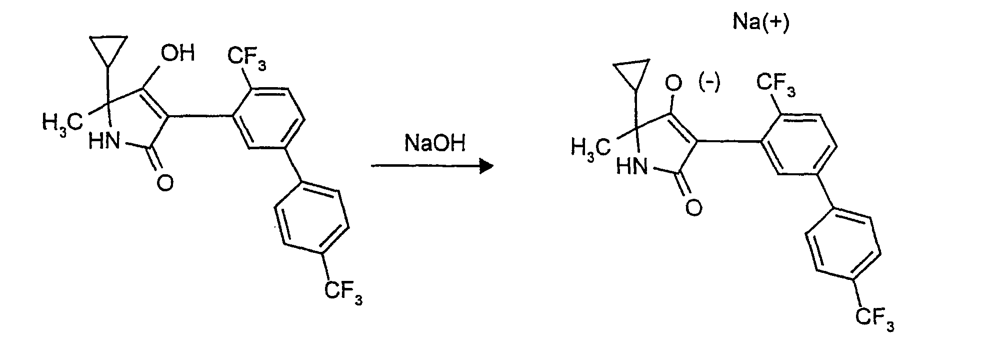

Verwendet man gemäß Verfahren (P) (Variante α) 3-[(6-Methyl-6-(3-trifluormethylphenyl))-phenyl]-4-hydroxy-5-tetramethylen-Δ³-dihydro-furan-2-on und Ethylisocyanat als Ausgangsprodukte, so kann der Reaktionsverlauf durch folgendes Reaktionsschema wiedergegeben werden:

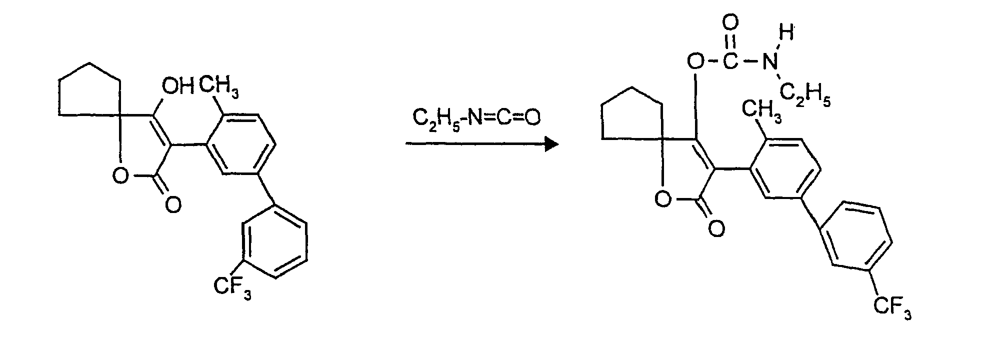

Verwendet man gemäß Verfahren (P) (Variante β) 3-[(2-Chlor-5-phenyl)-phenyl]-5-methyl-pyrrolidin-2,4-dion und Dimethylcarbamidsäurechlorid als Ausgangsprodukte, so kann der Reaktionsverlauf durch folgendes Schema wiedergegeben werden:

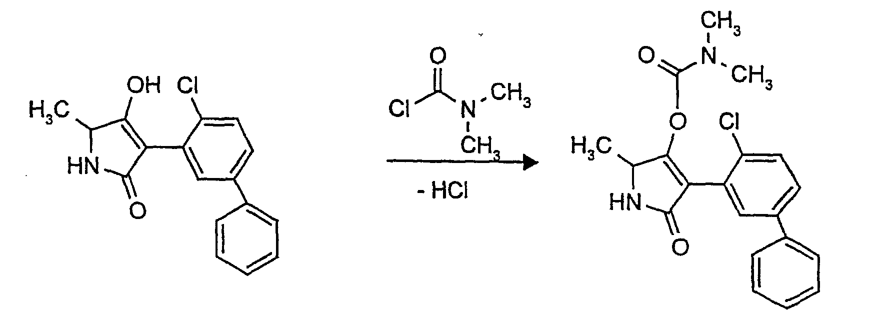

Die beim erfindungsgemäßen Verfahren (a) als Ausgangsstoffe benötigten Verbindungen der Formel (II) 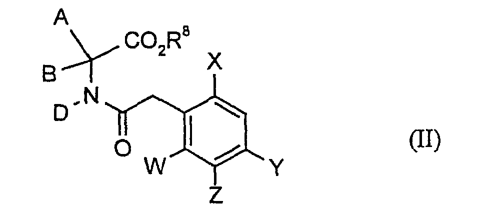 in welcher
A, B, D, W, X, Y, Z und R⁸ die oben angegebenen Bedeutungen haben,
sind neu.

Man erhält die Acylaminosäureester der Formel (II) beispielsweise, wenn man Aminosäurederivate der Formel (XXIII) 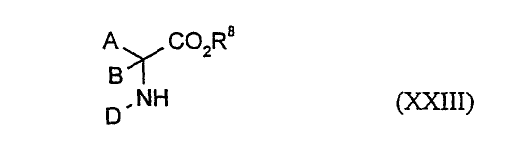 in welcher
- A, B, R⁸ und D: die oben angegebenen Bedeutungen haben,
mit substituierten Phenylessigsäurehalogeniden der Formel (XXIV) 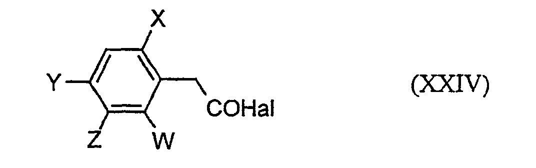 in welcher
- W, X ,Y und Z: die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968)
oder wenn man Acylaminosäuren der Formel (XXV) 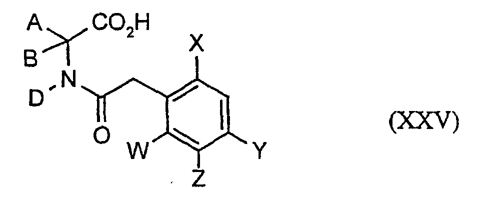 in welcher
- A, B, D, W, X, Y und Z: die oben angegebenen Bedeutungen haben,
verestert (Chem. Ind. (London) 1568 (1968)).

Die Verbindungen der Formel (XXV) 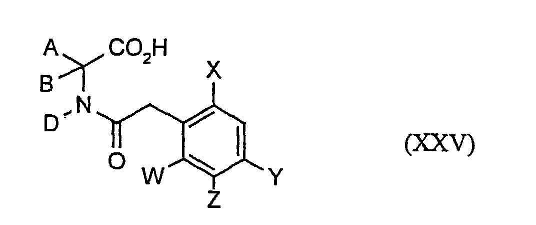 in welcher
- A, B, D, W, X, Y und Z: die oben angegebenen Bedeutungen haben,
sind neu.

Man erhält die Verbindungen der Formel (XXV), wenn man Aminosäuren der Formel (XXVI) 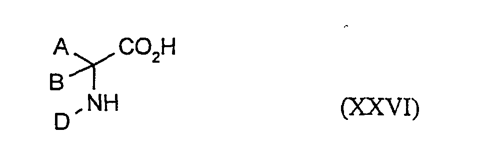 in welcher
- A, B und D: die oben angegebenen Bedeutungen haben,
mit substituierten Phenylessigsäurehalogeniden der Formel (XXIV) 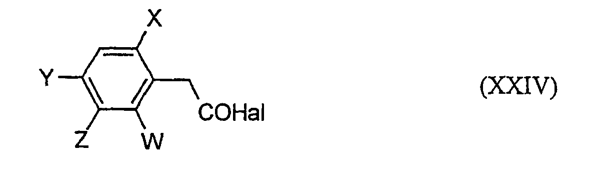 in welcher
- W, X, Y und Z: die oben angegebenen Bedeutungen haben und
- Hal: für Chlor oder Brom steht,
beispielsweise nach Schotten-Baumann acyliert (Organikum, VEB Deutscher Verlag der Wissenschaften, Berlin 1977, S. 505).

Die Verbindungen der Formel (XXIV) sind neu. Sie lassen sich nach im Prinzip bekannten Verfahren darstellen (s. z.B. H. Henecka, Houben-Weyl, Methoden der Organischen Chemie, Bd. 8, S. 467-469 (1952)).

Man erhält die Verbindungen der Formel (XXIV) beispielsweise, indem man substituierte Phenylessigsäuren der Formel (XXVII) 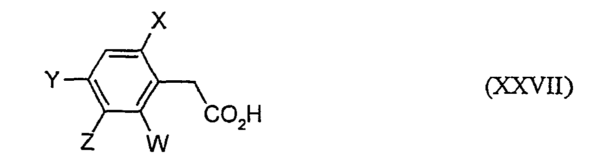 in welcher
- W, X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXIII) und (XXVI) sind teilweise bekannt und/oder lassen sich nach bekannten Verfahren darstellen (siehe z.B. Compagnon, Miocque Ann. Chim. (Paris) [14] 5, S. 11-22,23-27 (1970)).

Die substituierten cyclischen Aminocarbonsäuren der Formel (XXVIa), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als ß bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 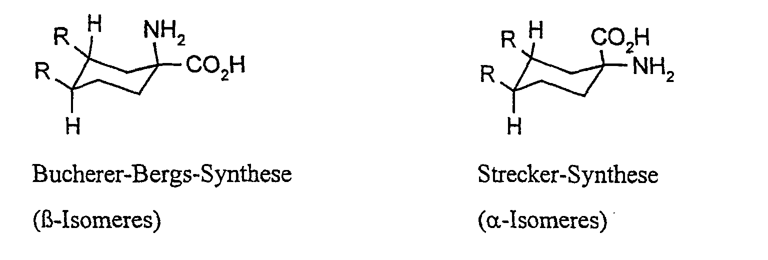 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 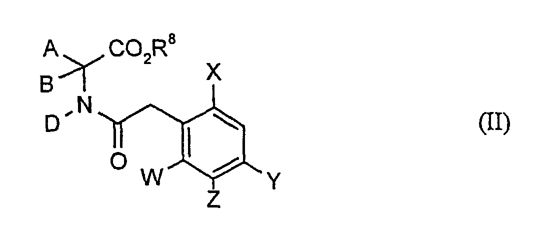 in welcher
- A, B, D, W, X, Y, Z und R⁸: die oben angegebenen Bedeutungen haben,
herstellen, indem man Aminonitrile der Formel (XXVIII) 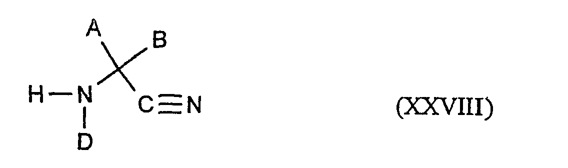 in welcher
- A, B und D: die oben angegebenen Bedeutungen haben,
mit substituierten Phenylessigsäurehalogeniden der Formel (XXIV) 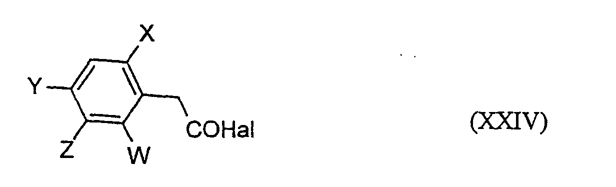 in welcher
- W, X, Y, Z und Hal: die oben angegebenen Bedeutungen haben,
zu Verbindungen der Formel (XXIX) 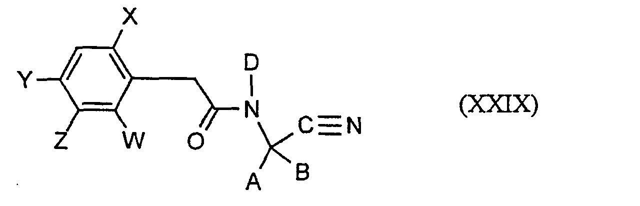 in welcher
- A, B, D, W, X, Y und Z: die oben angegebenen Bedeutungen haben,
umsetzt,
und diese anschließend einer sauren Alkoholyse unterwirft.

Die Verbindungen der Formel (XXIX) sind ebenfalls neu.

Die bei dem erfindungsgemäßen Verfahren (B) als Ausgangstoffe benötigten Verbindungen der Formel (III) 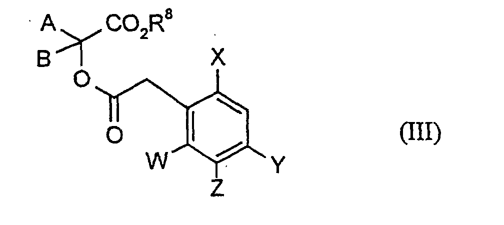 in welcher
- A, B, W, X, Y, Z und R⁸: die oben angegebenen Bedeutungen haben,
sind neu.

Sie lassen sich nach im Prinzip bekannten Methoden herstellen.

So erhält man die Verbindungen der Formel (III) beispielsweise, wenn man
2-Hydroxycarbonsäureester der Formel (XXX) 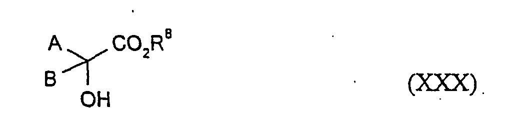 in welcher
- A, B und R⁸: die oben angegebenen Bedeutungen haben,
mit substituierten Phenylessigsäurehalogeniden der Formel (XXIV) 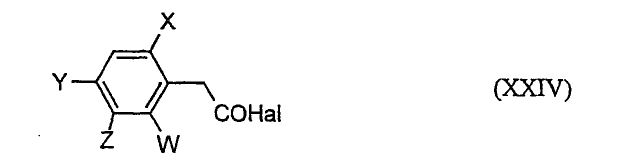 in welcher
- W, X, Y, Z und Hal: die oben angegebenen Bedeutungen haben,
acyliert (Chem. Reviews 52, 237-416 (1953) und eingangs zitierte Anmeldungen).

Weiterhin erhält man Verbindungen der Formel (III), wenn man
substituierte Phenylessigsäuren der Formel (XXVII) 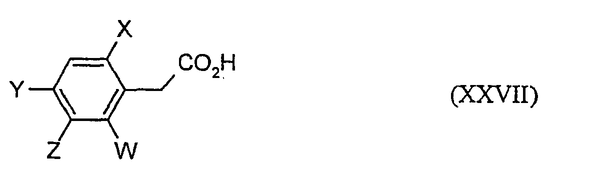 in welcher
- W, X, Y und Z: die oben angegebenen Bedeutungen haben,
mit α-Halogencarbonsäureestern der Formel (XXXI) 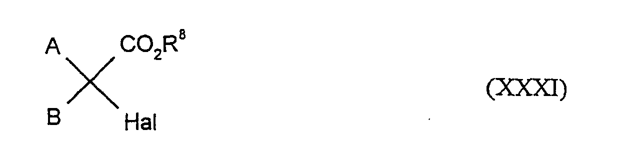 in welcher
- A, B und R⁸: die oben angegebenen Bedeutungen haben und
- Hal: für Chlor oder Brom steht,
alkyliert.

Die Verbindungen der Formel (XXVII) sind neu.

Die Verbindungen der Formel (XXXI) sind käuflich.

Beispielsweise erhält man die Verbindungen der Formel (XXVII), 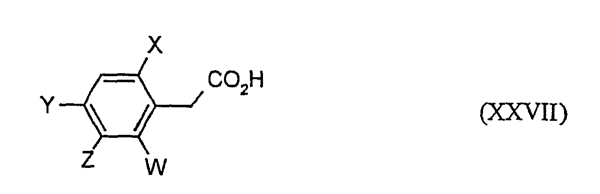 in welcher
- W, X, Y und Z: die oben angegebenen Bedeutungen haben,

α) wenn man Verbindungen der Formel (XXVII-a) 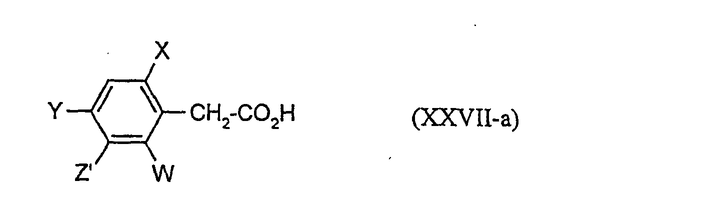 in welcher
   - X und Y: die oben angegebene Bedeutung haben,
   - Z': für Chlor oder Brom, bevorzugt für Brom steht,
   mit Boronsäuren der Formel (XII) 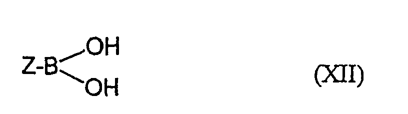 in welcher
   - Z: die oben angegebene Bedeutung hat,
   in Gegenwart eines Lösungsmittels, einer Base und eines Katalysators (bevorzugt eines Palladiumkomplexes, wie z.B. Palladium-tetrakis(triphenylphosphin)) umsetzt oder
β) wenn man Phenylessigsäureester der Formel (XXXII) 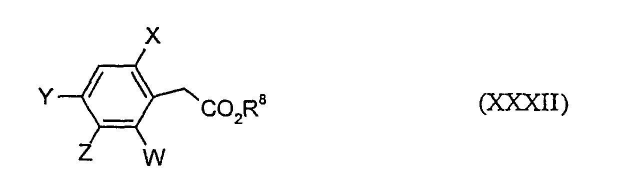 in welcher
   - W, X, Y, Z und R⁸: die oben angegebene Bedeutung haben,
   in Gegenwart von Säuren oder Basen, in Gegenwart eines Lösungsmittels unter allgemein bekannten Standardbedingungen verseift oder
γ) wenn man Phenylessigsäuren der Formel (XXVII-b) 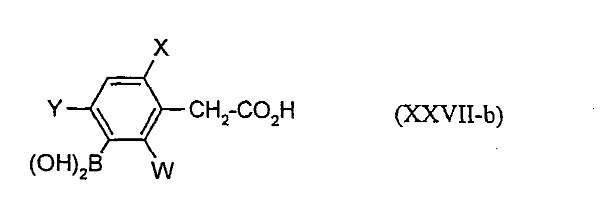 in welcher
   W, X und Z die oben angegebene Bedeutung haben,
   mit Halogenverbindungen der Formel (XXXIII),

   Z-Hal (XXXIII)

   in welcher
   - Z: die oben angegebene Bedeutung hat und
   - Hal: für Chlor, Brom oder Iod, bevorzugt für Brom und Iod steht,
   in Gegenwart eines Lösungsmittels, einer Base und eines Katalysators (bevorzugt eines der oben genannten Palladiumkomplexe) umsetzt.

Die Verbindungen der Formeln (XII) und (XXXIII) sind teilweise bekannt, teilweise käuflich oder lassen sich nach im Prinzip bekannten Verfahren herstellen. Die Phenylessigsäuren der Formel (XXVII-a) sind teilweise aus WO 97/01 535, WO 97/36 868 und WO 98/05 638 bekannt oder lassen sich nach den dort beschriebenen Verfahren herstellen.

Die Verbindungen der Formeln (XXVII-b) und (XXXII) sind neu.

Man erhält die Verbindungen der Formel (XXVII-b) 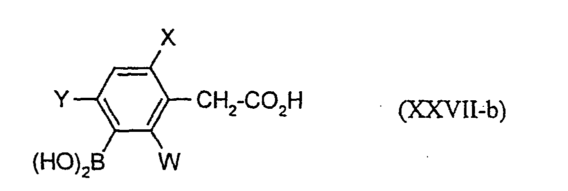 in welcher
- W, X und Y: die oben angegebene Bedeutung haben,
beispielsweise, wenn man Phenylessigsäuren der Formel (XXVII-a) 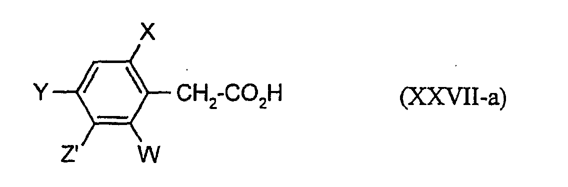 in welcher
- W, X, Y und Z': die oben angegebene Bedeutung haben,
mit Lithiumverbindungen der Formel (XXXIV)

Li-R²¹ (XXXIV)

in welcher
- R²¹: für C₁-C₈-Alkyl oder Phenyl, bevorzugt für n-C₄H₉ steht,
und Boronsäureestem der Formel (XXXV)

B(OR⁸)₃ (XXXV)

in welcher
- R⁸: die oben angegebene Bedeutung hat,
in Gegenwart eines Verdünnungsmittels umsetzt.

Die Verbindungen der Formeln (XXXIV) und (XXXV) sind käufliche Verbindungen.

Die Verbindungen der Formel (XXXII) 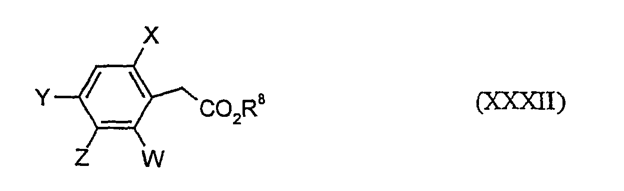 in welcher
- W, X, Y, Z und R⁸: die oben angegebene Bedeutung haben,
erhält man beispielsweise,
wenn man Phenylessigsäureester der Formel (XXXII-a) 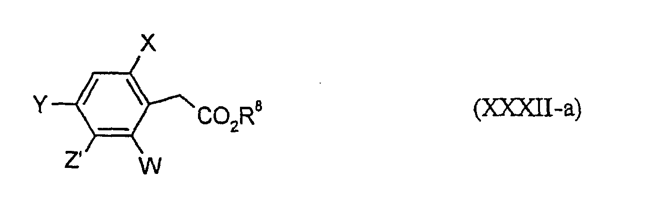 in welcher
- R⁸, W, X, Y und Z': die oben angegebene Bedeutung haben,
mit Boronsäuren der Formel (XII) 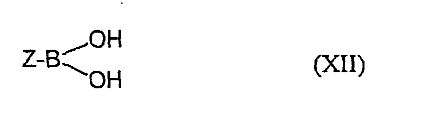 in welcher
- Z: die oben angegebene Bedeutung hat,
in Gegenwart eines Lösungsmittels, einer Base und eines Katalysators (bevorzugt eines der oben genannten Palladiumkomplexe) umsetzt.

Die Phenylessigsäureester der Formel (XXXII-a) sind teilweise aus den Anmeldungen WO 97/01535, WO 97/36868 und WO 98/0563 bekannt oder lassen sich nach den dort beschriebenen Verfahren herstellen.

Die bei dem obigen Verfahren (C) als Ausgangsstoffe benötigten Verbindungen der Formel (IV) 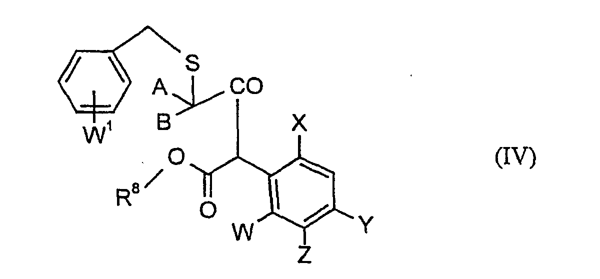 in welcher
- A, B, W, W¹, X, Y, Z und R⁸: die oben angegebenen Bedeutungen haben,
sind neu.

Sie lassen sich nach im Prinzip bekannten Methoden herstellen.

Man erhält die Verbindungen der Formel (IV) beispielsweise, wenn man
substituierte Phenylessigsäureester der Formel (XXXII) 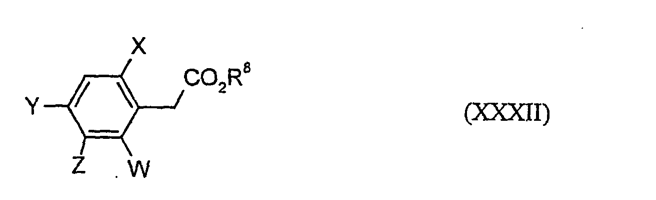 in welcher
- W, X, Y, R⁸ und Z: die oben angegebenen Bedeutungen haben,
mit 2-Benzylthio-carbonsäurehalogeniden der Formel (XXXVI) 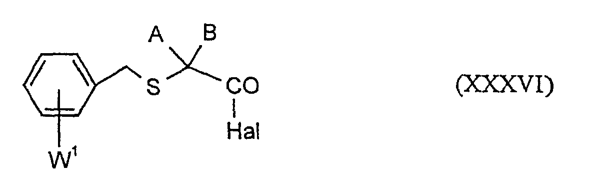 in welcher
- A,B und W¹: die oben angegebenen Bedeutungen haben und
- Hal: für Halogen (insbesondere Chlor oder Brom) steht,
in Gegenwart von starken Basen acyliert (siehe z.B. M.S. Chambers, E.J. Thomas, D.J. Williams, J. Chem. Soc. Chem. Commun., (1987), 1228).

Die Verbindungen der Formel (XXXII) sind neu. Man erhält Verbindungen der Formel (XXXII) auch wenn man Verbindungen der Formel (XXVII) 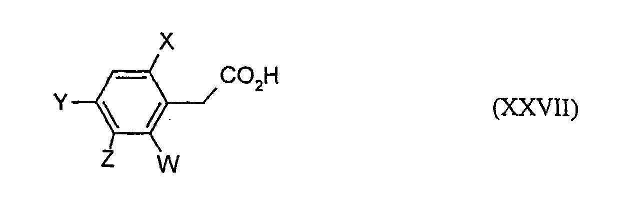 in welcher
- W, X, Y und Z: die oben angegebene Bedeutung haben,
in Gegenwart von Alkoholen und wasserentziehenden Mitteln (z.B. konz. Schwefelsäure) verestert,
oder Alkohole mit Verbindungen der Formel (XXIV) 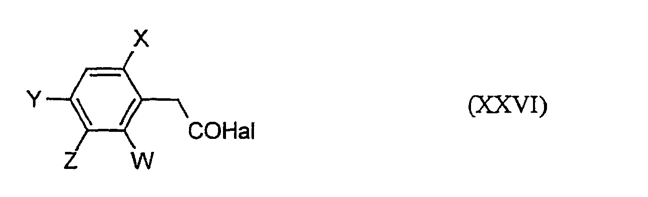 in welcher
- W, X, Y, Z und Hal: die oben angegebenen Bedeutungen haben
acyliert (Chem. Reviews 52, 237-416 (1953)).

Die Benzylthio-carbonsäurehalogenide der Formel (XXXVI) sind teilweise bekannt und/oder lassen sich nach bekannten Verfahren herstellen (J. Antibiotics (1983), 26, 1589).

Die bei den obigen Verfahren (D), (E) und (F) als Ausgangsstoffe benötigten Halogencarbonylketene der Formel (V) sind neu. Sie lassen sich nach im Prinzip bekannten Methoden in einfacher Weise herstellen (vgl. beispielsweise Org. Prep. Proced. Int., 7, (4), 155-158, 1975 und DE 1 945 703). So erhält man z.B. die Verbindungen der Formel (V) 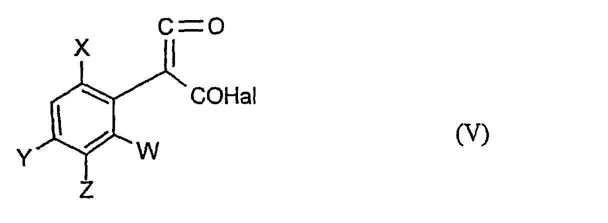 in welcher
- W, X, Y und Z: die oben angegebenen Bedeutungen haben und
- Hal: für Chlor oder Brom steht,
wenn man
substituierte Phenylmalonsäuren der Formel (XXXVII) 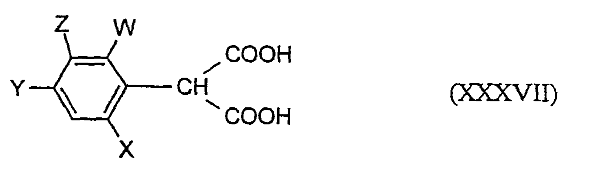 in welcher
- W, X, Y und Z: die oben angegebenen Bedeutungen haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Dimethylformamid, Methyl-Sterylformamid oder Triphenylphosphin und gegebenenfalls in Gegenwart von Basen wie z.B. Pyridin oder Triethylamin, umsetzt.

Die substituierten Phenylmalonsäuren der Formel (XXXVII) sind neu. Sie lassen sich in einfacher Weise nach bekannten Verfahren herstellen (vgl. z.B. Organikum, VEB Deutscher Verlag der Wissenschaften, Berlin 1977, S. 517 ff, EP-A-528 156, WO 97/36868, WO 97/01535 und WO 98/05638).

So erhält man Phenylmalonsäuren der Formel (XXXVII) 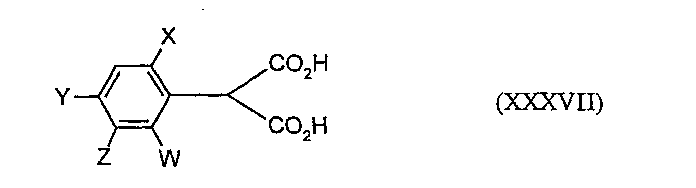 in welcher
- W, X, Y und Z: die oben angegebenen Bedeutungen haben,
wenn man Phenylmalonsäureester der Formel (VI) 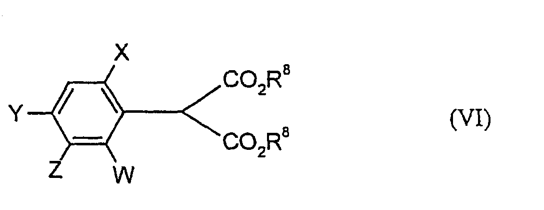 in welcher
- W, X, Y, Z und R⁸: die oben angegebene Bedeutung haben,
zunächst in Gegenwart einer Base und einem Lösungsmittel verseift und anschließend vorsichtig ansäuert (EP-528 156, WO 97/36868, WO 97/01535).

Die Malonsäureester der Formel (VI) 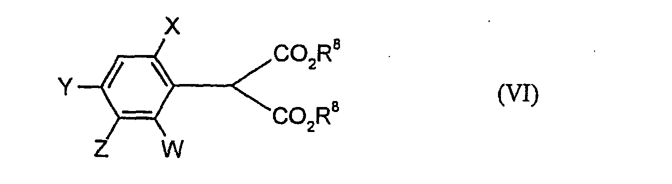 in welcher
- W, X, Y, Z und R⁸: die oben angegebene Bedeutung haben,
sind neu.

Sie lassen sich nach allgemein bekannten Methoden der Organischen Chemie darstellen (vgl. z.B. Tetrahedron Lett. 27, 2763 (1986) und Organikum VEB Deutscher Verlag der Wissenschaften, Berlin 1977, S. 587 ff.).

Die für das erfindungsgemäße Verfahren (D) als Ausgangsstoffe benötigten Hydrazine der Formel (VII)

A-NH-NH-D (VII),

in welcher
- A und D: die oben angegebenen Bedeutungen haben,
sind teilweise bekannt und/oder nach literaturbekannten Methoden herstellbar (vgl. beispielsweise Liebigs Ann. Chem. 585, 6 (1954); Reaktionen der organischen Synthese, C. Ferri, Seite 212, 513; Georg Thieme Verlag Stuttgart, 1978; Liebigs Ann. Chem. 443, 242 (1925); Chem. Ber. 98, 2551 (1965), EP-508 126).

Die für das erfindungsgemäße Verfahren (E) als Ausgangsstoffe benötigten Carbonylverbindungen der Formel (VIII) 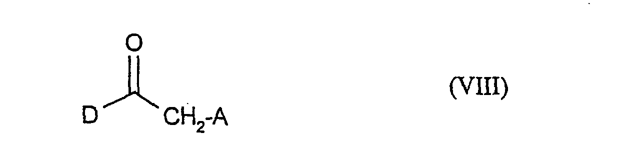 in welcher
- A und D: die oben angegebenen Bedeutungen haben,
oder deren Silylenolether der Formel (VIIIa) 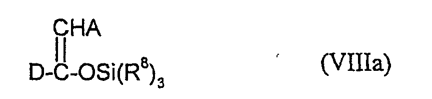 in welcher
- A, D und R⁸: die oben angegebenen Bedeutungen haben,
sind käufliche, allgemeine bekannte oder nach bekannten Verfahren zugängliche Verbindungen.

Die Herstellung der zur Durchführung des erfindungsgemäßen Verfahrens (F) als Ausgangsstoffe benötigten Ketensäurechloride der Formel (V) wurde bereits oben beschrieben. Die zur Durchführung des erfindungsgemäßen Verfahrens (F) benötigten Thioamide der Formel (IX) 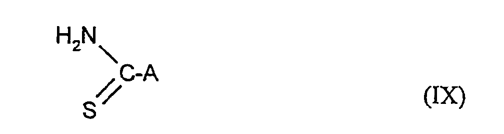 in welcher
- A: die oben angegebene Bedeutung hat,
sind allgemein in der Organischen Chemie bekannte Verbindungen.

Die bei dem obigen Verfahren (G) als Ausgangsstoffe benötigten Verbindungen der Formel (X) 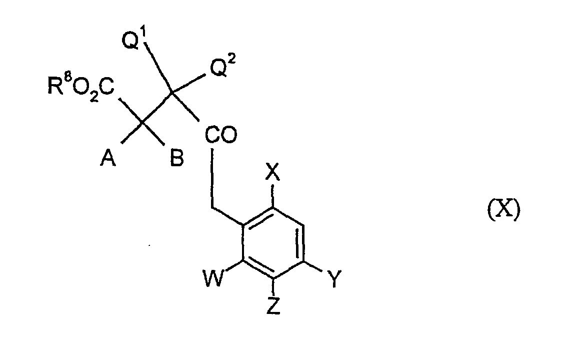 in welcher
- A, B, Q¹, Q², W, X, Y, Z und R⁸: die oben angegebene Bedeutung haben,
sind neu.

Sie lassen sich nach im Prinzip bekannten Methoden herstellen.

Man erhält die Verbindungen der Formel (X) beispielsweise, wenn man Verbindungen der Formel (XXXVIII) 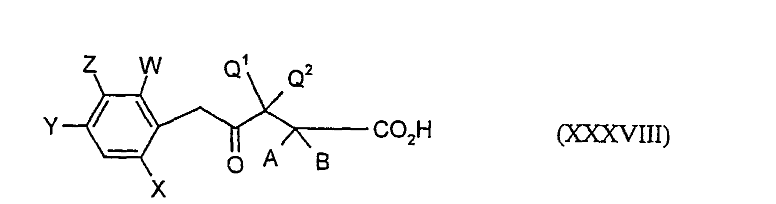 in welcher
W, X, Y, Z, A, B, Q¹ und Q² die oben angegebene Bedeutung haben,
verestert (vgl. z.B. Organikum, 15. Auflage, Berlin, 1977, Seite 499) oder alkyliert (siehe Herstellungsbeispiel).

Die Verbindungen der Formel (XXXVIII) 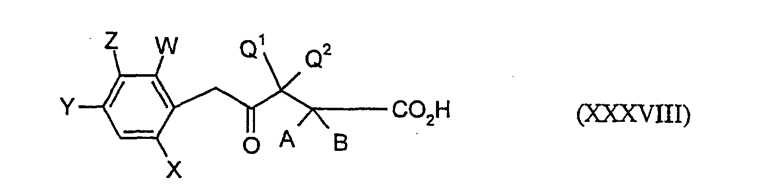 in welcher
A, B, Q¹, Q², W, X, Y und Z die oben angegebene Bedeutung haben,
sind neu, lassen sich aber nach im Prinzip bekannten Methoden herstellen (siehe Herstellungsbeispiel).

Man erhält die 5-Aryl-4-ketocarbonsäuren der Formel (XXXVIII) beispielsweise, wenn man 2-Phenyl-3-oxo-adipinsäureester der Formel (XXXIX) 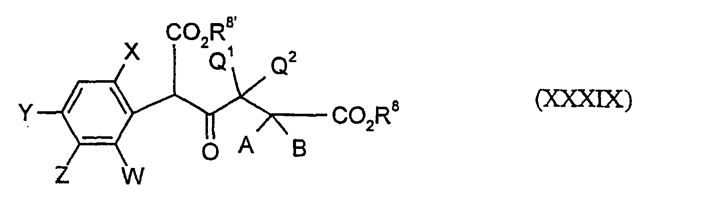 in welcher
A, B, D¹, D², W, X, Y und Z die oben angegebene Bedeutung haben und
- R⁸ und R^{8'}: für Alkyl (insbesondere C₁-C₈-Alkyl) stehen,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521).

Die Verbindungen der Formel (XXXIX) 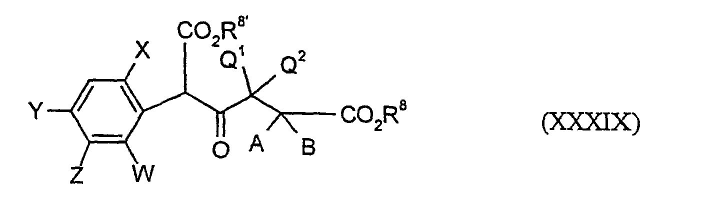 in welcher
- A, B, Q¹, Q², W, X, Y, Z, R⁸, R^{8'}: die oben angegebene Bedeutung haben,
sind neu.

Man erhält die Verbindungen der Formel (XXXIX) beispielsweise,
wenn man Dicarbonsäurehalbesterchloride der Formel (XL), 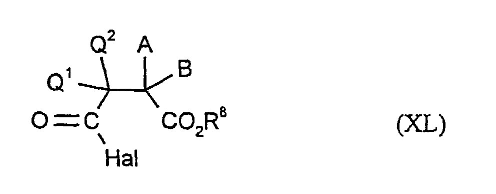 in welcher
- A, B, Q¹, Q² und R⁸: die oben angegebene Bedeutung haben und
- Hal: für Chlor oder Brom steht,
oder Carbonsäureanhydride der Formel (XLI-A) 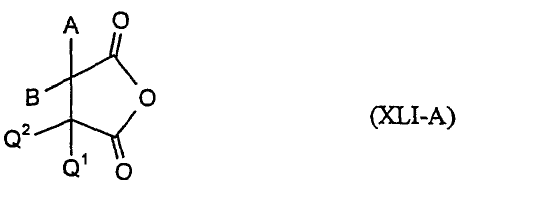 in welcher
A, B, Q¹ und Q² die oben angegebene Bedeutung haben,
mit einem Phenylessigsäureester der Formel (XXXII) 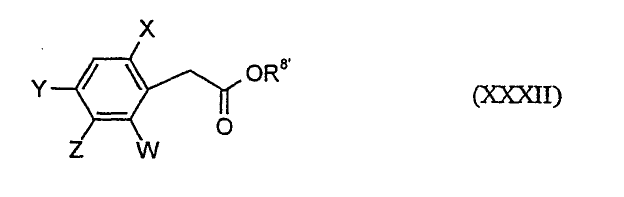 in welcher
- W, X, Y, Z und R^{8'}: die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228, vgl. auch die Herstellungsbeispiele).

Die Verbindungen der Formeln (XL) und (XLI-A) sind teilweise bekannte oder käufliche Verbindungen der Organischen Chemie und/oder lassen sich nach im Prinzip bekannten Methoden in einfacher Weise herstellen.

Die bei dem obigen Verfahren (H) als Ausgangsstoffe benötigten Verbindungen der Formel (XI) 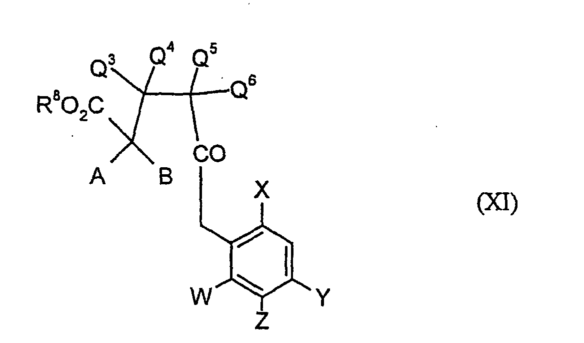 in welcher
- A, B, Q³, Q⁴, Q⁵, Q⁶, W, X, Y, Z und R⁸: die oben angegebene Bedeutung haben,
sind neu.

Sie lassen sich nach im Prinzip bekannten Methoden herstellen.

Man erhält die 6-Aryl-5-ketocarbonsäureester der Formel (XI) beispielsweise, wenn man 6-Aryl-5-ketocarbonsäuren der Formel (XLII) 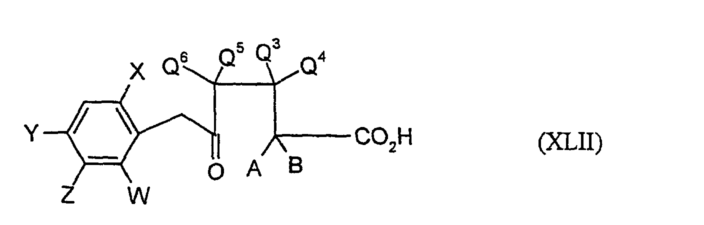 in welcher
- A, B, Q³, Q⁴, Q⁵, Q⁶, W, X, Y und Z: die oben angegebene Bedeutung haben,
verestert, (vgl. z.B. Organikum, 15. Auflage, Berlin, 1977, Seite 499).

Die Verbindungen der Formel (XLII) 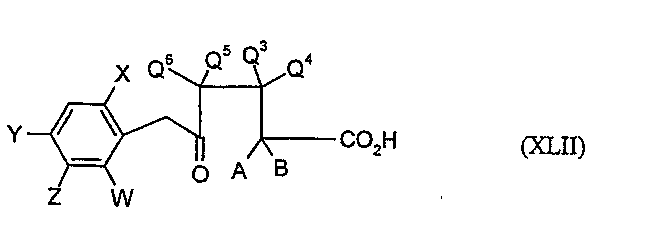 in welcher
- A, B, Q³, Q⁴, Q⁵, Q⁶, W, X, Y und Z: die oben angegebene Bedeutung haben,
sind neu. Sie lassen sich nach im Prinzip bekannten Methoden herstellen, beispielsweise wenn man
substituierte 2-Phenyl-3-oxo-heptandisäureester der Formel (XLIII) 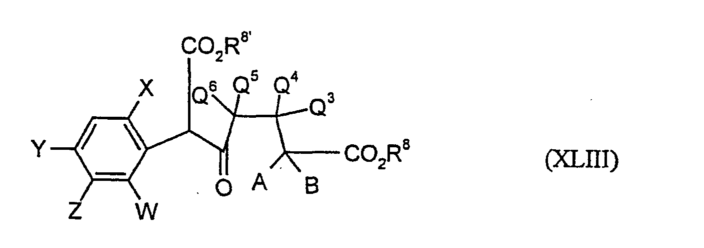 in welcher
A, B, Q³, Q⁴, Q⁵, Q⁶, W, X, Y und Z die oben angegebene Bedeutung haben und
- R⁸ und R^{8'}: für Alkyl (bevorzugt C₁-C₆-Alkyl), stehen,
gegebenenfalls in Gegenwart eines Verdünnungsmittels und gegebenenfalls in Gegenwart einer Base oder Säure verseift und decarboxyliert (vgl. z.B. Organikum, 15. Auflage, Berlin, 1977, Seite 519 bis 521) (siehe auch Herstellungsbeispiel).

Die Verbindungen der Formel (XLIII) 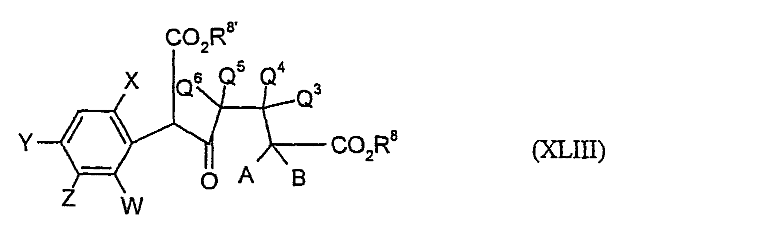 in welcher
A, B, Q³, Q⁴, Q⁵, Q⁶, W, X, Y, Z, R⁸ und R^{8'} die oben angegebene Bedeutung haben,
sind neu. Man erhält sie beispielsweise
wenn man Dicarbonsäureester der Formel (XLIV), 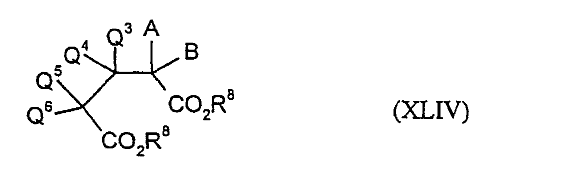 in welcher
A, B, Q³, Q⁴, Q⁵, Q⁶ und R⁸ die oben angegebene Bedeutung haben,
mit einem substituierten Phenylessigsäureester der Formel (XXXII) 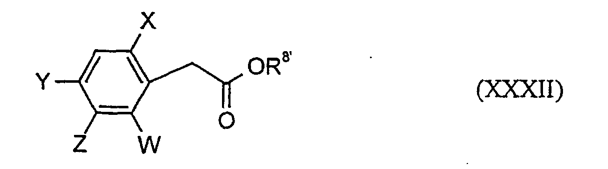 in welcher
- W, X, Y, Z und R^{8'}: die oben angegebene Bedeutung haben,
in Gegenwart eines Verdünnungsmittels und in Gegenwart einer Base kondensiert (siehe auch Herstellungsbeispiel).

Die Verbindungen der Formel (XLIV) sind teilweise bekannt und/oder lassen sich nach bekannten Verfahren darstellen.

Anstelle der Verbindungen der Formel (XLIV) lassen sich auch die entsprechenden Anhydride einsetzen. Man verfährt dann wie bei der Herstellung der Verbindungen der Formel (X), bei der man von den Anhydriden der Formel (XLI-B) ausgeht. Die Verbindungen der Formel (XLI-B) sind teilweise bekannte oder käufliche Verbindungen.

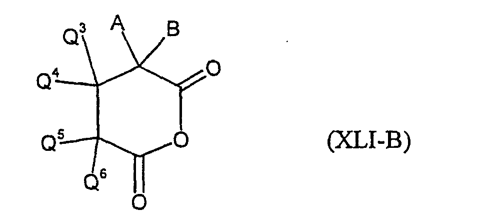

Die Verbindungen der Formel (XXXII) wurden bereits bei den Vorstufen für das Verfahren (B) beschrieben.

Man erhält die Verbindungen der Formel (XXXII) beispielsweise auch, indem man substituierte 1,1,1-Trichlor-2-phenylethane der Formel (XLV) 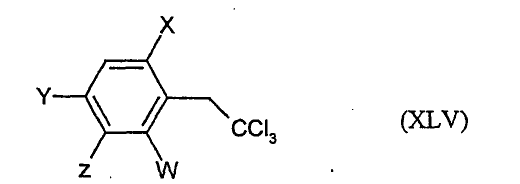 in welcher
- W, X, Y und Z: die oben angegebene Bedeutung haben,
zunächst mit Alkoholaten (z.B. Alkalimetallalkoholaten wie Natriummethylat oder Natriumethylat) in Gegenwart eines Verdünnungsmittels (z.B. dem vom Alkoholat abgeleiteten Alkohol) bei Temperaturen zwischen 0°C und 150°C, bevorzugt zwischen 20°C und 120°C, und anschließend mit einer Säure (bevorzugt eine anorganische Säure wie z.B. Schwefelsäure) bei Temperaturen zwischen - 20°C und 150°C, bevorzugt 0°C und 100°C, umsetzt (vgl. DE 3 314 249).

Die Verbindungen der Formel (LXV) sind neu, sie lassen sich nach im Prinzip bekannten Verfahren herstellen.

Man erhält die Verbindungen der Formel (XLV) beispielsweise, wenn man Aniline der Formel (XLVI) 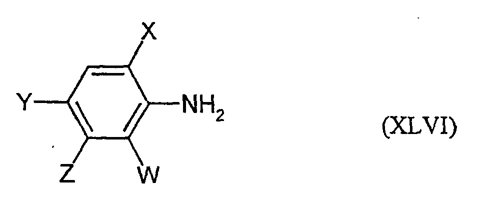 in welcher
- W, X, Y und Z: die oben angegebene Bedeutung haben,
in Gegenwart eines Alkylnitrits der Formel (XLVII)

R²¹-ONO (XLVII)

in welcher
- R²¹: für Alkyl, bevorzugt C₁-C₆-Alkyl steht,
in Gegenwart von Kupfer(II)-chlorid und gegebenenfalls in Gegenwart eines Verdünnungsmittels (z.B. eines aliphatischen Nitrils wie Acetonitril) bei einer Temperatur von -20°C bis 80°C, bevorzugt 0°C bis 60°C, mit Vinylidenchlorid (CH₂=CCl₂) umsetzt.

Die Verbindungen der Formel (XLVII) sind bekannte Verbindungen der Organischen Chemie. Kupfer(II)-chlorid und Vinylidenchlorid sind lange bekannt und käuflich.

Die Aniline der Formel (XLVI) sind teilweise neu.

Man erhält die Aniline der Formel (XLVI) beispielsweise indem man Aniline der Formel (XLVI-a) 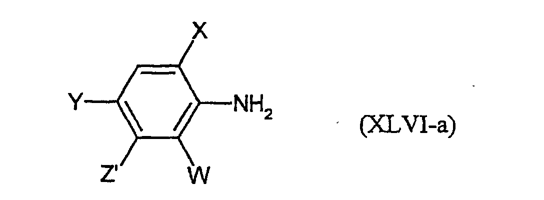 in welcher
W, X und Y die oben angegebene Bedeutung haben, und
- Z': für Chlor oder Brom, bevorzugt für Brom steht,
mit Boronsäuren der Formel (XII) 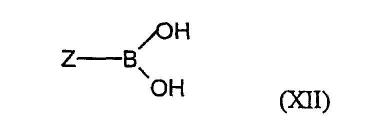 in welcher
- Z: die oben angegebene Bedeutung hat,
in Gegenwart eines Lösungsmittels, einer Base und eines Katalysators (bevorzugt eines Palladiumkomplexes, wie z.B. Palladium-tetrakis (triphenylphosphin)) umsetzt.

Die Aniline der Formel (XLVI-a) sind bekannte Verbindungen oder lassen sich im allgemeinen nach bekannten Verfahren herstellen.

Die bei dem obigen Verfahren (I) als Ausgangsstoffe benötigten Verbindungen der Formeln (I-1'a) bis (I-8'-a), in welchen A, B, D, Q¹, Q², Q³, Q⁴, Q⁵, Q⁶, W, X und Y die oben angegebene Bedeutung haben und Z' für Chlor und Brom, bevorzugt für Brom steht, sind teilweise bekannt (WO 96/35 664, WO 97/02 243 und WO 98/05 638) oder lassen sich gemäß den dort beschriebenen Verfahren oder nach Verfahren (A) bis (H) herstellen.

Die Boronsäuren der Formel (XII) 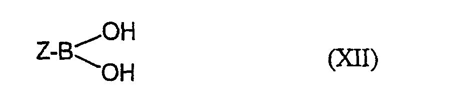 in welcher
- Z: die oben angegebene Bedeutung hat,
sind teilweise käuflich oder lassen sich nach allgemein bekannten Verfahren in einfacher Weise herstellen.

Die zur Durchführung der erfindungsgemäßen Verfahren (J), (K), (L), (M), (N), (O) und (P) außerdem als Ausgangsstoffe benötigten Säurehalogenide der Formel (XIII), Carbonsäureanhydride der Formel (XIV), Chlorameisensäureester oder Chlorameisensäurethioester der Formel (XV), Chlormonothioameisensäureester oder Chlordithioameisensäureester der Formel (XVI), Sulfonsäurechloride der Formel (XVII), Phosphorverbindungen der Formel (XVIII) und Metallhydroxide, Metallalkoxide oder Amine der Formel (XIX) und (XX) und Isocyanate der Formel (XXI) und Carbamidsäurechloride der Formel (XXII) sind allgemein bekannte Verbindungen der Organischen bzw. Anorganischen Chemie.

Die Verbindungen der Formeln (VII), (VIII), (IX), (XIII) bis (XXIII), (XXVI), (XXVIII), (XXX), (XXXVI), (XL), (XLI) und (XLIV) sind darüber hinaus aus den eingangs zitierten Patentanmeldungen bekannt und/oder lassen sich nach den dort angegebenen Methoden herstellen.

Das Verfahren (A) ist dadurch gekennzeichnet, daß man Verbindungen der Formel (II), in welcher A, B, D, W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa doppeltäquimolaren Mengen ein. Es ist jedoch auch möglich, die eine oder andere Komponente in einem größeren Überschuß (bis zu 3 Mol) zu verwenden.

Das Verfahren (B) ist dadurch gekennzeichnet, daß Verbindungen der Formel (III), in welcher A, B, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (B) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (C) ist dadurch gekennzeichnet, daß man Verbindungen der Formel (IV) in welcher A, B, W, X, Y, Z und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden wie z.B. Halogenwasserstoffsäuren, Schwefelsäure, Alkyl-, Aryl- und Haloalkylsulfonsäuren, insbesondere halogenierte Alkylcarbonsäuren wie z.B. Trifluoressigsäure.

Die Reaktionstemperaturen können bei der Durchführung des erfindungsgemäßen Verfahrens (C) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (C) wird im allgemeinen unter Normaldruck durchgeführt.

Bei der Durchführung des erfindungsgemäßen Verfahrens (C) setzt man die Reaktionskomponenten der Formeln (IV) und die Säure z.B. in äquimolaren Mengen ein. Es ist jedoch gegebenenfalls auch möglich, die Säure als Lösungsmittel oder als Katalysator zu verwenden.

Die Verfahren (D-α) und (D-β) sind dadurch gekennzeichnet, daß man Verbindungen der Formeln (V) oder (VI), in welchen W, X, Y, Z, R⁸ und Hal die oben angegebenen Bedeutungen haben mit Verbindungen der Formel (VII), in welcher A und D die oben angegebenen Bedeutungen haben, gegebenenfalls in Gegenwart einer Base und gegebenenfalls in Gegenwart eines Verdünnungsmittels umsetzt.

Als Verdünnungsmittel können bei den erfindungsgemäßen Verfahren (D-α) und (D-β) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie, nur im Fall, daß Verbindungen der Formel (VI) eingesetzt werden, Alkohole wie Methanol, Ethanol, Propanol, Iso-Propanol, Butanol, Iso-Butanol und tert.-Butanol.

Als Base kommen in dem Fall, daß Verbindungen der Formel (V) eingesetzt werden, anorganische Basen, insbesondere Alkali- oder Erdalkalicarbonate wie Natriumcarbonat, Kaliumcarbonat oder Calciumcarbonat sowie organische Basen wie beispielsweise Pyridin oder Triethylamin in Betracht und in dem Fall, daß Verbindungen der Formel (VI) eingesetzt werden, Alkalimetall- und Erdalkalimetalloxide, -hydroxide und -carbonate, wie Natriumhydroxid, Kaliumhydroxid, Magnesiumhydroxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)-ammoniumchlorid) oder TDA 1 (= Tris-(methoxyethoxyethyl)-amin) in Betracht, ferner Alkalimetalle wie Natrium oder Kalium, Alkalimmetall- und Erdalkalimetallamide und -hydride, wie Natriumamid, Natriumhydrid und Calciumhydrid, und außerdem auch Alkalimetallalkoholate, wie Natriummethylat und Kalium-tert.-butylat.

Die Reaktionstemperaturen können bei der Durchführung des erfindungsgemäßen Verfahrens (D-α) und (D-β) innerhalb eines größeren Bereiches variiert werden. Im allgemeinen arbeitet man bei Temperaturen zwischen -20°C und 250°C, vorzugsweise zwischen 0°C und 150°C.

Die erfindungsgemäßen Verfahren (D-α) und (D-β) werden im allgemeinen unter Normaldruck durchgeführt.

Bei der Durchführung der erfindungsgemäßen Verfahren (D-α) und (D-β) setzt man die Reaktionskomponenten der Formeln (V) und (VII) oder (VI) und (VII) und die gegebenenfalls eingesetzte deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das erfindungsgemäße Verfahren (E) ist dadurch gekennzeichnet, daß man Carbonylverbindungen der Formel (VIII) oder deren Enolether der Formel (VIII-a) mit Ketensäurehalogeniden der Formel (V) in Gegenwart eines Verdünnungsmittels und gegebenenfalls in Gegenwart eines Säureakzeptors umsetzt.

Als Verdünnungsmittel können bei dem erfindungsgemäßen Verfahren (E) alle inerten organischen Solventien eingesetzt werden. Vorzugsweise verwendbar sind Kohlenwasserstoffe, wie Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid oder N-Methyl-pyrrolidon.

Als Säureakzeptoren können bei der Durchführung der erfindungsgemäßen Verfahrensvariante E) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante E) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (E) wird zweckmäßigerweise unter Normaldruck durchgeführt.

Bei der Durchführung des erfindungsgemäßen Verfahrens (E) setzt man die Reaktionskomponenten der Formeln (VIII) und (V), in welchen A, D, W, X, Y und Z die oben angegebenen Bedeutungen haben und Hal für Halogen steht, und gegebenenfalls die Säureakzeptoren im allgemeinen in etwa äquimolaren Mengen ein. Es ist jedoch auch möglich, die eine oder andere Komponente in einem größeren Überschuß (bis zu 5 Mol) zu verwenden.

Das erfindungsgemäße Verfahren (F) ist dadurch gekennzeichnet, daß man Thioamide der Formel (IX) mit Ketensäurehalogeniden der Formel (V) in Gegenwart eines Verdünnungsmittels und gegebenenfalls in Gegenwart eines Säureakzeptors umsetzt.

Als Verdünnungsmittel können bei der erfindungsgemäßen Verfahrensvariante F) alle inerten organischen Solventien eingesetzt werden. Vorzugsweise verwendbar sind Kohlenwasserstoffe, wie Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung des erfindungsgemäßen Verfahrens (F) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung des erfindungsgemäßen Verfahrens (F) innerhalb eines größeren Bereiches variiert werden. Zweckmäßigerweise arbeitet man bei Temperaturen zwischen 0°C und 250°C, vorzugsweise zwischen 20°C und 220°C.

Das erfindungsgemäße Verfahren (F) wird zweckmäßigerweise unter Normaldruck durchgeführt.

Bei der Durchführung des erfindungsgemäßen Verfahrens (F) setzt man die Reaktionskomponenten der Formeln (IX) und (V), in welchen A, W, X, Y und Z die oben angegebenen Bedeutungen haben und Hal für Halogen steht und gegebenenfalls die Säureakzeptoren im allgemeinen in etwa äquimolaren Mengen ein. Es ist jedoch auch möglich, die eine oder andere Komponente in einem größeren Überschuß (bis zu 5 Mol) zu verwenden.

Das Verfahren (G) ist dadurch gekennzeichnet, daß man Verbindungen der Formel (X), in welcher A, B, Q¹, Q², W, X, Y, Z und R⁸ die oben angegebene Bedeutung haben, in Gegenwart einer Base einer intramolekularen Kondensation unterwirft.

Als Verdünnungsmittel können bei dem erfindungsgemäßen Verfahren (G) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (G) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (G) innerhalb eines größeren Bereiches variiert werden. Im allgemeinen arbeitet man bei Temperaturen zwischen -75°C und 250°C, vorzugsweise zwischen -50°C und 150°C.

Das erfindungsgemäße Verfahren (G) wird im allgemeinen unter Normaldruck durchgeführt.

Bei der Durchführung des erfindungsgemäßen Verfahrens (G) setzt man die Reaktionskomponenten der Formel (X) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (H) ist dadurch gekennzeichnet, daß man Verbindungen der Formel (XI), in welcher A, B, Q³, Q⁴, Q⁵, Q⁶, W, X, Y, Z und R⁸ die oben angegebene Bedeutung haben, in Gegenwart von Basen einer intramolekularen Kondensation unterwirft.

Als Verdünnungsmittel können bei dem erfindungsgemäßen Verfahren (H) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (H) alle üblichen Protonenakzeptoren eingesetzt werden.

Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (H) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (H) wird im allgemeinen unter Normaldruck durchgeführt.

Bei der Durchführung des erfindungsgemäßen Verfahrens (H) setzt man die Reaktionskomponenten der Formel (XII) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Zur Durchführung des erfindungsgemäßen Verfahrens (I) sind Palladium(O)-Komplexe als Katalysator geeignet. Bevorzugt wird beispielsweise Tetrakis-(triphenylphosphin)palladium. Gegebenenfalls können auch Palladium(II)-Verbindungen eingesetzt werden, beispielsweise PdCl₂.

Als Säureakzeptoren zur Durchführung des erfindungsgemäßen Verfahrens (I) kommer anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium- oder Ammoniumcarbonat, Natriumhydrogen- oder Kaliumhydrogencarbonat, Alkalifluoride, wie beispielsweise Cäsiumfluorid, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (I) kommen Wasser, organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Dicalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Alkohole, wie Methanol, Ethanol, n- oder i-Propanol, n-, iso-, sek.- oder tert.-Butanol, Ethandiol, Propan-1,2-diol, Ethoxyethanol, Methoxyethanol, Diethylenglykolmonomethylether, Diethylenglykolmonomethylether; Wasser.

Die Reaktionstemperatur kann bei dem erfindungsgemäßen Verfahren (I) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (I) werden Boronsäure der Formel (XII), in welcher Z die oben angegebene Bedeutung hat und Verbindungen der Formeln (I-1-a) bis (I-8-a), in welchen A, B, D, Q¹, Q², Q³, Q⁴, Q⁵, Q⁶, W, X, Y und Z die oben angegebene Bedeutung haben, im molaren Verhältnis 1:1 bis 3:1, vorzugsweise 1:1 bis 2:1 eingesetzt. Vom Katalysator setzt man im allgemeinen 0,005 bis 0,5 Mol, vorzugsweise 0,01 Mol bis 0,1 Mol pro Mol der Verbindungen der Formeln (I-1-a) bis (I-8-a) ein. Die Base setzt man im allgemeinen in einem Überschuß ein.

Das Verfahren (J-α) ist dadurch gekennzeichnet, daß man Verbindungen der Formeln (I-1-a) bis (I-8-a) jeweils mit Carbonsäurehalogeniden der Formel (XIII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (J-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (J-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (J-α) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (J-α) werden die Ausgangsstoffe der Formeln (I-1-a) bis (I-8-a) und das Carbonsäurehalogenid der Formel (XIII) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (J-β) ist dadurch gekennzeichnet, daß man Verbindungen der Formeln (I-1-a) bis (I-8-a) mit Carbonsäureanhydriden der Formel (XIV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (J-β) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (J-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (J-β) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (J-β) werden die Ausgangsstoffe der Formeln (I-1-a) bis (I-8-a) und das Carbonsäureanhydrid der Formel (XIV) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (K) ist dadurch gekennzeichnet, daß man Verbindungen der Formeln (I-1-a) bis (I-8-a) jeweils mit Chlorameisensäureestem oder Chlorameisensäurethiolestern der Formel (XV) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (K) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (K) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (K) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (K) wird im allgemeinen unter Normaldruck durchgeführt.

Bei der Durchführung des erfindungsgemäßen Verfahrens (K) werden die Ausgangsstoffe der Formeln (I-1-a) bis (I-8-a) und der entsprechende Chlorameisensäureester bzw. Chlorameisensäurethiolester der Formel (XV) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (L) ist dadurch gekennzeichnet, daß man Verbindungen der Formeln (I-1-a) bis (I-8-a) jeweils mit Verbindungen der Formel (XVI) in Gegenwart eines Verdünnungsmittels und gegebenenfalls in Gegenwart eines Säurebindemittels umsetzt.

Beim Herstellungsverfahren (L) setzt man pro Mol Ausgangsverbindung der Formeln (I-1-a) bis (I-8-a) ca. 1 Mol Chlormonothioameisensäureester bzw. Chlordithioameisensäureester der Formel (XVI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-8-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (M) ist dadurch gekennzeichnet, daß man Verbindungen der Formeln (I-1-a) bis (I-8-a) jeweils mit Sulfonsäurechloriden der Formel (XVII) gegebenenfalls in Gegenwart eines Verdünnungsmittels und gegebenenfalls in Gegenwart eines Säurebindemittels umsetzt.

Beim Herstellungsverfahren (M) setzt man pro Mol Ausgangsverbindung der Formel (I-1-a bis I-8-a) ca. 1 Mol Sulfonsäurechlorid der Formel (XVII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-8-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (N) ist dadurch gekennzeichnet, daß man Verbindungen der Formeln (I-1-a) bis (I-8-a) jeweils mit Phosphorverbindungen der Formel (XVIII) gegebenenfalls in Gegenwart eines Verdünnungsmittels und gegebenenfalls in Gegenwart eines Säurebindemittels umsetzt.

Beim Herstellungsverfahren (N) setzt man zum Erhalt von Verbindungen der Formeln (I-1-e) bis (I-8-e) auf 1 Mol der Verbindungen (I-1-a) bis (I-8-a), 1 bis 2, vorzugsweise 1 bis 1,3 Mol der Phosphorverbindung der Formel (XVIII) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (O) ist dadurch gekennzeichnet, daß man Verbindungen der Formeln (I-1-a) bis (I-8-a) mit Metallhydroxiden bzw. Metallalkoxiden der Formel (XIX) oder Aminen der Formel (XX), gegebenenfalls in Gegenwart eines Verdünnungsmittels, umsetzt.

Als Verdünnungsmittel können bei dem erfindungsgemäßen Verfahren (O) vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (O) wird im allgemeinen unter Normaldruck durchgerührt.

Die Reaktionstemperaturen liegen im allgemeinen zwischen -20°C und 100°C, vorzugsweise zwischen 0°C und 50°C.

Das erfindungsgemäße Verfahren (P) ist dadurch gekennzeichnet, daß man Verbindungen der Formeln (I-1-a) bis (I-8-a) jeweils mit (P-α) Verbindungen der Formel (XXI) gegebenenfalls in Gegenwart eines Verdünnungsmittels und gegebenenfalls in Gegenwart eines Katalysators oder (P-β) mit Verbindungen der Formel (XXII) gegebenenfalls in Gegenwart eines Verdünnungsmittels und gegebenenfalls in Gegenwart eines Säurebindemittels umsetzt.

Bei Herstellungsverfahren (P-α) setzt man pro Mol Ausgangsverbindung der Formeln (I-1-a) bis (I-8-a) ca. 1 Mol Isocyanat der Formel (XXI) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (P-β) setzt man pro Mol Ausgangsverbindung der Formeln (I-1-a) bis (I-8-a) ca. 1 Mol Carbamidsäurechlorid der Formel (XXII) bei -20 bis 150°C, vorzugsweise bei 0 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-8-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus
   Aus der Ordnung der Chilopoda z.B. Geophilus carpophagus, Scutigera spec.
   Aus der Ordnung der Symphyla z.B. Scutigerella immaculata.
   Aus der Ordnung der Thysanura z.B. Lepisma saccharina.
   Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
   Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp..
   Aus der Ordnung der Mallophaga z.B. Trichodectes spp., Damalinea spp.
   Aus der Ordnung der Thysanoptera z.B. Frankliniella occidentalis, Hercinothrips femoralis, Thrips palmi, Thrips tabaci.
   Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Liriomyza spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
   Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit nach Blatt- und Bodenanwendung aus.

Sie lassen sich mit besonders gutem Erfolg zur Bekämpfung von pflanzenschädigenden Insekten einzetzen, wie beispielsweise gegen die Larven des Meerrettichblattkäfers (Phaedon cochleariae), gegen die Larven der grünen Reiszikade (Nephotettix cincticeps) und gegen die Larven der grünen Pfirsichblattlaus (Myzus persicae).

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:
Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindemia, Lamium, Veronica,
Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-FettsäureEster, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 % und daneben bevorzugt Streckmittel und/oder oberflächenaktive Mittel.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2- {2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung, Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol, Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen, Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuronmethyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändem, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirexjuvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie
   Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen Bauholz, Holzbalken, Eisenbahnschwellen, Brückenteile, Bootsstege, Holzahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise - Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octyl-isothiazolin-3-on genannt.

Die Herstellung und die Verwendung der erfindungsgemäßen Wirkstoffe geht aus den nachfolgenden Beispielen hervor.

### Beispiel I-1a-1

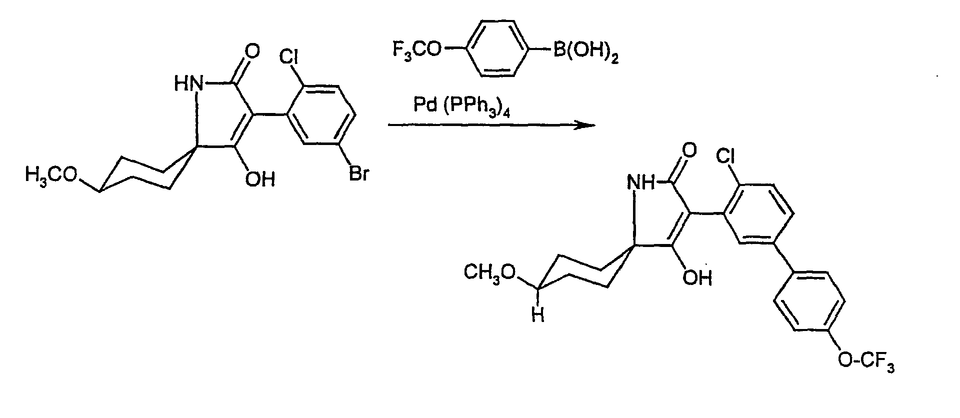

1,93 g der oben gezeigten Bromverbindung in 20 ml 1,2-Dimethoxyethan werden bei 20°C mit 1,6 g 4-Trifluormethoxyphenylboronsäure und 0,29 g Tetrakis(trisphenylphosphin)palladium versetzt. Man rührt 15 Minuten bei 20°C, gibt 15 ml 20 %ige Natriumcarbonatlösung zu und rührt einen Tag bei 80°C. Die Lösung wird filtriert und mit Essigsäureethylester extrahiert. Die wäßrige Phase wird bei 0 bis 10°C mit konzentrierter Salzsäure angesäuert, bis zur Trockne im Vakuum eingeengt und der Rückstand mit Methylenchlorid/Essigester 3:1 als Laufmittel an Kieselgel chromatographiert.
Ausbeute 0,40 g (17 % der Theorie); Fp.: 143°C.

### Beispiel I-1a-14

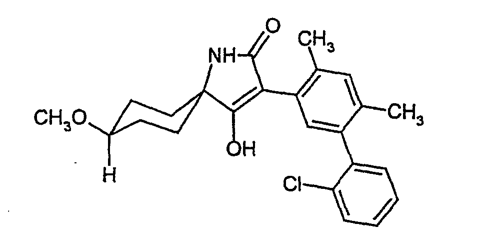

Zu 2,6 g Kalium-tert.-butylat in 7 ml absolutem Dimethylformamid (DMF) tropft man bei 80°C 4,42 g der Verbindung gemäß Beispiel (II-10) in 9 ml absolutem DMF und rührt bei dieser Temperatur, bis die Reaktion beendet ist (dünnschichtchromatographische (DC) Kontrolle). Nach dem Abkühlen gibt man 90 ml Eiswasser zu und säuert bei 0°C bis 10°C mit konz. Salzsäure auf pH 2 an. Man saugt ab, wäscht mit Eiswasser, trocknet und kocht mit Methyl-tert.-butylether (MTBE)/n-Hexan aus.
Ausbeute 2,81 g (68 % der Theorie), Fp.: 204°C.

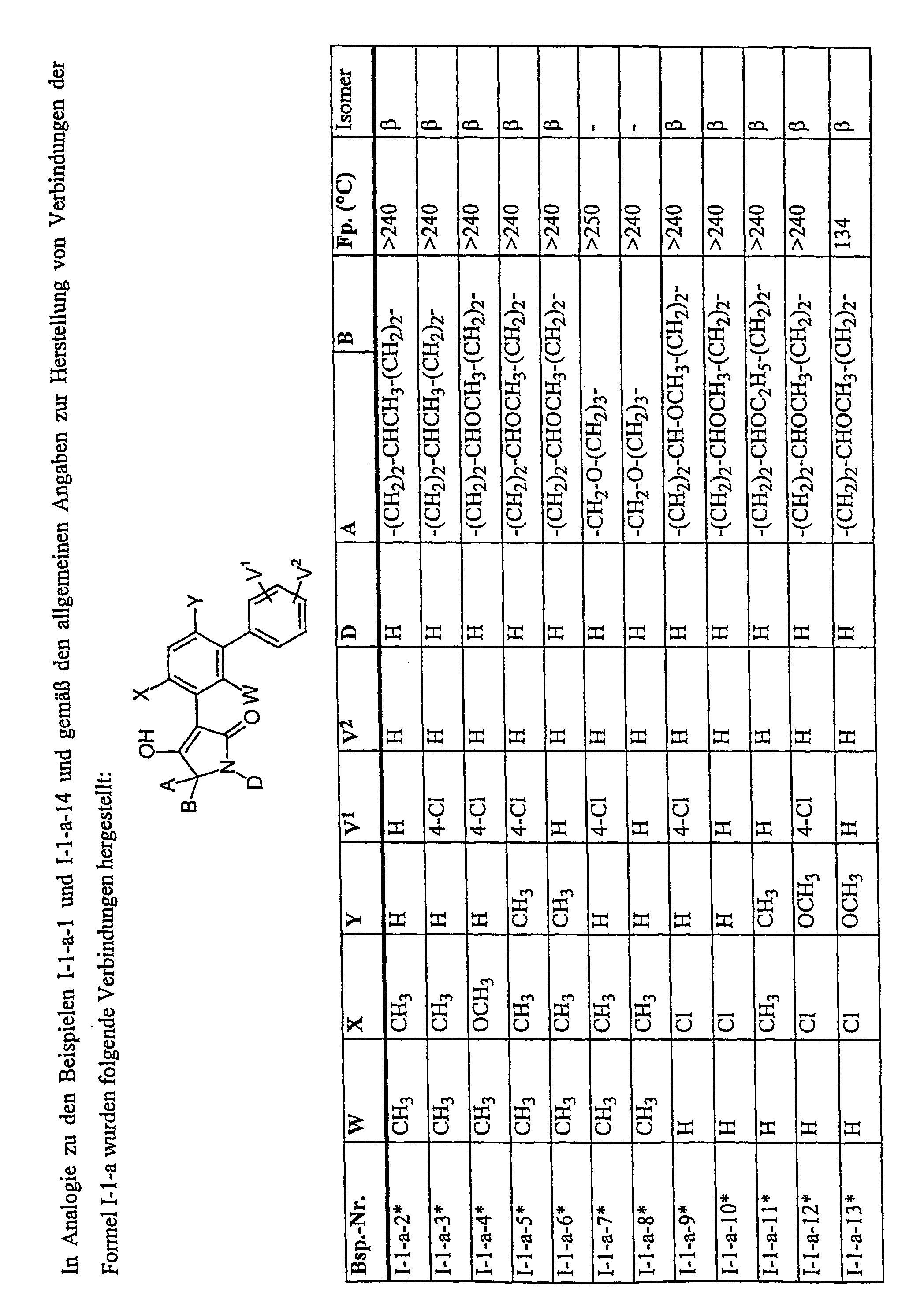

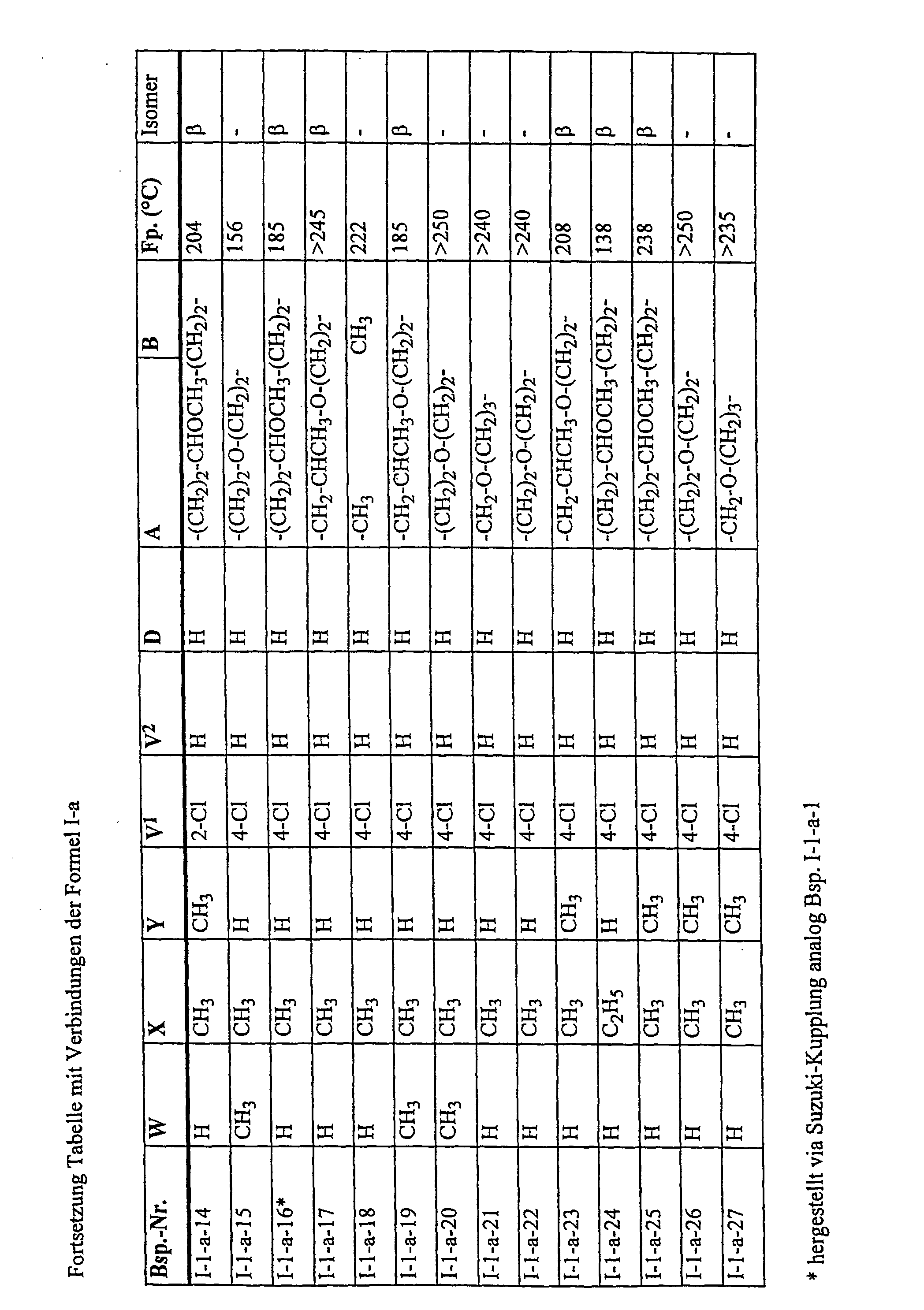

### Beispiel I-1b-1

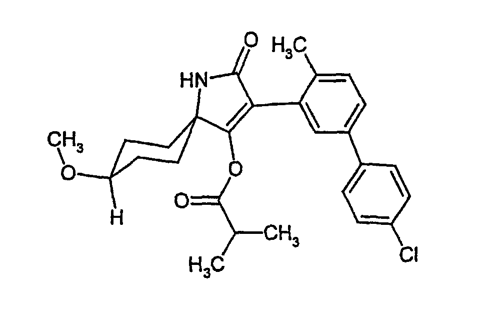

Zu 1 g der Verbindung gemäß Beispiel (I-1-a-16) und 0,5 ml Triethylamin in 30 ml Essigsäureethylester (EE) gibt man unter Rückfluß 0,4 ml Isobuttersäurechlorid und rührt, bis die Reaktion beendet ist (DC-Kontrolle). Es wird eingeengt und chromatographisch an Kieselgel gereinigt.
Laufmittel Methylenchlorid/EE 10/1
Ausbeute 0,15 g (11 % der Theorie), Fp.: 201°C.

Analog zu Beispiel (I-1-b-1) und gemäß den allgemeinen Angaben zur Herstellung von Verbindungen der Formel (I-1-b) wurden folgende Verbindungen hergestellt:

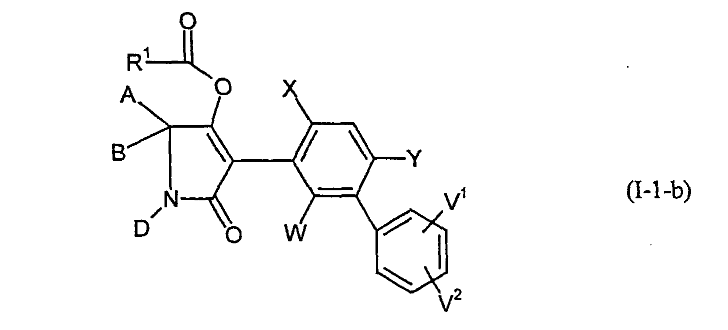

| Bsp.-Nr. | W | X | Y | V¹ | V² | D | A | B | R¹ | Fp.°C | Isomer |
|---|---|---|---|---|---|---|---|---|---|---|---|
| I-1b-2 | H | CH₃ | H | 4-Cl | H | H | CH₂-CHCH₃-O-(CH₂)₂- | | i-C₃H₇ | >230 | β |

### Beispiel I-1c-1

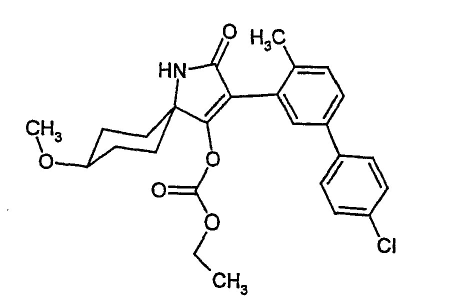

Zu 1 g der Verbindung gemäß Beispiel (I-1-a-16) und 0,5 ml Triethylamin in 30 ml Methylenchlorid gibt man bei 0°C 0,35 g Chlorameisensäureethylester und rührt einen Tag bei 20°C. Es wird eingeengt und der Rückstand chromatographisch an Kieselgel gereinigt.
Laufmittel Methylenchlorid/EE 10/1
Ausbeute 0,12 g, Fp.: 194°C.

Analog zu Beispiel (I-1-c-1) und gemäß den allgemeinen Angaben zur Herstellung von Verbindungen der Formel (I-1-c) wurden folgende Verbindungen hergestellt:

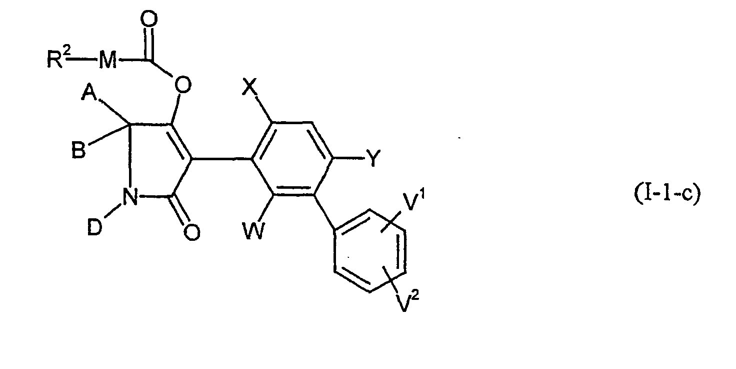

| Bsp.-Nr. | W | X | Y | V¹ | V² | D | A | B | M | R² | Fp.°C | Isomer |
|---|---|---|---|---|---|---|---|---|---|---|---|---|
| I-1c-2 | H | CH₃ | H | 4-Cl | H | H | -CH₂-CHCH₃-O-(CH₂)₂- | | 0 | C₂H₅ | 187 | β |
| I-1c-3 | H | CH₃ | H | 4-Cl | H | H | -CH₂-O-(CH₂)₃- | | O | C₂H₅ | >240 | - |
| I-1c-4 | H | CH₃ | H | 4-Cl | H | H | -(CH₂)₂-CHOCH₃-(CH₂)₂- | | O | i-C₄H₉ | 168 | β |
| I-1c-5 | H | CH₃ | H | 4-Cl | H | H | CH₃ CH₃ | | O | C₂H₅ | 149 | - |

### Beispiel II-1

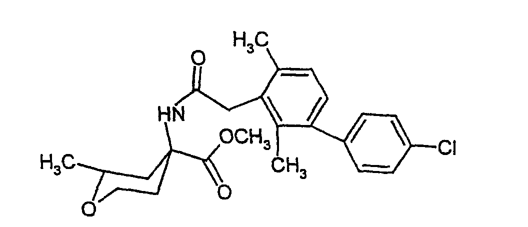

5 g der Verbindung gemäß Beispiel (XXVII-5) und 7,6 ml Thionylchlorid werden auf 80°C erhitzt, bis die Gasentwicklung beeendet ist. Überschüssiges Thionylchlorid wird entfernt, der Rückstand in 20 ml Acetonitril aufgenommen (Lösung 1).

Man legt 4,61 g der Verbindung 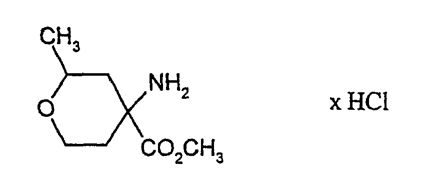 in 20 ml Acetonitril vor, gibt 6,1 g gemahlenes Kaliumcarbonat zu, tropft bei 0-10°C Lösung 1 zu und rührt noch 1 Stunde bei Raumtemperatur. Die Mischung wird auf 250 ml Eiswasser gegossen. Man extrahiert mit Methylenchlorid, wäscht mit 0,5 N HCl und engt ein. Der Rückstand wird an Kieselgel chromatographiert (Laufmittel Methylenchlorid/EE 3/1), Ausbeute 5 g, Fp.: 91°C.

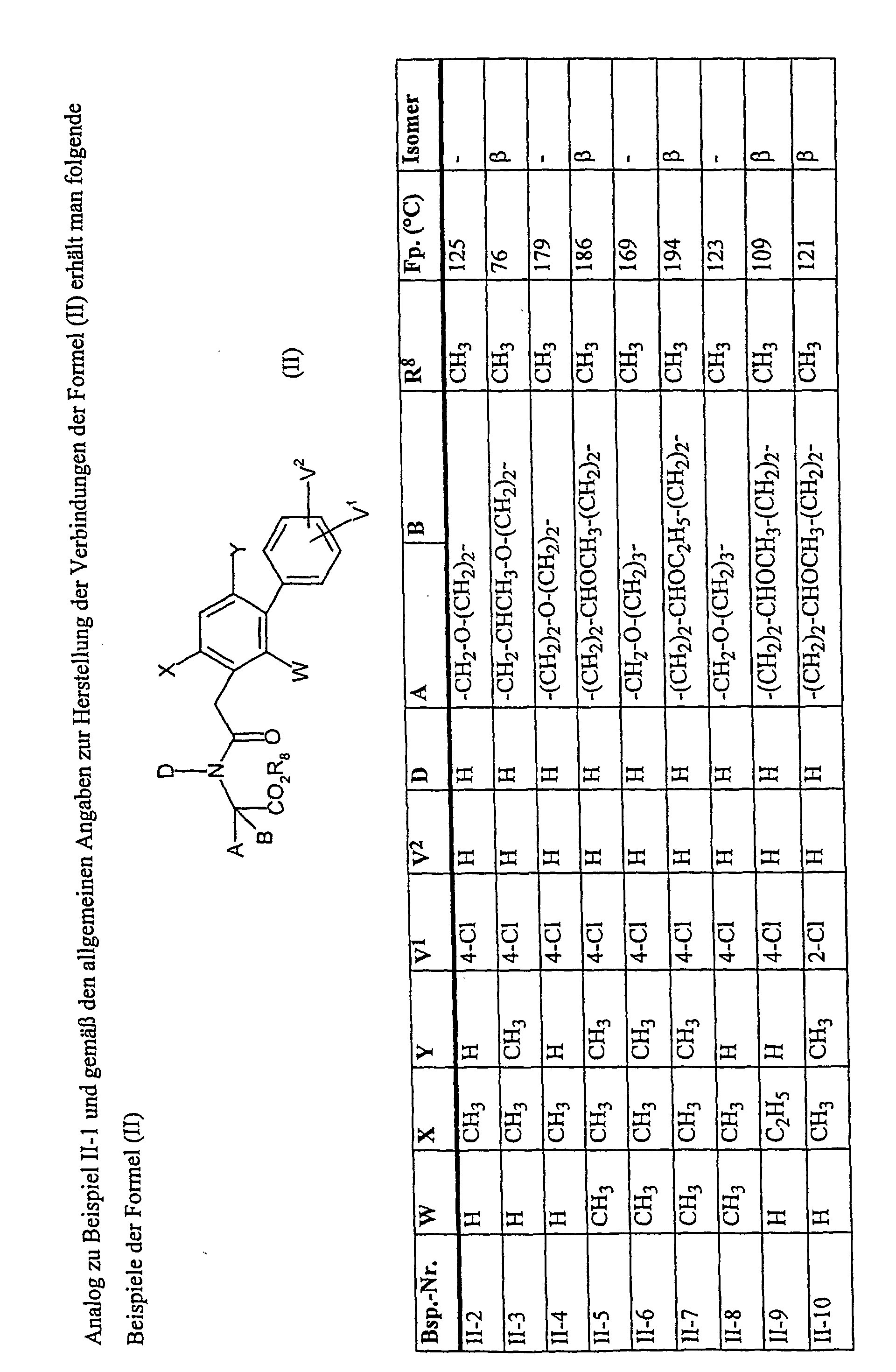

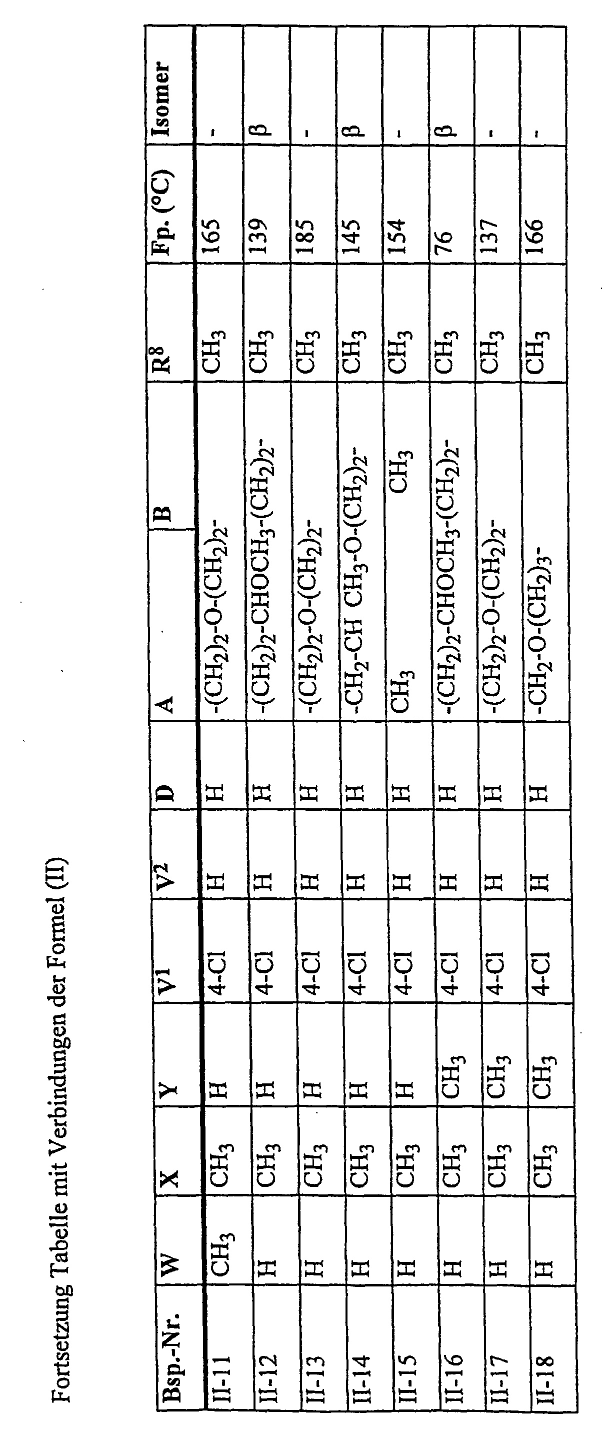

### Beispiel (I-2-a-1)

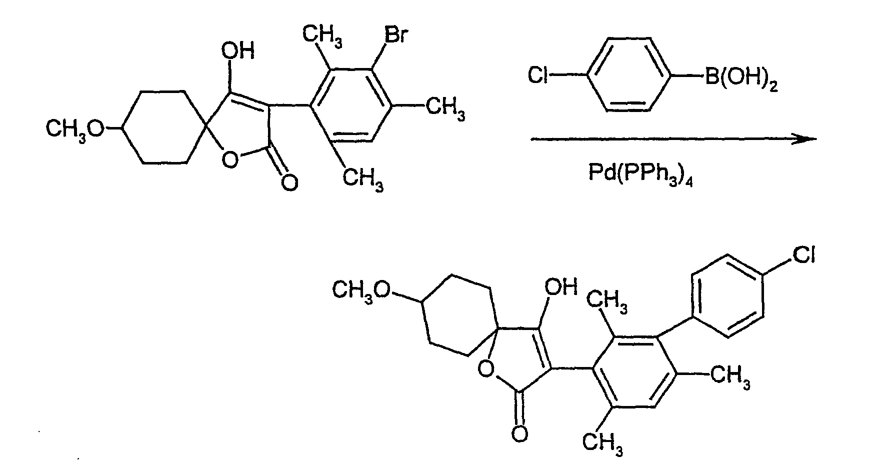

Zu 1,55 g der o.g. Brom-Verbindung erhältlich gemäß den Beispielen aus WO 97/36868, 0,73 g 4-Chlorphenylboronsäure und 0,27 g Tetrakis-triphenylphoshinpalladium in 20 ml Dimethoxyethan gibt man 19,5 ml 20 %ige Natriumcarbonatlösung und rührt 4 Stunden bei 80°C. Man versetzt mit 1N NaOH und extrahiert 2 mal mit Ether. Die alkalische Phase wird filtriert und mit vedünnter Salzsäure angesäuert. Man saugt ab und trocknet. Ausbeute 0,36 g, Fp. 260-263°C.

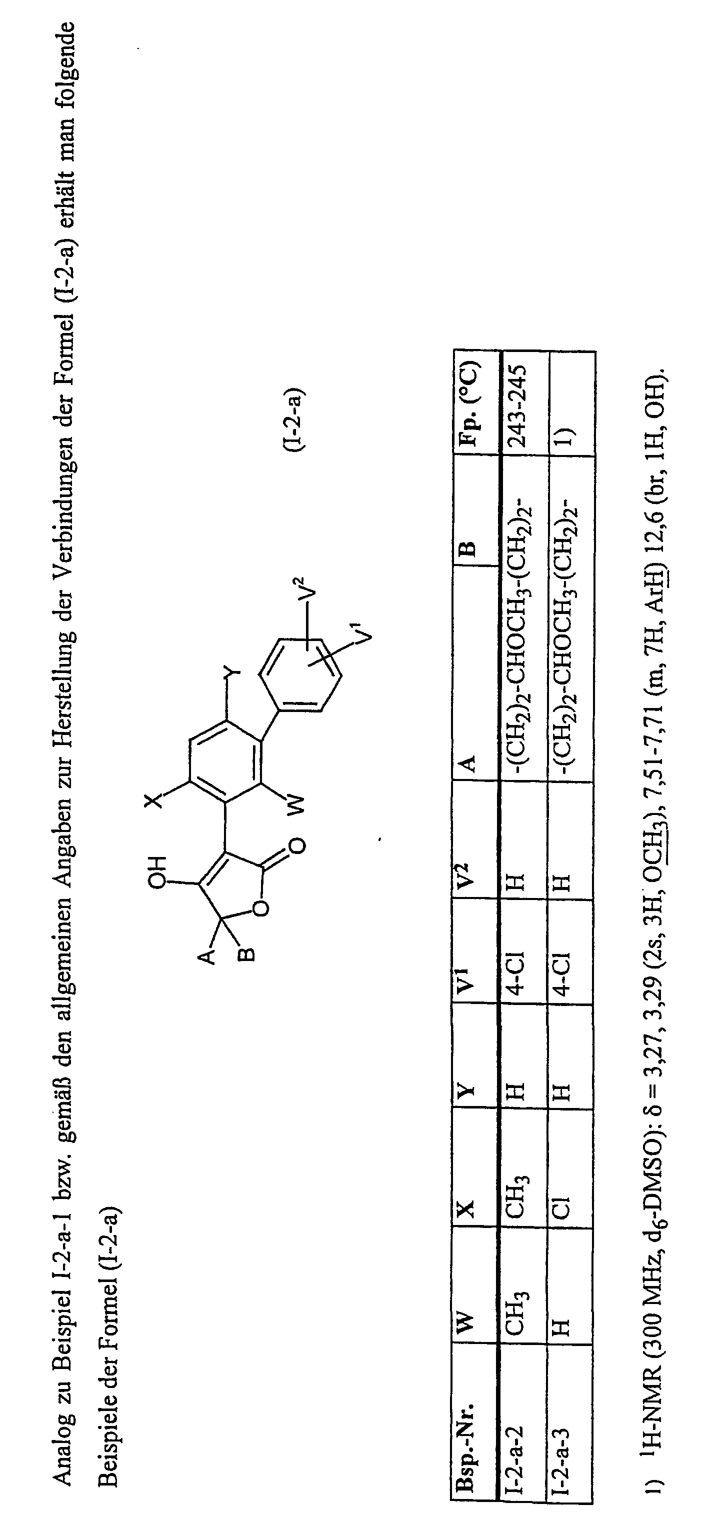

### Beispiel I-2-b-1

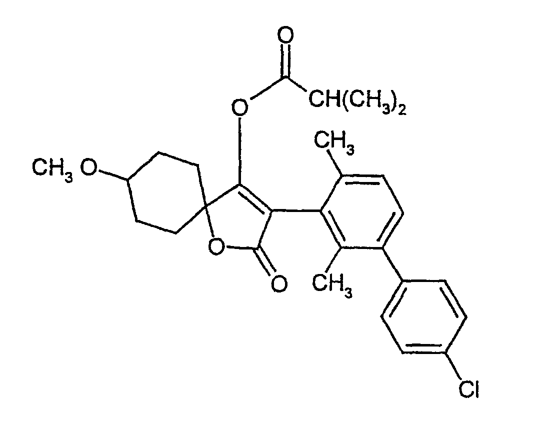

0,125 g (0,3 mmol) der Verbindung I-2-a-2 werden in absolutem Methylenchlorid vorgelegt mit 0,04 g (0,36 mmol) Triethylamin versetzt und bei 0-10°C 0,04 g (0,36 mmol) Isobuttersäurechlorid zugetropft. Die Mischung wird über Nacht gerührt, anschließend einmal mit 10 %iger Zitronensäure und einmal mit 10 %iger Natronlauge gewaschen, die organische Phase getrocknet und eingeengt.
Ausbeute: 0,1 g Öl
¹H-NMR (300 MHz, CDCl₃): δ = 2,06, 2,07 (2s, 3H, Ar CH₃), 2,24, 2,25 (2s, 3H, Ar CH₃), 2,61 (m, 1H, CH(CH₃)₂), 3,35, 3,40 (2s, 3H, OCH₃), 7,09 (s, 2H, ArH), 7,16, 7,19 (AA', BB', 1H, Ar-H), 7,35, 7,37 (AA', BB', 2H, ArH).

### Beispiel I-2-c-1

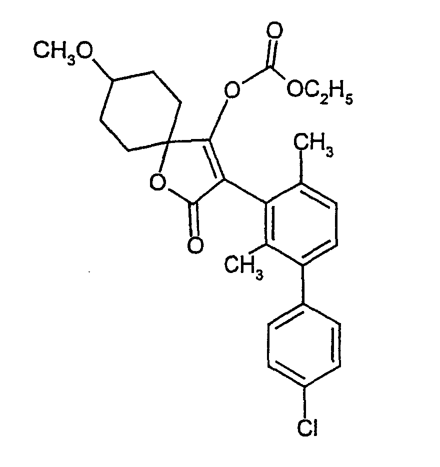

Herstellung analog zu Beispiel I-2-b-1 durch Umsetzung der Verbindung gemäß Beispiel I-2-a-2 mit Chlorameisensäure-ethylester. Öl
¹H-NMR (300 MHz, CDCl₃): δ = 1,10, (t, 3H, O-CH₂CH₃), 2,08, (2s, 3H, Ar CH₃), 2,24, 2,25 (2s, 3H, ArCH₃), 3,36, 3,40 (2s, 3H, OCH₃), 7,11 (2s, 2H, ArH), 7,18, 7,21 (AA', BB', 2H, ArH), 7,35, 7,38 (AA', BB', 2H, ArH).

### Beispiel I-3-a-1

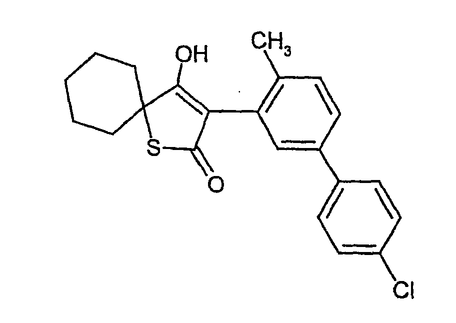

44 g der Verbindung gemäß Beispiel IV-1 und 92 ml Trifluoressigsäure werden in 210 ml Toluol über Nacht unter Rückfluß erhitzt. Es wird eingeengt und der Rückstand in 600 ml Wasser und 200 ml MTBE aufgenommen. Durch Zugabe von NaOH wird auf pH 14 eingestellt und dann 2 mal mit MTBE extrahiert. Die wässrige Phase wird in 1 l 1N HCl getropft. Man rührt 2 Stunden, extrahiert mit Methylenchlorid und engt ein. Zur Reinigung wird der Rückstand mt ca. 200 ml MTBE/Cyclohexan 8/1 verrührt, abgesaugt und getrocknet. Ausbeute 5,9 g, Fp. 232-235°C.

Analog zu Beispiel (I-3a-1) bzw. gemäß den allgemeinen Angaben zur Herstellung der Verbindungen der Formel (I-3-a) erhält man folgende Verbindungen

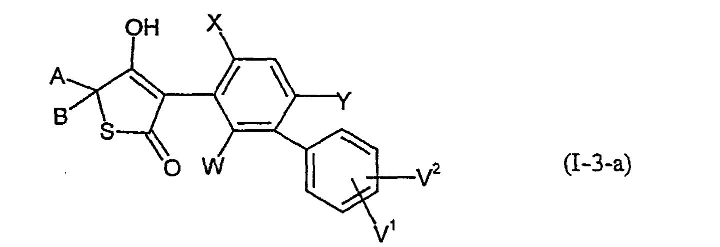

| **Bsp.-Nr.** | **W** | **X** | **Y** | **V**^{**1**} | **V**^{**2**} | **A** | **B** | **Fp. (°C)** |
|---|---|---|---|---|---|---|---|---|
| I-3-a-2 | H | CH₃ | CH₃ | 4-Cl | H | -(CH₂)₅- | | 245-247 |
| I-3-a-3 | CH₃ | CH₃ | CH₃ | 4-Cl | H | -(CH₂)₅- | | 248-250 |

### Beispiel I-3-b-1

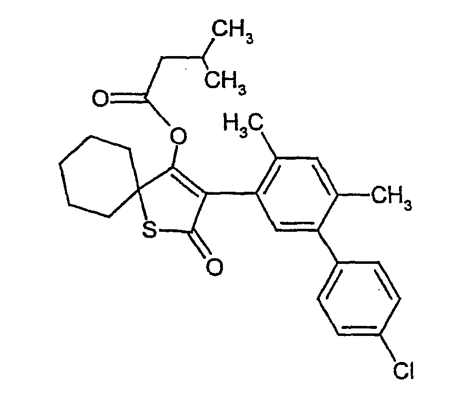

Herstellung analog Beispiel I-2-b-1, durch Umsetzung der Verbindung gemäß Beispiel I-3-a-2 mit Isovaleriansäurechlorid.
¹H-NMR (400 MHz, d₆-DMSO): δ=0,9 (d, 6H, CH-CH₃); 1,2-2,0 (m, 10H, Cyclohexyl-H); 2,1, 2,2 (s, 6H, 2xArCH₃); 6,85-7,5 (m, 6H, ArH) ppm.

### Beispiel I-3-c-1

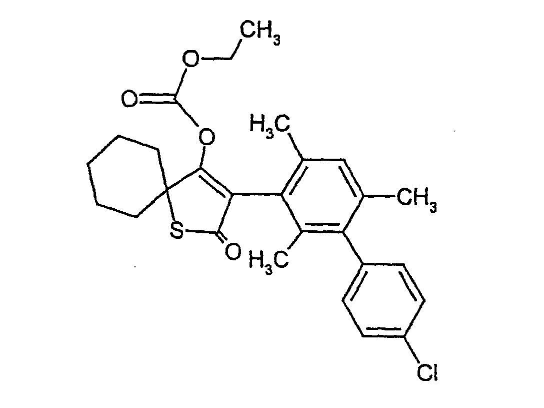

Herstellung analog Beispiel I-2-c-1, durch Umsetzung der Verbindung gemäß Beispiel I-3-a-3 mit Chlorameisensäureethylester. Öl.
¹H-NMR (400 MHz, d₆-DMSO): δ=0,95 (t, 3H, CH₂CH₃); 1,3-1,9 (m, 10H, Cyclohexyl-H); 1,9 (s, 3H, ArCH₃); 2,1 (s, 6H, 2 x Ar-CH₃); 4,0 (g, 2H, OCH₂); 7,0-7,5 (m, 5H, Ar-H) ppm.

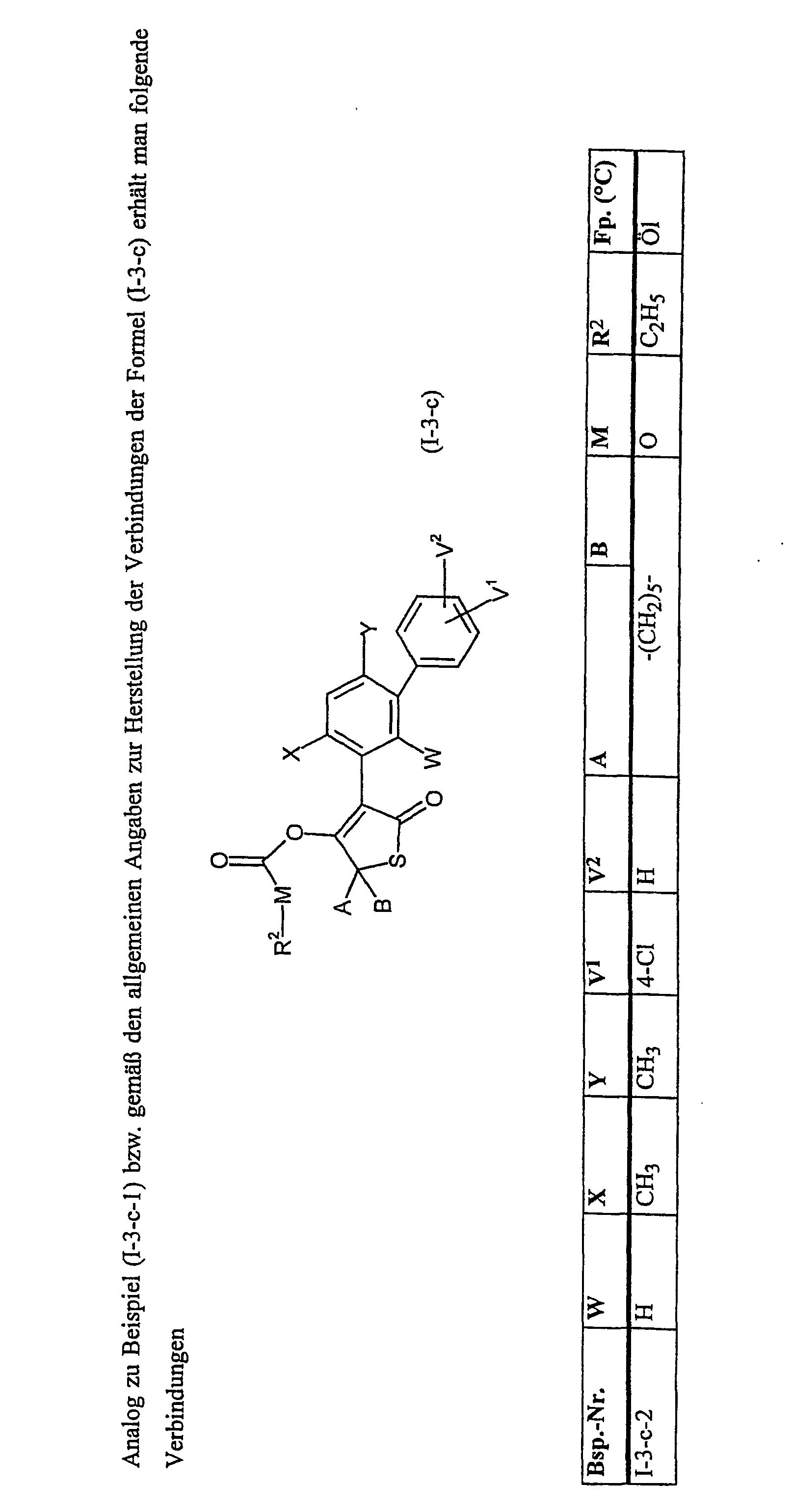

### Beispiel IV-1

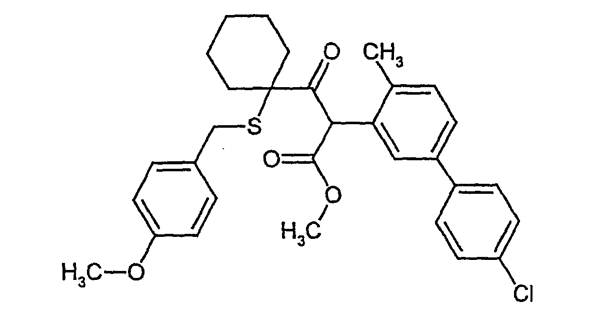

A: 15 g (53,5 mmol) der Verbindung 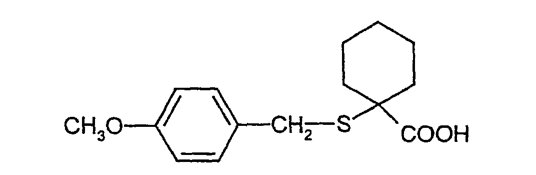 werden auf übliche Weise mit 9,63 g (80,3 mmol) Thionylchlorid in das entsprechende Säurechlorid überführt und in 30 ml Tetrahydrofuran (THF) gelöst.
B: Zu 45,8 ml (96,3 mmol, 1,1 eq) Lithiumdiisopropylamid (LDA)-Lösung in 100 ml THF tropft man bei 0°C 27 g (87,5 mmol) der Verbindung gemäß Beispiel XXXII-2, rührt 30 Minuten bei dieser Temperatur und tropft dann die unter A hergestellte Lösung zu. Ohne Kühlung wird noch 1 Stunde gerührt. Man setzt 300 ml MTBE und einige Tropfen Wasser zu, wäscht 2 mal mit je 300 ml 10 %iger NH₄Cl-Lösung und engt ein. Ausbeute 44 g. Öl.
¹H-NMR (400 MHz, d₆-DMSO): δ=1,2-2,0 (m, 10H, Cyclohexyl-H); 2,25 (s, 3H, Ar-CH₃); 3,1 (dd, 2H, SCH₂); 3,6-3,7 (s, 6H, 2xOCH₃); 6,7-7,7 (m, 12H, Ar-H) ppm.

### Beispiel I-7-a-1

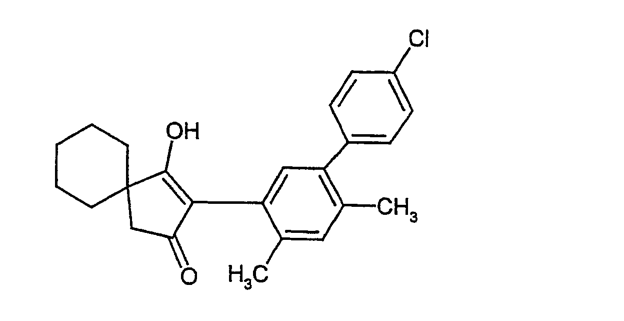

5,3 g (12,8 mmol) der Verbindung gemäß Beispiel X-1 werden in 20 ml DMF vorgelegt mit 2,2 g (19,2 mmol, 1,5 eq) Kalium-ter.-butylat versetzt und 1 Stunde auf 80°C erwärmt (DC-Kontrolle),

Dann läßt man das Gemisch langsam in ca. 0,6 l 1N HCl (unter Eiskühlung) zulaufen, saugt ab und trocknet. Ausbeute 4,85 g, Fp. 224-226°C.

Analog zu Beispiel (I-7-a-1) bzw. gemäß den allgemeinen Angaben zur Herstellung der Verbindungen der Formel (I-7-a) erhält man folgende Verbindungen

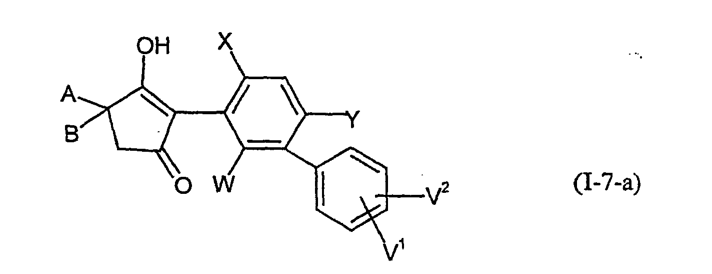

| **Bsp.-Nr.** | **W** | **X** | **Y** | **V**^{**1**} | **V**^{**2**} | **A** | **B** | **Fp. (°C)** |
|---|---|---|---|---|---|---|---|---|
| I-7-a-2 | H | CH₃ | H | 4-Cl | H | -(CH₂)₅- | | 223-224 |
| I-7-a-3 | CH₃ | CH₃ | CH₃ | 4-Cl | H | -(CH₂)₅- | | >250 |

### Beispiel I-7-b-1

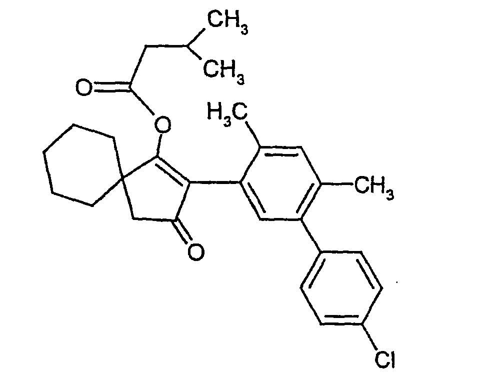

Herstellung analog Beispiel (I-2-b-1) durch Umsetzung der Verbindung gemäß Beispiel (I-7-a-1) mit Isovaleriansäurechlorid. Ausbeute 1,26 g eines Öls.
¹H-NMR (400 MHz, d₆-DMSO): δ=0,95 (d, 6H, 2xCH-CH3); 1,2-1,8 (m, 10H, Cyclohexyl-H); 2,1, 2,2 (s, 6H, 2xAr-CH₃); 6,8-7,5 (m, 6H, Ar-H) ppm.

### Beispiel I-7-c-1

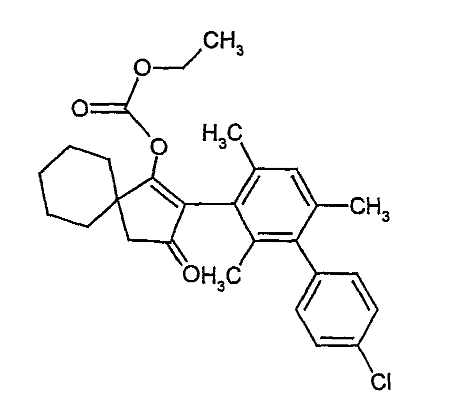

Herstellung analog Beispiel (I-2-c-1) durch Umsetzung der Verbindung gemäß Beispiel (I-7-a-3) mit Chlorameisensäureethylester. Ausbeute 1,3 g. Wachs.
¹H-NMR (400 MHz, d₆-DMSO): 1,2 (t, 3H, CH₂CH₃); 1,3-1,8 (m, 10H, Cyclohexyl); 1,65, 1,9, 2,05 (s, 3 x 3H, Ar-CH₃); 3,05 (bs, 2H, Cyclopentyl CH₂); 4,2 (q, 2H, OCH₂); 7,0 - 7,5 (m, 5H, Ar-H).

Analog zu Beispiel (I-7-c-1) bzw. gemäß den allgemeinen Angaben zur Herstellung der Verbindungen der Formel (I-7-c) erhält man folgende Verbindungen

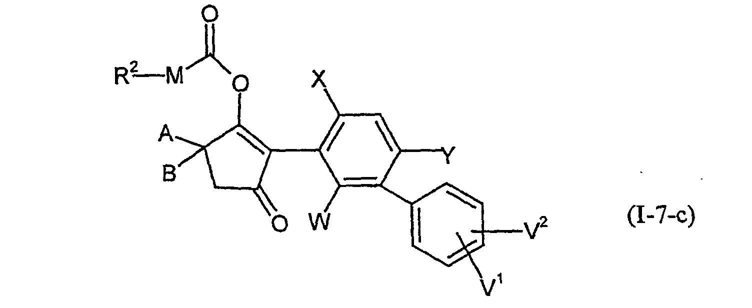

| **Bsp.-Nr.** | **W** | **X** | **Y** | **V**^{**1**} | **V**^{**2**} | **A** | **B** | **M** | **R**^{**2**} | **Fp.(°C)** |
|---|---|---|---|---|---|---|---|---|---|---|
| I-7-c-2 | H | CH₃ | CH₃ | 4-Cl | H | -(CH₂)₂- | | O | C₂H₅ | Öl |

### Beispiel X-1

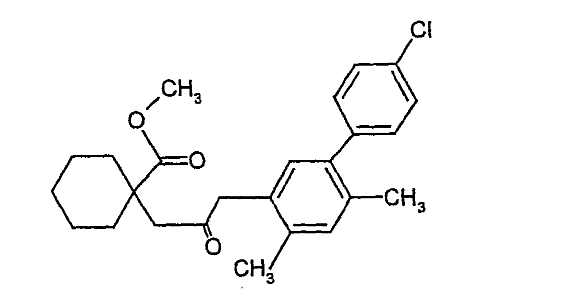

24 g Rohprodukt der Verbindung gemäß Beispiel (XXXVIII-1) werden in 140 ml Aceton mit 8,2 g Kaliumcarbonat und 25,4 g Methyliodid 16 Stunden unter Rückfluß erhitzt. Man filtriert, engt ein und reinigt den Rückstand an Kieselgel: Laufmittel, Methylenchlorid/Petrolether 2/1, schließlich reines Methylenchlorid. Ausbeute 5,6 g.
¹H-NMR (400 MHz, d₆-DMSO): δ=1,2-1,8 (m, 10H, Cyclohexyl H); 2,1, 2,2 (s, 6H, 2xAr-CH₃); 2,9, 3,8 (s, 4H, 2xCOCH₂); 3,5 (s, 3H, OCH₃); 6,95-7,5 (s, 6H, Ar-H) ppm.

### Beispiel XXXVIII-1

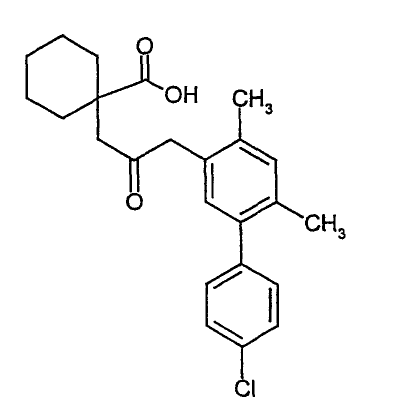

Zu einer Lösung von 30 ml LDA-Lösung (2 m, 1,1 eq) in 60 ml THF wird eine Lösung von 17,9 g der Verbindung gemäß Beispiel XXXII-1 bei -15°C getropft und 1 Stunde bei 0°C gerührt. Dann wird bei -15°C eine Lösung von 6,1 g der Verbindung gemäß Beispiel XLI-1 in 10 ml THF getropft. Man rührt 2 Stunden bei Raumtemperatur, gibt 100 ml Wasser und 24 g Ammoniumchlorid zu und säuert mit konz. HCl an. Das Zwischenprodukt wird mit Ether extrahiert. Man engt ein und erhitzt den Rückstand zwei Tage mit 60 g KOH in 220 ml Wasser. Nach dem Abkühlen wird mit konz. HCl angesäuert und mit Ether extrahiert. Das Rohprodukt wird ohne weitere Reinigung weiter umgesetzt. Ausbeute 24 g.

### Beispiel XLI-1

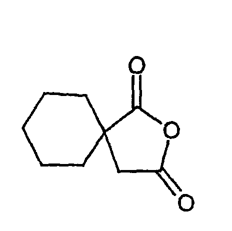

100 g der Verbindung 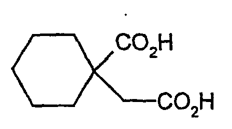 werden über Nacht in 500 ml Essigsäureanhydrid unter Rückfluß erhitzt. Man engt ein, löst in wenig Methylenchlorid und versetzt mit n-Hexan. Man beläßt über Nacht im Kühlschrank, saugt ab und trocknet. Ausbeute 74,8 g.

### Beispiel I-8-a-1

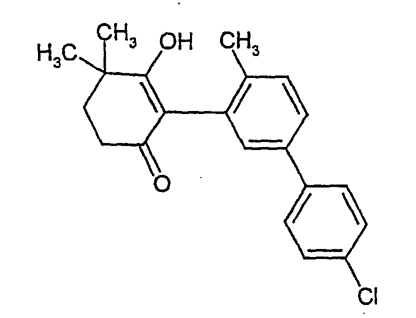

Zu 2,1 g der Verbindung gemäß Beispiel (XI-1) in 10 ml DMF gibt man 0,95 g Kalium-tert.-butylat und erhitzt 2 Stunden auf 80°C. Unter Eiskühlung läßt man das Gemisch langsam in ca. 1l 1N HCl laufen und saugt den Niederschlag ab.
Ausbeute 0,2 g.
¹H-NMR, (400 MHz, d₆-DMSO): δ=1,1 (s, 6H, C-CH₃); 1,85 (m, 2H, COCH₂CH₂); 2,0 (s, 3H, ArCH₃); 2,65 (m, 2H, COCH₂CH₂); 7,1-7,6 (m, 7H, Ar-H) ppm.

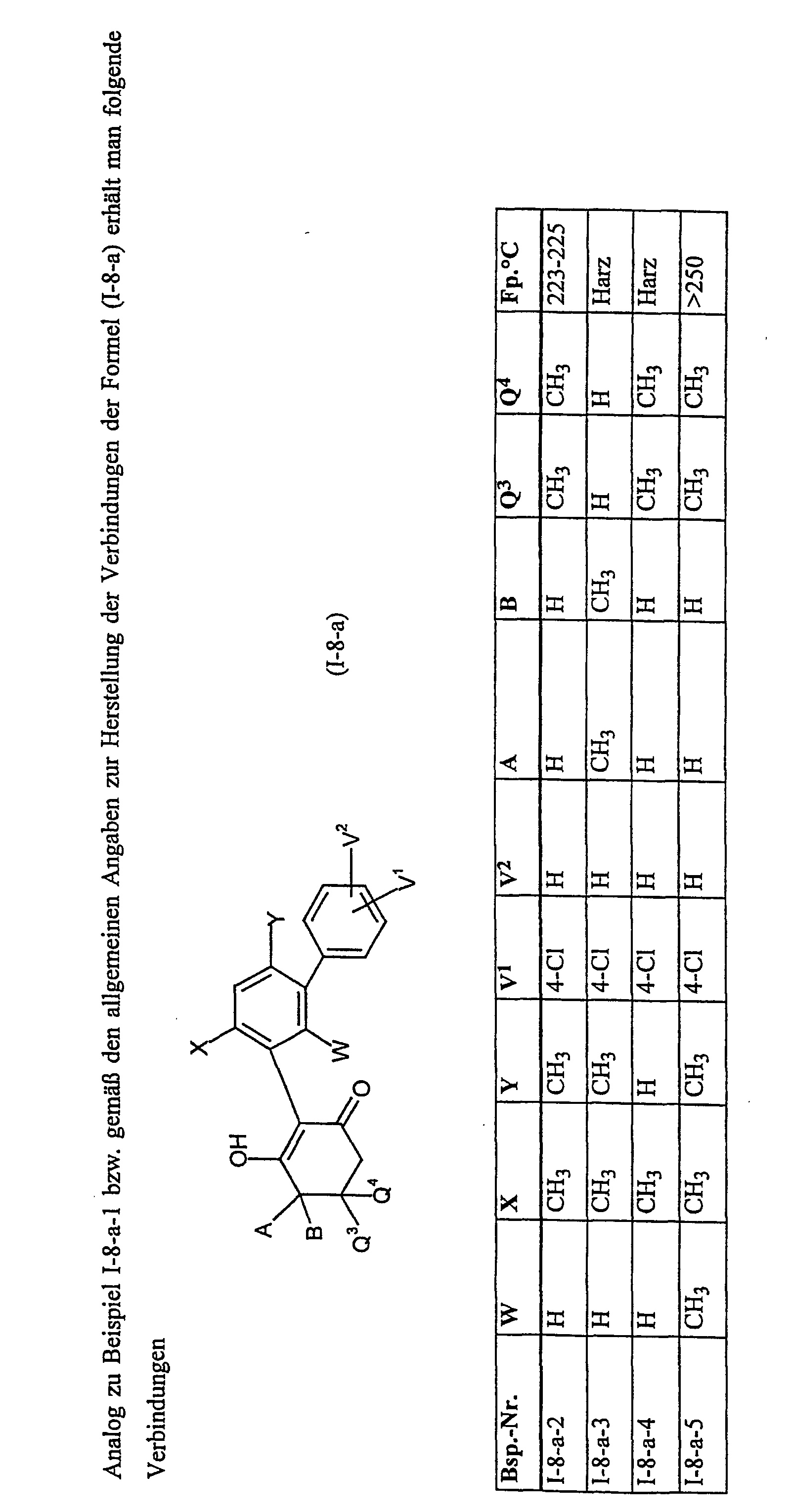

### Beispiel I-8-b-1

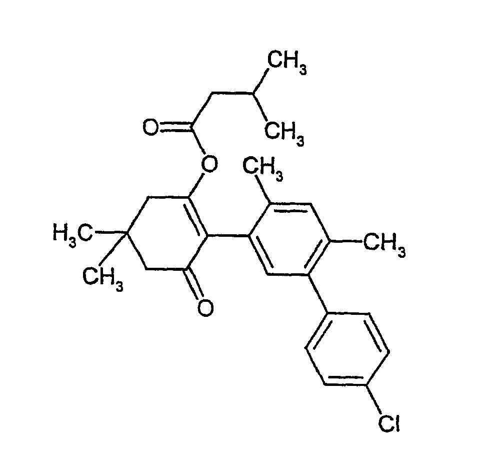

Herstellung analog zu Beispiel (I-2-b-1), durch Umsetzung der Verbindung gemäß Beispiel I-8-a-2 mit Isovaleriansäurechlorid. Öl.
¹H-NMR (400 MHz, d₆-DMSO): 1,1 (δ, 6H, 2xCHCH₃); 1,1 (s, 6H, 2xCCH₃); 1,65 (m, 1H, CHCH₃); 2,0, 2,2 (s, 6H, ArCH3); 6,7-7,5 (m, 6H, Ar-H) ppm.

### Beispiel I-8-c-1

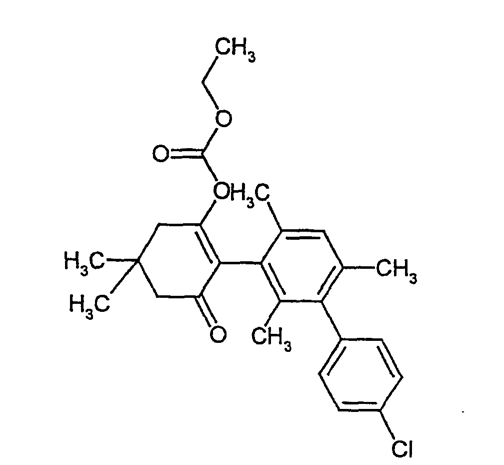

Herstellung analog zu Beispiel (I-2-c-1), durch Umsetzung der Verbindung gemäß (I-8-a-5) mit Chlorameisensäureethylester.
1H-NMR (400 MHz, d₆-DMSO): δ=1,1 (t,3H, CH₂CH₃); 1,14 1,18 (s,2x3H, C-CH₃); 1,9 2,0 2,1 (s, 3x3H, ArCH₃); 2,45 2,7 (d,2x2H, Cyclohexyl-CH₂); 4,1 (q,2H, OCH₂); 7,0 - 7,5 (m,5H, Ar-H) ppm.

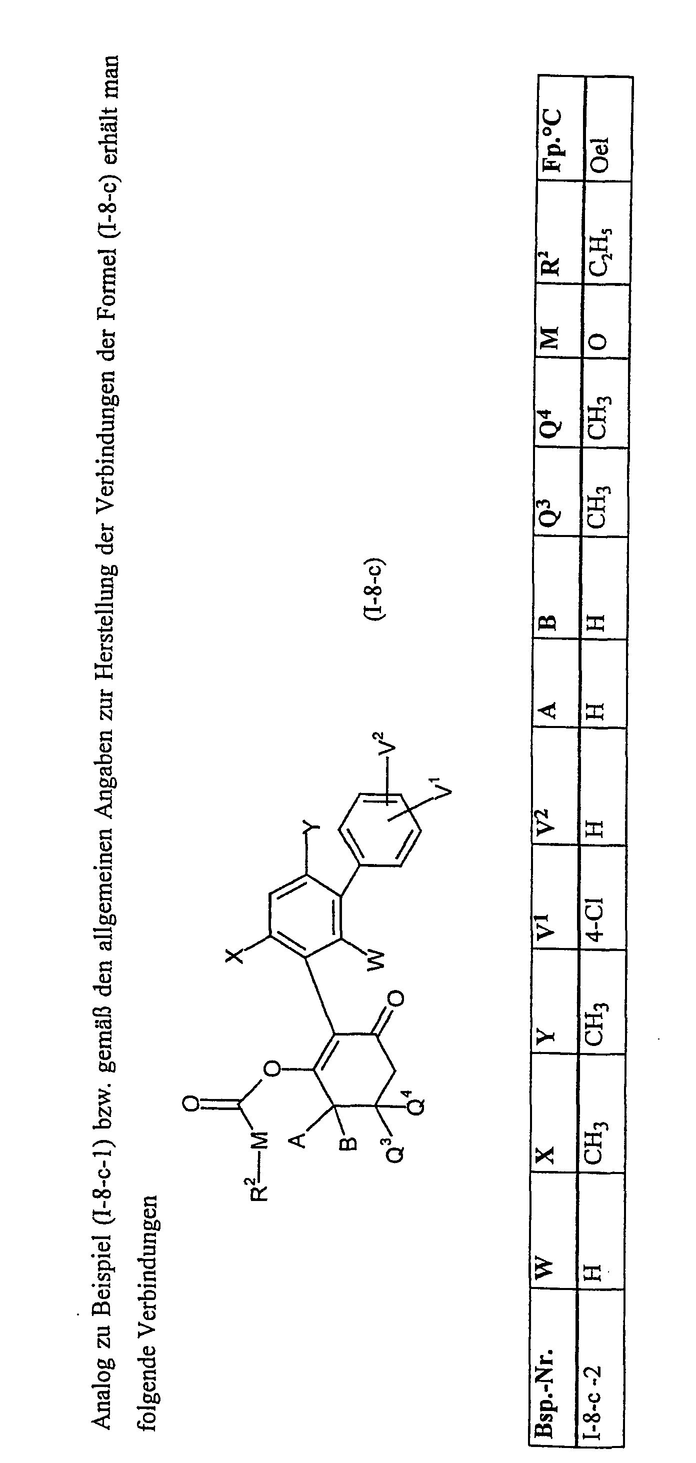

### Beispiel XI-1

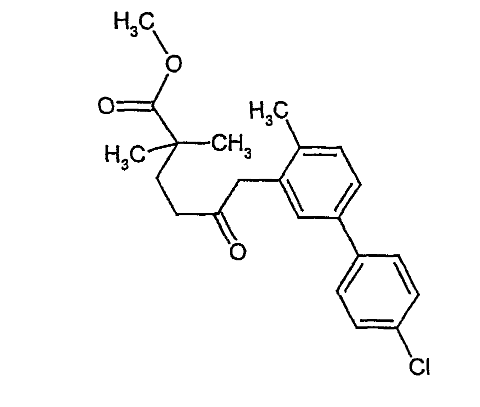

38,2 g der Verbindung gemäß Beispiel XLII-1, 14,6 g Kaliumcarbonat und 45,25 g Methyliodid werden in 250 ml Aceton 16 Stunden unter Rückfluß erhitzt. Man filtriert, engt ein und reinigt den Rückstnad an Kieselgel (Laufmittel Methylenchlorid/Petrolether 2/1, schließlich reines Methylenchlorid. Ausbeute 2,1g Öl.
¹H-NMR (400 MHz, d₆-DMSO): 1,1 (s, 6H, 2xCCH₃); 1,7 (m, 2H, COCH₂CH₂); 2,15 (s, 3H, ArCH₃); 2,55 (m, 2H, COCH₂CH₂); 3,55 (s, 3H, OCH₃); 7,2-7,7 (m, 7H, Ar-H) ppm.

### Beispiel XLII-1

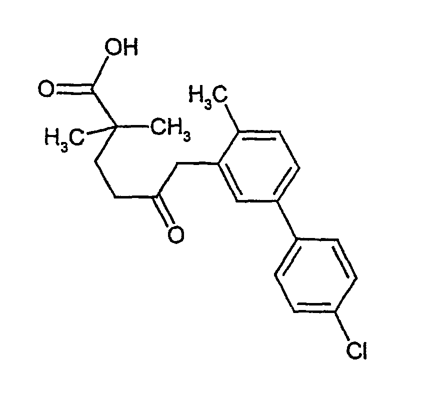

Zu einer Lösung von 60 ml LDA-Lösung (2 molar, 1,1 eq) in 120 ml THF tropft man bei -15°C eine Lösung von 34 g der Verbindung gemäß Beispiel XXXII-2 in 30 ml THF und rührt noch 1 Stunde bei 0°C. Dann tropft man bei -15°C eine Lösung von 10,3 g 2,2-Dimethylglutarsäureanhydrid in 20 ml THF. Man rührt 2 Stunden bei Raumtemperatur, gibt dann 180 ml Wasser und 48 g Ammoniumchlorid zu, säuert mit konzentrierter HCl an und extrahiert das Zwischenprodukt mit Ether. Der Ether wird entfernt und der Rückstand mit 120 ml KOH in 400 ml Wasser zwei Tage unter Rückfluß erhitzt.

Nach dem Abkühlen säuert man mit konzentrierter HCl an und extrahiert mit Ether. Das nach dem Entfernen des Ethers verbleibende Rohprodukt wird ohne Reinigung weiter umgesetzt. Ausbeute 38,4 g Öl.

### Beispiel XXIV-1

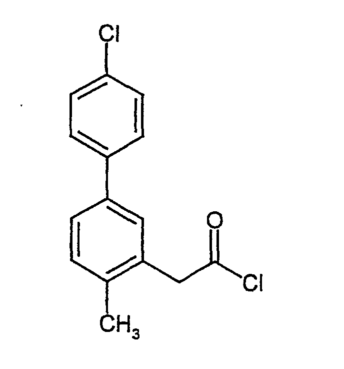

65,2 g der Verbindung gemäß Beispiel XXVII-2 werden in 55 ml Thionylchlorid auf 70°C erhitzt, bis die Gasentwicklung beendet ist. Das überschüssige Thionylchlorid wird entfernt und der Rückstand im Hochvakuum destilliert. Ausbeute: 32 g, Fp. 46°C.

Analog zu Beispiel (XXIV-1) bzw. gemäß den allgemeinen Angaben zur Herstellung von Verbindungen der Formel (XXIV) erhält man folgende Verbindungen der Formel (XXIV)

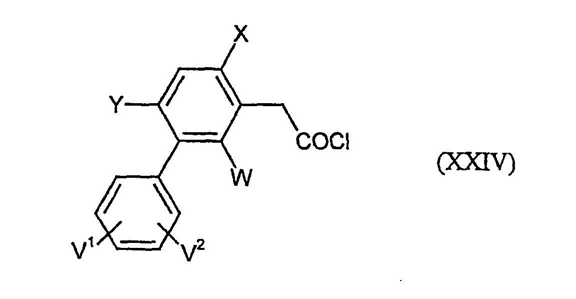

| **Bsp.-Nr.** | **W** | **X** | **Y** | **V**^{**1**} | **V**^{**2**} | **Fp.°C** |
|---|---|---|---|---|---|---|
| XXIV-2 | H | CH₃ | H | 4-Cl | H | * |
| XXIV-3 | CH₃ | CH₃ | CH₃ | 4-Cl | H | * |
| XXIV-4 | H | CH₃ | CH₃ | 2-Cl | H | * |

| | | | | | | |
|---|---|---|---|---|---|---|
| * Die Verbindungen wurden ohne weitere Reinigung zur Herstellung von Verbindungen der Formel (II) bzw. (III) eingesetzt. | | | | | | |

### Beispiel XXVII-1

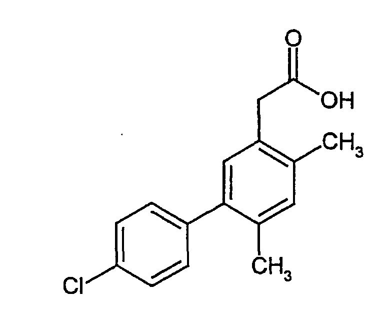

Zu 35 g der Verbindung gemäß Bsp. XXXII-1 in 280 ml THF tropft man 3,04 g LiOH in 280 ml Wasser, gibt 10 ml Ethanol zu und rührt über Nacht bei Raumtemperatur. Dann wird eingeengt, der Rückstand mit Wasser versetzt und mit MTBE extrahiert. Die wässrige Phase wird mit konzentrierter HCl angesäuert. Man saugt ab und wäscht mit Hexan nach. Ausbeute: 21 g, Fp. 133°C.

Analog zu Beispiel (XXVII-1) bzw. gemäß den allgemeinen Angaben zur Herstellung von Verbindungen der Formel (XXVII) erhält man folgende Verbindungen der Formel (XXVII)

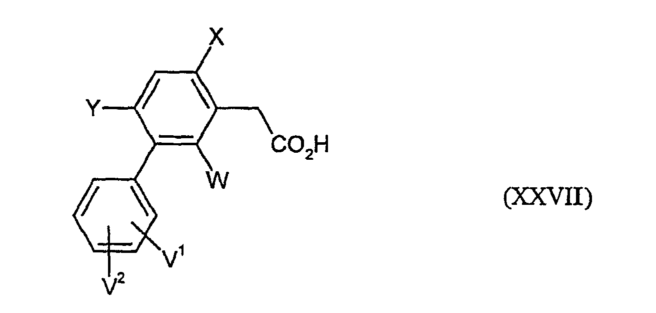

| **Bsp.-Nr.** | **W** | **X** | **Y** | **V**^{**1**} | **V**^{**2**} | **Fp.°C** |
|---|---|---|---|---|---|---|
| XXVII-2 | H | CH₃ | H | 4-Cl | H | 137 |
| XXVII-3 | CH₃ | CH₃ | CH₃ | 4-Cl | H | 138 |
| XXVII-4 | H | CH₃ | CH₃ | 2-Cl | H | 134 |
| XXVII-5 | CH₃ | CH₃ | H | 4-Cl | H | 153 |

### Beispiel XXXII-1

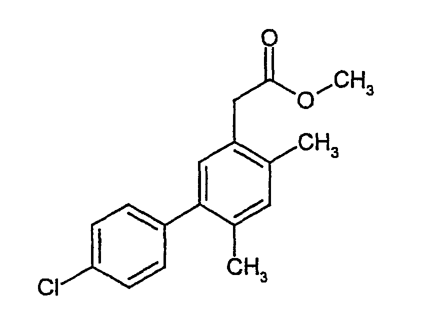

### Variante A

Zu 31 g KOH in 1l Methanol gibt man 54 g der Verbindung gemäß Beispiel XLV-1 und rührt über Nacht bei Raumtemperatur. Man saugt ab und wäscht mit Methanol nach. Das Filtrat wird eingeengt, der Rückstand mit Wasser versetzt und mit Methylenchlorid extrahiert. Die organische Phase wird eingeengt. Ausbeute 10 g.

### Variante B

96 g 3-Brom-4,6-dimethylphenylessigsäure-methylester, 65 g 4-Chlorphenylboronsäure und 1,5 g Bis(triphenylphosphin)palladium(II)chlorid in 1 l Dimethoxyethan und 700 ml 1 M Na₂CO₃-Lösung werden über Nacht unter Rückfluß erhitzt. Man verteilt zwischen Wasser und EE, wäscht die organische Phase mit gesättigter Ammoniumchloridlösung, Wasser und gesättigter Kochsalzlösung und engt ein. Ausbeute 61 g.
¹H-NMR (400 MHz, COCl₃): δ= 2,21, 2,31 (2s, 6H, Ar-CH₃); 3,63 (s, 2H, CH₂); 3,69 (s, 3H, OCH₃); 7,03-7,1 (2s, 2H, Ar-H); 7,25, 7,38 (AA', BB', 4H, Ar-H) ppm.

Analog zu Beispiel (XXXII-1), Variante B bzw. gemäß den allgemeinen Angaben zur Herstellung von Verbindungen der Formel (XXXII) erhält man folgende Verbindungen der Formel (XXXII)

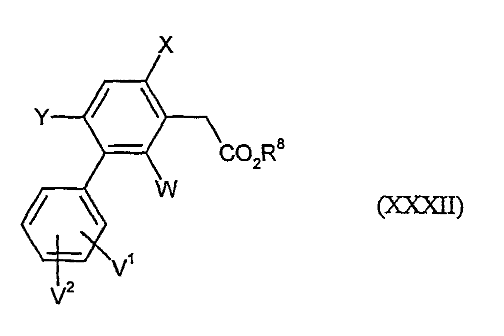

| **Bsp.-Nr.** | **W** | **X** | **Y** | **V**^{**1**} | **V**^{**2**} | **R**^{**8**} | **Fp.°C** |
|---|---|---|---|---|---|---|---|
| XXXII-2 | H | CH₃ | H | 4-Cl | H | CH₃ | Öl |
| XXXII-3 | CH₃ | CH₃ | CH₃ | 4-Cl | H | CH₃ | 68 |
| XXXII-4 | H | CH₃ | CH₃ | 2-Cl | H | CH₃ | Öl |
| XXXII-5 | CH₃ | CH₃ | H | 4-Cl | H | CH₃ | Öl |

### Beispiel XLV-1

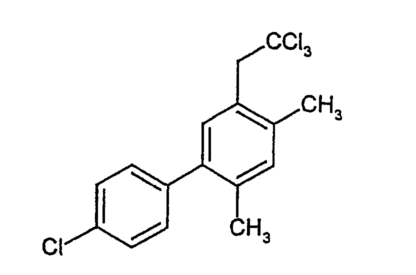

29,5 g wasserfreies Kupfer(II)chlorid werden in die Lösung von 33 g Isopentylnitrit in 120 ml Acetonitril eingetragen. Man versetzt mit 271 g Dichlorethen (Vinylidenchlorid) und anschließend mit 43g der Verbindung gemäß Beispiel XLVI-1 gelöst in Acetonitril und rührt bei Raumtemperatur bis zum Ende der Gasentwicklung. Dann gießt man auf 800 ml eisgekühlte 20%ige Salzsäure und extrahiert mehrfach mit MTBE. Die organische Phase wird mit 20%iger HCl gewaschen und eingeengt. Ausbeute 19 g. Wurde ohne Reinigung direkt weiter umgesetzt.

### Beispiel XLVI-1

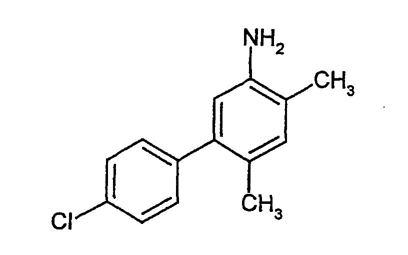

erhält man ausgehend von 3-Brom-4,6-dimethylanilin und 4-Chlorphenylboronsäure durch Suzuki-Kupplung, die wie bei Beispiel XXXII-1, Variante B durchgeführt wird. Ausbeute 12 g.
¹H-NMR (400 MHz, CDCl₃): δ = 2,12 (s, 3H, Ar-CH₃), 2,18 (s, 3H, Ar-CH₃), 3,35 (br, 2H, NH₂), 6,53 (s, 1H, Ar-H), 6,94 (s, 1H, Ar-H), 7,23 (AA',BB', 2H, Ar-H), 7,35 (AA',BB', 2H, Ar-H).

### Beispiel XLVI-2

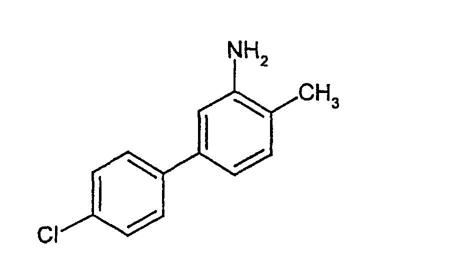

wird analog ausgehend von 3-Brom-6-methylanilin erhalten. Fp. 184°C

### Beispiel

### Myzus-Test

| | |
|---|---|
| Lösungsmittel | 1 Gewichtsteil Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigt z.B. die folgende Verbindung der Herstellungsbeispiele gute Wirksamkeit:

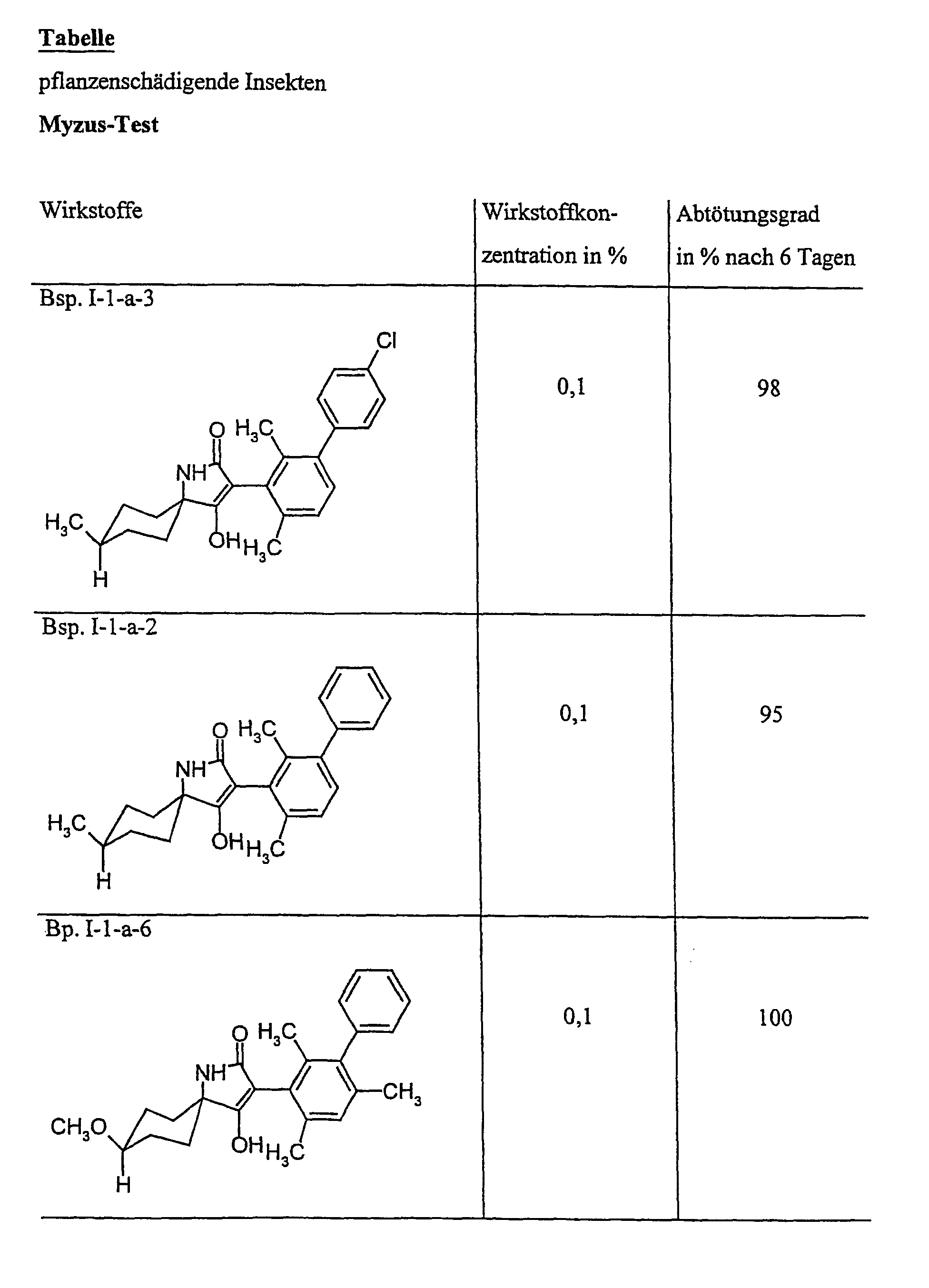

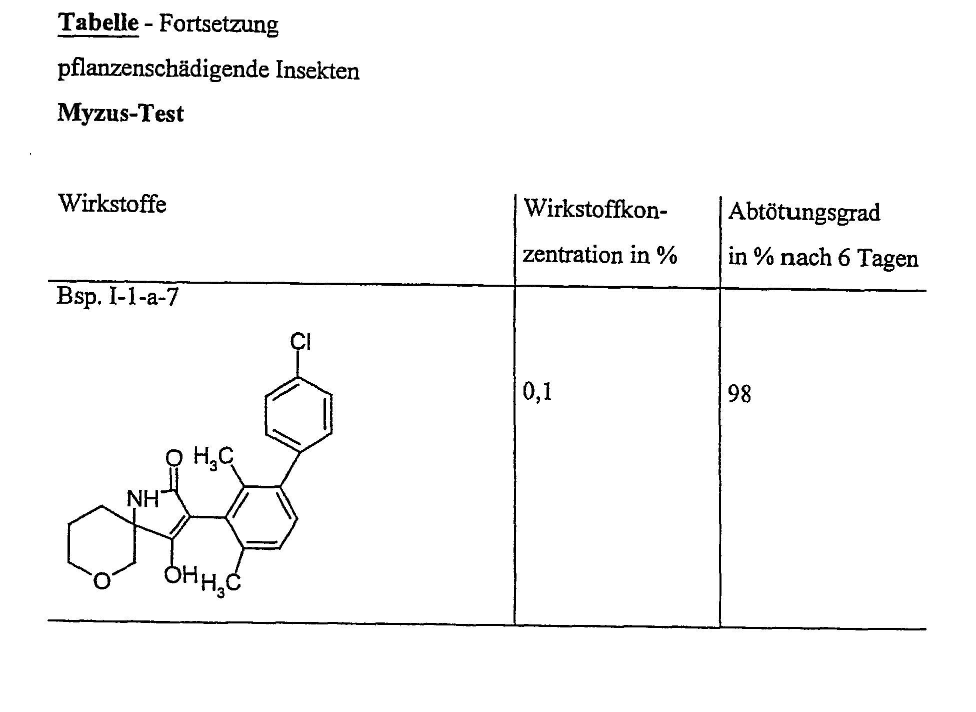

### Beispiel

### Phaedon-Larven-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käferlarven abgetötet wurden.

In diesem Test zeigen z.B. die folgenden Verbindungen der Herstellungsbeispiele gute Wirksamkeit:

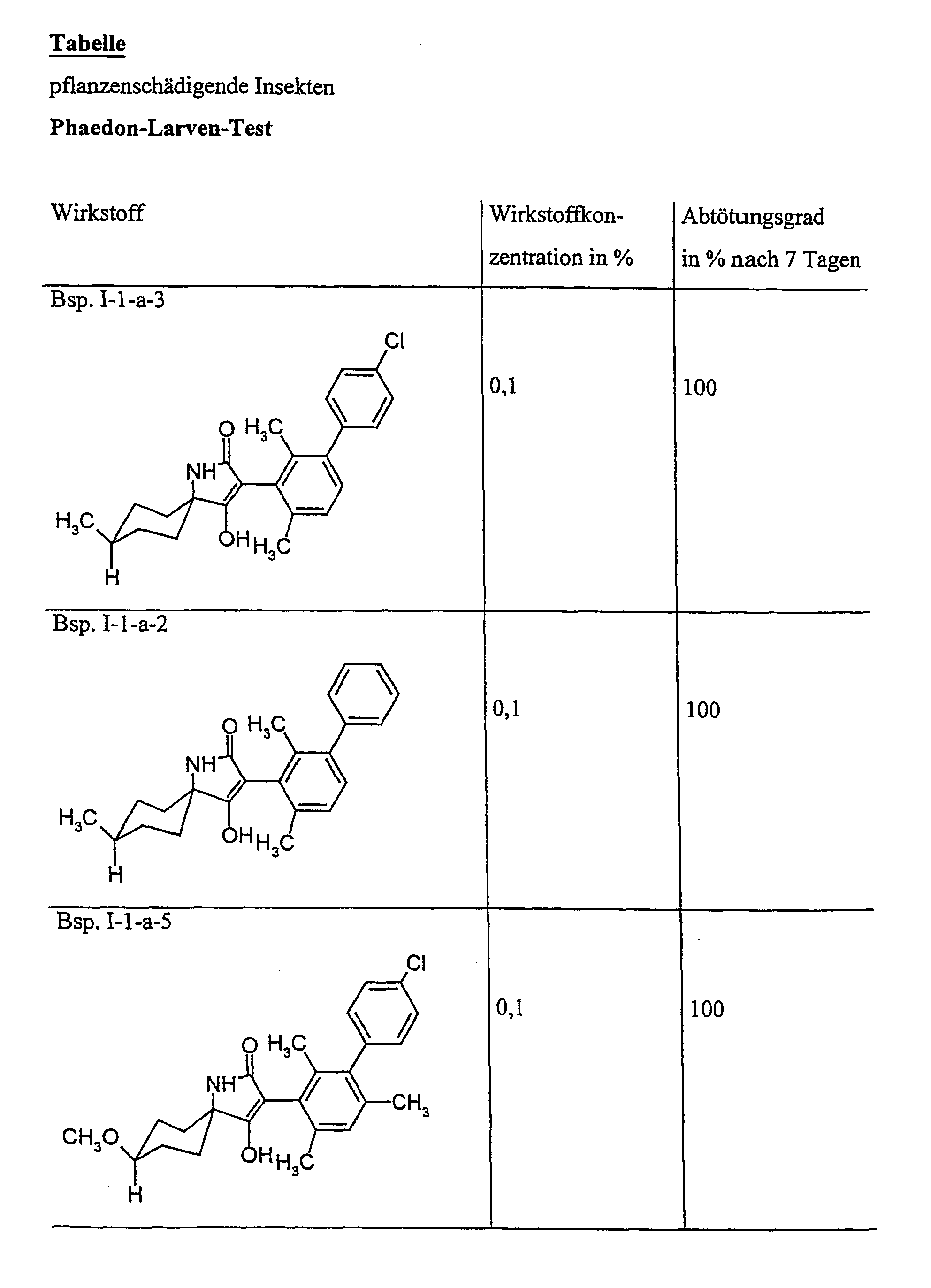

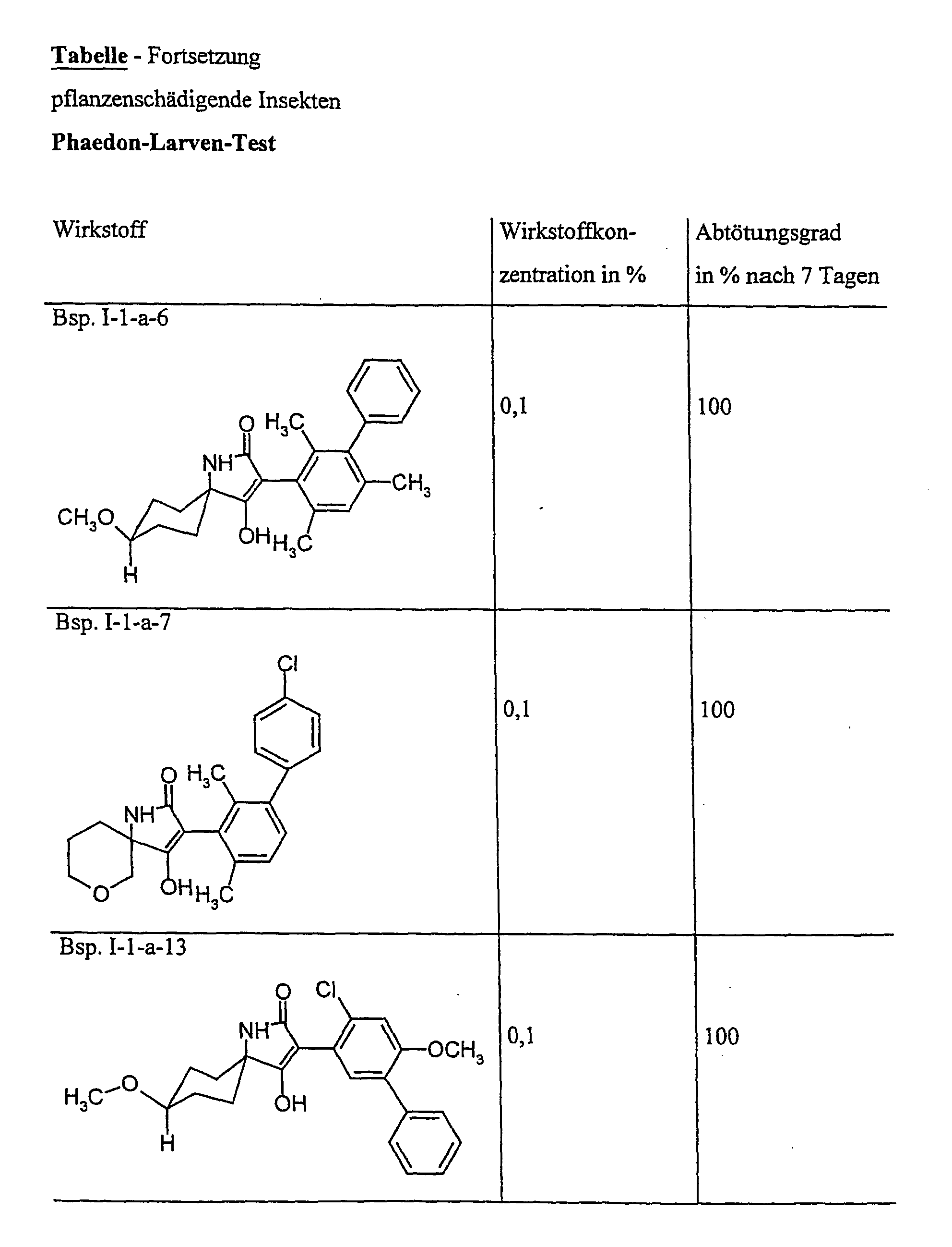

### Beispiel

### Spodoptera frugiperda-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele gute Wirksamkeit.

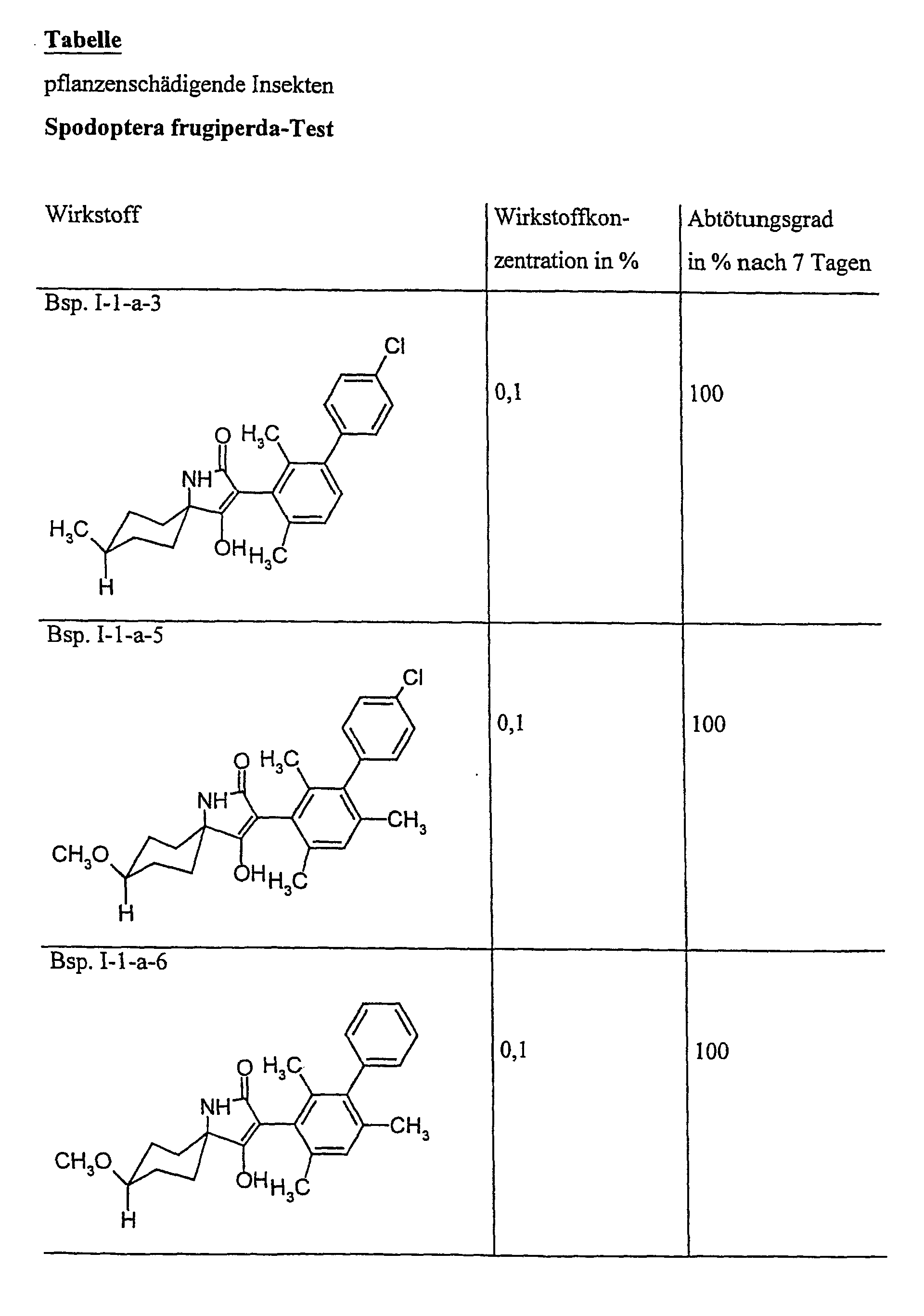

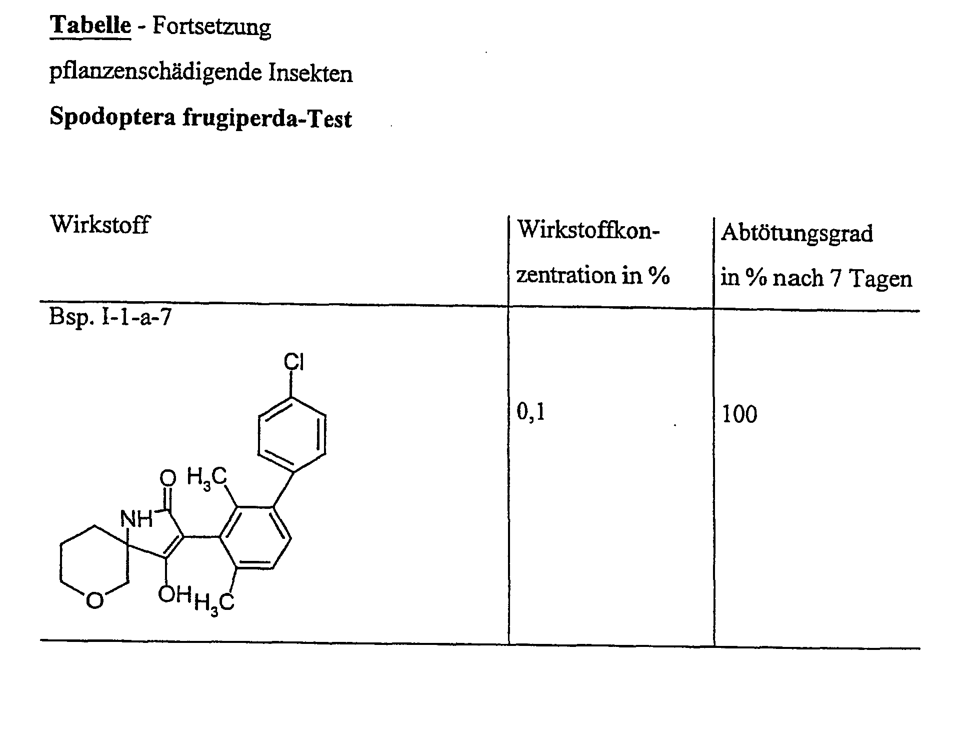

### Beispiel

### Tetranychus-Test (OP-resistent/Tauchbehandlung)

| | |
|---|---|
| Lösungsmittel | 7 Gewichtsteile Dimethylformamid |
| Emulgator | 1 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele gute Wirksamkeit:

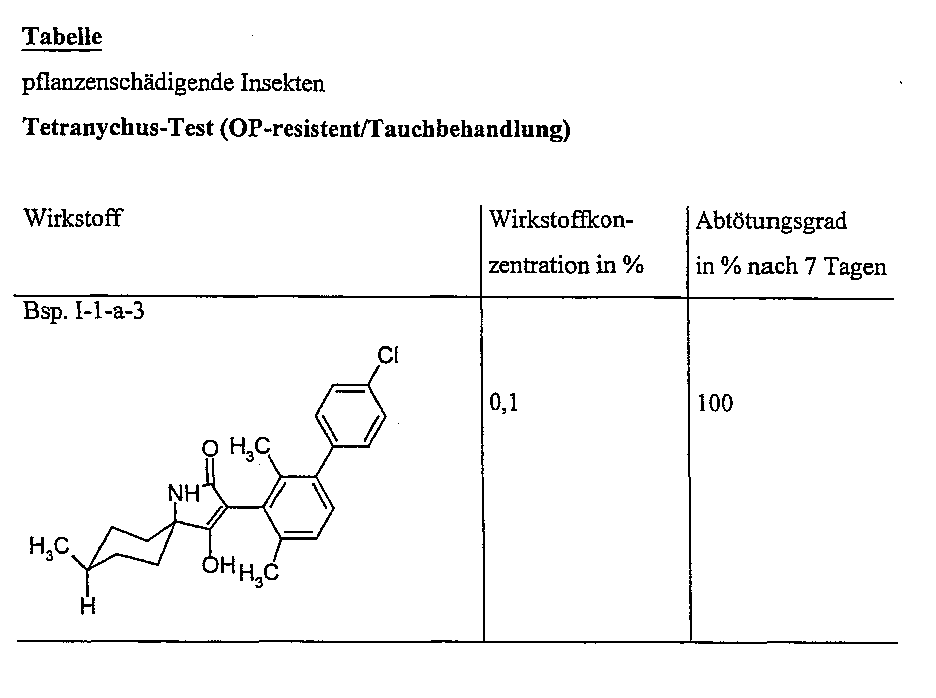

## Patentansprüche

1. Verbindungen der Formel (I) 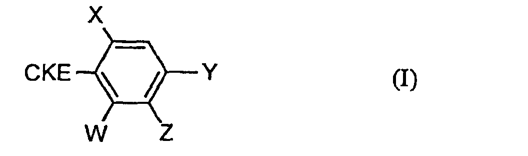 in welcher
X für Halogen, C₁-C₆-Alkyl, C₁-C₆-Halogenalkyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkoxy, C₃-C₆-Halogenalkenyloxy, Nitro, Cyano oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Benzyloxy oder Benzylthio steht,
Z für einen der Reste 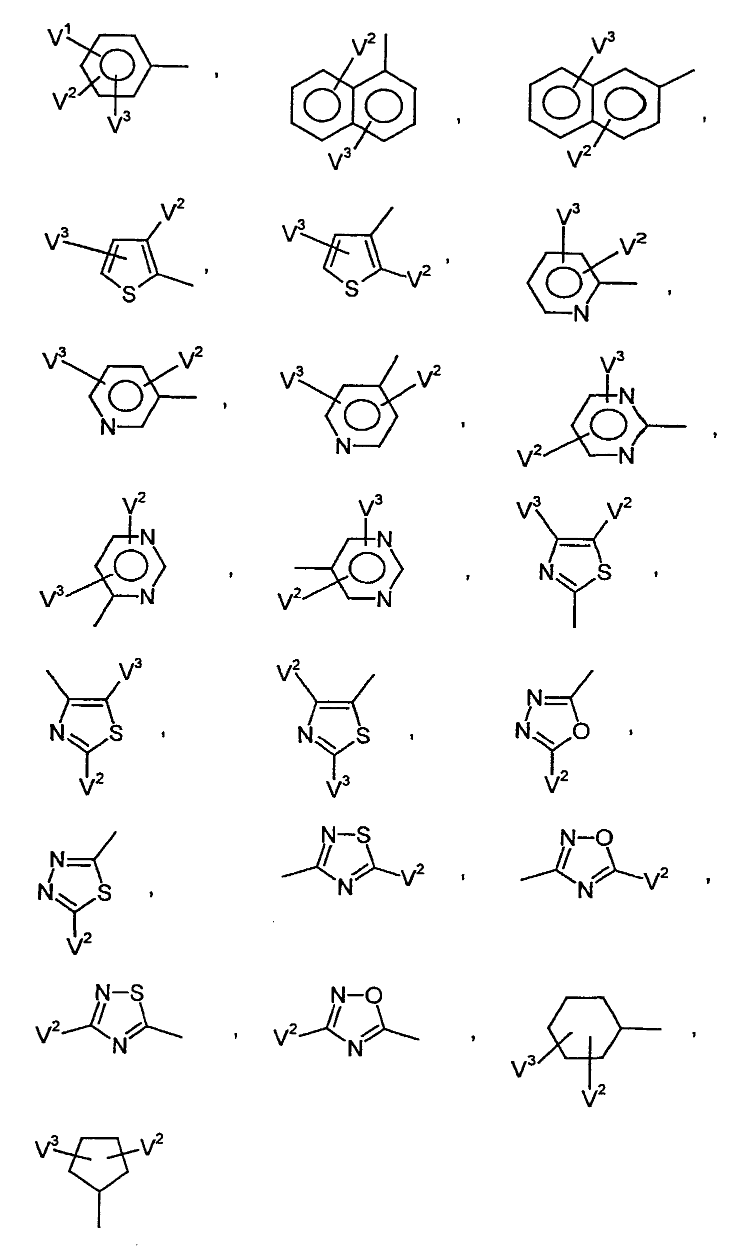 steht,
V¹ für Wasserstoff, Halogen, C₁-C₁₂-Alkyl, C₁-C₆-Alkoxy, C₁-C₆-Alkylthio, C₁-C₆-Alkylsulfinyl, C₁-C₆-Alkylsulfonyl, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio steht,
V² und V³ unabhängig voneinander für Wasserstoff, Halogen, C₁-C₆-Alkyl, C₁-C₆-Alkoxy, C₁-C₄-Halogenalkyl oder C₁-C₄-Halogenalkoxy stehen,
W und Y unabhängig voneinander für Wasserstoff, Halogen, C₁-C₆-Alkyl, C₁-C₆-Halogenalkyl, C₁-C₆-Alkoxy, C₁-C₆-Halogenalkoxy, Nitro oder Cyano stehen,
CKE für eine der Gruppen 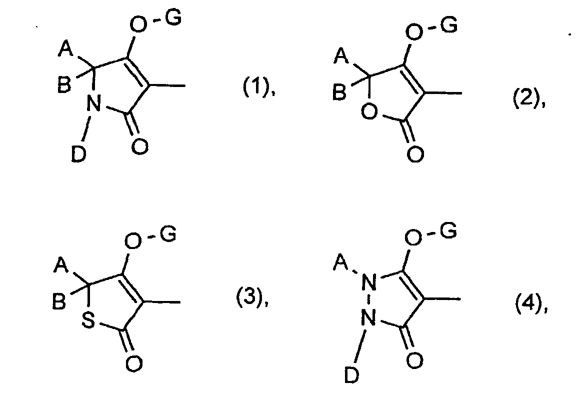 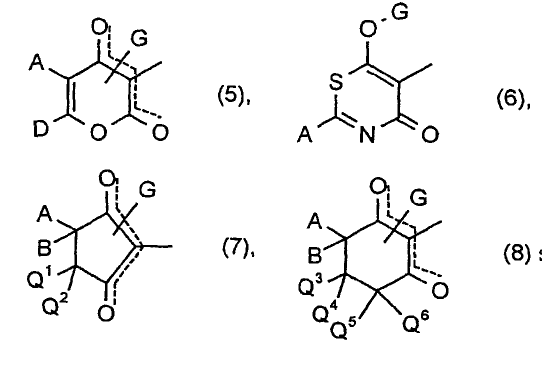 steht,
A für Wasserstoff oder für jeweils gegebenenfalls durch Halogen substituiertes C₁-C₁₂-Alkyl, C₃-C₈-Alkenyl, C₁-C₁₀-Alkoxy-C₁-C₈-alkyl, Poly-C₁-C₈-alkoxy-C₁-C₈-alkyl, C₁-C₁₀-Alkylthio-C₁-C₆-alkyl, gegebenenfalls durch Halogen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes C₆- oder C₁₀-Aryl, Hetaryl mit 5 oder 6 Ringatomen oder C₆- oder C₁₀-Aryl-C₁-C₆-alkyl steht,
B für Wasserstoff, C₁-C₁₂-Alkyl oder C₁-C₈-Alkoxy-C₁-C₆-alkyl steht oder
A, B und das Kohlenstoffatom an das sie gebunden sind, für gesättigtes C₃-C₁₀-Cycloalkyl oder ungesättigtes C₅-C₁₀-Cycloalkyl stehen, worin gegebenenfalls ein Ringglied durch Sauerstoff oder Schwefel ersetzt ist und welche gegebenenfalls einfach oder zweifach durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
A, B und das Kohlenstoffatom, an das sie gebunden sind, für C₃-C₆-Cycloalkyl stehen, welches durch eine gegebenenfalls ein oder zwei nicht direkt benachbarte Sauerstoff- und/oder Schwefelatome enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
A, B und das Kohlenstoffatom, an das sie gebunden sind, für C₃-C₈-Cycloallcyl oder C₅-C₈-Cycloalkenyl stehen, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
D für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl, C₁-C₁₀-Alkylthio-C₂-C₈-akyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 oder 6 Ringatomen, Phenyl-C₁-C₆-alkyl oder Hetaryl-C₁-C₆-alkyl mit 5 oder 6 Ringatomen steht, oder
A und D gemeinsam für jeweils gegebenenfalls substituiertes C₃-C₆-Alkandiyl oder C₃-C₆-Alkendiyl stehen, in welchen gegebenenfalls eine Methylengruppe durch Sauerstoff oder Schwefel ersetzt ist und wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppe, C₃-C₆-Alkendiylgruppe oder eine Butadienylgruppe, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Ring mit 5 oder 6 Ringatomen bilden (im Fall der Verbindung der Formel (I-1) stehen A und D dann gemeinsam mit den Atomen, an die sie gebunden sind beispielsweise für die weiter unten genannten Gruppen AD-1 bis AD-10), der Sauerstoff oder Schwefel enthalten kann, oder worin gegebenenfalls eine der folgenden Gruppen 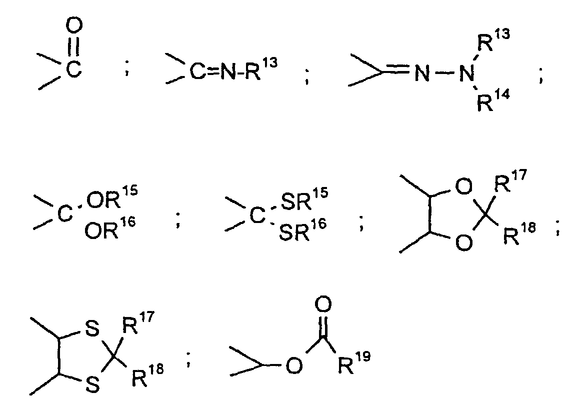 oder 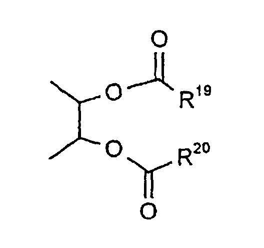
enthalten ist, oder
A und Q¹ gemeinsam für jeweils gegebenenfalls einfach oder zweifach, gleich oder verschieden durch Halogen, Hydroxy, durch jeweils gegebenenfalls einfach bis dreifach, gleich oder verschieden durch Halogen substituiertes C₁-C₁₀-Alkyl, C₁-C₆-Alkoxy, C₁-C₆-Alkylthio, C₃-C₇-Cycloalkyl oder durch jeweils gegebenenfalls einfach bis dreifach, gleich oder verschieden durch Halogen, C₁-C₆-Alkyl oder C₁-C₆-Alkoxy substituiertes Benzyloxy oder Phenyl substituiertes C₃-C₆-Alkandiyl oder C₄-C₆-Alkendiyl stehen, welches außerdem gegebenenfalls eine der nachstehenden Gruppen 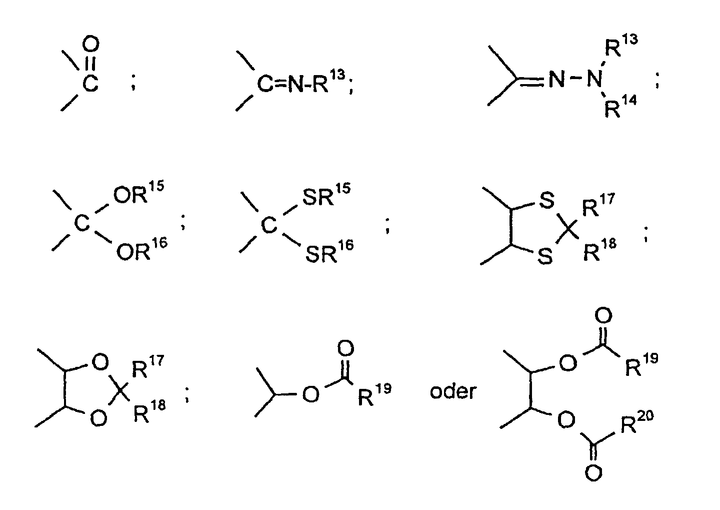 enthält oder durch eine C₁-C₂-Alkandiylgruppe oder durch ein Sauerstoffatom überbrückt ist oder
Q¹ für Wasserstoff oder C₁-C₄-Alkyl steht,
Q², Q⁴, Q⁵ und Q⁶ unabhängig voneinander für Wasserstoff oder C₁-C₄-Alkyl stehen,
Q³ für Wasserstoff, C₁-C₆-Alkyl, C₁-C₆-Alkoxy-C₁-C₂-alkyl, C₁-C₆-Alkylthio-C₁-C₂-alkyl, gegebenenfalls durch C₁-C₄-Alkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl steht oder
Q³ und Q⁴ gemeinsam mit dem Kohlenstoffatom, an das sie gebunden sind, für einen gegebenenfalls durch C₁-C₄-Alkyl, C₁-C₄-Alkoxy oder C₁-C₂-Halogenalkyl substituierten C₃-C₇-Ring stehen, in welchem gegebenenfalls ein Ringglied durch Sauerstoff oder Schwefel ersetzt ist,
G für Wasserstoff (a) oder für eine der Gruppen 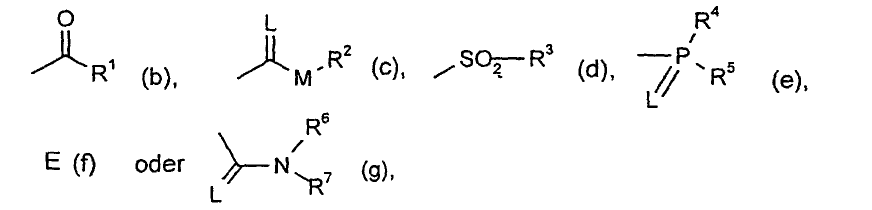 steht,
in welchen
E für ein Metallion oder ein Ammoniumion steht,
L für Sauerstoff oder Schwefel steht und
M für Sauerstoff oder Schwefel steht,
R¹ für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht,
R² für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇--Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest stehen, in welchem gegebenenfalls eine Methylengruppe durch Sauerstoff oder Schwefel ersetzt ist,
R¹³ für Wasserstoff, für jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl oder Phenyl-C₁-C₄-alkoxy steht,
R¹⁴ für Wasserstoff oder C₁-C₈-Alkyl steht oder
R¹³ und R¹⁴ gemeinsam für C₄-C₆-Alkandiyl stehen,
R¹⁵ und R¹⁶ gleich oder verschieden sind und für C₁-C₆-Alkyl stehen oder
R¹⁵ und R¹⁶ gemeinsam für einen C₂-C₄-Alkandiylrest stehen, der gegebenenfalls durch C₁-C₆-Alkyl, C₁-C₆-Halogenalkyl oder durch gegebenenfalls durch Halogen, C₁-C₆-Alkyl, C₁-C₄-Halogenalkyl, C₁-C₆-Alkoxy, C₁-C₄-Halogenalkoxy, Nitro oder Cyano substituiertes Phenyl substituiert ist,
R¹⁷ und R¹⁸ unabhängig voneinander für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl stehen oder
R¹⁷ und R¹⁸ gemeinsam mit dem Kohlenstoffatom, an das sie gebunden sind, für eine Carbonylgruppe oder für gegebenenfalls durch Halogen, C₁-C₄-Alkyl oder C₁-C₄-Akoxy substituiertes C₅-C₇-Cycloalkyl stehen, in dem gegebenenfalls eine Methylengruppe durch Sauerstoff oder Schwefel ersetzt ist und
R¹⁹ und R²⁰ unabhängig voneinander für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino stehen.

2. Verbindungen der Formel (I) gemäß Anspruch 1, worin
X für Fluor, Chlor, Brom, C₁-C₄-Alkyl, C₁-C₄-Alkoxy, C₃-C₄-Alkenyloxy, C₁-C₄-Halogenalkyl, C₁-C₄-Halogenalkoxy, C₃-C₄-Halogenalkenyloxy, Nitro oder Cyano stehe,
Z für einen der Reste 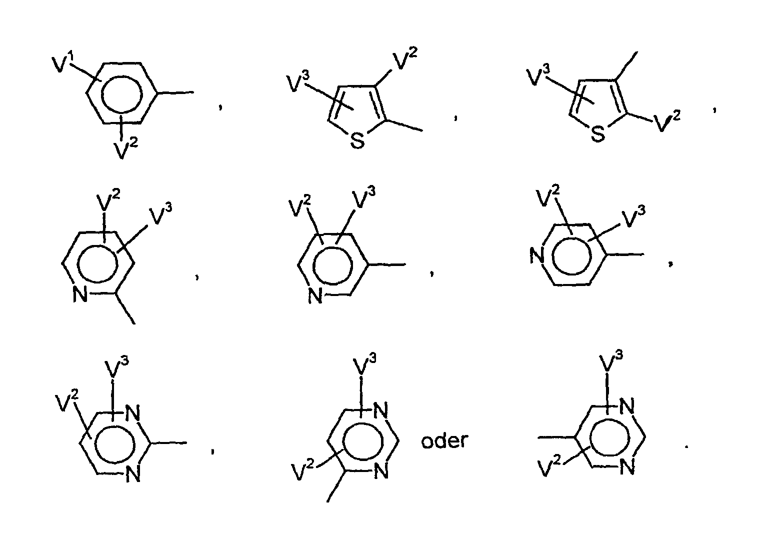
V¹ für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro,Cyano oder jeweils gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Phenoxy-C₁-C₂-alkyl, Phenyl-C₁-C₂-alkoxy, Phenylthio-C₁-C₂-alkyl oder Phenyl-C₁-C₂-alkylthio steht,
V² und V³ unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy stehen,
W und Y unabhängig voneinander für Wasserstoff, Fluor, Chlor, Brom, C₁-C₄-Alkyl, C₁-C₄-Halogenalkyl, C₁-C₄-Alkoxy oder C₁-C₄-Halogenalkoxy stehen,
CKE für eine der Gruppen 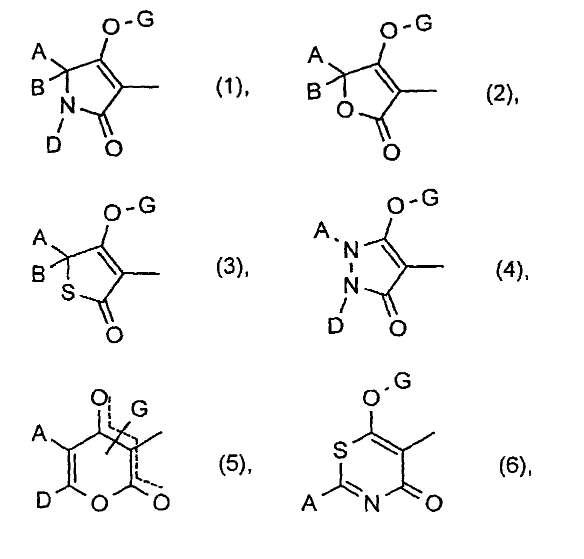 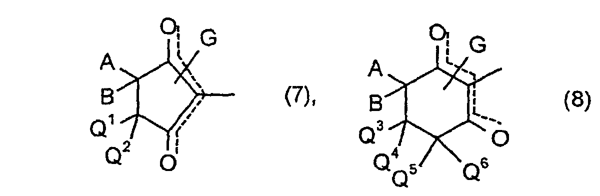 steht,
A für Wasserstoff oder für jeweils gegebenenfalls durch Fluor oder Chlor substituiertes C₁-C₁₀-Alkyl, C₁-C₈-Alkoxy-C₁-C₆-alkyl, gegebenenfalls durch Fluor, Chlor, C₁-C₄-Alkyl oder C₁-C₄-Alkoxy substituiertes C₃-C₇-Cycloalkyl, in welchem gegebenenfalls ein Ringglied durch Sauerstoff oder Schwefel ersetzt ist oder (jedoch nicht im Fall der Verbindungen der Formeln (1-5), (I-7) und (I-8)) jeweils gegebenenfalls durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl, Furanyl, Pyridyl, Imidazolyl, Triazolyl, Pyrazolyl, Pyrimidyl, Thiazolyl, Thienyl oder Phenyl-C₁-C₄-alkyl steht,
B für Wasserstoff oder C₁-C₆-Alkyl steht oder
A, B und das Kohlenstoffatom an das sie gebunden sind, für gesättigtes oder ungesättigtes C₅-C₇-Cycloalkyl stehen, worin gegebenenfalls ein Ringglied durch Sauerstoff oder Schwefel ersetzt ist und welches gegebenenfalls einfach durch C₁-C₆-Alkyl, C₅-C₈-Cycloalkyl, C₁-C₃-Halogenalkyl, C₁-C₆-Alkoxy, Fluor, Chlor oder Phenyl substituiert ist oder
A, B und das Kohlenstoffatom, an das sie gebunden sind, für C₅-C₆-Cycloalkyl stehen, welches durch eine gegebenenfalls ein oder zwei nicht direkt benachbarte Sauerstoff- oder Schwefelatome enthaltende Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet oder
A, B und das Kohlenstoffatom, an das sie gebunden sind, für C₃-C₆-Cycloalkyl oder C₅-C₆-Cycloalkenyl stehen, in welchen zwei Substituenten gemeinsam mit den Kohlenstoffatomen, an die sie gebunden sind, für jeweils gegebenenfalls durch C₁-C₅-Alkyl, C₁-C₅-Alkoxy, Fluor, Chlor oder Brom substituiertes C₂-C₄-Alkandiyl oder C₂-C₄-Alkendiyl, in welchem gegebenenfalls eine Methylengruppe durch Sauerstoff oder Schwefel ersetzt ist oder für Butadiendiyl stehen,
D für Wasserstoff, für jeweils gegebenenfalls durch Fluor oder Chlor substituiertes C₁-C₁₀-Alkyl, C₃-C₆-Alkenyl, C₁-C₈-Alkoxy-C₂-C₆-alkyl oder C₁-C₈-Alkylthio-C₂-C₆-alkyl, für gegebenenfalls durch Fluor, Chlor, C₁-C₄-Alkyl, C₁-C₄-Akoxy oder C₁-C₂-Halogenalkyl substituiertes C₃-C₇-Cycloalkyl, in welchem gegebenenfalls eine Methylengruppe durch Sauerstoff oder Schwefel ersetzt ist oder (jedoch nicht im Fall der Verbindungen der Formeln (I-1) und (I-4)) für jeweils gegebenenfalls durch Fluor, Chlor, Brom, C₁-C₄-Alkyl, C₁-C₄-Halogenalkyl, C₁-C₄-Alkoxy oder C₁-C₄-Halogenalkoxy substitiuiertes Phenyl, Furanyl, Imidazolyl, Pyridyl, Thiazolyl, Pyrazolyl, Pyrimidyl, Pyrrolyl, Thienyl, Triazolyl oder Phenyl-C₁-C₄-alkyl steht, oder
A und D gemeinsam für gegebenenfalls substituiertes C₃-C₅-Alkandiyl stehen, in welchem eine Methylengruppe durch eine Carbonylgruppe, Sauerstoff oder Schwefel ersetzt sein kann, wobei als Substituenten Hydroxy, C₁-C₆-Alkyl oder C₁-C₄-Alkoxy in Frage kommen oder
A und D (im Fall der Verbindungen der Formel (I-1)) gemeinsam mit den Atomen, an die sie gebunden sind, für eine der Gruppen AD-1 bis AD-10: 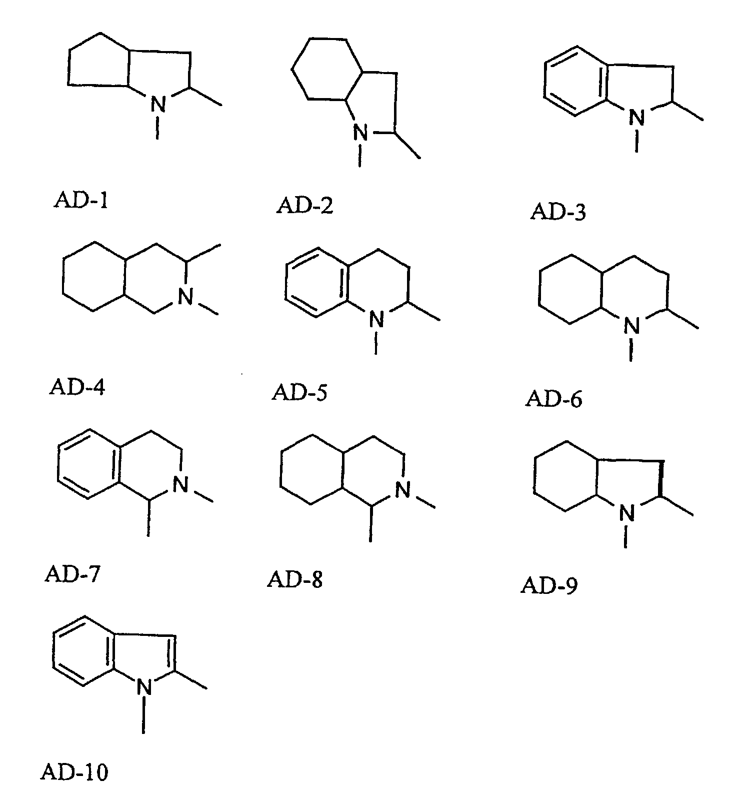 stehen,
oder
A und Q¹ gemeinsam für jeweils gegebenenfalls einfach oder zweifach, gleich oder verschieden durch Fluor, Chlor, Hydroxy, durch jeweils gegebenenfalls einfach bis dreifach durch Fluor substituiertes C₁-C₈-Alkyl oder C₁-C₄-Alkoxy substituiertes C₃-C₄-Alkandiyl oder C₃-C₄-Alkendiyl stehen oder
Q¹ für Wasserstoff steht,
Q² für Wasserstoff steht,
Q⁴, Q⁵ und Q⁶ unabhängig voneinander für Wasserstoff oder C₁-C₃-Alkyl stehen,
Q³ für Wasserstoff, C₁-C₄-Alkyl, C₁-C₄-Alkoxy-C₁-C₂-alkyl, C₁-C₄-Alkylthio-C₁-C₂-alkyl oder gegebenenfalls durch Methyl oder Methoxy substituiertes C₃-C₆-Cycloalkyl steht, worin gegebenenfalls eine Methylengruppe durch Sauerstoff oder Schwefel ersetzt ist oder
Q³ und Q⁴ gemeinsam mit dem Kohlenstoff, an das sie gebunden sind, für einen gegebenenfalls durch C₁-C₄-Alkyl oder C₁-C₄-Alkoxy substituierten gesättigten C₅-C₆-Ring stehen, in welchem gegebenenfalls ein Ringglied durch Sauerstoff oder Schwefel ersetzt ist,
G für Wasserstoff (a) oder für eine der Gruppen 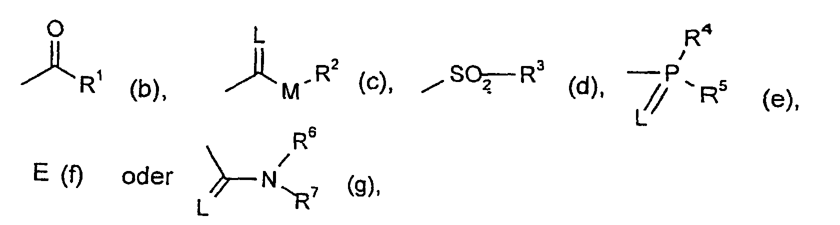 steht,
in welchen
E für ein Metallion oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C₆--Alkylthio-C₁-C₆-alkyl, Poly-C₁-C₆-alkoxy-C₁-C₆-alkyl oder gegebenenfalls durch Fluor, Chlor, C₁-C₅-Alkyl oder C₁-C₅-Alkoxy substituiertes C₃-C₇-Cycloalkyl, in welchem gegebenenfalls ein oder zwei nicht direkt benachbarte Ringglieder durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halegenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₃-alkyl oder
für jeweils gegebenenfalls durch Fluor, Chlor, Brom, Amino oder C₁-C₄-Alkyl substituiertes Pyridyloxy-C₁-C₃-alkyl, Pyrimidyloxy-C₁-C₃-alkyl oder Thiazolyloxy-C₁-C₃-alkyl steht,
R² für jeweils gegebenenfalls durch Fluor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht,
R³ für gegebenenfalls durch Fluor substituiertes C₁-C₆-Alkyl oder für jeweils gegebenenfalls durch Fluor, Chlor, Brom, C₁-C₄-Alkyl, C₁-C₄-Alkoxy, C₁-C₃-Halogenalkyl, C₁-C₃-Halogenalkoxy, Cyano oder Nitro substituiertes Phenyl oder Benzyl steht,
R⁴ und R⁵ unabhängig voneinander für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio stehen und
R⁶ und R⁷ unabhängig voneinander für Wasserstoff, C₁-C₆-Alkyl, C₃-C₆-Cycloalkyl, C₁-C₆-Alkoxy, C₃-C₆-Alkenyl, C₁-C₆-Alkoxy-C₁-C₆-alkyl, für gegebenenfalls durch Fluor, Chlor, Brom, C₁-C₃-Halogenalkyl, C₁-C₄-Alkyl oder C₁-C₄-Alkoxy substituiertes Phenyl, für gegebenenfalls durch Fluor, Chlor, Brom, C₁-C₄-Alkyl, C₁-C₃-Halogenalkyl oder C₁-C₄-Alkoxy substituiertes Benzyl, oder zusammen für einen gegebenenfalls durch Methyl oder Ethyl substituierten C₄-C₅-Alkylenrest stehen, in welchem gegebenenfalls eine Methylengruppe durch Sauerstoff oder Schwefel ersetzt ist.

3. Verbindungen der Formel (I) gemäß Anspruch 1, worin
X für Fluor, Chlor, Methyl, Ethyl, Propyl, iso-Propyl, Methoxy, Ethoxy, Propoxy, iso-Propoxy, Trifluormethyl, Difluormethoxy, Trifluormethoxy, Nitro oder Cyano steht,
Z für einen der Reste 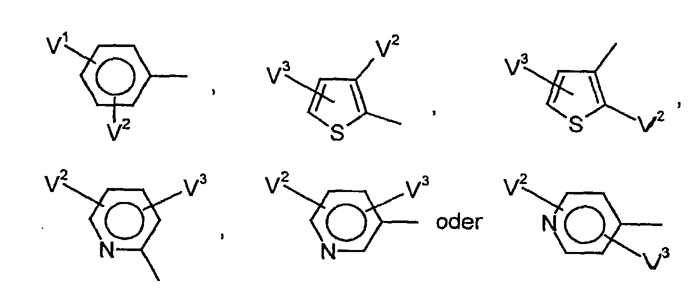 steht,
V¹ für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Nitro, Cyano oder gegebenenfalls einfach durch Fluor, Chlor, Methyl, Methoxy, Trifluormethyl oder Trifluormethoxy substituiertes Phenyl steht,
V² und V³ unabhängig voneinander für Wasserstoff, Fluor, Chlor, Methyl, Ethyl, n-Propyl, iso-Propyl, Methoxy, Ethoxy, Trifluormethyl oder Trifluormethoxy stehen,
W und Y unabhängig voneinander für Wasserstoff, Fluor, Chlor, Methyl, Ethyl, n-Propyl, Methoxy, Ethoxy oder Propoxy stehen,
CKE für eine der Gruppen 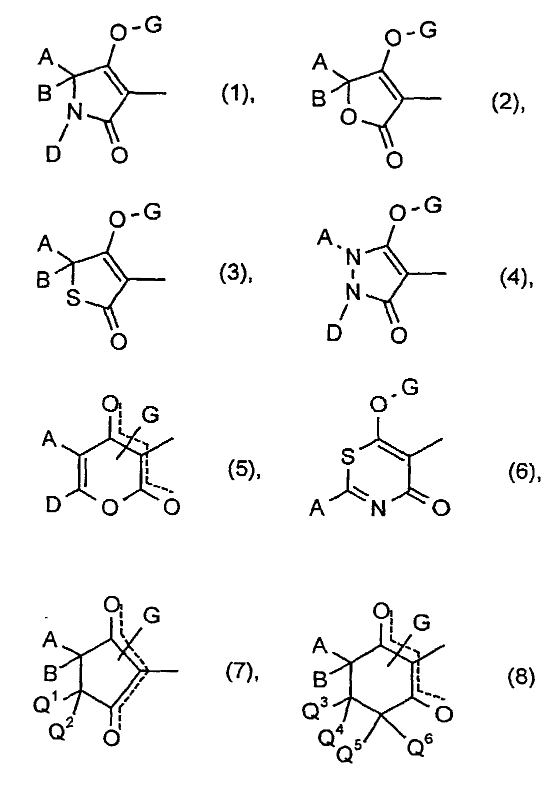 steht,
A für Wasserstoff oder für jeweils gegebenenfalls durch Fluor substituiertes C₁-C₈-Alkyl oder C₁-C₆-Alkoxy-C₁-C₄-alkyl, gegebenenfalls durch Fluor, Methyl, Ethyl oder Methoxy substituiertes C₃-C₆-Cycloalkyl, in welchem gegebenenfalls ein Ringglied durch Sauerstoff oder Schwefel ersetzt ist oder (jedoch nicht im Fall der Verbindungen der Formeln (I-5), (1-7) und (I-8)) für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl oder Benzyl steht,
B für Wasserstoff oder C₁-C₄-Alkyl steht oder
A, B und das Kohlenstoffatom an das sie gebunden sind, für gesättigtes C₅-C₆-Cycloalkyl stehen, in welchem gegebenenfalls ein Ringglied durch Sauerstoff oder Schwefel ersetzt ist und welches gegebenenfalls einfach durch Methyl, Ethyl, Propyl, Isopropyl, Butyl, iso-Butyl, sec.-Butyl, tert.-Butyl, Trifluormethyl, Methoxy, Ethoxy, Propoxy, iso-Propoxy, Butoxy, iso-Butoxy, sek.-Butoxy, tert.-Butoxy, Fluor oder Chlor substituiert ist oder
A, B und das Kohlenstoffatom, an das sie gebunden sind, für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder für Butadiendiyl stehen,
D für Wasserstoff, für jeweils gegebenenfalls durch Fluor oder Chlor substituiertes C₁-C₈-Alkyl, C₃-C₄-Alkenyl, C₁-C₆-Alkoxy-C₂-C₄-alkyl, C₁-C₄-Alkylthio-C₂-C₄-alkyl oder C₃-C₆-Cycloalkyl, in welchem gegebenenfalls eine Methylengruppe durch Sauerstoff oder Schwefel ersetzt ist oder (jedoch nicht im Fall der Verbindungen der Formeln (I-1) und (I-4)) für jeweils gegebenenfalls durch Fluor, Chlor, Methyl, Ethyl, n-Propyl, iso-Propyl, Methoxy, Ethoxy, Trifluormethyl oder Trifluormethoxy substituiertes Phenyl, Furanyl, Pyridyl, Thienyl oder Benzyl steht,
oder
A und D gemeinsam für gegebenenfalls substituiertes C₃-C₄-Alkandiyl stehen, worin gegebenenfalls ein Kohlenstoffatom durch Schwefel ersetzt ist und welches gegebenenfalls durch Hydroxy, Methyl, Ethyl, Methoxy oder Ethoxy substituiert ist oder
A und D (im Fall der Verbindungen der Formel (I-1)) gemeinsam mit den Atomen, an die sie gebunden sind, für eine der folgenden Gruppen AD: 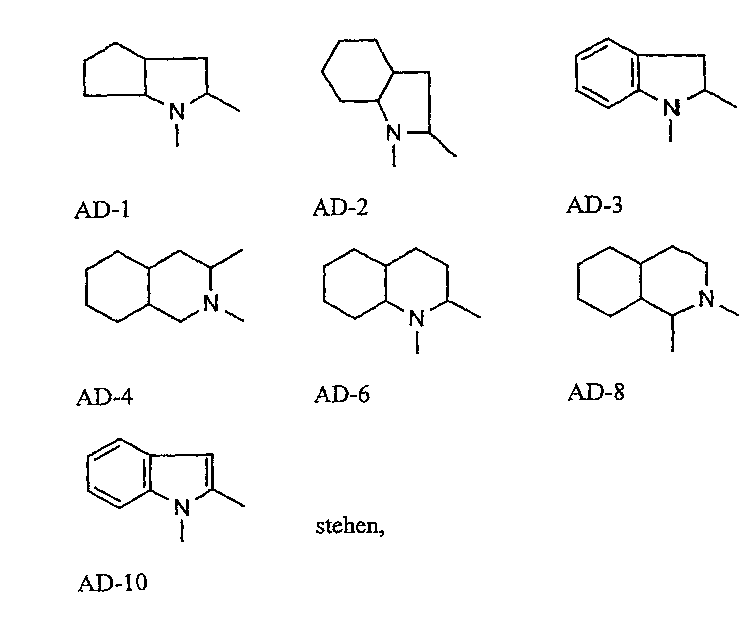 stehen,
A und Q¹ gemeinsam für gegebenenfalls einfach oder zweifach durch Fluor, Hydroxy, Methyl oder Methoxy substituiertes C₃-C₄-Alkandiyl oder Butendiyl stehen oder
Q¹ für Wasserstoff steht,
Q² für Wasserstoff steht,
Q⁴, Q⁵ und Q⁶ unabhängig voneinander für Wasserstoff, Methyl oder Ethyl stehen,
Q³ für Wasserstoff, Methyl, Ethyl oder C₃-C₆-Cycloalkyl steht, worin gegebenenfalls eine Methylengruppe durch Sauerstoff oder Schwefel ersetzt ist oder
Q³ und Q⁴ gemeinsam mit dem Kohlenstoff, an den sie gebunden sind, für einen gegebenenfalls durch Methyl oder Methoxy substituierten gesättigten C₅-C₆-Ring stehen, in welchem gegebenenfalls ein Ringglied durch Sauerstoff oder Schwefel ersetzt ist,
G für Wasserstoff (a) oder für eine der Gruppen 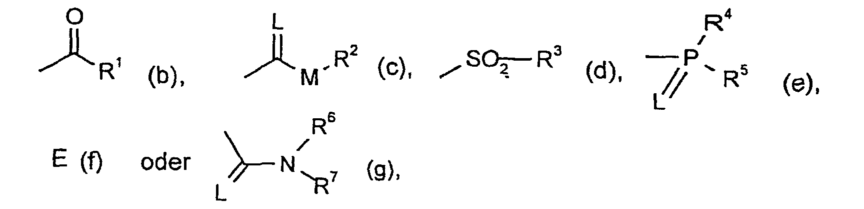 steht,
in welchen
E für ein Metallion oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder gegebenenfalls durch Fluor, Chlor, Methyl, Ethyl, Propyl, i-Propyl, Butyl, i-Butyl, tert.-Butyl, Methoxy, Ethoxy, n-Propoxy oder iso-Propoxy substituiertes C₃-C₆-Cycloalkyl, in welchem gegebenenfalls ein oder zwei nicht direkt benachbarte Ringglieder durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl, Pyridyl, Pyrimidyl, Thiazolyl oder Pyrazolyl,
für gegebenenfalls durch Fluor, Chlor, Methyl oder Ethyl substituiertes Phenoxy-C₁-C₂-alkyl oder
für jeweils gegebenenfalls durch Fluor, Chlor, Amino, Methyl oder Ethyl substituiertes Pyridyloxy-C₁-C₂-alkyl, Pyrimidyloxy-C₁-C₂-alkyl oder Thiazolyloxy-C₁-C₂-alkyl steht,
R² für jeweils gegebenenfalls durch Fluor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
R³ für gegebenenfalls durch Fluor substituiertes Methyl, Ethyl, n-Propyl, Isopropyl oder jeweils gegebenenfalls durch Fluor, Chlor, Brom, Methyl, tert.-Butyl, Methoxy, Trifluormethyl, Trifluormethoxy, Cyano oder Nitro substituiertes Phenyl oder Benzyl steht,
R⁴ und R⁵ unabhängig voneinander für C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio oder C₁-C₃-Alkyl substituiertes Phenyl, Phenoxy oder Phenylthio stehen und
R⁶ und R⁷ unabhängig voneinander für Wasserstoff, für C₁-C₄-Alkyl, C₃-C₆-Cycloalkyl, C₁-C₄-Alkoxy, C₃-C₄-Alkenyl, C₁-C₄-Alkoxy-C₁-C₄-alkyl, für gegebenenfalls durch Fluor, Chlor, Brom, Trifluormethyl, Methyl oder Methoxy substituiertes Phenyl, für gegebenenfalls durch Fluor, Chlor, Brom, Methyl, Trifluormethyl oder Methoxy substituiertes Benzyl, oder zusammen für einen C₅-C₆-Alkylenrest stehen, in welchem gegebenenfalls eine Methylengruppe durch Sauerstoff oder Schwefel ersetzt ist.

4. Verfahren zur Herstellung von Verbindungen der Formel (I) gemäß Anspruch 1, **dadurch gekennzeichnet, daß** man
(A) Verbindungen der Formel (I-1-a) 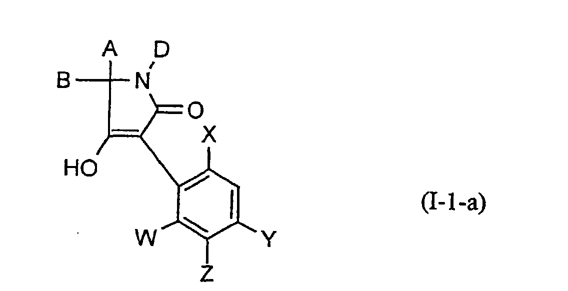 in welcher
A, B, D, W, X, Y und Z die in Anspruch 1 angegebenen Bedeutungen haben,
erhält, wenn man
N-Acylaminosäureester der Formel (II) 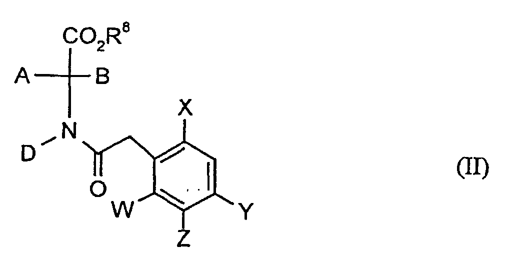 in welcher
A, B, D, W, X, Y und Z die oben angegebenen Bedeutungen haben,
und
R⁸ für Alkyl steht,
in Gegenwart eines Verdünnungsmittels und in Gegenwart einer Base intramolekular kondensiert,
(B) Verbindungen der Formel (I-2-a) 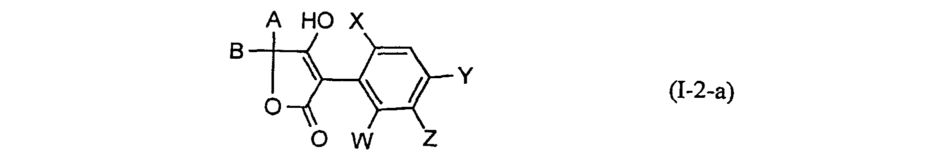 in welcher
A, B, W, X, Y und Z die oben angegebenen Bedeutungen haben,
erhält, wenn man
Carbonsäureester der Formel (III) 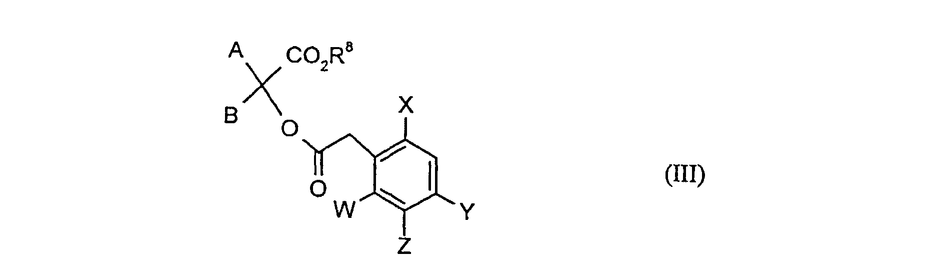 in welcher
A, B, W, X, Y, Z und R⁸ die oben angegebenen Bedeutungen haben,
in Gegenwart eines Verdünnungsmittels und in Gegenwart einer Base intramolekular kondensiert,
(C) Verbindungen der Formel (I-3-a) 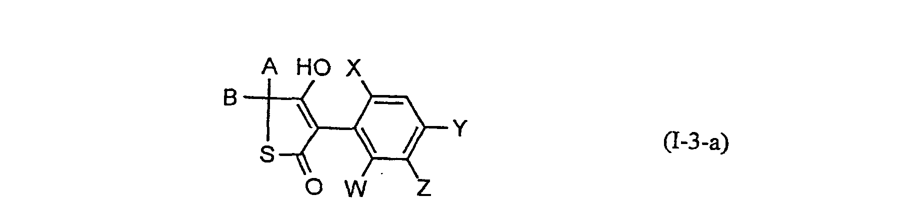 in welcher
A, B, W, X, Y und Z die oben angegebenen Bedeutungen haben,
erhält, wenn man
β-Ketocarbonsäureester der Formel (IV) 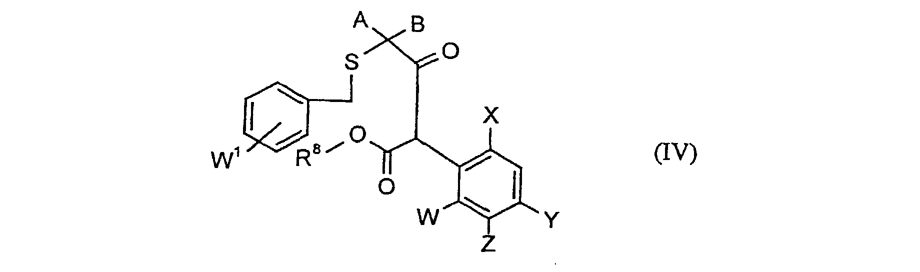 in welcher
A, B, W, X, Y, Z und R⁸ die oben angegebenen Bedeutungen haben und
W¹ für Wasserstoff, Halogen, Alkyl oder Alkoxy steht,
gegebenenfalls in Gegenwart eines Verdünnungsmittels und in Gegenwart einer Säure intramolekular cyclisiert,
(D) Verbindungen der Formel (I-4-a) 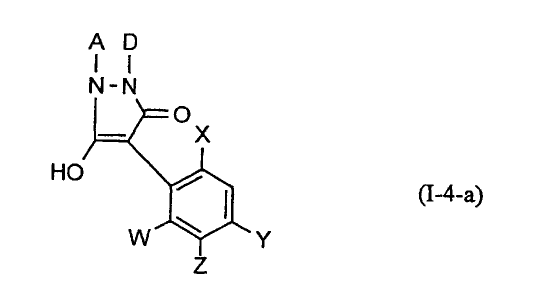 in welcher
A, D, W, X, Y und Z die oben angegebenen Bedeutungen haben,
erhält, wenn man
(α) Halogencarbonylketene der Formel (V) 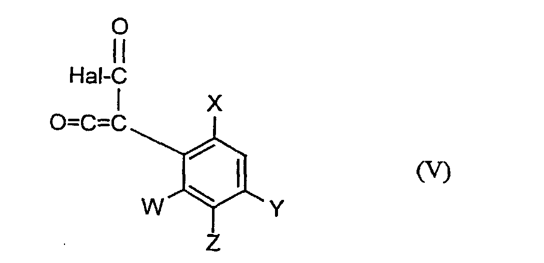 in welcher
W, X, Y und Z die oben angegebenen Bedeutungen haben
und
Hal für Halogen steht,
oder
(β) Malonsäurederivate der Formel (VI) 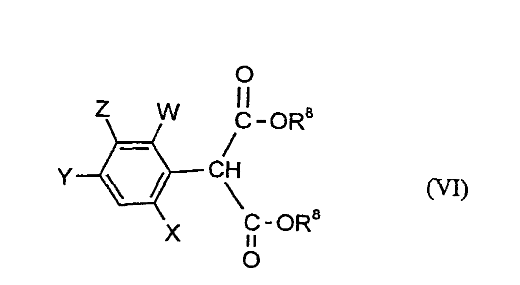 in welcher
R⁸, W, X, Y und Z die oben angegebenen Bedeutungen haben,
mit Hydrazinen der Formel (VII)
A-NH-NH-D (VII)
in welcher
A und D die oben angegebenen Bedeutungen haben,
gegebenenfalls in Gegenwart eines Verdünnungsmittels und gegebenenfalls in Gegenwart einer Base umsetzt,
(E) Verbindungen der Formel (I-5-a) 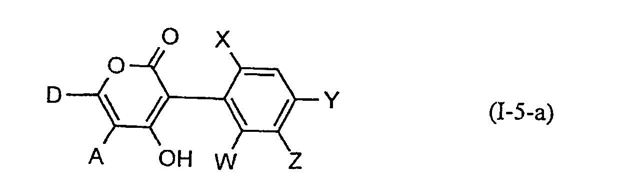 in welcher
A, D, W, X, Y und Z die oben angegebenen Bedeutungen haben,
erhält, wenn man
Carbonylverbindungen der Formel (VIII) 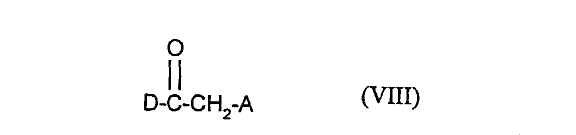 in welcher
A und D die oben angegebenen Bedeutungen haben,
oder deren Silylenolether der Formel (VIIIa) 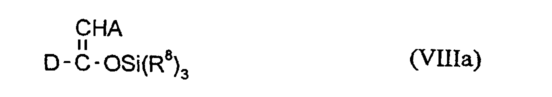 in welcher
A, D und R⁸ die oben angegebene Bedeutung haben,
mit Ketensäurehalogeniden der Formel (V) 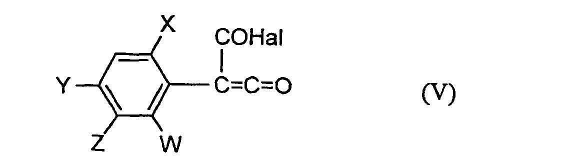 in welcher
W, X, Y und Z die oben angegebenen Bedeutungen haben und
Hal für Halogen steht,
gegebenenfalls in Gegenwart eines Verdünnungsmittels und gegebenenfalls in Gegenwart eines Säureakzeptors umsetzt,
(F) Verbindungen der Formel (I-6-a) 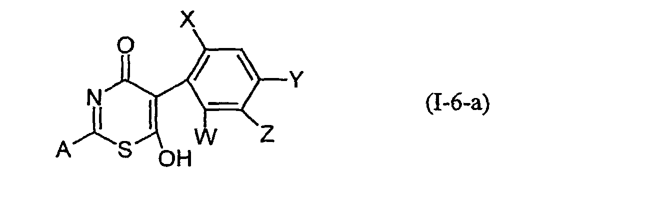 in welcher
A, W, X, Y und Z die oben angegebene Bedeutung haben,
erhält, wenn man Thioamide der Formel (IX) 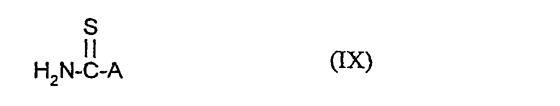 in welcher
A die oben angegebene Bedeutung hat,
mit Ketensäurehalogeniden der Formel (V) 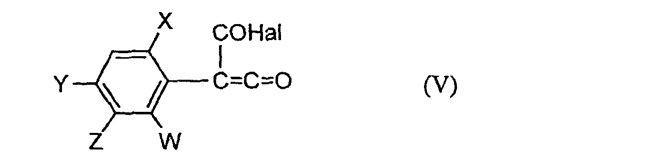 in welcher
Hal, W, X, Y und Z die oben angegebenen Bedeutungen haben,
gegebenenfalls in Gegenwart eines Verdünnungsmittels und gegebenenfalls in Gegenwart eines Säureakzeptors umsetzt,
(G) Verbindungen der Formel (I-7-a) 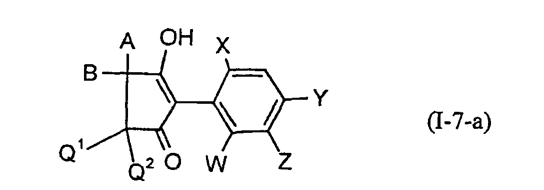 in welcher
A, B, Q¹, Q², W, X, Y und Z die in Anspruch 1 angegebene Bedeutung haben,
erhält, wenn man
Ketocarbonsäureester der Formel (X) 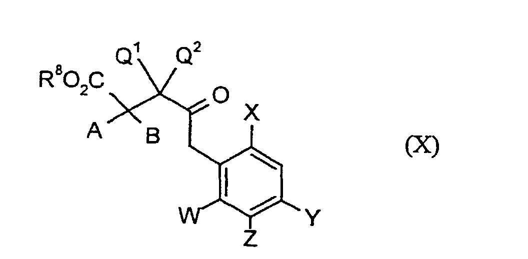 in welcher
A, B, Q¹, Q², W, X, Y und Z die oben angegebene Bedeutung haben, und
R⁸ für Alkyl steht,
gegebenenfalls in Gegenwart eines Verdünnungsmittels und in Gegenwart einer Base intramolekular cyclisiert,
(H) daß man Verbindungen der Formel (I-8-a) 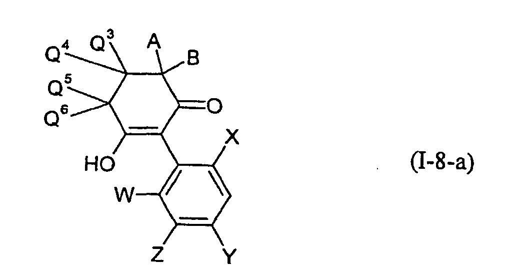 in welcher
A, B, Q³, Q⁴, Q⁵, Q⁶, W, X, Y und Z die in Anspruch 1 angegebene Bedeutung haben,
erhält, wenn man
6-Aryl-5-keto-hexansäureester der Formel (XI) 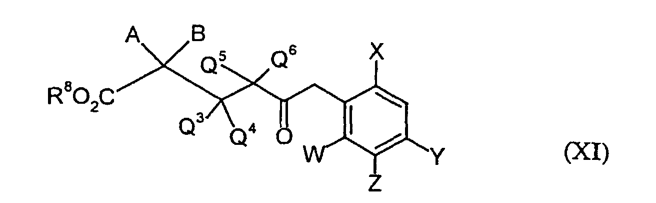 in welcher
A, B, Q³, Q⁴, Q⁵, Q⁶, W, X, Y und Z die oben angegebene Bedeutung haben
und
R⁸ für Alkyl steht,
in Gegenwart eines Verdünnungsmittels und in Gegenwart einer Base intramolekular kondensiert,
(I) Verbindungen der oben gezeigten Formeln (I-8-a), in welchen A, B, D, Q¹, Q², Q³, Q⁴, Q⁵, Q⁶, X, Y und Z die oben angegebene Bedeutung haben, erhält, wenn man Verbindungen der Formel (I-1'-a) bis (I-8'-a), 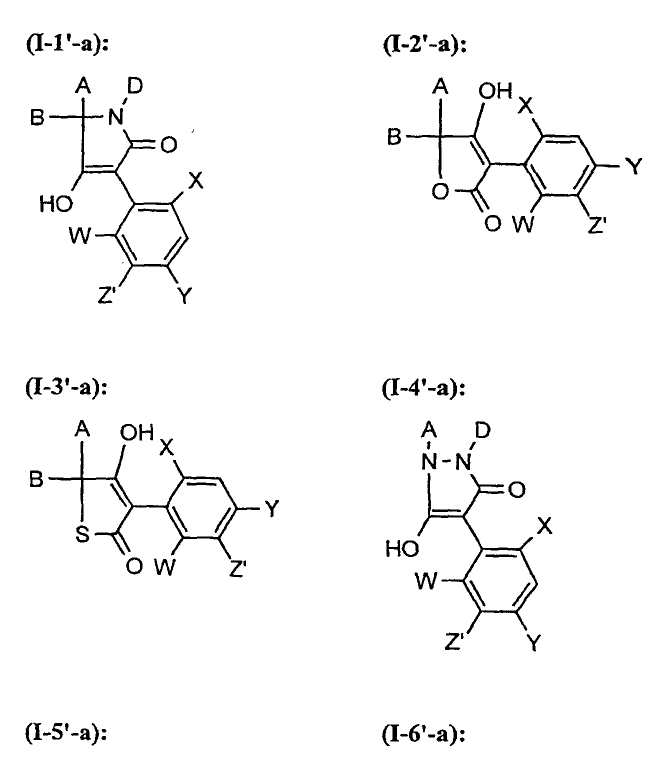 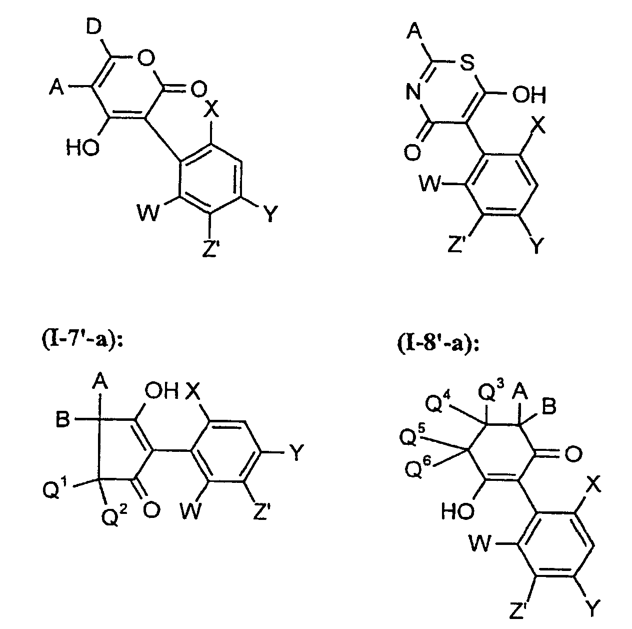 in welchen
A, B, D, Q¹, Q², Q³, Q⁴, Q⁵, Q⁶, W, X und Y die oben angegebene Bedeutung haben und
Z' für Chlor, Brom oder Jod steht,
mit Boronsäuren der Formel (XII) 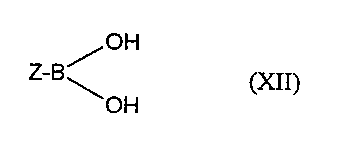 in welcher
Z die oben angegebene Bedeutung hat,
in Gegenwart eines Lösungsmittels, einer Base und eines Katalysators umsetzt und anschließend gegebenenfalls die so erhaltenen Verbindungen der Formeln (I-1-a) bis (I-8-a) jeweils
(Jα) mit Säurehalogeniden der Formel (XIII) 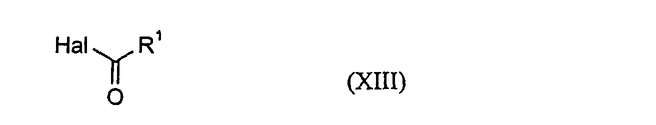 in welcher
R¹ die in Anspruch 1 angegebene Bedeutung hat und
Hal für Halogen steht
oder
(β) mit Carbonsäureanhydriden der Formel (XIV)
R¹-CO-O-CO-R¹ (XIV)
in welcher
R¹ die oben angegebene Bedeutung hat,
gegebenenfalls in Gegenwart eines Verdünnungsmittels und gegebenenfalls in Gegenwart eines Säurebindemittels umsetzt, oder jeweils
(K) mit Chlorameisensäureestern oder Chlorameisensäurethioestern der Formel (XV)
R²-M-CO-Cl (XV)
in welcher
R² und M die in Anspruch 1 angegebenen Bedeutungen haben,
gegebenenfalls in Gegenwart eines Verdünnungsmittels und gegebenenfalls in Gegenwart eines Säurebindemittels umsetzt, oder jeweils
(L) mit Chlormonothioameisensäureestern oder Chlordithioameisensäureestern der Formel (XVI) 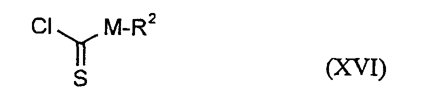 in welcher
M und R² die oben angegebenen Bedeutungen haben,
gegebenenfalls in Gegenwart eines Verdünnungsmittels und gegebenenfalls in Gegenwart eines Säurebindemittels umsetzt, oder jeweils
(M) mit Sulfonsäurechloriden der Formel (XVII)
R³-SO₂-Cl (XVII)
in welcher
R³ die in Anspruch 1 angegebene Bedeutung hat,
gegebenenfalls in Gegenwart eines Verdünnungsmittels und gegebenenfalls in Gegenwart eines Säurebindemittels umsetzt, oder jeweils
(N) mit Phosphorverbindungen der Formel (XVIII) 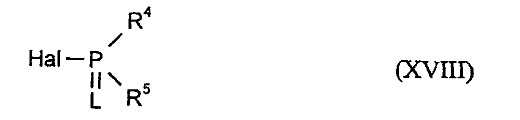 in welcher
L, R⁴ und R⁵ die in Anspruch 1 angegebenen Bedeutungen haben und
Hal für Halogen steht,
gegebenenfalls in Gegenwart eines Verdünnungsmittels und gegebenenfalls in Gegenwart eines Säurebindemittels umsetzt, oder jeweils
(L) mit Metallverbindungen oder Aminen der Formeln (XIX) oder (XX)
Me(OR¹⁰)ₜ (XIX) 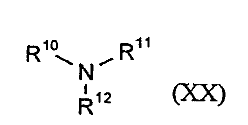
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt, oder jeweils
(Pα) mit Isocyanaten oder Isothiocyanaten der Formel (XXI)
R⁶-N=C=L (XXI)
in welcher
R⁶ und L die in Anspruch 1 angegebenen Bedeutungen haben,
gegebenenfalls in Gegenwart eines Verdünnungsmittels und gegebenenfalls in Gegenwart eines Katalysators umsetzt, oder jeweils
(β) mit Carbamidsäurechloriden oder Thiocarbamidsäurechloriden der Formel (XXII) 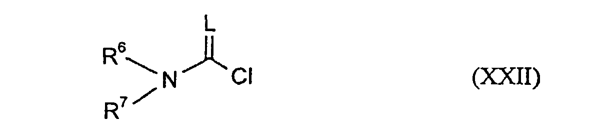 in welcher
L, R⁶ und R⁷ die in Anspruch 1 angegebenen Bedeutungen haben,
gegebenenfalls in Gegenwart eines Verdünnungsmittels und gegebenenfalls in Gegenwart eines Säurebindemittels, umsetzt.

5. Verbindungen der Formel (II) 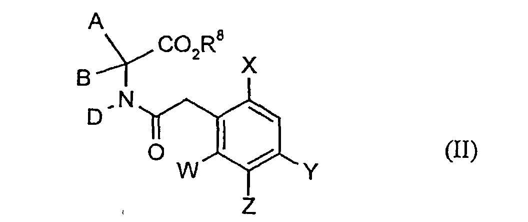 in welcher
A, B, D, W, X, Y und Z die in Anspruch 1 angegebenen Bedeutungen haben und
R⁸ für Alkyl steht.

6. Verbindungen der Formel (XXIV) 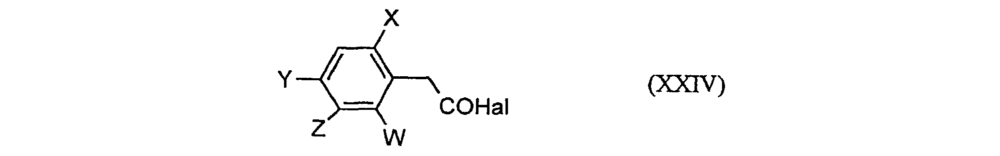 in welcher
W, X, Y und Z die in Anspruch 1 angegebenen Bedeutungen haben und
Hal für Chlor oder Brom steht.

7. Verbindungen der Formel (XXV) 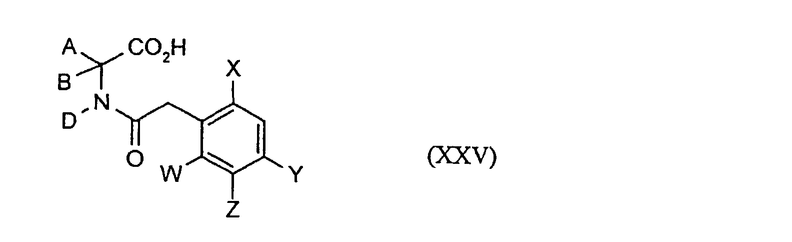 in welcher
A, B, D, W, X, Y und Z die in Anspruch 1 angegebenen Bedeutungen haben.

8. Verbindungen der Formel (XXIX) 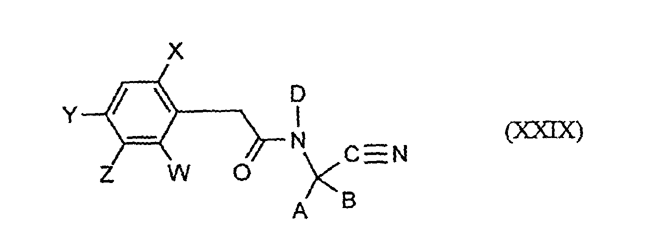 in welcher
A, B, D, W, X, Y und Z die in Anspruch 1 angegebenen Bedeutungen haben.

9. Verbindungen der Formel (III) 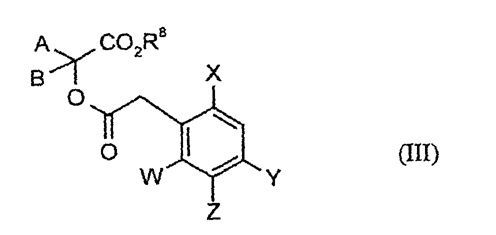 in welcher
A, B, W, X, Y und Z die oben angegebenen Bedeutungen haben und
R⁸ für Alkyl steht.

10. Verbindungen der Formel (XXVII) 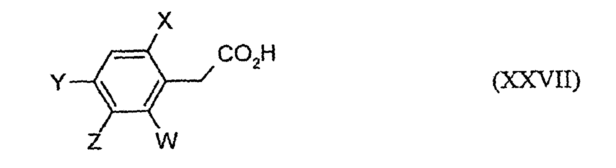 in welcher
W, X, Y und Z die in Anspruch 1 angegebenen Bedeutungen haben.

11. Verbindungen der Formel (XXXII) 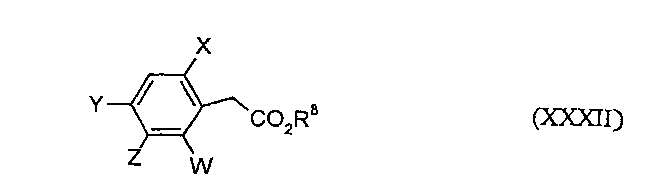 in welcher
W, X, Y und Z die in Anspruch 1 angegebene Bedeutung haben und
R⁸ für Alkyl steht.

12. Verbindungen der Formel (XXVII-b) 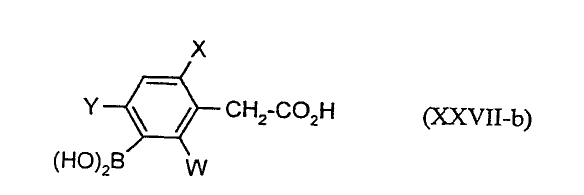 in welcher
W, X und Y die in Anspruch 1 angegebene Bedeutung haben.

13. Verbindungen der Formel (IV) 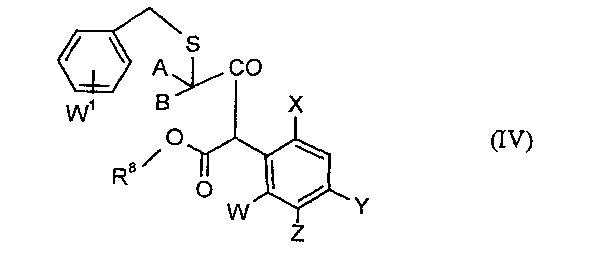 in welcher
A, B, W, W¹, X, Y und Z die in Anspruch 1 angegebenen Bedeutungen haben und
R⁸ für Alkyl steht

14. Verbindungen der Formel (V) 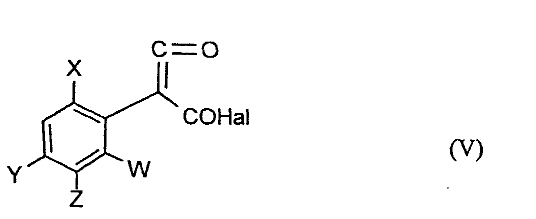 in welcher
W, X, Y und Z die in Anspruch 1 angegebenen Bedeutungen haben und
Hal für Chlor oder Brom steht.

15. Verbindungen der Formel (XXXVII) 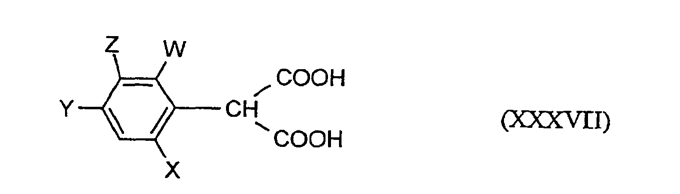 in welcher
W, X, Y und Z die in Anspruch 1 angegebenen Bedeutungen haben.

16. Verbindungen der Formel (VI)
in welcher
W, X, Y und Z die in Anspruch 1 angegebene Bedeutung haben und
R⁸ für Alkyl steht.

17. Verbindungen der Formel (X) 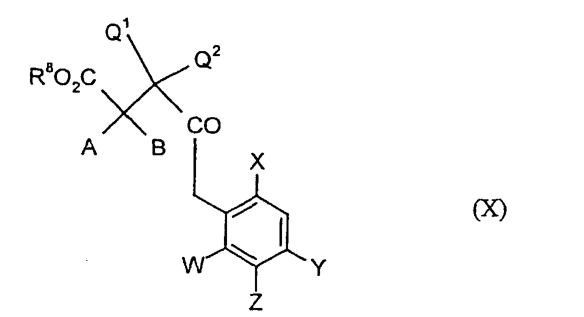 in welcher
A, B, Q¹, Q², W, X, Y und Z die in Anspruch 1 angegebene Bedeutung haben und
R⁸ für Alkyl steht.

18. Verbindungen der Formel (XXXVIII) 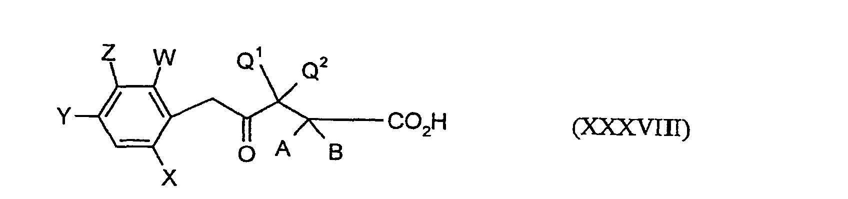 in welcher
W, X, Y, Z, A, B, Q¹ und Q² die in Anspruch 1 angegebene Bedeutung haben.

19. Verbindungen der Formel (XXXIX) 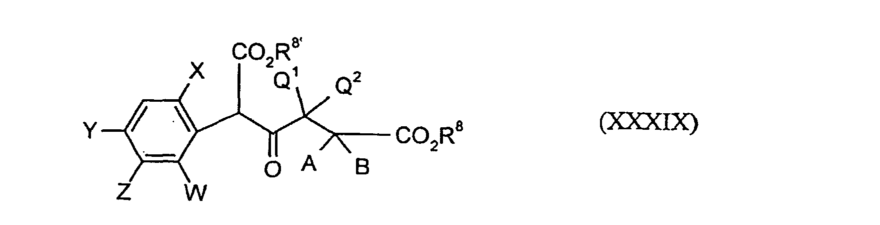 in welcher
A, B, D¹, D², W, X, Y und Z die in Anspruch 1 angegebene Bedeutung haben und
R⁸ und R^{8'} für Alkyl stehen.

20. Verbindungen der Formel (XI) 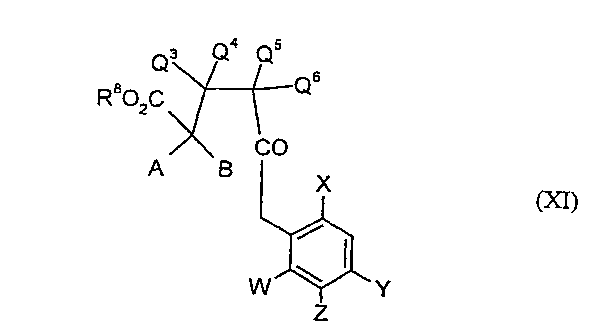 in welcher
A, B, Q³, Q⁴, Q⁵, Q⁶, W, X, Y und Z die in Anspruch 1 angegebene Bedeutung haben und
R⁸ für Alkyl steht.

21. Verbindungen der Formel (XLII) 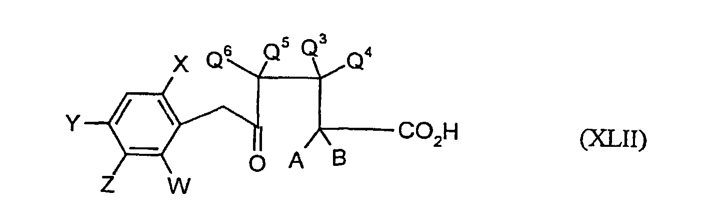 in welcher
A, B, Q³, Q⁴, Q⁵, Q⁶, W, X, Y und Z die in Anspruch 1 angegebene Bedeutung haben.

22. Verbindungen der Formel (XLIII) 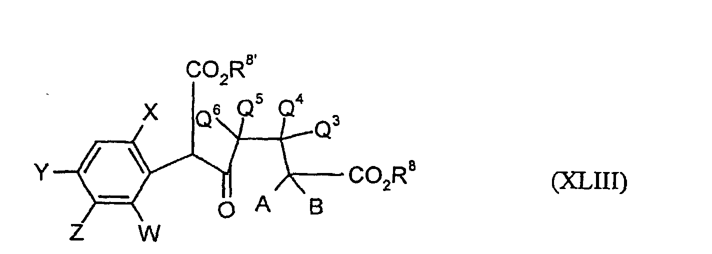 in welcher
A, B, Q³, Q⁴, Q⁵, Q⁶, W, X, Y und Z die in Anspruch 1 angegebene Bedeutung haben und
R⁸ und R^{8'} für Alkyl stehen.

23. Schädlingsbekämpfungsmittel und/oder Unkrautbekämpfungsmittel, **gekennzeichnet durch** einen Gehalt an mindestens einer Verbindung der Formel (I) gemäß Anspruch 1.

24. Verwendung von Verbindungen der Formel (I) gemäß Anspruch 1 zur Bekämpfung von Schädlingen im Pflanzenschutz, Haushaltsbereich, Hygienebereich und Vorratsschutz.

25. Verfahren zur Bekämpfung von Schädlingen im Pflanzenschutz, Haushaltsbereich, Hygienebereich und Vorratsschutz, ausgenommen am menschliden oder tierischen körper, **dadurch gekennzeichnet, daß** man Verbindungen der Formel (I) gemäß Anspruch 1 auf die Schädlinge und/oder ihren Lebensraum einwirken läßt.

26. Verfahren zur Herstellung von Schädlingsbekämpfungsmitteln und/oder Unkrautbekämpfungsmitteln, **dadurch gekennzeichnet, daß** man Verbindungen der Formel (I) gemäß Anspruch 1 mit Streckmitteln und/oder oberflächenaktiven Mitteln vermischt.

## Claims

1. Compounds of the formula (I) 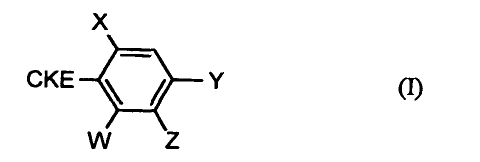 in which
X represents halogen, C₁-C₆-alkyl, C₁-C₆-halogenoalkyl, C₁-C₆-alkoxy, C₃-C₆-alkenyloxy, C₁-C₆-alkylthio, C₁-C₆-alkylsulphinyl, C₁-C₆-alkylsulphonyl, C₁-C₆-halogenoalkoxy, C₃-C₆-halogenoalkenyloxy, nitro, cyano or in each case optionally halogen-, C₁-C₆-alkyl-, C₁-C₆-alkoxy-, C₁-C₄-halogenoalkyl-, C₁-C₄-halogenoalkoxy-, nitro- or cyano-substituted phenyl, phenoxy, phenylthio, benzyloxy or benzylthio,
Z represents one of the radicals 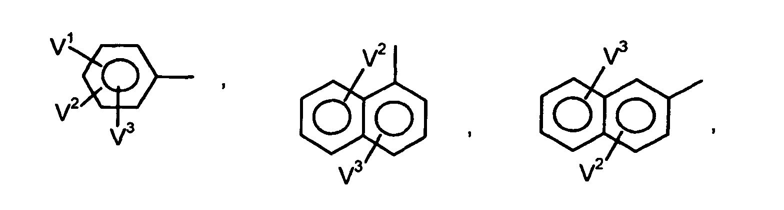 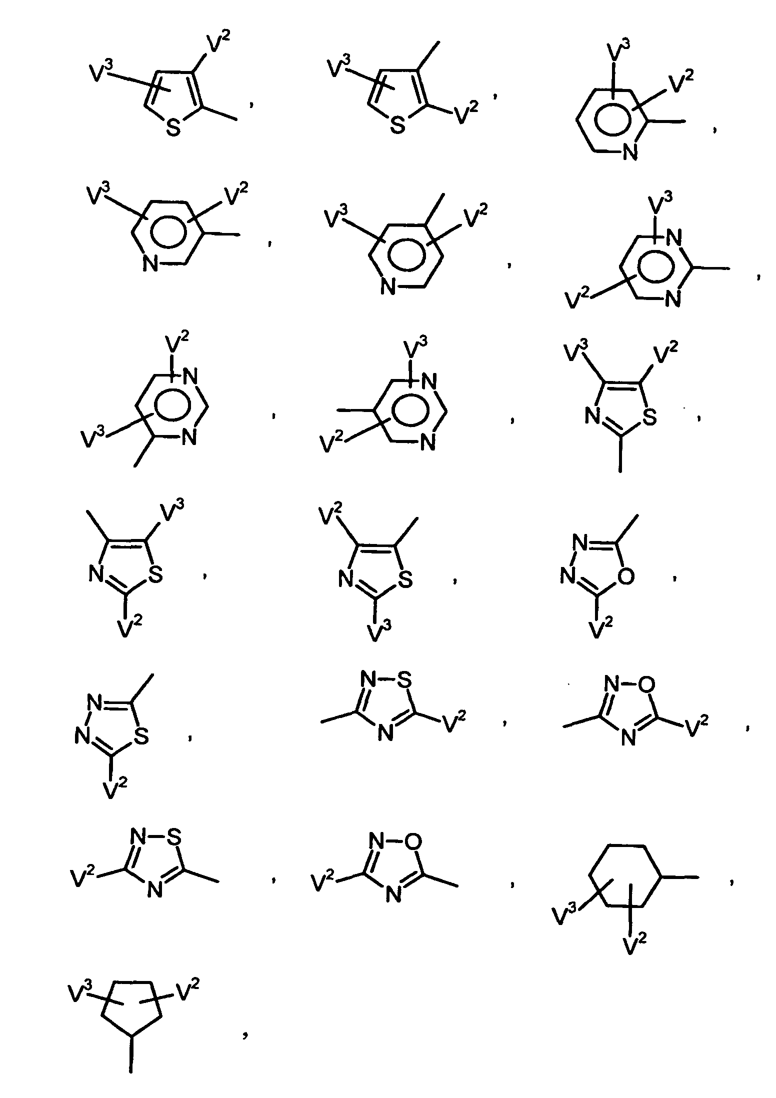
V¹ represents hydrogen, halogen, C₁-C₁₂-alkyl, C₁-C₆-alkoxy, C₁-C₆-alkylthio, C₁-C₆-alkylsulphinyl, C₁-C₆-alkylsulphonyl, C₁-C₄-halogenoalkyl, C₁-C₄-halogenoalkoxy, nitro, cyano or phenyl, phenoxy, phenoxy-C₁-C₄-alkyl, phenyl-C₁-C₄-alkoxy, phenylthio-C₁-C₄-alkyl or phenyl-C₁-C₄-alkylthio, each of which is optionally mono- or polysubstituted by halogen, C₁-C₆-alkyl, C₁-C₆-alkoxy, C₁-C₄-halogenoalkyl, C₁-C₄-halogenoalkoxy, nitro or cyano,
V² and V³ independently of one another each represent hydrogen, halogen. C₁-C₆-alkyl, C₁-C₆-alkoxy, C₁-C₄-halogenoalkyl or C₁-C₄-halogenoalkoxy,
W and Y independently of one another each represent hydrogen, halogen, C₁-C₆-alkyl, C₁-C₆-halogenoalkyl, C₁-C₆-alkoxy, C₁-C₆-halogenoalkoxy, nitro or cyano,
CKE represents one of the groups 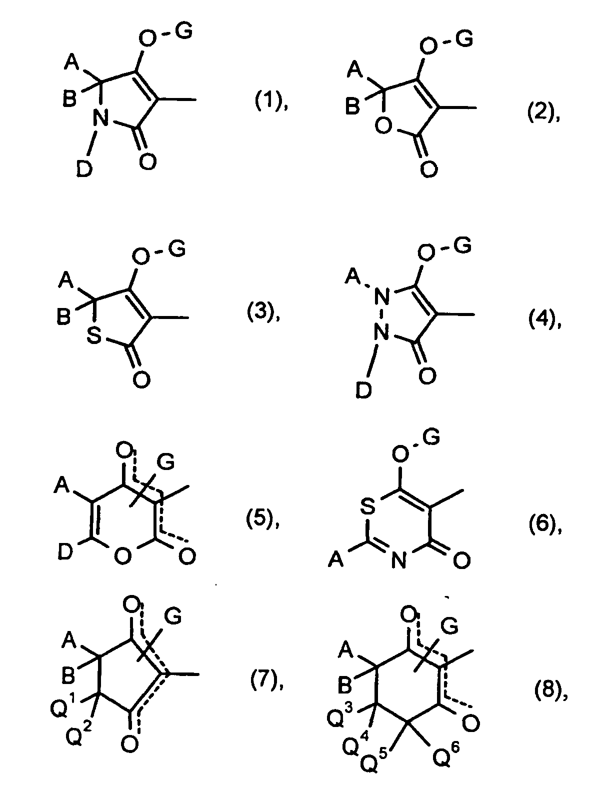
A represents hydrogen or in each case optionally halogen-substituted C₁-C₁₂-alkyl, C₃-C₈-alkenyl, C₁-C₁₀-alkoxy-C₁-C₈-alkyl, poly-C₁-C₈-alkoxy-C₁-C₈-alkyl, C₁-C₁₀-alkylthio-C₁-C₆-alkyl, optionally halogen-, C₁-C₆-alkyl- or C₁-C₆-alkoxy-substituted C₃-C₈-cycloalkyl in which optionally one or two not directly adjacent ring members are replaced by oxygen and/or sulphur or represents in each case optionally halogen-, C₁-C₆-alkyl-, C₁-C₆-halogenoalkyl-, C₁-C₆-alkoxy-, C₁-C₆-halogenoalkoxy-, cyano- or nitro-substituted C₆-or C₁₀-aryl, hetaryl having 5 or 6 ring atoms or C₆- or C₁₀-aryl-C₁-C₆-alkyl,
B represents hydrogen, C₁-C₁₂-alkyl or C₁-C₈-alkoxy-C₁-C₆-alkyl or
A, B and the carbon atom to which they are attached represent saturated C₃-C₁₀-cycloalkyl or unsaturated C₅-C₁₀-cycloalkyl in which optionally one ring member is replaced by oxygen or sulphur and which are optionally mono- or disubstituted by C₁-C₈-alkyl, C₃-C₁₀-cycloalkyl, C₁-C₈-halogenoalkyl, C₁-C₈-alkoxy, C₁-C₈-alkylthio, halogen or phenyl or
A, B and the carbon atom to which they are attached represent C₃-C₆-cycloalkyl which is substituted by an alkylenediyl group which optionally contains one or two not directly adjacent oxygen and/or sulphur atoms, or by an alkylenedioxyl group or by an alkylenedithioyl group which, together with the carbon atom to which it is attached, forms a further five- to eight-membered ring, or
A, B and the carbon atom to which they are attached represent C₃-C₈-cycloalkyl or C₅-C₈-cycloalkenyl, in which two substituents together with the carbon atoms to which they are attached represent in each case optionally C₁-C₆-alkyl-, C₁-C₆-alkoxy- or halogen-substituted C₂-C₆-alkanediyl, C₂-C₆-alkenediyl or C₄-C₆-alkanedienediyl in which optionally one methylene group is replaced by oxygen or sulphur,
D represents hydrogen, in each case optionally halogen-substituted C₁-C₁₂-alkyl, C₃-C₈-alkenyl, C₃-C₈-alkinyl, C₁-C₁₀-alkoxy-C₂-C₈-alkyl, poly-C₁-C₈-alkoxy-C₂-C₈-alkyl, C₁-C₁₀-alkylthio-C₂-C₈-alkyl, optionally halogen-, C₁-C₄-alkyl-, C₁-C₄-alkoxy- or C₁-C₄-halogenoalkyl-substituted C₃-C₈-cycloalkyl in which optionally one ring member is replaced by oxygen or sulphur or in each case optionally halogen-, C₁-C₆-alkyl-, C₁-C₆-halogenoalkyl-, C₁-C₆-alkoxy-, C₁-C₆-halogenoalkoxy-, cyano- or nitro-substituted phenyl, hetaryl having 5 or 6 ring atoms, phenyl-C₁-C₆-alkyl or hetaryl-C₁-C₆-alkyl having 5 or 6 ring atoms, or
A and D together represent in each case optionally substituted C₃-C₆-alkanediyl or C₃-C₆-alkenediyl in which optionally one methylene group is replaced by oxygen or sulphur,
possible substituents in each case being:
halogen, hydroxyl, mercapto or in each case optionally halogen-substituted C₁-C₁₀-alkyl, C₁-C₆-alkoxy, C₁-C₆-alkylthio, C₃-C₇-cycloalkyl, phenyl or benzyloxy, or a further C₃-C₆-alkanediyl group, C₃-C₆-alkenediyl group or a butadienyl group which is optionally substituted by C₁-C₆-alkyl or in which optionally two adjacent substituents together with the carbon atoms to which they are attached form a further saturated or unsaturated ring having 5 or 6 ring atoms (in the case of the compound of the formula (I-1), A and D, together with the atoms to which they are attached, then represent, for example, the groups AD-1 to AD-10 mentioned further below) which ring may contain oxygen or sulphur, or which may optionally contain one of the groups below 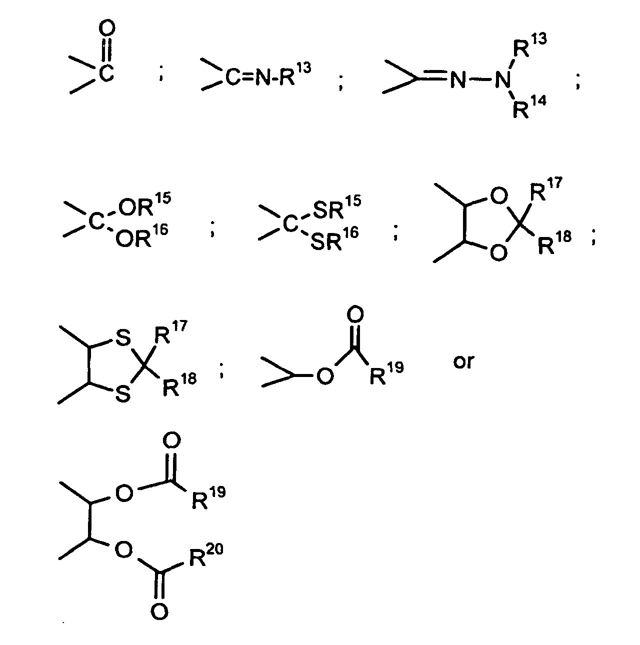
or
A and Q¹ together represent C₃-C₆-alkanediyl or C₄-C₆-alkenediyl, each of which is optionally mono- or disubstituted by identical or different substituents selected from the group consisting of halogen, hydroxyl; C₁-C₁₀-alkyl, C₁-C₆-alkoxy, C₁-C₆-alkylthio, C₃-C₇-cycloalkyl, each of which is optionally mono- to trisubstituted by identical or different halogens; and benzyloxy and phenyl, each of which is optionally mono- to trisubstituted by identical or different substituents selected from the group consisting of halogen, C₁-C₆-alkyl or C₁-C₆-alkoxy, and which furthermore optionally contains one of the groups below 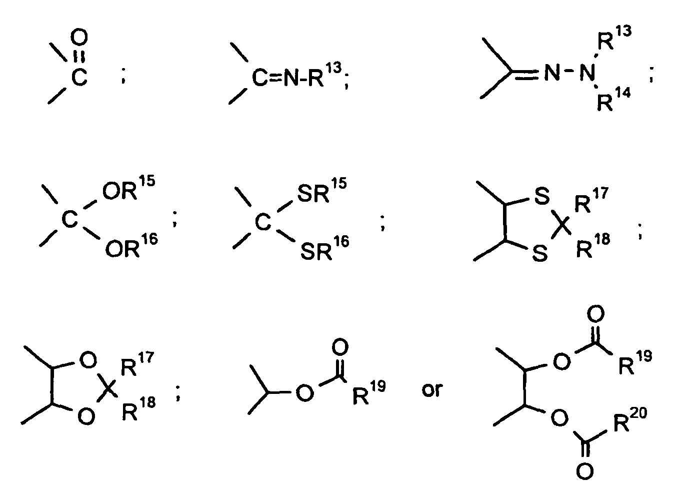 or is bridged by a C₁-C₂-alkanediyl group or by an oxygen atom, or
Q¹ represents hydrogen or C₁-C₄-alkyl,
Q², Q⁴, Q⁵ and Q⁶ independently of one another each represent hydrogen or C₁-C₄-alkyl,
Q³ represents hydrogen, C₁-C₆-alkyl, C₁-C₆-alkoxy-C₁-C₂-alkyl, C₁-C₆-alkylthio-C₁-C₂-alkyl, optionally C₁-C₄-alkyl- or C₁-C₄-alkoxy-substituted C₃-C₈-cycloalkyl in which optionally one methylene group is replaced by oxygen or sulphur or optionally halogen-, C₁-C₄-alkyl-, C₁-C₄-alkoxy-, C₁-C₂-halogenoalkyl-, C₁-C₂-halogenoalkoxy-, cyano- or nitro-substituted phenyl, or
Q³ and Q⁴ together with the carbon atom to which they are attached represent an optionally C₁-C₄-alkyl-, C₁-C₄-alkoxy- or C₁-C₂-halogenoalkyl-substituted C₃-C₇-ring in which optionally one ring member is replaced by oxygen or sulphur,
G represents hydrogen (a) or represents one of the groups 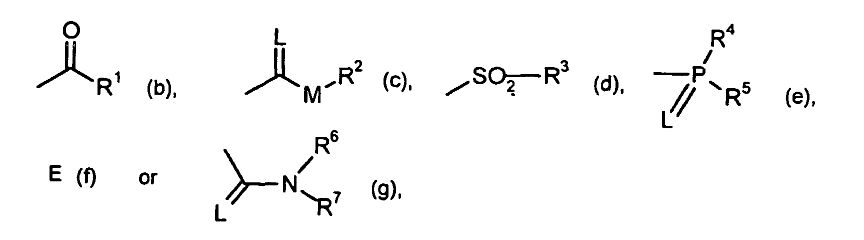 in which
E represents a metal ion or an ammonium ion,
L represents oxygen or sulphur and
M represents oxygen or sulphur,
R¹ represents in each case optionally halogen-substituted C₁-C₂₀-alkyl, C₂-C₂₀-alkenyl, C₁-C₈-alkoxy-C₁-C₈-alkyl, C₁-C₈-alkylthio-C₁-C₈-alkyl, poly-C₁-C₈-alkoxy-C₁-C₈-alkyl or optionally halogen-, C₁-C₆-alkyl- or C₁-C₆-alkoxy-substituted C₃-C₈-cycloalkyl in which optionally one or more not directly adjacent ring members are replaced by oxygen and/or sulphur,
represents optionally halogen-, cyano-, nitro-, C₁-C₆-alkyl-, C₁-C₆-alkoxy-, C₁-C₆-halogenoalkyl-, C₁-C₆-halogenoalkoxy-, C₁-C₆-alkylthio- or C₁-C₆-alkylsulphonyl-substituted phenyl,
represents optionally halogen-, nitro-, cyano-, C₁-C₆-alkyl-, C₁-C₆-alkoxy-, C₁-C₆-halogenoalkyl- or C₁-C₆-halogenoalkoxy-substituted phenyl-C₁-C₆-alkyl,
represents optionally halogen- or C₁-C₆-alkyl-substituted 5- or 6-membered hetaryl,
represents optionally halogen- or C₁-C₆-alkyl-substituted phenoxy-C₁-C₆-alkyl or
represents optionally halogen-, amino- or C₁-C₆-alkyl-substituted 5- or 6-membered hetaryloxy-C₁-C₆-alkyl,
R² represents in each case optionally halogen-substituted C₁-C₂₀-alkyl, C₂-C₂₀-alkenyl, C₁-C₈-alkoxy-C₂-C₈-alkyl, poly-C₁-C₈-alkoxy-C₂-C₈-alkyl,
represents optionally halogen-, C₁-C₆-alkyl- or C₁-C₆-alkoxy-substituted C₃-C₈-cycloalkyl or
represents in each case optionally halogen-, cyano-, nitro-, C₁-C₆-alkyl-, C₁-C₆-alkoxy-, C₁-C₆-halogenoalkyl- or C₁-C₆-halogenoalkoxy-substituted phenyl or benzyl,
R³ represents optionally halogen-substituted C₁-C₈-alkyl or represents in each case optionally halogen-, C₁-C₆-alkyl-, C₁-C₆-alkoxy-, C₁-C₄-halogenoalkyl-, C₁-C₄-halogenoalkoxy-, cyano- or nitro-substituted phenyl or benzyl,
R⁴ and R⁵ independently of one another each represent in each case optionally halogen-substituted C₁-C₈-alkyl, C₁-C₈-alkoxy, C₁-C₈-alkylamino, di-(C₁-C₈-alkyl)amino, C₁-C₈-alkylthio, C₂-C₈-alkenylthio, C₃-C₇-cycloalkylthio or represent in each case optionally halogen-, nitro-, cyano-, C₁-C₄-alkoxy-, C₁-C₄-halogenoalkoxy-, C₁-C₄-alkylthio-, C₁-C₄-halogenoalkylthio-, C₁-C₄-alkyl- or C₁-C₄-halogenoalkyl-substituted phenyl, phenoxy or phenylthio,
R⁶ and R⁷ independently of one another each represent hydrogen, represent in each case optionally halogen-substituted C₁-C₈-alkyl, C₃-C₈-cycloalkyl, C₁-C₈-alkoxy, C₃-C₈-alkenyl, C₁-C₈-alkoxy-C₁-C₈-alkyl, represent optionally halogen-, C₁-C₈-halogenoalkyl-, C₁-C₈-alkyl- or C₁-C₈-alkoxy-substituted phenyl, optionally halogen-, C₁-C₈-alkyl-, C₁-C₈-halogenoalkyl- or C₁-C₈-alkoxy-substituted benzyl or together represent an optionally C₁-C₄-alkyl-substituted C₃-C₆-alkylene radical in which optionally one methylene group is replaced by oxygen or sulphur,
R¹³ represents hydrogen, represents in each case optionally halogen-substituted C₁-C₈-alkyl or C₁-C₈-alkoxy, represents optionally halogen-, C₁-C₄-alkyl- or C₁-C₄-alkoxy-substituted C₃-C₈-cycloalkyl in which optionally one methylene group is replaced by oxygen or sulphur, or represents in each case optionally halogen-, C₁-C₆-alkyl-, C₁-C₆-alkoxy-, C₁-C₄-halogenoalkyl-, C₁-C₄-halogenoalkoxy-, nitro- or cyano-substituted phenyl, phenyl-C₁-C₄-alkyl or phenyl-C₁-C₄-alkoxy,
R¹⁴ represents hydrogen or C₁-C₈-alkyl, or
R¹³ and R¹⁴ together represent C₄-C₆-alkanediyl,
R¹⁵ and R¹⁶ are identical or different and each represent C₁-C₆-alkyl, or
R¹⁵ and R¹⁶ together represent a C₂-C₄-alkanediyl radical which is optionally substituted by C₁-C₆-alkyl, C₁-C₆-halogenoalkyl or by optionally halogen-, C₁-C₆-alkyl-, C₁-C₄-halogenoalkyl-, C₁-C₆-alkoxy-, C₁-C₄-halogenoalkoxy-, nitro- or cyano-substituted phenyl,
R¹⁷ and R¹⁸ independently of one another each represent hydrogen, represent optionally halogen-substituted C₁-C₈-alkyl or represent optionally halogen-, C₁-C₆-alkyl-, C₁-C₆-alkoxy-, C₁-C₄-halogenoalkyl-, C₁-C₄-halogenoalkoxy-, nitro- or cyano-substituted phenyl, or
R¹⁷ and R¹⁸ together with the carbon atom to which they are attached represent a carbonyl group or represent optionally halogen-, C₁-C₄-alkyl- or C₁-C₄-alkoxy-substituted C₅-C₇-cycloalkyl in which optionally one methylene group is replaced by oxygen or sulphur and
R¹⁹ and R²⁰ independently of one another each represent C₁-C₁₀-alkyl, C₂-C₁₀-alkenyl, C₁-C₁₀-alkoxy, C₁-C₁₀-alkylamino, C₃-C₁₀-alkenylamino, di-(C₁-C₁₀-alkyl)amino or di-(C₃-C₁₀-alkenyl)amino.

2. Compounds of the formula (I) according to Claim 1 in which
X represents fluorine, chlorine, bromine, C₁-C₄-alkyl, C₁-C₄-alkoxy, C₃-C₄-alkenyloxy, C₁-C₄-halogenoalkyl, C₁-C₄-halogenoalkoxy, C₃-C₄-halogenoalkenyloxy, nitro or cyano,
Z represents one of the radicals 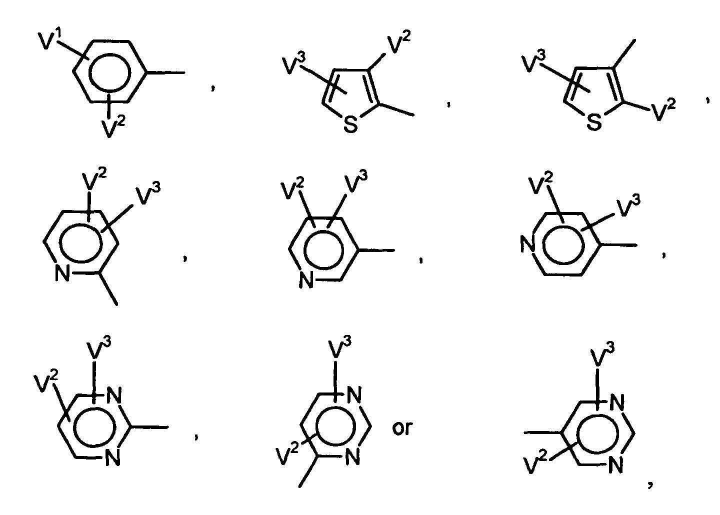
V¹ represents hydrogen, fluorine, chlorine, bromine, C₁-C₆-alkyl, C₁-C₄-alkoxy, C₁-C₂-halogenoalkyl, C₁-C₂-halogenoalkoxy, nitro, cyano or phenyl, phenoxy, phenoxy-C₁-C₂-alkyl, phenyl-C₁-C₂-alkoxy, phenylthio-C₁-C₂-alkyl or phenyl-C₁-C₂-alkylthio, each of which is optionally mono- or disubstituted by fluorine, chlorine, bromine, C₁-C₄-alkyl, C₁-C₄-alkoxy, C₁-C₂-halogenoalkyl, C₁-C₂-halogenoalkoxy, nitro or cyano,
V² and V³ independently of one another each represent hydrogen, fluorine, chlorine, bromine, C₁-C₄-alkyl, C₁-C₄-alkoxy, C₁-C₂-halogenoalkyl or C₁-C₂-halogenoalkoxy,
W and Y independently of one another each represent hydrogen, fluorine, chlorine, bromine, C₁-C₄-alkyl, C₁-C₄-halogenoalkyl, C₁-C₄-alkoxy or C₁-C₄-halogenoalkoxy,
CKE represents one of the groups 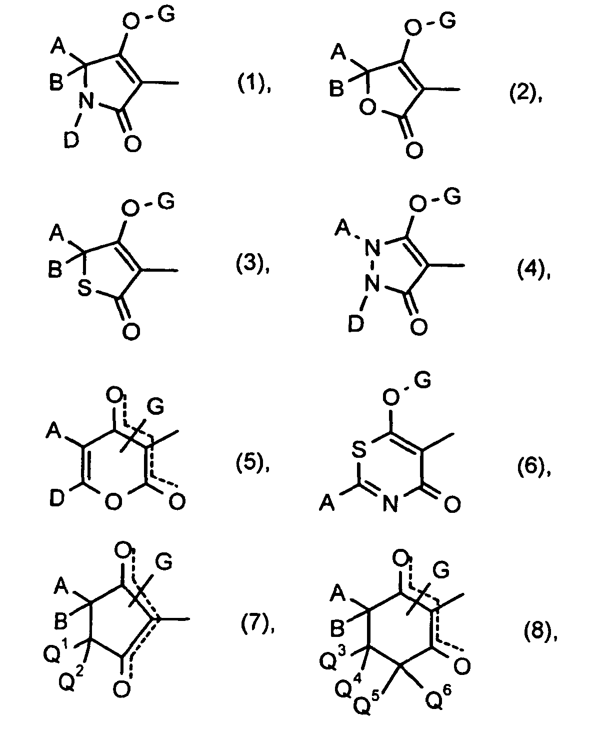
A represents hydrogen or represents in each case optionally fluorine- or chlorine-substituted C₁-C₁₀-alkyl, C₁-C₈-alkoxy-C₁-C₆-alkyl, optionally fluorine-, chlorine-, C₁-C₄-alkyl- or C₁-C₄-alkoxy-substituted C₃-C₇-cycloalkyl in which optionally one ring member is replaced by oxygen or sulphur or (but not in the case of the compounds of the formulae (I-5), (I-7) and (I-8)) in each case optionally fluorine-, chlorine-, bromine-, C₁-C₄-alkyl-, C₁-C₄-halogenoalkyl-, C₁-C₄-alkoxy- or C₁-C₄-halogenoalkoxy-substituted phenyl, furanyl, pyridyl, imidazolyl, triazolyl, pyrazolyl, pyrimidyl, thiazolyl, thienyl or phenyl-C₁-C₄-alkyl,
B represents hydrogen or C₁-C₆-alkyl, or
A,B and the carbon atom to which they are attached represent saturated or unsaturated C₅-C₇-cycloalkyl in which optionally one ring member is replaced by oxygen or sulphur and which is optionally monosubstituted by C₁-C₆-alkyl, C₅-C₈-cycloalkyl, C₁-C₃-halogenoalkyl, C₁-C₆-alkoxy, fluorine, chlorine or phenyl, or
A,B and the carbon atom to which they are attached represent C₅-C₆-cycloalkyl which is substituted by an alkylenediyl group which optionally contains one or two not directly adjacent oxygen or sulphur atoms or by an alkylenedioxyl group or by an alkylenedithiol group which, together with the carbon atom to which it is attached, forms a further five- or six-membered ring, or
A, B and the carbon atom to which they are attached represent C₃-C₆-cycloalkyl or C₅-C₆-cycloalkenyl in which two substituents together with the carbon atoms to which they are attached represent in each case optionally C₁-C₅-alkyl-, C₁-C₅-alkoxy-, fluorine-, chlorine- or bromine-substituted C₂-C₄-alkanediyl or C₂-C₄-alkenediyl, in which optionally one methylene group is replaced by oxygen or sulphur, or represent butadienediyl,
D represents hydrogen, represents in each case optionally fluorine- or chlorine-substituted C₁-C₁₀-alkyl, C₃-C₆-alkenyl, C₁-C₈-alkoxy-C₂-C₆-alkyl or C₁-C₈-alkylthio-C₂-C₆-alkyl, represents optionally fluorine-, chlorine-, C₁-C₄-alkyl-, C₁-C₄-alkoxy- or C₁-C₂-halogenoalkyl-substituted C₃-C₇-cycloalkyl in which optionally one methylene group is replaced by oxygen or sulphur or (but not in the case of the compounds of the formulae (I-1) and (I-4)) represents in each case optionally fluorine-, chlorine-, bromine-, C₁-C₄-alkyl-, C₁-C₄-halogenoalkyl-, C₁-C₄-alkoxy- or C₁-C₄-halogenoalkoxy-substituted phenyl, furanyl, imidazolyl, pyridyl, thiazolyl, pyrazolyl, pyrimidyl, pyrrolyl, thienyl, triazolyl orphenyl-C₁-C₄-alkyl, or
A and D together represent optionally substituted C₃-C₅-alkanediyl in which one methylene group may be replaced by a carbonyl group, oxygen or sulphur, possible substituents being hydroxyl, C₁-C₆-alkyl or C₁-C₄-alkoxy, or
A and D (in the case of the compounds of the formula (I-1)) together with the atoms to which they are attached represent one of the groups AD-1 to AD-10: 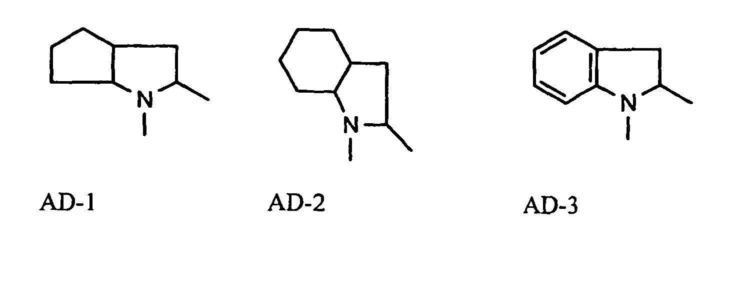 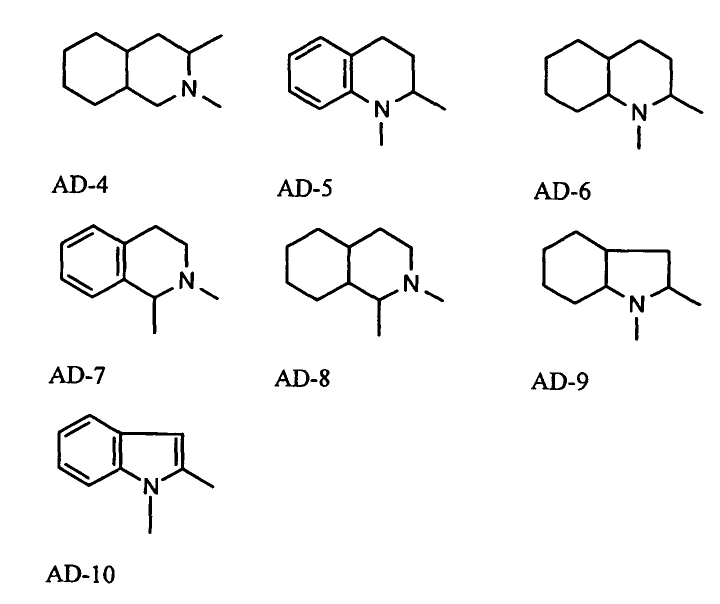 or
A and Q¹ together represent C₃-C₄-alkanediyl or C₃-C₄-alkenediyl, each of which is optionally mono- or disubstituted by identical or different substituents selected from the group consisting of fluorine, chlorine, hydroxyl, and C₁-C₈-alkyl and C₁-C₄-alkoxy, each of which is optionally mono- to trisubstituted by fluorine, or
Q¹ represents hydrogen,
Q² represents hydrogen,
Q⁴, Q⁵ and Q⁶ independently of one another each represent hydrogen or C₁-C₃-alkyl,
Q³ represents hydrogen, C₁-C₄-alkyl, C₁-C₄-alkoxy-C₁-C₂-alkyl, C₁-C₄-alkylthio-C₁-C₂-alkyl or optionally methyl- or methoxy-substituted C₃-C₆-cycloalkyl in which optionally one methylene group is replaced by oxygen or sulphur, or
Q³ and Q⁴ together with the carbon atom to which they are attached represent an optionally C₁-C₄-alkyl- or C₁-C₄-alkoxy-substituted saturated C₅-C₆-ring in which optionally one ring member is replaced by oxygen or sulphur,
G represents hydrogen (a) or represents one of the groups 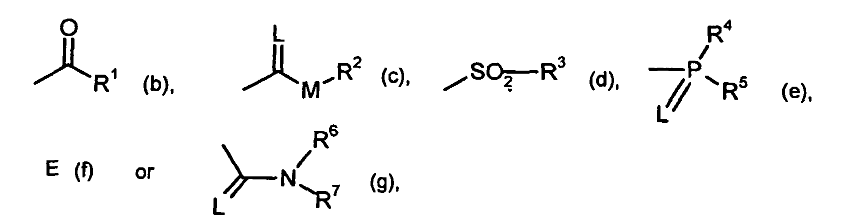 in which
E represents a metal ion or an ammonium ion,
L represents oxygen or sulphur and
M represents oxygen or sulphur,
R¹ represents in each case optionally fluorine- or chlorine-substituted C₁-C₁₆-alkyl, C₂-C₁₆-alkenyl, C₁-C₆-alkoxy-C₁-C₆-alkyl, C₁-C₆-alkylthio-C₁-C₆-alkyl, poly-C₁-C₆-alkoxy-C₁-C₆-alkyl or optionally fluorine-, chlorine-, C₁-C₅-alkyl- or C₁-C₅-alkoxy-substituted C₃-C₇-cycloalkyl in which optionally one or two not directly adjacent ring members are replaced by oxygen and/or sulphur,
represents optionally fluorine-, chlorine-, bromine-, cyano-, nitro-, C₁-C₄-alkyl-, C₁-C₄-alkoxy-, C₁-C₃-halogenoalkyl-, C₁-C₃-halogenoalkoxy-, C₁-C₄-alkylthio- or C₁-C₄-alkylsulphonyl-substituted phenyl,
represents optionally fluorine-, chlorine-, bromine-, C₁-C₄-alkyl-, C₁-C₄-alkoxy-, C₁-C₃-halogenoalkyl- or C₁-C₃-halogenoalkoxy-substituted phenyl-C₁-C₄-alkyl,
represents in each case optionally fluorine-, chlorine-, bromine- or C₁-C₄-alkyl-substituted pyrazolyl, thiazolyl, pyridyl, pyrimidyl, furanyl or thienyl,
represents optionally fluorine-, chlorine-, bromine- or C₁-C₄-alkylsubstituted phenoxy-C₁-C₃-alkyl or
represents in each case optionally fluorine-, chlorine-, bromine-, amino- or C₁-C₄-alkyl-substituted pyridyloxy-C₁-C₃-alkyl, pyrimidyloxy-C₁-C₃-alkyl or thiazolyloxy-C₁-C₃-alkyl,
R² represents in each case optionally fluorine-substituted C₁-C₁₆-alkyl, C₂-C₁₆-alkenyl, C₁-C₆-alkoxy-C₂-C₆-alkyl or poly-C₁-C₆-alkoxy-C₂-C₆-alkyl,
represents optionally fluorine-, chlorine-, C₁-C₄-alkyl- or C₁-C₄-alkoxy-substituted C₃-C₇-cycloalkyl or
represents in each case optionally fluorine-, chlorine-, bromine-, cyano-, nitro-, C₁-C₄-alkyl-, C₁-C₃-alkoxy-, C₁-C₃-halogenoalkyl- or C₁-C₃-halogenoalkoxy-substituted phenyl or benzyl,
R³ represents optionally fluorine-substituted C₁-C₆-alkyl or represents in each case optionally fluorine-, chlorine-, bromine-, C₁-C₄-alkyl-, C₁-C₄-alkoxy-, C₁-C₃-halogenoalkyl-, C₁-C₃-halogenoalkoxy-, cyano- or nitro-substituted phenyl or benzyl,
R⁴ and R⁵ independently of one another each represent C₁-C₆-alkyl, C₁-C₆-alkoxy, C₁-C₆-alkylamino, di-(C₁-C₆-alkyl)amino, C₁-C₆-alkylthio, C₃-C₄-alkenylthio, C₃-C₆-cycloalkylthio or represent in each case optionally fluorine-, chlorine-, bromine-, nitro-, cyano-, C₁-C₃-alkoxy-, C₁-C₃-halogenoalkoxy-, C₁-C₃-alkylthio-, C₁-C₃-halogenoalkylthio-, C₁-C₃-alkyl- or C₁-C₃-halogenoalkyl-substituted phenyl, phenoxy or phenylthio, and
R⁶ and R⁷ independently of one another each represent hydrogen, C₁-C₆-alkyl, C₃-C₆-cycloalkyl, C₁-C₆-alkoxy, C₃-C₆-alkenyl, C₁-C₆-alkoxy-C₁-C₆-alkyl, represent optionally fluorine-, chlorine-, bromine-, C₁-C₃-halogenoalkyl-, C₁-C₄-alkyl- or C₁-C₄-alkoxy-substituted phenyl, represent optionally fluorine-, chlorine-, bromine-, C₁-C₄-alkyl-, C₁-C₃-halogenoalkyl- or C₁-C₄-alkoxy-substituted benzyl, or together represent an optionally methyl- or ethyl-substituted C₄-C₅-alkylene radical in which optionally one methylene group is replaced by oxygen or sulphur.

3. Compounds of the formula (I) according to Claim 1 in which
X represents fluorine, chlorine, methyl, ethyl, propyl, iso-propyl, methoxy, ethoxy, propoxy, iso-propoxy, trifluoromethyl, difluoromethoxy, trifluoromethoxy, nitro or cyano,
Z represents one of the radicals 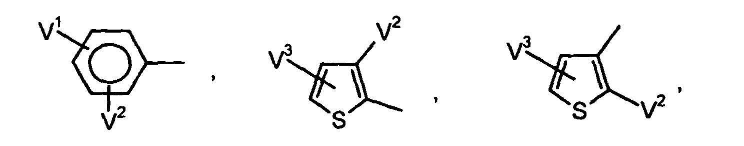 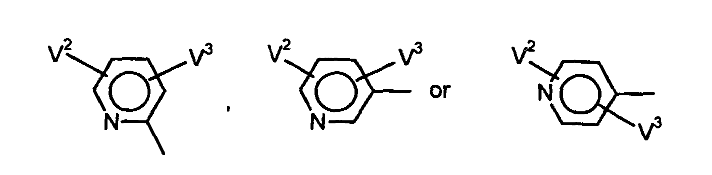
V¹ represents hydrogen, fluorine, chlorine, bromine, methyl, ethyl, n-propyl, iso-propyl, n-butyl, iso-butyl, tert-butyl, methoxy, ethoxy, n-propoxy, iso-propoxy, trifluoromethyl, trifluoromethoxy, nitro, cyano or phenyl which is optionally monosubstituted by fluorine, chlorine, methyl, methoxy, trifluoromethyl or trifluoromethoxy,
V² and V³ independently of one another each represent hydrogen, fluorine, chlorine, methyl, ethyl, n-propyl, iso-propyl, methoxy, ethoxy, trifluoromethyl or trifluoromethoxy,
W and Y independently of one another each represent hydrogen, fluorine, chlorine, methyl, ethyl, n-propyl, methoxy, ethoxy or propoxy,
CKE represents one of the groups 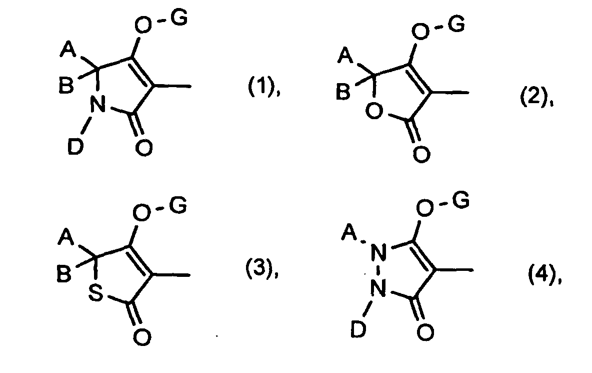 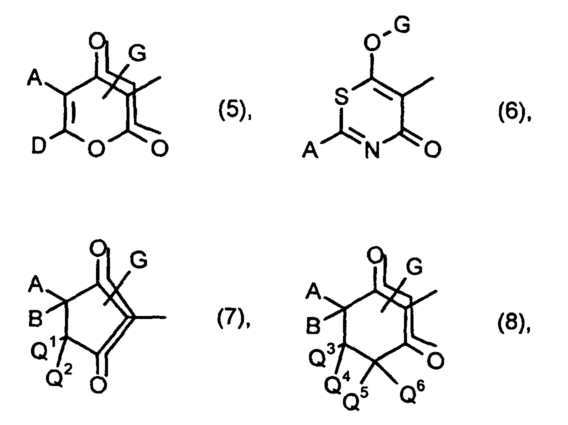
A represents hydrogen or represents in each case optionally fluorine-substituted C₁-C₈-alkyl or C₁-C₆-alkoxy-C₁-C₄-alkyl, optionally fluorine-, methyl-, ethyl- or methoxy-substituted C₃-C₆-cycloalkyl in which optionally one ring member is replaced by oxygen or sulphur or (but not in the case of the compounds of the formulae (I-5), (I-7) and (1-8)) represents in each case optionally fluorine-, chlorine-, bromine-, methyl-, ethyl-, n-propyl-, iso-propyl-, methoxy-, ethoxy-, trifluoromethyl-, trifluoromethoxy-, cyano- or nitro-substituted phenyl or benzyl,
B represents hydrogen or C₁-C₄-alkyl, or
A, B and the carbon atom to which they are attached represent saturated C₅-C₆-cycloalkyl in which optionally one ring member is replaced by oxygen or sulphur and which is optionally monosubstituted by methyl, ethyl, propyl, isopropyl, butyl, iso-butyl, sec-butyl, tert-butyl, trifluoromethyl, methoxy, ethoxy, propoxy, iso-propoxy, butoxy, iso-butoxy, sec-butoxy, tert-butoxy, fluorine or chlorine, or
A, B and the carbon atom to which they are attached represent C₅-C₆-cycloalkyl or C₅-C₆-cycloalkenyl in which two substituents together with the carbon atoms to which they are attached represent C₂-C₄-alkanediyl or C₂-C₄-alkenediyl in which in each case optionally one methylene group is replaced by oxygen or sulphur, or represent butadienediyl,
D represents hydrogen, represents in each case optionally fluorine- or chlorine-substituted C₁-C₈-alkyl, C₃-C₄-alkenyl, C₁-C₆-alkoxy-C₂-C₄-alkyl, C₁-C₄-alkylthio-C₂-C₄-alkyl or C₃-C₆-cycloalkyl in which optionally one methylene group is replaced by oxygen or sulphur or (but not in the case of the compounds of the formulae (I-1) and (I-4)) represents in each case optionally fluorine-, chlorine-, methyl-, ethyl-, n-propyl-, iso-propyl-, methoxy-, ethoxy-, trifluoromethyl- or trifluoromethoxy-substituted phenyl, furanyl, pyridyl, thienyl or benzyl,
or
A and D together represent optionally substituted C₃-C₄-alkanediyl in which optionally one carbon atom is replaced by sulphur and which is optionally substituted by hydroxyl, methyl, ethyl, methoxy or ethoxy, or
A and D (in the case of the compounds of the formula (I-1)) together with the atoms to which they are attached represent one of the following groups AD: 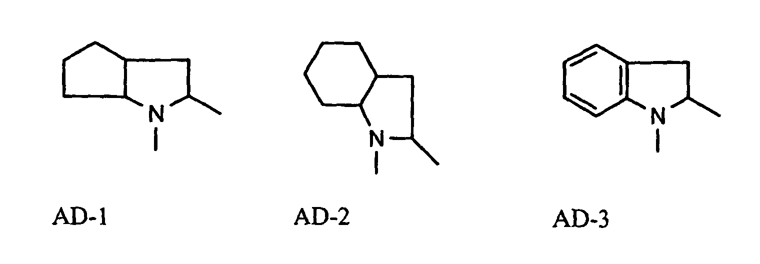 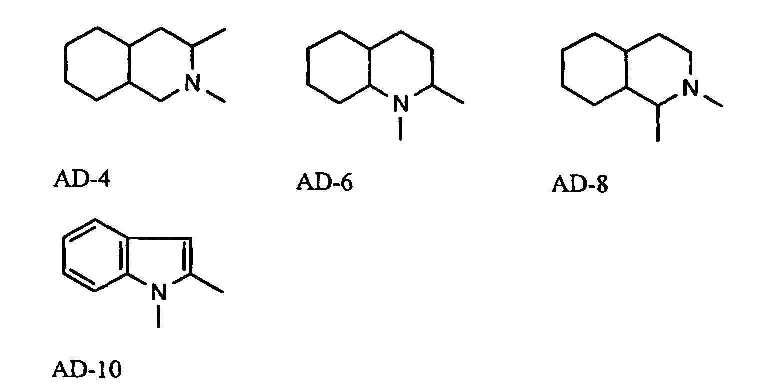
A and Q¹ together represent C₃-C₄-alkanediyl or butenediyl, each of which is optionally mono- or disubstituted by fluorine, hydroxyl, methyl or methoxy, or
Q¹ represents hydrogen,
Q² represents hydrogen,
Q⁴, Q⁵ and Q⁶ independently of one another each represent hydrogen, methyl or ethyl,
Q³ represents hydrogen, methyl, ethyl or C₃-C₆-cycloalkyl in which optionally one methylene group is replaced by oxygen or sulphur, or
Q³ and Q⁴ together with the carbon atom to which they are attached represent an optionally methyl- or methoxy-substituted saturated C₅-C₆-ring in which optionally one ring member is replaced by oxygen or sulphur,
G represents hydrogen (a) or represents one of the groups 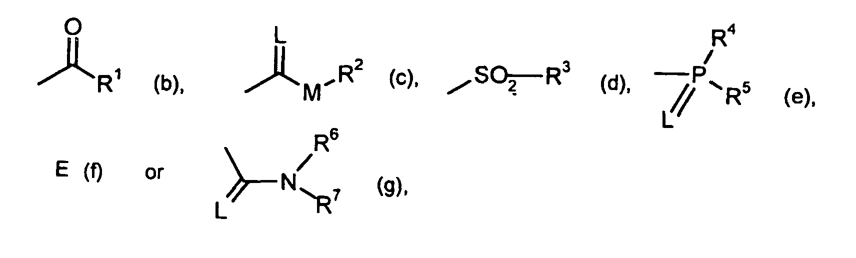 in which
E represents a metal ion or an ammonium ion,
L represents oxygen or sulphur and
M represents oxygen or sulphur,
R¹ represents in each case optionally fluorine- or chlorine-substituted C₁-C₁₄-alkyl, C₂-C₁₄-alkenyl, C₁-C₄-alkoxy-C₁-C₆-alkyl, C₁-C₄-alkylthio-C₁-C₆-alkyl, poly-C₁-C₄-alkoxy-C₁-C₄-alkyl or optionally fluorine-, chlorine-, methyl-, ethyl-, propyl-, i-propyl-, butyl-, i-butyl-, tert-butyl-, methoxy-, ethoxy-, n-propoxy- or iso-propoxy-substituted C₃-C₆-cycloalkyl in which optionally one or two not directly adjacent ring members are replaced by oxygen and/or sulphur,
represents optionally fluorine-, chlorine-, bromine-, cyano-, nitro-, methyl-, ethyl-, n-propyl-, i-propyl-, methoxy-, ethoxy-, trifluoromethyl-, trifluoromethoxy-, methylthio-, ethylthio-, methylsulphonyl- or ethylsulphonyl-substituted phenyl,
represents optionally fluorine-, chlorine-, bromine-, methyl-, ethyl-, n-propyl-, i-propyl-, methoxy-, ethoxy-, trifluoromethyl- or trifluoromethoxy-substituted benzyl,
represents in each case optionally fluorine-, chlorine-, bromine-, methyl- or ethyl-substituted furanyl, thienyl, pyridyl, pyrimidyl, thiazolyl or pyrazolyl,
represents optionally fluorine-, chlorine-, methyl- or ethyl-substituted phenoxy-C₁-C₂-alkyl or
represents in each case optionally fluorine-, chlorine-, amino-, methyl- or ethyl-substituted pyridyloxy-C₁-C₂-alkyl, pyrimidyloxy-C₁-C₂-alkyl or thiazolyloxy-C₁-C₂-alkyl,
R² represents in each case optionally fluorine-substituted C₁-C₁₄-alkyl, C₂-C₁₄-alkenyl, C₁-C₄-alkoxy-C₂-C₆-alkyl or poly-C₁-C₄-alkoxy-C₂-C₆-alkyl,
represents optionally fluorine-, chlorine-, methyl-, ethyl-, n-propyl-, iso-propyl- or methoxy-substituted C₃-C₆-cycloalkyl,
or represents in each case optionally fluorine-, chlorine-, cyano-, nitro-, methyl-, ethyl-, n-propyl-, i-propyl-, methoxy-, ethoxy-, trifluoromethyl- or trifluoromethoxy-substituted phenyl or benzyl,
R³ represents in each case optionally fluorine-substituted methyl, ethyl, n-propyl, isopropyl or in each case optionally fluorine-, chlorine-, bromine-, methyl-, tert-butyl-, methoxy-, trifluoromethyl-, trifluoromethoxy-, cyano- or nitro-substitued phenyl or benzyl,
R⁴ and R⁵ independently of one another each represent C₁-C₄-alkyl, C₁-C₄-alkoxy, C₁-C₄-alkylamino, di-(C₁-C₄-alkyl)amino, C₁-C₄-alkylthio or represent in each case optionally fluorine-, chlorine-, bromine-, nitro-, cyano-, C₁-C₂-alkoxy-, C₁-C₂-fluoroalkoxy-, C₁-C₂-alkylthio-, C₁-C₂-fluoroalkylthio- or C₁-C₃-alkyl-substituted phenyl, phenoxy or phenylthio, and
R⁶ and R⁷ independently of one another each represent hydrogen, represent C₁-C₄-alkyl, C₃-C₆-cycloalkyl, C₁-C₄-alkoxy, C₃-C₄-alkenyl, C₁-C₄-alkoxy-C₁-C₄-alkyl, represent optionally fluorine-, chlorine-, bromine-, trifluoromethyl-, methyl- or methoxy-substituted phenyl, represent optionally fluorine-, chlorine-, bromine-, methyl-, trifluoromethyl- or methoxy-substituted benzyl, or together represent a C₅-C₆-alkylene radical in which optionally one methylene group is replaced by oxygen or sulphur.

4. Process for preparing compounds of the formula (I) according to Claim 1, **characterized in that**
(A) Compounds of the formula (I-1-a) 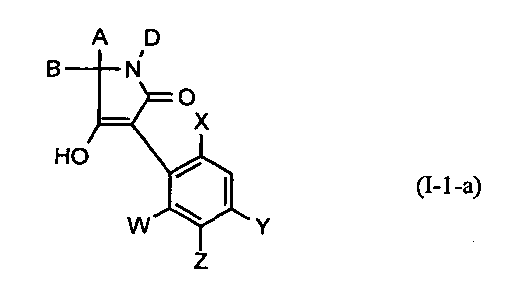 in which
A, B, D, W, X, Y and Z are each as defined in Claim 1
are obtained when
N-acylamino acid esters of the formula (II) 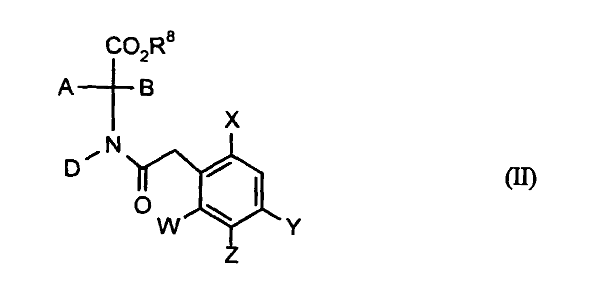 in which
A, B, D, W, X, Y and Z are each as defined above
and
R⁸ represents alkyl
are condensed intramolecularly in the presence of a diluent and in the presence of a base,
(B) Compounds of the formula (I-2-a) 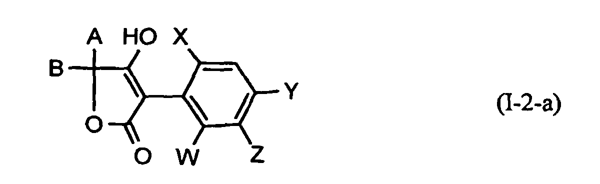 in which
A, B, W, X, Y and Z are each as defined above
are obtained when
carboxylic esters of the formula (III) 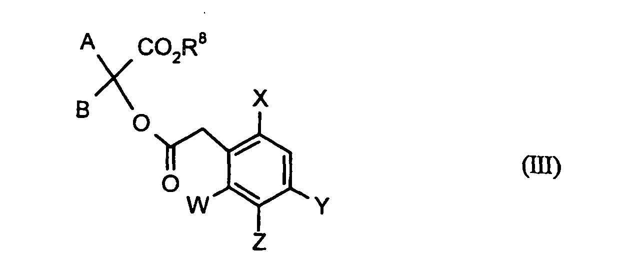 in which
A, B, W, X, Y, Z and R⁸ are each as defined above
are condensed intramolecularly in the presence of a diluent and in the presence of a base,
(C) Compounds of the formula (I-3-a) 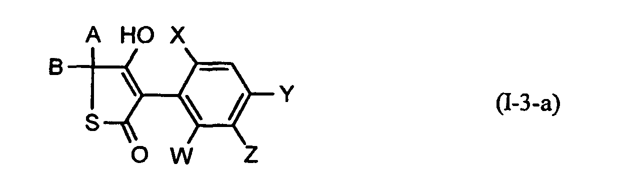 in which
A, B, W, X, Y and Z are each as defined above
are obtained when
β-ketocarboxylic esters of the formula (IV) 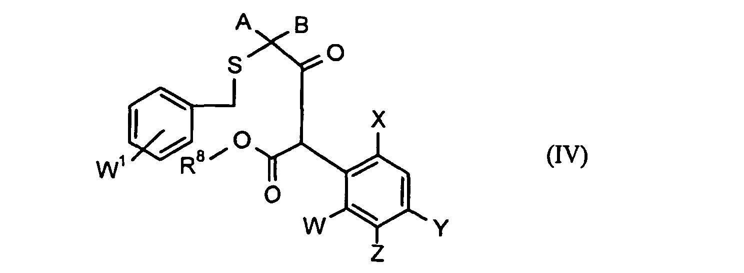 in which
A, B, W, X, Y, Z and R⁸ are each as defined above and
W¹ represents hydrogen, halogen, alkyl or alkoxy
are cyclized intramolecularly, if appropriate in the presence of a diluent and in the presence of an acid,
(D) Compounds of the formula (I-4-a) 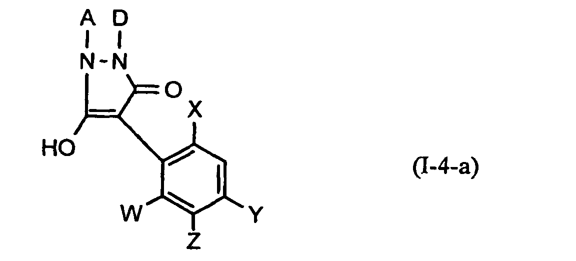 in which
A, D, W, X, Y and Z are each as defined above
are obtained when
(α) halogenocarbonyl ketenes of the formula (V) 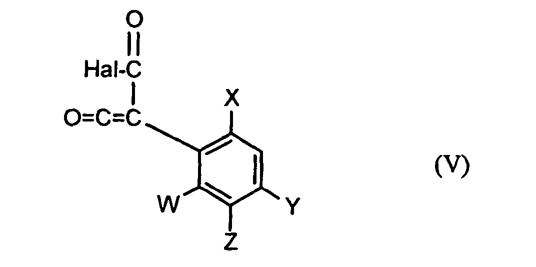 in which
W, X, Y and Z are each as defined above
and
Hal represents halogen
or
(β) malonic acid derivatives of the formula (VI) 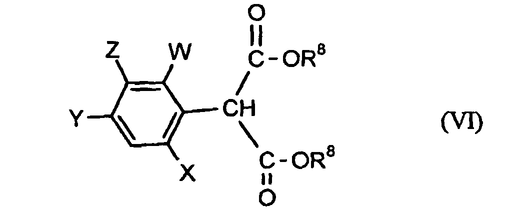 in which
R⁸, W, X, Y and Z are each as defined above
are reacted with hydrazines of the formula (VII)
A-NH-NH-D (VII)
in which
AandD are each as defined above
if appropriate in the presence of a diluent and if appropriate in the presence of a base,
(E) Compounds of the formula (I-5-a) 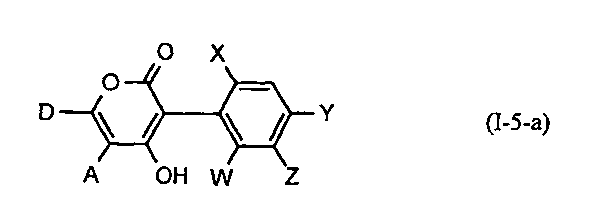 in which
A, D, W, X, Y and Z are each as defined above,
are obtained when
carbonyl compounds of the formula (VIII) 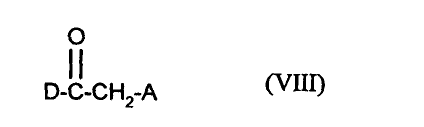 in which
AandD are each as defined above
or their silyl enol ethers of the formula (VIIIa) 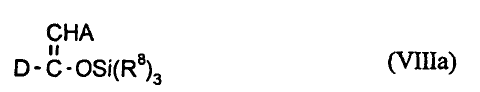 in which
A, D and R⁸ are each as defined above
are reacted with ketene acid halides of the formula (V) 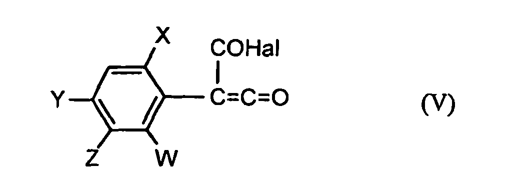 in which
W, X, Y and Z are each as defined above and
Hal represents halogen,
if appropriate in the presence of a diluent and if appropriate in the presence of an acid acceptor,
(F) Compounds of the formula (I-6-a) 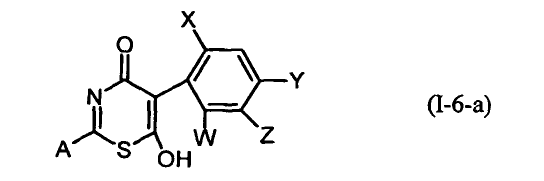 in which
A, X, Y and Z are each as defined above
are obtained when thioamides of the formula (IX) 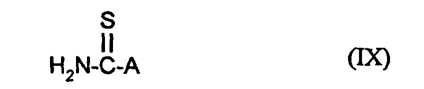 in which
A is as defined above
are reacted with ketene acid halides of the formula (V) 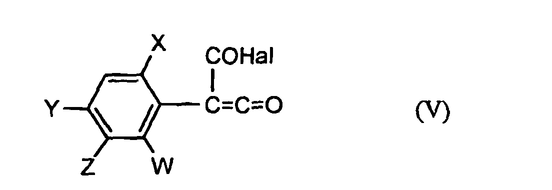 in which
Hal, W, X, Y and Z are each as defined above,
if appropriate in the presence of a diluent and if appropriate in the presence of an acid acceptor,
(G) Compounds of the formula (I-7-a) 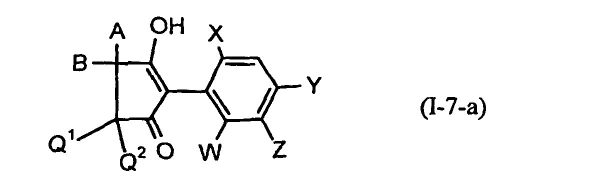 in which
A, B, Q¹, Q², W, X, Y and Z are each as defined in Claim 1
are obtained when
ketocarboxylic esters of the formula (X) 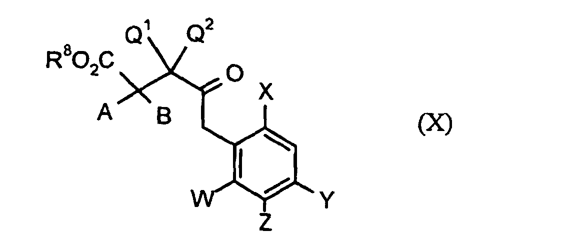 in which
A, B, Q¹, Q², W, X, Y and Z are each as defined above and
R⁸ represents alkyl
are cyclized intramolecularly, if appropriate in the presence of a diluent and in the presence of a base,
(H) Compounds of the formula (I-8-a) 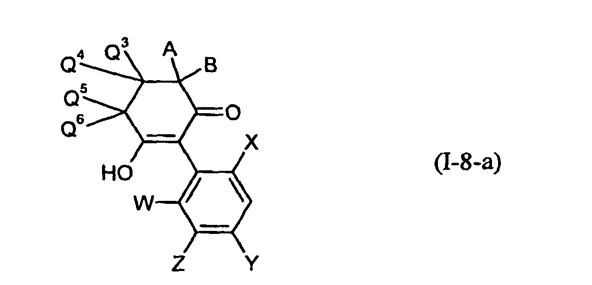 in which
A, B, Q³, Q⁴, Q⁵, Q⁶, W, X, Y and Z are each as defined in Claim 1
are obtained when
6-aryl-5-keto-hexanoic esters of the formula (XI) 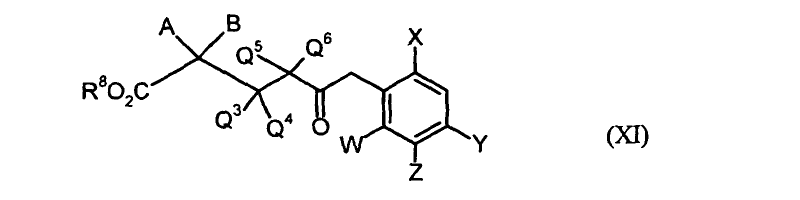 in which
A, B, Q³, Q⁴, Q⁵, Q⁶, W, X, Y and Z are each as defined above
and
R⁸ represents alkyl
are condensed intramolecularly in the presence of a diluent and in the presence of a base,
(I) Compounds of the formulae (I-8-a) shown above in which A, B, D, Q¹, Q², Q³, Q⁴, Q⁵, Q⁶, W, X, Y and Z are each as defined above are obtained when compounds of the formulae (I-1'-a) to (I-8'-a), 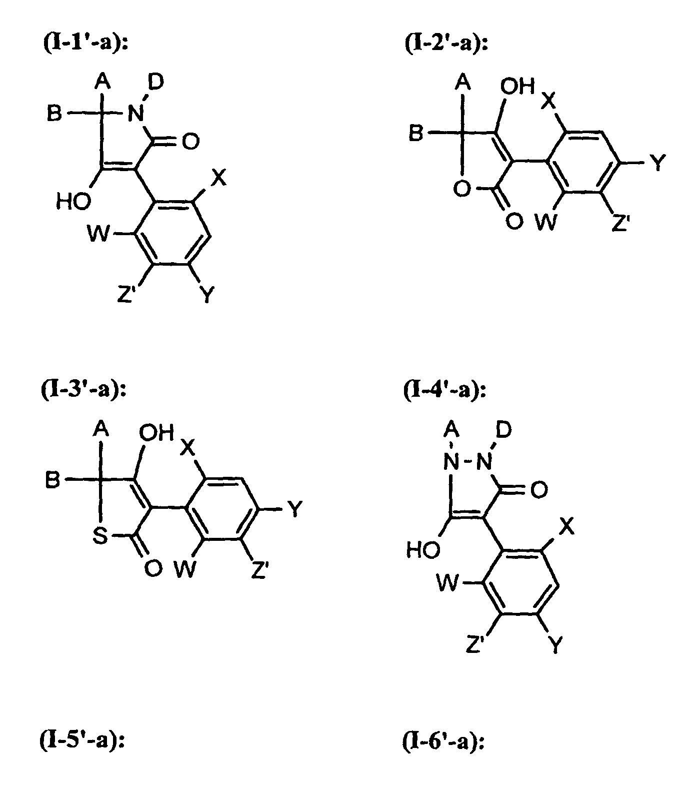 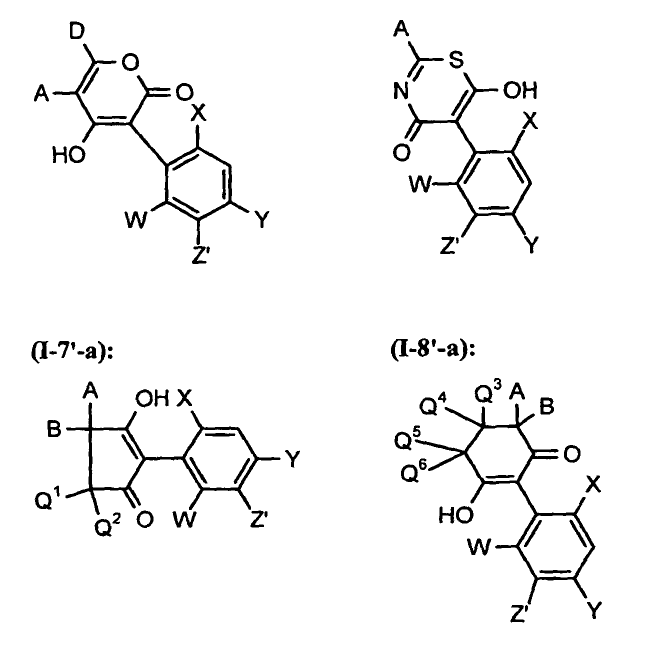 in which
A, B, D, Q¹, Q², Q³, Q⁴, Q⁵, Q⁶, W, X, Y and Z are each as defined above and
Z' represents chlorine, bromine or iodine
are reacted with boronic acids of the formula (XII) 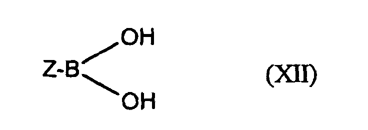 in which
Z is as defined above
in the presence of a solvent, a base and a catalyst and the resulting compounds of the formulae (I-1-a) to (I-8-a) are subsequently in each case
(Jα) reacted with acyl halides of the formula (XIII) 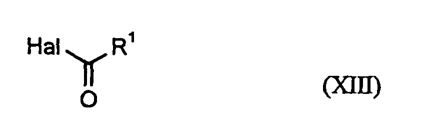 in which
R¹ is as defined in Claim 1 and
Hal represents halogen
or
(β) reacted with carboxylic anhydrides of the formula (XIV)
R¹-CO-O-CO-R¹ (XIV)
in which
R¹ is as defined above,
if appropriate in the presence of a diluent and if appropriate in the presence of an acid binder, or in each case
(K) reacted with chloroformic esters or chloroformic thioesters of the formula (XV)
R²-M-CO-Cl (XV)
in which
R² and M are each as defined in Claim 1,
if appropriate in the presence of a diluent and if appropriate in the presence of an acid binder, or in each case
(L) reacted with chloromonothioformic esters or chlorodithioformic esters of the formula (XVI) 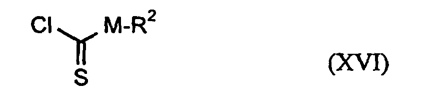 in which
M and R² are each as defined above
if appropriate in the presence of a diluent and if appropriate in the presence of an acid binder, or in each case
(M) reacted with sulphonyl chlorides of the formula (XVII)
R³-SO₂-Cl (XVII)
in which
R³ is as defined in Claim 1,
if appropriate in the presence of a diluent and if appropriate in the presence of an acid binder, or in each case
(N) reacted with phosphorus compounds of the formula (XVIII) 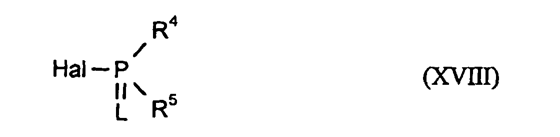 in which
L, R⁴ and R⁵ are each as defined in Claim 1 and
Hal represents halogen,
if appropriate in the presence of a diluent and if appropriate in the presence of an acid binder, or in each case
(O) reacted with metal compounds or amines of the formulae (XIX) or (XX)
Me(OR¹⁰)ₜ (XIX) 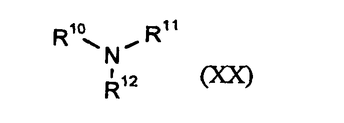
in which
Me represents a mono- or divalent metal,
t represents the number 1 or 2 and
R¹⁰, R¹¹, R¹² independently of one another each represent hydrogen or alkyl,
if appropriate in the presence of a diluent, or in each case
(Pα) reacted with isocyanates or isothiocyanates of the formula (XXI)
R⁶-N=C=L (XXi)
in which
R⁶ and L are each as defined in Claim 1,
if appropriate in the presence of a diluent and if appropriate in the presence of a catalyst, or in each case
(β) reacted with carbamoyl chlorides or thiocarbamoyl chlorides of the formula (XXII) 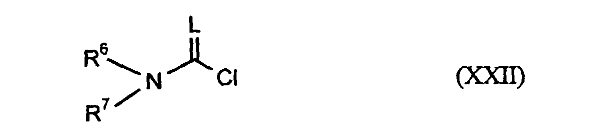 in which
L, R⁶ and R⁷ are each as defined in Claim 1,
if appropriate in the presence of a diluent and if appropriate in the presence of an acid binder.

5. Compounds of the formula (II) 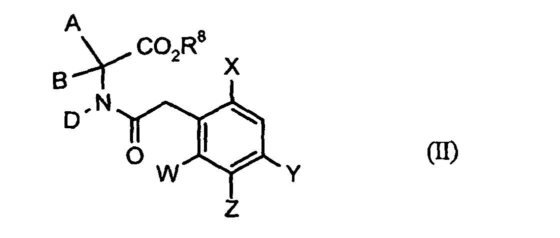 in which
A, B, D, W, X, Y and Z are each as defined in Claim 1 and
R⁸ represents alkyl.

6. Compounds of the formula (XXIV) 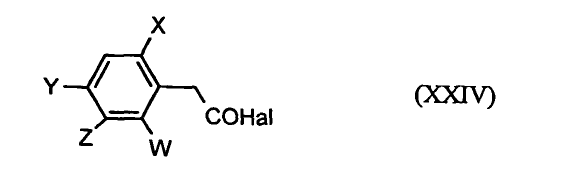 in which
W, X, Y and Z are each as defined in Claim 1 and
Hal represents chlorine or bromine.

7. Compounds of the formula (XXV) 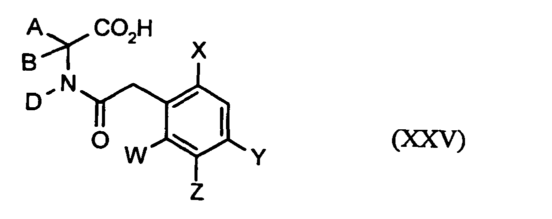 in which
A, B, D, W, X, Y and Z are each as defined in Claim 1.

8. Compounds of the formula (XXIX) 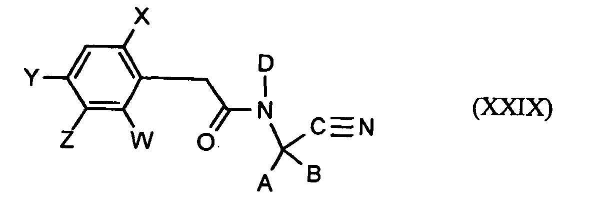 in which
A, B, D, W, X, Y and Z are each as defined in Claim 1.

9. Compounds of the formula (III) 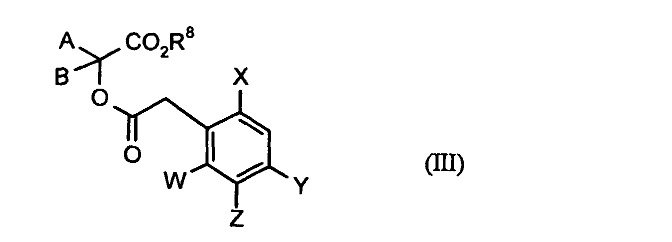 in which
A, B, W, X, Y and Z are each as defined above and
R⁸ represents alkyl.

10. Compounds of the formula (XXVII) 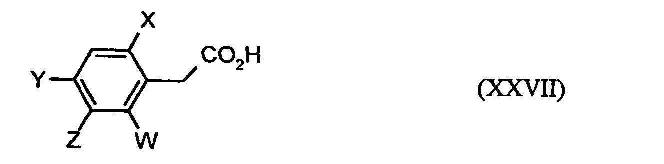 in which
W, X, Y and Z are each as defined in Claim 1.

11. Compounds of the formula (XXXII) 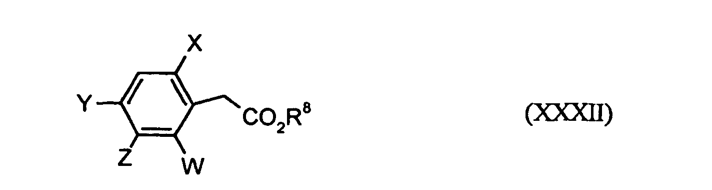 in which
W, X, Y and Z are each as defined in Claim 1 and
R⁸ represents alkyl.

12. Compounds of the formula (XXVII-b) 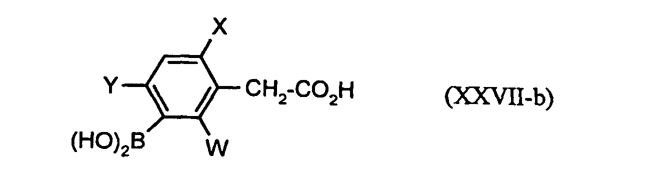 in which
W, X and Y are each as defined in Claim 1.

13. Compounds of the formula (IV) 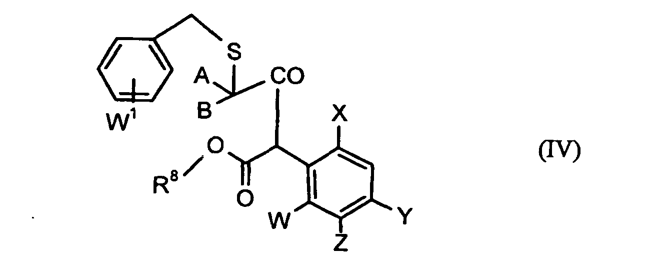 in which
A, B, W, W¹, X, Y and Z are each as defined in Claim 1 and
R⁸ represents alkyl.

14. Compounds of the formula (V) 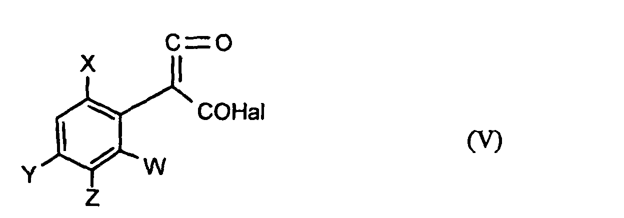 in which
W, X, Y and Z are each as defined in Claim 1 and
Hal represents chlorine or bromine.

15. Compounds of the formula (XXXVII) 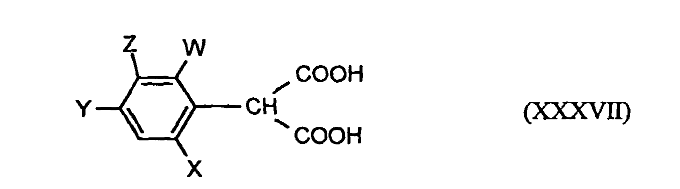 in which
W, X, Y and Z are each as defined in Claim 1.

16. Compounds of the formula (VI) 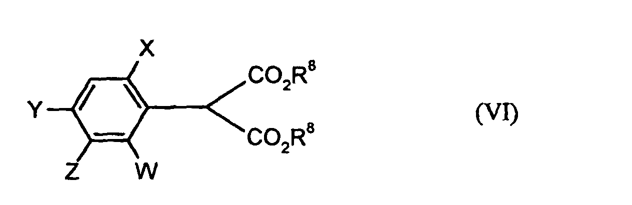 in which
W, X, Y and Z are each as defined in Claim 1 and
R⁸ represents alkyl.

17. Compounds of the formula (X) 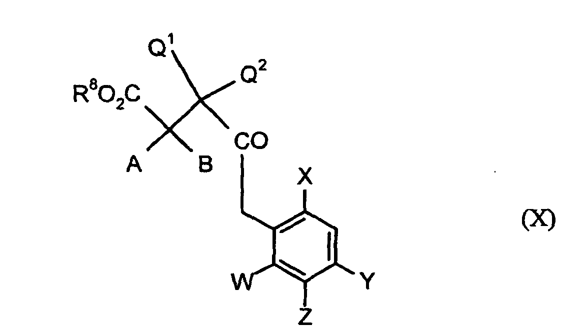 in which
A, B, Q¹, Q², W, X, Y and Z are each as defined in Claim 1 and
R8 represents alkyl.

18. Compounds of the formula (XXXVIII) 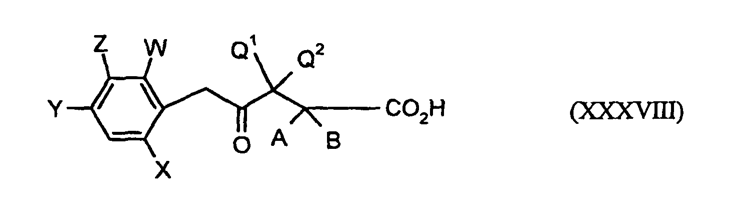 in which
W, X, Y, Z, A, B, Q¹ and Q² are each as defined in Claim 1.

19. Compounds of the formula (XXXIX) 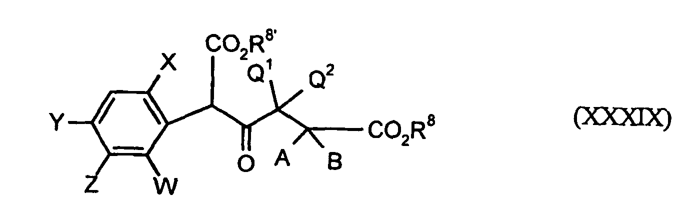 in which
A, B, D¹, D², W, X, Y and Z are each as defined in Claim 1 and
R⁸ and R^{8'} each represent alkyl.

20. Compounds of the formula (XI) 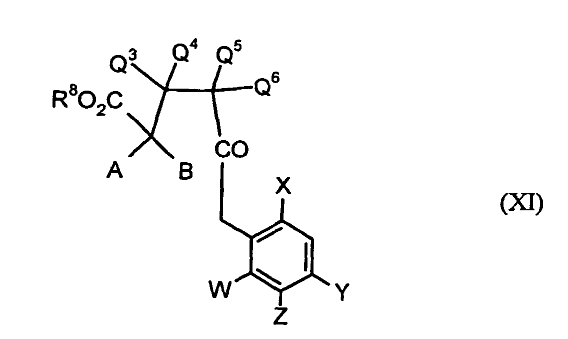 in which
A, B, Q³, Q⁴, Q⁵, Q⁶, W, X, Y and Z are each as defined in Claim 1 and
R⁸ represents alkyl.

21. Compounds of the formula (XLII) 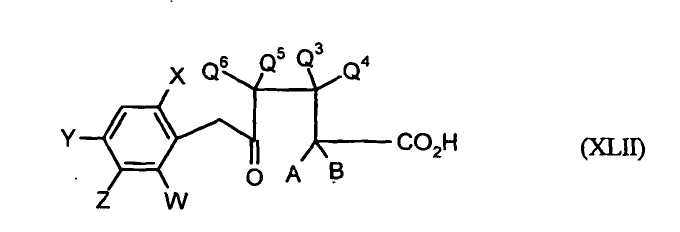 in which
A, B, Q³, Q⁴, Q⁵, Q⁶, W, X, Y and Z are each as defined in Claim 1.

22. Compounds of the formula (XLIII) 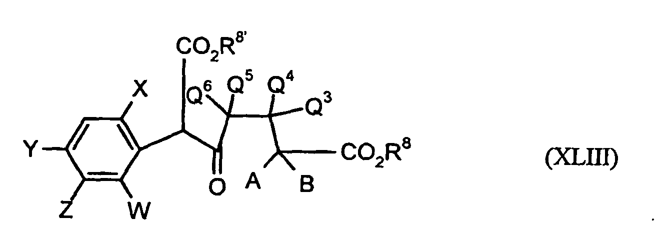 in which
A, B, Q³, Q⁴, Q⁵, Q⁶, W, X, Y and Z are each as defined in Claim 1 and
R⁸ and R^{8'} each represent alkyl.

23. Pesticides and/or herbicides, **characterized in that** they contain at least one compound of the formula (I) according to Claim 1.

24. Use of compounds of the formula (I) according to Claim 1 for controlling pests in crop protection, in the domestic sector, in the hygiene sector and in the protection of stored products, apart from on the human body or on the body of an animal.

25. Method for controlling pests in crop protection, in the domestic sector, in the hygiene sector and in the protection of stored products, apart from on the human body or on the body of an animal, **characterized in that** compounds of the formula (I) according to Claim 1 are allowed to act on the pests and/or their habitat.

26. Method for preparing pesticides and/or herbicides, **characterized in that** compounds of the formula (I) according to Claim 1 are mixed with extenders and/or surfactants.

## Revendications

1. Composés de formule (I) 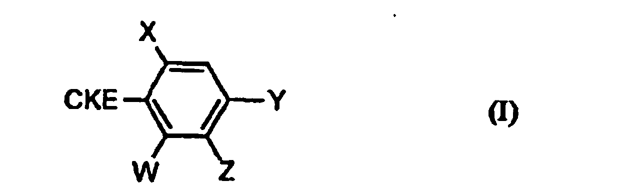 dans laquelle
X représente un halogène, un reste alkyle en C₁ à C₆, halogénalkyle en C₁ à C₆, alkoxy en C₁ à C₆, alcényloxy en C₃ à C₆, alkylthio en C₁ à C₆, alkylsulfinyle en C₁ à C₆, alkylsulfonyle en C₁ à C₆, halogénalkoxy en C₁ à C₆, halogénalcényloxy en C₃ à C₆, nitro, cyano ou un reste phényle, phénoxy, phénylthio, benzyloxy ou benzylthio, chacun éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
Z représente l'un des restes 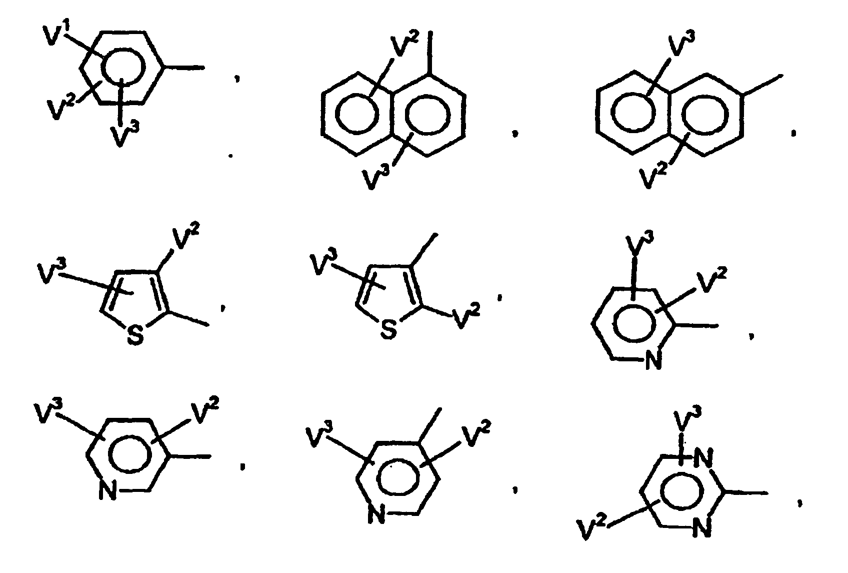 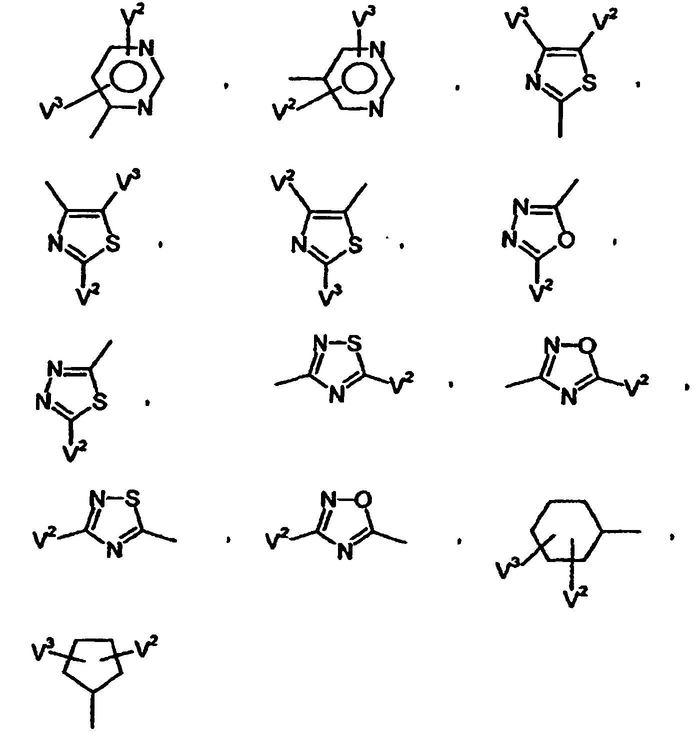
V¹ représente l'hydrogène, un halogène, un reste alkyle en C₁ à C₁₂, alkoxy en C₁ à C₆, alkylthio en C₁ à C₆, alkylsulfinyle en C₁ à C₆, alkylsulfonyle en C₁ à C₆, halogénalkyle en C₁ à C₄, halogénalkoxy en C₁ à C₄, nitro, cyano, ou un reste phényle, phénoxy, phénoxy-(alkyle en C₁ à C₄), phényl-(alkoxy en C₁ à C₄), phénylthio-(alkyle en C₁ à C₄) ou phényl-(alkylthio en C₁ à C₄), chacun éventuellement substitué une ou plusieurs fois par un radical halogéno, alkyle en C₁ à C₆, alkoxy en C₁ à C₆, halogénalkyle en C₁ à C₄, halogénalkoxy en C₁ à C₄, nitro ou cyano,
V² et V³ représentent, indépendamment l'un de l'autre, l'hydrogène, un halogène, un reste alkyle en C₁ à C₆, alkoxy en C₁ à C₆, halogénalkyle en C₁ à C₄ ou halogénalkoxy en C₁ à C₄,
W et Y représentent, indépendamment l'un de l'autre, l'hydrogène, un halogène, un reste alkyle en C₁ à C₆, halogénalkyle en C₁ à C₆, alkoxy en C₁ à C₆, halogénalkoxy en C₁ à C₆, nitro ou cyano,
CKE représente l'un des groupes 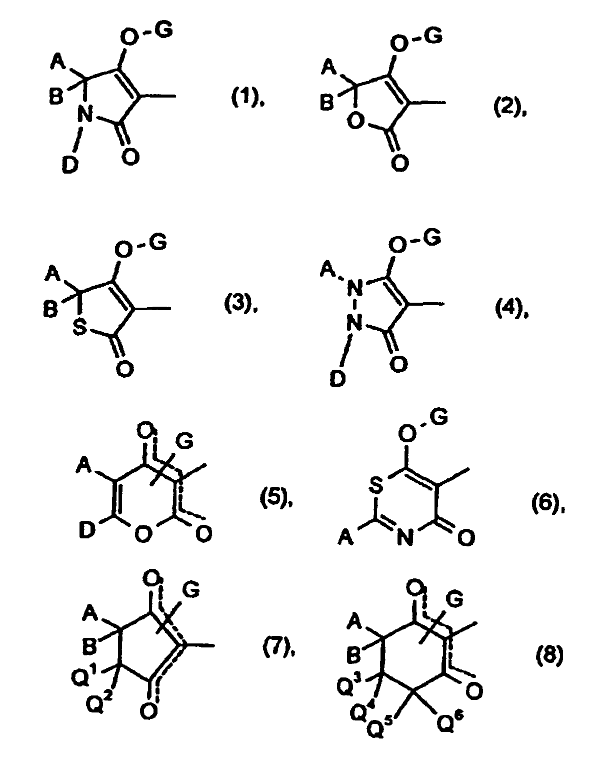
A représente l'hydrogène ou un reste alkyle en C₁ à C₁₂, alcényle en C₃ à C₈, (alkoxy en C₁ à C₁₀) - (alkyle en C₁ à Ce), poly (alkoxy en C₁ à C₈) - (alkyle en C₁ à C₈), (alkylthio en C₁ à C₁₀) - (alkyle en C₁ à C₆), chacun éventuellement substitué par un halogène, un reste cycloalkyle en C₃ à C₈ éventuellement substitué par un radical halogéno, alkyle en C₁ à C₆ ou alkoxy en C₁ à C₆ et dans lequel, le cas échéant, un ou deux atomes non directement contigus du noyau sont remplacés par de l'oxygène et/ou du soufre, ou un reste aryle en C₆ ou C₁₀, hétaryle à noyau de 5 ou 6 atomes ou (aryle en C₆ à C₁₀)-(alkyle en C₁ à C₆), chacun éventuellement substitué par un radical halogéno, alkyle en C₁ à C₆, halogénalkyle en C₁ à C₆, alkoxy en C₁ à C₆, halogénalkoxy en C₁ à C₆, cyano ou nitro,
B représente l'hydrogène, un reste alkyle en C₁ à C₁₂ ou (alkoxy en C₁ à C₈)-(alkyle en C₁ à C₆), ou bien
A, B et l'atome de carbone auquel ils sont liés représentent un groupe cycloalkyle en C₃ à C₁₀ saturé ou un groupe cycloalkyle en C₅ à C₁₀ non saturé dans lequel, le cas échéant, un atome du noyau est remplacé par de l'oxygène ou du soufre et qui sont éventuellement substitués une ou deux fois par un radical alkyle en C₁ à C₈, cycloalkyle en C₃ à C₁₀, halogénalkyle en C₁ à C₈, alkoxy en C₁ à C₈, alkylthio en C₁ à C₈, halogéno ou phényle,
A, B et l'atome de carbone auquel ils sont liés représentent un groupe cycloalkyle en C₃ à C₆, qui est substitué par un groupe alkylènediyle contenant éventuellement un ou deux atomes d'oxygène et/ou de soufre non directement contigus, ou par un groupe alkylènedioxyle ou par un groupe alkylènedithioyle qui forme avec l'atome de carbone auquel il est lié un autre noyau pentagonal à octogonal, ou bien
A, B et l'atome de carbone auquel ils sont liés représentent un groupe cycloalkyle en C₃ à C₈ ou un groupe cycloalcényle en C₅ à Ce dans lequel deux substituants forment conjointement avec les atomes de carbone auxquels ils ont liés un groupe alcanediyle en C₂ à C₆, alcènediyle en C₂ à C₆ ou alcanediènediyle en C₄ à C₆, chacun éventuellement substitué par un radical alkyle en C₁ à C₆, alkoxy en C₁ à C₆ ou halogéno, et dans lesquels, le cas échéant, un groupe méthylène est remplacé par de l'oxygène ou du soufre,
D représente l'hydrogène, un reste alkyle en C₁ à C₁₂, alcényle en C₃ à C₈, alcynyle en C₃ à C₈, (alkoxy en C₁ à C₁₀)-(alkyle en C₂ à C₈), poly (alkoxy en C₁ à C₈)-(alkyle en C₂ à C₈), (alkylthio en C₁ à C₁₀)-(alkyle en C₂ à C₈), chacun éventuellement substitué par un halogène, un reste cycloalkyle en C₃ à C₈ éventuellement substitué par un radical halogéno, alkyle en C₁ à C₄, alkoxy en C₁ à C₄ ou halogénalkyle en C₁ à C₄ et dans lequel, le cas échéant, un atome du noyau est remplacé par de l'oxygène ou du soufre, ou bien un reste phényle, hétaryle pentagonal ou hexagonal, phényl-(alkyle en C₁ à C₆) ou hétaryl-(alkyle en C₁ à C₈) à noyau de 5 ou 6 atomes, chacun éventuellement substitué par un radical halogéno, alkyle en C₁ à C₆, halogénalkyle en C₁ à C₆, alkoxy en C₁ à C₆, halogénalkoxy en C₁ à C₆, cyano ou nitro, ou bien
A et D forment ensemble un reste alcanediyle en C₃ à C₆ ou un reste alcènediyle en C₃ à C₆, chacun éventuellement substitué, dans lesquels, le cas échéant, un groupe méthylène est remplacé par de l'oxygène ou du soufre et pour chacun desquels on considère comme substituants :
un halogène, un groupe hydroxy, un groupe mercapto ou un reste alkyle en C₁ à C₁₀, alkoxy en C₁ à C₆, alkylthio en C₁ à C₆, cycloalkyle en C₃ à C₇, phényle ou benzyloxy, chacun éventuellement substitué par un halogène, ou bien un autre groupe alcanediyle en C₃ à C₆, un groupe alcènediyle en C₃ à C₆ ou un groupe butadiényle qui est éventuellement substitué par un radical alkyle en C₁ à C₆ ou dans lequel, le cas échéant, deux substituants voisins forment, avec les atomes de carbone auxquels ils sont liés, un autre noyau saturé ou non saturé, pentagonal ou hexagonal (dans le cas du composé de formule (I-I), A et D forment alors conjointement avec les atomes auxquels ils sont liés, par exemple les autres groupes AD-1 à AD-10 indiqués ci-dessous), qui peut contenir de l'oxygène ou du soufre, ou dans lequel est contenu, le cas échéant, l'un des groupes suivants 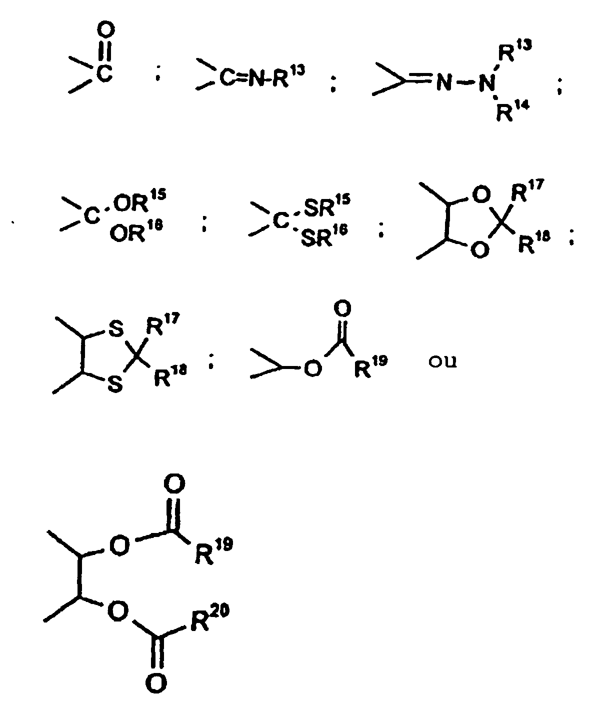
ou bien
A et Q¹ représentent ensemble un groupe alcanediyle en C₃ à C₆, ou un groupe alcènediyle en C₄ à C₆, qui est éventuellement substitué dans chaque cas une ou deux fois identiques ou différentes par un halogène, un radical hydroxy, par un reste alkyle en C₁ à C₁₀, alkoxy en C₁ à C₆, alkylthio en C₁ à C₆, cycloalkyle en C₃ à C₇, chacun éventuellement substitué une à trois fois identiques ou différentes par un halogène, ou bien par un reste benzyloxy ou phényle, chacun éventuellement substitué une à trois fois identiques ou différentes par un radical halogéno, alkyle en C₁ à C₆ ou alkoxy en C₁ à C₆, et qui contient en outre, le cas échéant, l'un des groupes ci-après 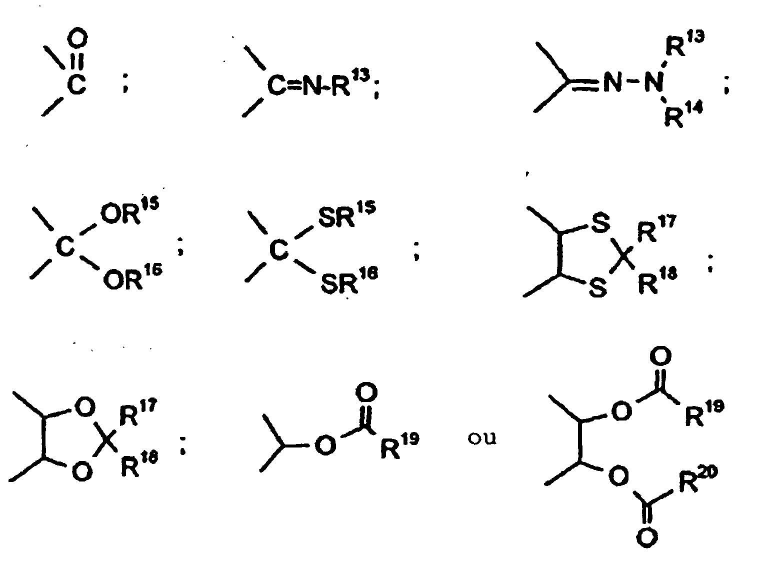 ou qui est ponté par un groupe alcanediyle en C₁ ou C₂ ou par un atome d'oxygène, ou bien
Q¹ représente l'hydrogène ou un reste alkyle en C₁ à C₄,
Q², Q⁴, Q⁵ et Q⁶ représentent indépendamment l'un de l'autre l'hydrogène ou un reste alkyle en C₁ à C₄,
Q³ représente l'hydrogène, un reste alkyle en C₁ à C₆, (alkoxy en C₁ à C₆) - (alkyle en C₁ ou C₂), (alkylthio en C₁ à C₆) - (alkyle en C₁ ou C₂), un reste cycloalkyle en C₃ à C₈ éventuellement substitué par un radical alkyle en C₁ à C₄ ou alkoxy en C₁ à C₄ et dans lequel, le cas échéant, un groupe méthylène est remplacé par de l'oxygène ou du soufre, ou un reste phényle éventuellement substitué par un radical halogéno, alkyle en C₁ à C₄, alkoxy en C₁ à C₄, halogénalkyle en C₁ ou C₂, halogénalkoxy en C₁ ou C₂, cyano ou nitro, ou bien
Q³ et Q⁴ forment, conjointement avec l'atome de carbone auquel ils sont liés, un noyau de 3 à 7 chaînons éventuellement substitué par un radical alkyle en C₁ à C₄, alkoxy en C₁ à C₄ ou halogénalkyle en C₁ ou C₂ et dans lequel, le cas échéant, un chaînon est remplacé par de l'oxygène ou du soufre,
G représente l'hydrogène (a) ou l'un des groupes 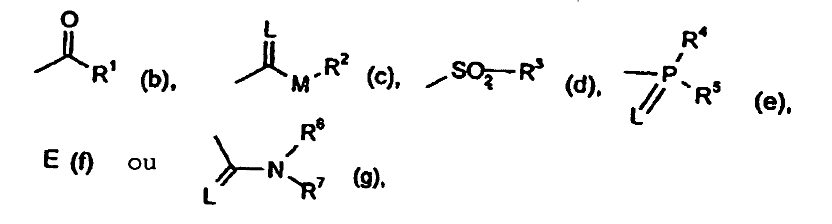 dans lesquels
E représente un ion métallique ou un ion ammonium,
L représente l'oxygène ou le soufre et
M représente l'oxygène ou le soufre,
R¹ représente un reste alkyle en C₁ à C₂₀, alcényle en C₂ à C₂₀, (alkoxy en C₁ à C₈) - (alkyle en C₁ à Ce), (alkylthio en C₁ à C₈) - (alkyle en C₁ à C₈), poly (alkoxy en C₁ à C₈) - (alkyle en C₁ à C₈), chacun éventuellement substitué par un halogène ou un reste cycloalkyle en C₃ à C₈ éventuellement substitué par un radical halogéno, alkyle en C₁ à C₆ ou alkoxy en C₁ à C₆ et dans lequel, le cas échéant, un ou plusieurs atomes non directement contigus du noyau sont remplacés par l'oxygène et/ou le soufre,
un reste phényle éventuellement substitué par un radical halogéno, cyano, nitro, alkyle en C₁ à C₆, alkoxy en C₁ à C₆, halogénalkyle en C₁ à C₆, halogénalkoxy en C₁ à C₆, alkylthio en C₁ à C₆ ou alkylsulfonyle en C₁ à C₆,
un reste phényl-(alkyle en C₁ à C₆) éventuellement substitué par un radical halogéno, nitro, cyano, alkyle en C₁ à C₆, alkoxy en C₁ à C₆, halogénalkyle en C₁ à C₆ ou halogénalkoxy en C₁ à C₆,
un reste hétaryle pentagonal ou hexagonal éventuellement substitué par un radical halogéno ou alkyle en C₁ à C₆,
un reste phénoxy-(alkyle en C₁ à C₆) éventuellement substitué par un radical halogéno ou alkyle en C₁ à C₆, ou bien
un reste hétaryloxy-(alkyle en C₁ à C₆) à noyau pentagonal ou hexagonal éventuellement substitué par un radical halogéno, amino ou alkyle en C₁ à C₆,
R² représente un reste alkyle en C₁ à C₂₀, alcényle en C₂ à C₂₀, (alkoxy en C₁ à C₈) - (alkyle en C₂ à C₈), poly (alkoxy en C₁ à C₈) - (alkyle en C₂ à C₈), chacun éventuellement substitué par un halogène,
un reste cycloalkyle en C₃ à C₈ éventuellement substitué par un radical halogéno, alkyle en C₁ à C₆ ou alkoxy en C₁ à C₆, ou bien
un reste phényle ou benzyle, chacun éventuellement substitué par un radical halogéno, cyano, nitro, alkyle en C₁ à C₆, alkoxy en C₁ à C₆, halogénalkyle en C₁ à C₆ ou halogénalkoxy en C₁ à C₆,
R³ représente un reste alkyle en C₁ à C₈ éventuellement substitué par un halogène, ou un reste phényle ou benzyle, chacun éventuellement substitué par un radical halogéno, alkyle en C₁ à C₆, alkoxy en C₁ à C₆, halogénalkyle en C₁ à C₄, halogénalkoxy en C₁ à C₄, cyano ou nitro,
R⁴ et R⁵ représentent indépendamment l'un de l'autre un reste alkyle en C₁ à C₈, alkoxy en C₁ à C₈, alkylamino en C₁ à C₈, di (alkyle en C₁ à C₈) amino, alkylthio en C₁ à C₈, alcénylthio en C₂ à C₈, cycloalkylthio en C₃ à C₇, chacun éventuellement substitué par un halogène, ou un reste phényle, phénoxy ou phénylthio, chacun éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄ ou halogénalkyle en C₁ à C₄,
R⁶ et R⁷ représentent indépendamment l'un de l'autre l'hydrogène, un reste alkyle en C₁ à C₈, cycloalkyle en C₃ à C₈, alkoxy en C₁ à C₈, alcényle en C₃ à C₈, (alkoxy en C₁ à C₈) - (alkyle en C₁ à C₈), chacun éventuellement substitué par un halogène, un reste phényle éventuellement substitué par un radical halogéno, halogénalkyle en C₁ à C₈, alkyle en C₁ à Ce ou alkoxy en C₁ à C₈, un reste benzyle éventuellement substitué par un radical halogéno, alkyle en C₁ à C₈, halogénalkyle en C₁ à C₈ ou alkoxy en C₁ à C₈, ou forment ensemble un reste alkylène en C₃ à C₆ éventuellement substitué par un radical alkyle en C₁ à C₄ et dans lequel, le cas échéant, un groupe méthylène est remplacé par de l'oxygène ou du soufre,
R¹³ représente l'hydrogène, un reste alkyle en C₁ à C₈ ou alkoxy en C₁ à C₈, chacun éventuellement substitué par un halogène, un reste cycloalkyle en C₃ à C₈ éventuellement substitué par un radical halogéno, alkyle en C₁ à C₄ ou alkoxy en C₁ à C₄ et dans lequel, le cas échéant, un groupe méthylène est remplacé par de l'oxygène ou du soufre, ou bien un reste phényle, phényl-(alkyle en C₁ à C₄) ou phényl-(alkoxy en C₁ à C₄), chacun éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
R¹⁴ représente l'hydrogène ou un reste alkyle en C₁ à C₈, ou bien
R¹³ et R¹⁴ forment ensemble un reste alcanediyle en C₄ à C₆,
R¹⁵ et R¹⁶ sont identiques ou différents et représentent un reste alkyle en C₁ à C₆,
R¹⁵ et R¹⁶ forment ensemble un reste alcanediyle en C₂ à C₄ qui est substitué le cas échéant par un radical alkyle en C₁ à C₆, halogénalkyle en C₁ à C₆ ou par un radical phényle éventuellement substitué par un radical halogéno, alkyle en C₁ à C₆, halogénalkyle en C₁ à C₄, alkoxy en C₁ à C₆, halogénalkoxy en C₁ à C₄, nitro ou cyano,
R¹⁷ et R¹⁸ représentent indépendamment l'un de l'autre l'hydrogène, un reste alkyle en C₁ à Ce éventuellement substitué par un halogène, ou un reste phényle éventuellement substitué par un radical halogéno, alkyle en C₁ à C₆, alkoxy en C₁ à C₆, halogénalkyle en C₁ à C₄, halogénalkoxy en C₁ à C₄, nitro ou cvano,
R¹⁷ et R¹⁸ forment, conjointement avec l'atome de carbone auquel ils sont liés, un groupe carbonyle ou un reste cycloalkyle en C₅ à C₇ éventuellement substitué par un radical halogéno, alkyle en C₁ à C₄ ou alkoxy en C₁ à C₄ et dans lequel, le cas échéant, un groupe méthylène est remplacé par de l'oxygène ou du soufre, et
R¹⁹ et R²⁰ représentent indépendamment l'un de l'autre un reste alkyle en C₁ à C₁₀, alcényle en C₂ à C₁₀, alkoxy en C₁ à C₁₀, alkylamino en C₁ à C₁₀, alcénylamino en C₃ à C₁₀, di (alkyle en C₁ à C₁₀) amino ou di (alcényle en C₃ à C₁₀) amino.

2. Composés de formule (I) suivant la revendication 1, formule dans laquelle
X représente le fluor, le chlore, le brome, un reste alkyle en C₁ à C₄, alkoxy en C₁ à C₄, alcényloxy en C₃ ou C₄, halogénalkyle en C₁ à C₄, halogénalkoxy en C₁ à C₄, halogénalcényloxy en C₃ ou C₄, nitro ou cyano,
Z représente l'un des restes 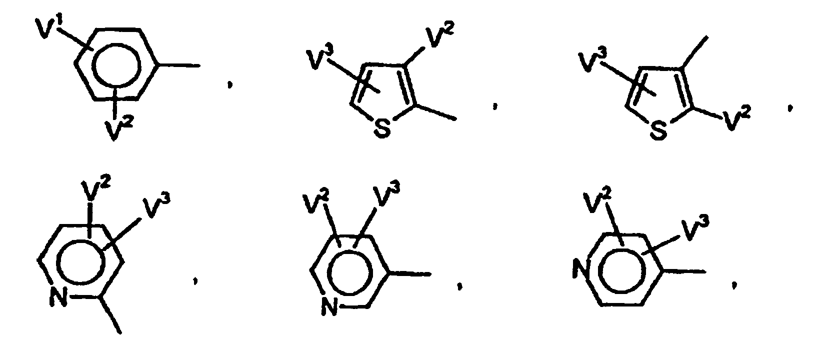 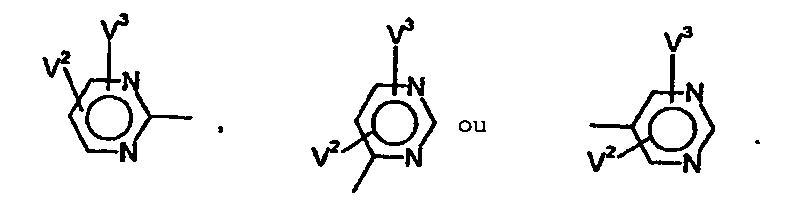
V¹ représente l'hydrogène, le fluor, le chlore, le brome, un reste alkyle en C₁ à C₆, alkoxy en C₁ à C₄, halogénalkyle en C₁ ou C₂, halogénalkoxy en C₁ ou C₂, nitro, cyano, ou un reste phényle, phénoxy, phénoxy-(alkyle en C₁ ou C₂), phényl- (alkoxy en C₁ ou C₂), phénylthio-(alkyle en C₁ ou C₂) ou phényl-(alkylthio en C₁ ou C₂), chacun éventuellement substitué une ou deux fois par un radical fluoro, chloro, bromo, alkyle en C₁ à C₄, alkoxy en C₁ à C₄, halogénalkyle en C₁ ou C₂, halogénalkoxy en C₁ ou C₂, nitro ou cyano,
V² et V³ représentent indépendamment l'un de l'autre l'hydrogène, le fluor, le chlore, le brome, un reste alkyle en C₁ à C₄, alkoxy en C₁ à C₉, halogénalkyle en C₁ ou C₂ ou halogénalkoxy en C₁ ou C₂,
W et Y représentent indépendamment l'un de l'autre, l'hydrogène, le fluor, le chlore, le brome, un reste alkyle en C₁ à C₄, halogénalkyle en C₁ à C₄, alkoxy en C₁ à C₄ ou halogénalkoxy en C₁ à C₄,
CKE représente l'un des groupes 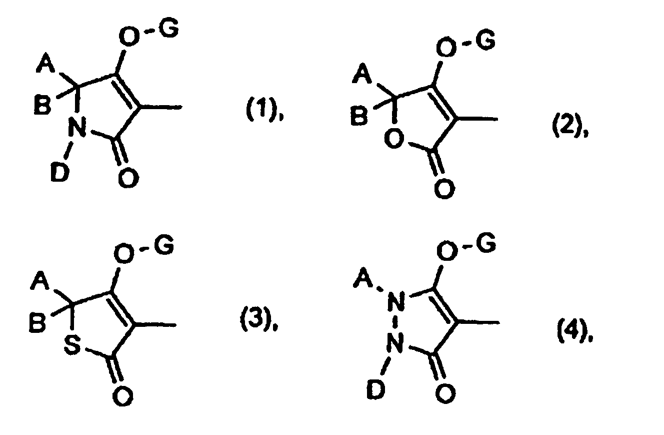 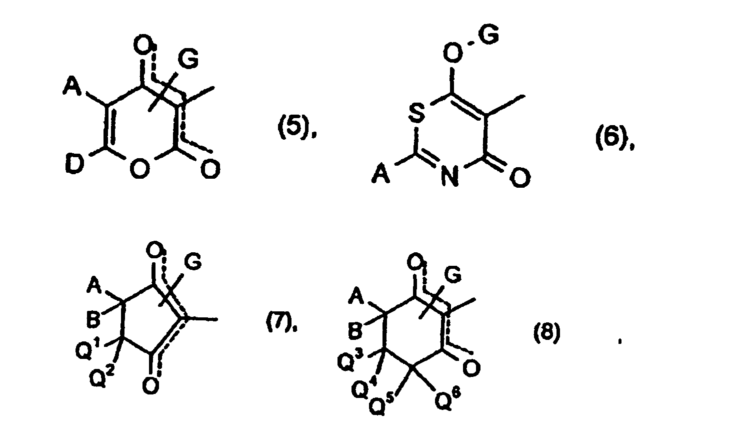
A représente l'hydrogène ou un reste alkyle en C₁ à C₁₀, (alkoxy en C₁ à C₈) - (alkyle en C₁ à C₆), chacun éventuellement substitué par du fluor ou du chlore, un reste cycloalkyle en C₃ à C₇ éventuellement substitué par un radical fluoro, chloro, alkyle en C₁ à C₄ ou alkoxy en C₁ à C₄ et dans lequel, le cas échéant, un chaînon du noyau est remplacé par de l'oxygène ou du soufre, ou bien (toutefois non dans le cas des composés de formules (1-5), (1-7) et (I-8)) un reste phényle, furannyle, pyridyle, imidazolyle, triazolyle, pyrazolyle, pyrimidyle, thiazolyle, thiényle ou phényl-(alkyle en C₁ à C₄), chacun éventuellement substitué par un radical fluoro, chloro, bromo, alkyle en C₁ à C₄, halogénalkyle en C₁ à C₄, alkoxy en C₁ à C₄ ou halogénalkoxy en C₁ à C₄,
B représente l'hydrogène ou un reste alkyle en C₁ à C₆, ou bien
A, B et l'atome de carbone auquel ils sont liés représentent un reste cycloalkyle en C₅ à C₇ saturé ou non saturé dans lequel, le cas échéant, un chaînon du noyau est remplacé par de l'oxygène ou du soufre et qui est éventuellement substitué une fois par un radical alkyle en C₁ à C₆, cycloalkyle en C₅ à C₈, halogénalkyle en C₁ à C₃, alkoxy en C₁ à C₆, fluoro, chloro ou phényle,
A, B et l'atome de carbone auquel ils sont liés représentent un reste cycloalkyle en C₅ ou C₆ qui est substitué par un groupe alkylènediyle contenant éventuellement un ou deux atomes d'oxygène ou de soufre non directement contigus, ou par un groupe alkylènedioxyle ou par un groupe alkylènedithiol, qui forme avec l'atome de carbone auquel il est lié, un autre noyau pentagonal ou hexagonal, ou bien,
A, B et l'atome de carbone auquel ils sont liés représentent un reste cycloalkyle en C₃ à C₆ ou un reste cycloalcényle en C₅ ou C₆, dans lesquels deux substituants représentent, conjointement avec les atomes auxquels ils sont liés, un reste alcanediyle en C₂ à C₄ ou alcènediyle en C₂ à C₄, chacun éventuellement substitué par un radical alkyle en C₁ à C₅, alkoxy en C₁ à C₅, fluoro, chloro ou bromo et dans lequel, le cas échéant, un groupe méthylène est remplacé par de l'oxygène ou du soufre, ou bien un reste butadiènediyle,
D représente l'hydrogène, un reste alkyle en C₁ à C₁₀, alcényle en C₃ à C₆, (alkoxy en C₁ à C₈) - (alkyle en C₂ à C₆) ou (alkylthio en C₁ à C₈)-(alkyle en C₂ à C₆), chacun éventuellement substitué par du fluor ou du chlore, un reste cycloalkyle en C₃ à C₇ éventuellement substitué par un radical fluoro, chloro, alkyle en C₁ à C₄, alkoxy en C₁ à C₄ ou halogénalkyle en C₁ ou C₂ dans lequel, le cas échéant, un groupe méthylène est remplacé par de l'oxygène ou du soufre, ou bien (toutefois non dans le cas des composés de formules (I-1) et (I-4)) un reste phényle, furannyle, imidazolyle, pyridyle, thiazolyle, pyrazolyle, pyrimidyle, pyrrolyle, thiényle, triazolyle ou phényl-(alkyle en C₁ à C₄), chacun éventuellement substitué par un radical fluoro, chloro, bromo, alkyle en C₁ à C₄, halogénalkyle en C₁ à C₄, alkoxy en C₁ à C₄ ou halogénalkoxy en C₁ à C₄, ou bien
A et D forment ensemble un reste alcanediyle en C₃ à C₅ éventuellement substitué, dans lequel un groupe méthylène peut être remplacé par un groupe carbonyle, de l'oxygène ou du soufre, les substituants considérés étant un radical hydroxy, alkyle en C₁ à C₆ ou alkoxy en C₁ à C₄, ou bien
A et D (dans le cas des composés de formules (I-I)) forment, conjointement avec les atomes auxquels ils sont liés, l'un des groupes AD-1 à AD-10 : 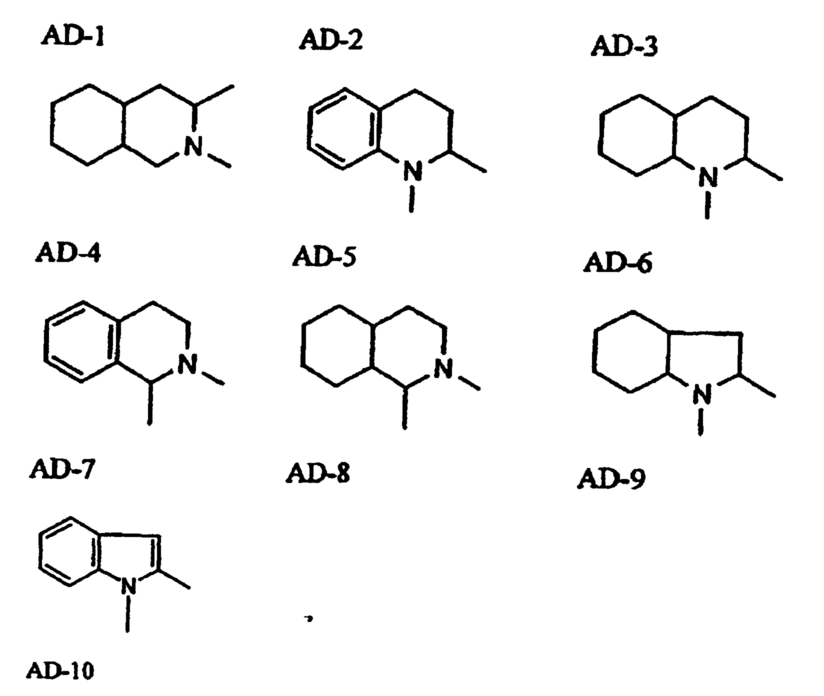 ou bien
A et Q¹ forment ensemble un groupe alcanediyle en C₃ ou C₄ ou alcènediyle en C₃ ou C₄, chacun éventuellement substitué une ou deux fois identiques ou différentes par un radical fluoro, chloro, hydroxy, par un reste alkyle en C₁ à C₈ ou alkoxy en C₁ à C₄, chacun éventuellement substitué une à trois fois par du fluor, ou bien
Q¹ représente l'hydrogène,
Q² représente l'hydrogène,
Q⁴, Q⁵ et Q⁶ représentent indépendamment l'un de l'autre l'hydrogène ou un reste alkyle en C₁ à C₃,
Q³ représente l'hydrogène, un reste alkyle en C₁ à C₄, (alkoxy en C₁ à C₉)-(alkyle en C₁ ou C₂), (alkylthio en C₁ à C₄)-(alkyle en C₁ ou C₂) ou un reste cycloalkyle en C₃ à C₆ éventuellement substitué par radical méthyle ou méthoxy et dans lequel, le cas échéant, un groupe méthylène est remplacé par de l'oxygène ou du soufre, ou bien
Q³ et Q⁴ forment, conjointement avec l'atome de carbone auquel ils sont liés, un noyau pentagonal ou hexagonal saturé éventuellement substitué par un radical alkyle en C₁ à C₄ ou alkoxy en C₁ à C₄ et dans lequel, le cas échéant, un chaînon du noyau est remplacé par de l'oxygène ou du soufre,
G représente l'hydrogène (a) ou l'un des groupes 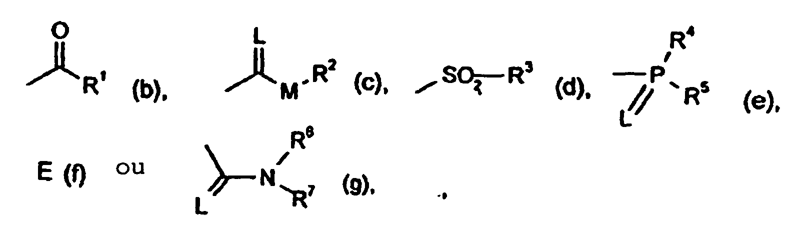 dans lesquels
E représente un ion métallique ou un ion ammonium,
L représente l'oxygène ou le soufre, et
M représente l'oxygène ou le soufre,
R¹ représente un reste alkyle en C₁ à C₁₆, alcényle en C₂ à C₁₆, (alkoxy en C₁ à C₆) - (alkyle en C₁ à C₆), (alkylthio en C₁ à C₆) - (alkyle en C₁ à C₆), poly(alkoxy en C₁ à C₆) - (alkyle en C₁ à C₆), chacun éventuellement substitué par du fluor ou du chlore ou un reste cycloalkyle en C₃ à C₇ éventuellement substitué par un radical fluoro, chloro, alkyle en C₁ à C₅ ou alkoxy en C₁ à C₅ et dans lequel, le cas échéant, un ou deux chaînons non directement contigus du noyau sont remplacés par de l'oxygène et/ou du soufre,
un reste phényle éventuellement substitué par un radical fluoro, chloro, bromo, cyano, nitro, alkyle en C₁ à C₄, alkoxy en C₁ à C₄, halogénalkyle en C₁ à C₃, halogénalkoxy en C₁ à C₃, alkylthio en C₁ à C₄ ou alkylsulfonyle en C₁ à C₄,
un reste phényl-(alkyle en C₁ à C₄) éventuellement substitué par un radical fluoro, chloro, bromo, alkyle en C₁ à C₉, alkoxy en C₁ à C₉, halogénalkyle en C₁ à C₃ ou halogénalkoxy en C₁ à C₃,
un reste pyrazolyle, thiazolyle, pyridyle, pyrimidyle, furannyle ou thiényle, chacun éventuellement substitué par un radical fluoro, chloro, bromo ou alkyle en C₁ à C₄,
un phénoxy-(alkyle en C₁ à C₃) éventuellement substitué par un radical fluoro, chloro, bromo ou alkyle en C₁ à C₄, ou bien
un reste pyridyloxy-(alkyle en C₁ à C₃), pyrimidyloxy- (alkyle en C₁ à C₃) ou thiazolyloxy-(alkyle en C₁ à C₃), chacun éventuellement substitué par un radical fluoro, chloro, bromo, amino ou alkyle en C₁ à C₄,
R² représente un reste alkyle en C₁ à C₁₆, alcényle en C₂ à C₁₆, (alkoxy en C₁ à C₆) - (alkyle en C₂ à C₆) ou poly(alkoxy en C₁ à C₆) - (alkyle en C₂ à C₆), chacun éventuellement substitué par du fluor,
un reste cycloalkyle en C₃ à C₇ éventuellement substitué par un radical fluoro, chloro, alkyle en C₁ à C₄ ou alkoxy en C₁ à C₄,
un reste phényle ou benzyle, chacun éventuellement substitué par un radical fluoro, chloro, bromo, cyano, nitro, alkyle en C₁ à C₄, alkoxy en C₁ à C₄, halogénalkyle en C₁ à C₃ ou halogénalkoxy en C₁ à C₃,
R³ représente un reste alkyle en C₁ à C₆ éventuellement substitué par du fluor ou un reste phényle ou benzyle chacun éventuellement substitué par un radical fluoro, chloro, bromo, alkyle en C₁ à C₄, alkoxy en C₁ à C₄, halogénalkyle en C₁ à C₃, halogénalkoxy en C₁ à C₃, cyano ou nitro,
R⁴ et R⁵ représentent indépendamment l'un de l'autre un reste alkyle en C₁ à C₆, alkoxy en C₁ à C₆, alkylamino en C₁ à C₆, di(alkyle en C₁ à C₆)amino, alkylthio en C₁ à C₆, alcénylthio en C₃ ou C₄, cycloalkylthio en C₃ à C₆ ou un reste phényle, phénoxy ou phénylthio, chacun éventuellement substitué par un radical fluoro, chloro, bromo, nitro, cyano, alkoxy en C₁ à C₃, halogénalkoxy en C₁ à C₃, alkylthio en C₁ à C₃, halogénalkylthio en C₁ à C₃, alkyle en C₁ à C₃ ou halogénalkyle en C₁ à C₃, et
R⁶ et R⁷ représentent indépendamment l'un de l'autre l'hydrogène, un reste alkyle en C₁ à C₆, cycloalkyle en C₃ à C₆, alkoxy en C₁ à C₆, alcényle en C₃ à C₆, (alkoxy en C₁ à C₆) - (alkyle en C₁ à C₆), un reste phényle éventuellement substitué par un radical fluoro, chloro, bromo, halogénalkyle en C₁ à C₃, alkyle en C₁ à C₄ ou alkoxy en C₁ à C₄, un reste benzyle éventuellement substitué par un radical fluoro, chloro, bromo, alkyle en C₁ à C₄, halogénalkyle en C₁ à C₃ ou alkoxy en C₁ à C₄, ou forment ensemble un reste alkylène en C₄ ou C₅ éventuellement substitué par un radical méthyle ou éthyle et dans lequel, le cas échéant, un groupe méthylène est remplacé par de l'oxygène ou du soufre.

3. Composés de formule (I) suivant la revendication 1, formule dans laquelle
X représente le fluor, le chlore, un reste méthyle, éthyle, propyle, isopropyle, méthoxy, éthoxy, propoxy, isopropoxy, trifluorométhyle, difluorométhoxy, trifluorométhoxy, nitro ou cyano,
Z représente l'un des restes 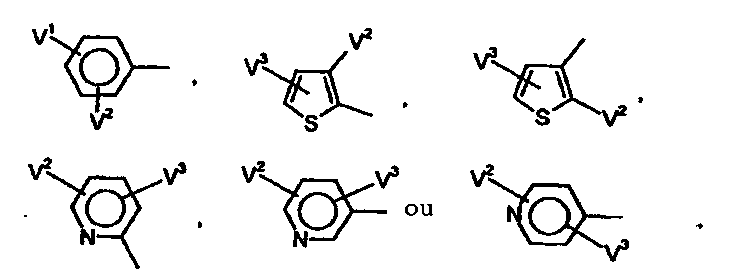
V¹ représente l'hydrogène, le fluor, le chlore, le brome un reste méthyle, éthyle, n-propyle, isopropyle, n-butyle, isobutyle, tertiobutyle, méthoxy, éthoxy, n-propoxy, isopropoxy, trifluorométhyle, trifluorométhoxy, nitro, cyano, ou un reste phényle éventuellement substitué une fois par un radical fluoro, chloro, méthyle, méthoxy, trifluorométhyle ou trifluorométhoxy,
V² et V³ représentent indépendamment l'un de l'autre l'hydrogène, le fluor, le chlore, un reste méthyle, éthyle, n-propyle, isopropyle, méthoxy, éthoxy, trifluorométhyle ou trifluorométhoxy,
W et Y représentent indépendamment l'un de l'autre l'hydrogène, le fluor, le chlore, un reste méthyle, éthyle, n-propyle, méthoxy, éthoxy ou propoxy,
CKE représente l'un des groupes 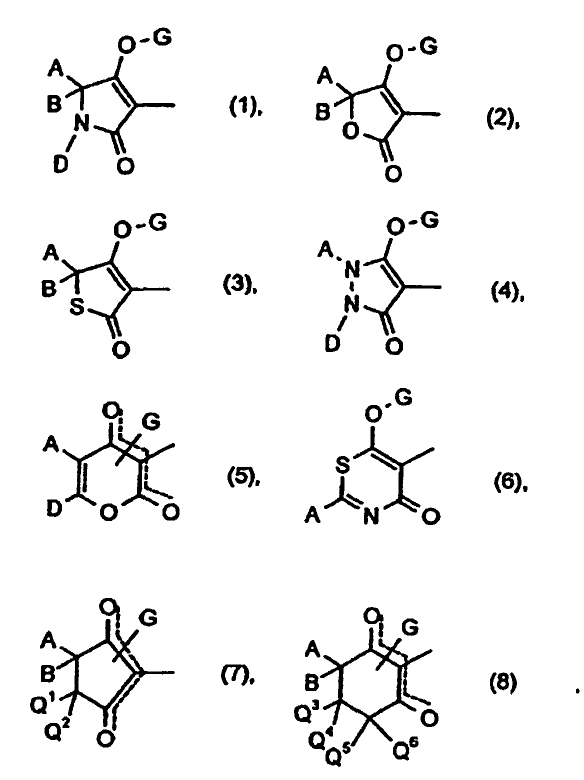
A représente l'hydrogène ou un reste alkyle en C₁ à C₈ ou (alkoxy en C₁ à C₆) - (alkyle en C₁ à C₄), chacun éventuellement substitué par du fluor, un reste cycloalkyle en C₃ à C₆ éventuellement substitué par un radical fluoro, méthyle, éthyle ou méthoxy et dans lequel, le cas échéant, un chaînon du noyau est remplacé par de l'oxygène ou du soufre, ou bien (toutefois non dans le cas des composés (I-5), (I-7) et (I-8)) un reste phényle ou benzyle, chacun éventuellement substitué par un radical fluoro, chloro, bromo, méthyle, éthyle, n-propyle, isopropyle, méthoxy, éthoxy, trifluorométhyle, trifluorométhoxy, cyano ou nitro,
B représente l'hydrogène ou un reste alkyle en C₁ à C₄,
A, B et l'atome de carbone auquel ils sont liés représentent un reste cycloalkyle saturé en C₅ ou C₆ dans lequel, le cas échéant, un chaînon du noyau est remplacé par de l'oxygène ou du soufre et qui est éventuellement substitué une fois par un radical méthyle, éthyle, propyle, isopropyle, butyle, isobutyle, sec.-butyle, tertiobutyle, trifluorométhyle, méthoxy, éthoxy, propoxy, isopropoxy, butoxy, isobutoxy, sec.-butoxy, tertiobutoxy, fluoro ou chloro, ou bien
A, B et l'atome de carbone auquel ils sont liés représentent un reste cycloalkyle en C₅ ou C₆ ou cycloalcényle en C₅ ou C₆, où deux substituants forment conjointement avec les atomes de carbone auxquels ils sont liés un groupe alcanediyle en C₂ à C₄ ou alcènediyle en C₂ à C₄ où, dans chaque cas, un groupe méthylène est éventuellement remplacé par de l'oxygène ou du soufre, ou forment un groupe butadiènediyle,
D représente l'hydrogène, un reste, éventuellement substitué dans chaque cas, par du fluor ou du chlore, alkyle en C₁ à C₈, alcényle en C₃ ou C₄, (alkoxy en C₁ à C₆) - (alkyle en C₁ à C₄), (alkylthio en C₁ à C₄)-(alkyle en C₂ à C₄) ou cycloalkyle en C₃ à C₆ dans lequel, le cas échéant, un groupe méthylène est remplacé par de l'oxygène ou du soufre, ou bien (toutefois non dans le cas des composés de formules (I-1) et (I-4)) un reste phényle, furannyle, pyridyle, thiényle ou benzyle, chacun éventuellement substitué par un radical fluoro, chloro, méthyle, éthyle, n-propyle, isopropyle, méthoxy, éthoxy, trifluorométhyle ou trifluorométhoxy,
ou bien
A et D forment ensemble un reste alcanediyle en C3 ou C4 éventuellement substitué, dans lequel, le cas échéant, un atome de carbone est remplacé par du soufre et qui est éventuellement substitué par un radical hydroxy, méthyle, éthyle, méthoxy ou éthoxy, ou bien
A et D (dans le cas des composés de formule (I-1)) forment conjointement avec les atomes de carbone auxquels ils sont liés, l'un des groupes AD suivant : 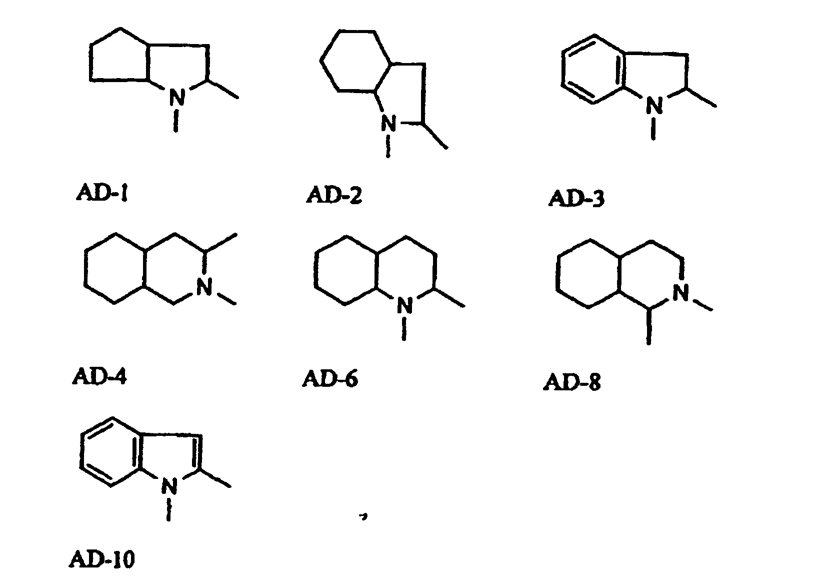
A et Q¹ forment ensemble un reste alcanediyle en C₃ ou C₄ éventuellement substitué une ou deux fois par un radical fluoro, hydroxy, méthyle ou méthoxy, ou un reste butènediyle, ou bien
Q¹ représente l'hydrogène,
Q² représente l'hydrogène,
Q₄, Q₅ et Q₆ représentent indépendamment les uns des autres, l'hydrogène, un reste méthyle ou éthyle,
Q³ représente l'hydrogène, un reste méthyle, éthyle, ou un reste cycloalkyle en C₃ à C₆ où, le cas échéant, un groupe méthylène est remplacé par de l'oxygène ou du soufre, ou bien
Q³ et Q⁴ forment, conjointement avec l'atome de carbone auquel ils sont liés, un noyau pentagonal ou hexagonal saturé éventuellement substitué par un radical méthyle ou méthoxy et dont un chaînon est éventuellement remplacé par de l'oxygène ou du soufre,
G représente l'hydrogène (a) ou l'un des groupes 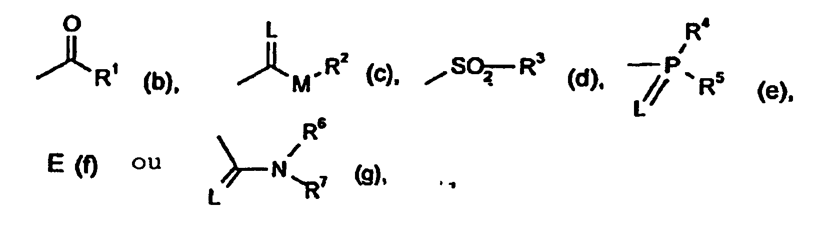 dans lesquels
E représente un ion métallique ou un ion ammonium,
L représente l'oxygène ou le soufre et
M représente l'oxygène ou le soufre,
R¹ représente un reste alkyle en C₁ à C₁₄, alcényle en C₂ à C₁₄, (alkoxy en C₁ à C₄) - (alkyle en C₁ à C₆), (alkylthio en C₁ à C₉)-(alkyle en C₁ à C₆), poly(alkoxy en C₁ à C₄)-(alkyle en C₁ à C₄), chacun éventuellement substitué par du fluor ou du chlore, ou un reste cycloalkyle en C₃ à C₆ éventuellement substitué par un radical fluoro, chloro, méthyle, éthyle, propyle, isopropyle, butyle, isobutyle, tertiobutyle, méthoxy, éthoxy, n-propoxy ou isopropoxy, et dont un ou deux chaînons non directement contigus sont éventuellement remplacés par de l'oxygène et/ou du soufre,
un reste phényle éventuellement substitué par un radical fluoro, chloro, bromo, cyano, nitro, méthyle, éthyle, n-propyle, isopropyle, méthoxy, éthoxy, trifluorométhyle, trifluorométhoxy, méthylthio, éthylthio, méthylsulfonyle ou éthylsulfonyle,
un reste benzyle éventuellement substitué par un radical fluoro, chloro, bromo, méthyle, éthyle, n-propyle, isopropyle, méthoxy, éthoxy, trifluorométhyle ou trifluorométhoxy,
un reste furannyle, thiényle, pyridyle, pyrimidyle, thiazolyle ou pyrazolyle, chacun éventuellement substitué par un radical fluoro, chloro, bromo, méthyle ou éthyle,
un reste phénoxy-(alkyle en C₁ ou C₂) éventuellement substitué par un radical fluoro, chloro, méthyle ou éthyle, ou bien
un reste pyridyloxy-(alkyle en C₁ ou C₂), pyrimidyloxy-(alkyle en C₁ ou C₂) ou thiazolyloxy-(alkyle en C₁ ou C₂), chacun éventuellement substitué par un radical fluoro, chloro, amino, méthyle ou éthyle,
R² représente un reste alkyle en C₁ à C₁₄, alcényle en C₂ à C₁₄, (alkoxy en C₁ à C₄) - (alkyle en C₁ ou C₂) ou poly (alkoxy en C₁ à C₉) - (alkyle en C₂ à C₆), chacun éventuellement substitué par du fluor,
un reste cycloalkyle en C₃ à C₆ éventuellement substitué par un radical fluoro, chloro, méthyle, éthyle, n-propyle, isopropyle ou méthoxy,
ou bien un reste phényle ou benzyle, chacun éventuellement substitué par un radical fluoro, chloro, cyano, nitro, méthyle, éthyle, n-propyle, isopropyle, méthoxy, éthoxy, trifluorométhyle ou trifluorométhoxy,
R³ représente un reste méthyle éventuellement substitué par du fluor, un reste éthyle, n-propyle, isopropyle, ou un reste phényle ou benzyle, chacun éventuellement substitué par un radical fluoro, chloro, bromo, méthyle, tertiobutyle, méthoxy, trifluorométhyle, trifluorométhoxy, cyano ou nitro,
R⁴ et R⁵ représentent, indépendamment l'un de l'autre, un reste alkyle en C₁ à C₄, alkoxy en C₁ à C₄, alkylamino en C₁ à C₄, di (alkyle en C₁ à C₄) amino, alkylthio en C₁ à C₄ ou un reste phényle, phénoxy ou phénylthio, chacun éventuellement substitué par un radical fluoro, chloro, bromo, nitro, cyano, alkoxy en C₁ ou C₂, fluoralkoxy en C₁ ou C₂, alkylthio en C₁ ou C₂, fluoralkylthio en C₁ ou C₂ ou alkyle en C₁ à C₃, et
R⁶ et R⁷ représentent indépendamment l'un de l'autre l'hydrogène, un reste alkyle en C₁ à C₄, cycloalkyle en C₃ à C₆, alkoxy en C₁ à C₄, alcényle en C₃ ou C₄, (alkoxy en C₁ à C₄) - (alkyle en C₁ à C₄), un reste phényle éventuellement substitué par un radical fluoro, chloro, bromo, trifluorométhyle, méthyle ou méthoxy, un reste benzyle éventuellement substitué par un radical fluoro, chloro, bromo, méthyle, trifluorométhyle ou méthoxy, ou forment ensemble un reste alkyle en C₅ ou C₆ dans lequel, le cas échéant, un groupe méthylène est remplacé par de l'oxygène ou du soufre.

4. Procédé de production de composés de formule (I) suivant la revendication 1, **caractérisé en ce que** :
(A) on obtient des composés de formule (I-1-a) 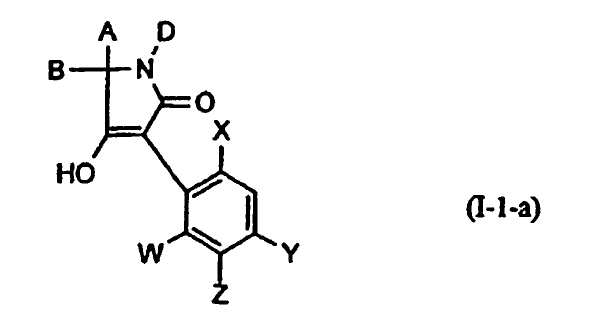 dans laquelle
A, B, D, W, X, Y et Z ont les définitions indiquées dans la revendication 1,
en effectuant la condensation intramoléculaire d'esters de N-acylaminoacides de formule (II) 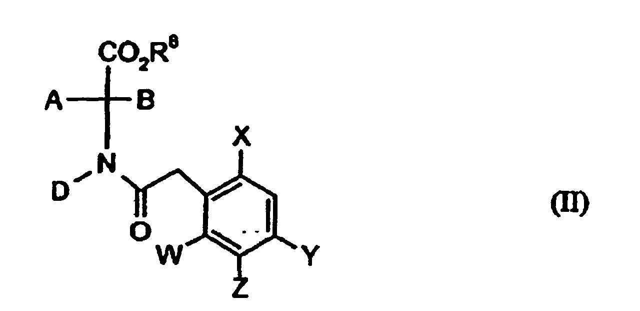 dans laquelle
A, B, D, W, X, Y et Z ont les définitions indiquées ci-dessus,
et
R⁸ représente un reste alkyle,
en présence d'un diluant et en présence d'un diluant et en présence d'une base
(B) on obtient des composés de formule (I-2-a) 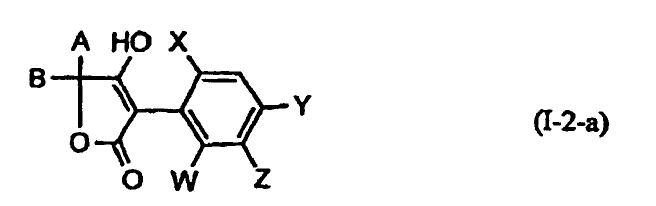 dans laquelle
A, B, W, X, Y et Z ont les définitions indiquées ci-dessus,
en effectuant la condensation intramoléculaire d'esters d'acides carboxyliques de formule (III) 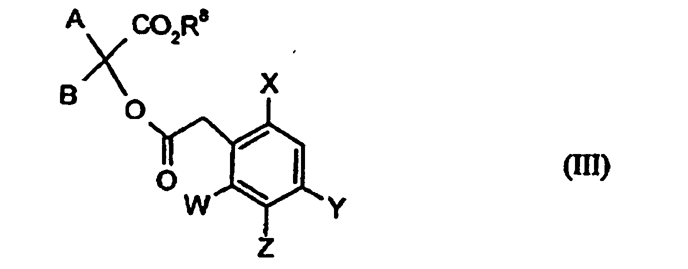 dans laquelle
A, B, W, X, Y, Z et R⁸ ont les définitions indiquées ci-dessus,
en présence d'un diluant et en présence d'une base,
(C) on obtient des composés de formule (I-3-a) 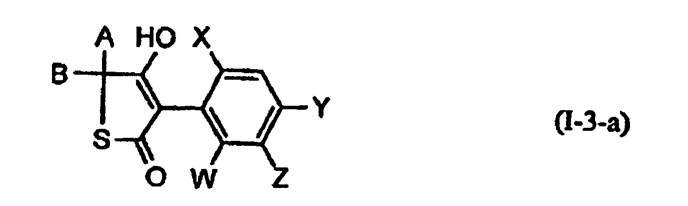 dans laquelle
A, B, W, X, Y et Z ont les définitions indiquées ci-dessus,
en effectuant la cyclisation intramoléculaire d'esters d'acides β-cétocarboxyliques de formule (IV) 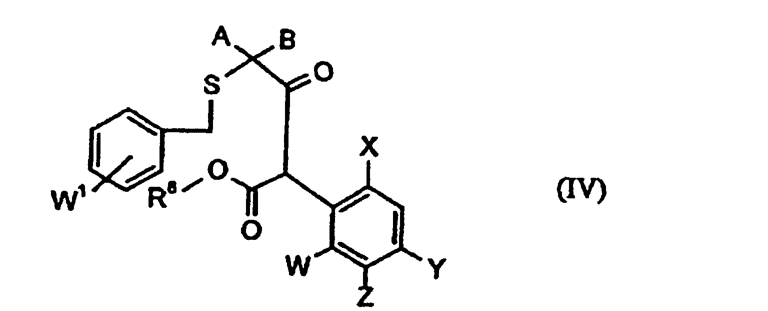 A, B, W, X, Y, Z et R⁸ ont les définitions indiquées ci-dessus, et
W¹ représente l'hydrogène, un halogène, un reste alkyle ou alkoxy,
éventuellement en présence d'un diluant et, le cas échéant, en présence d'un acide,
(D) on obtient des composés de formule (I-4-a) 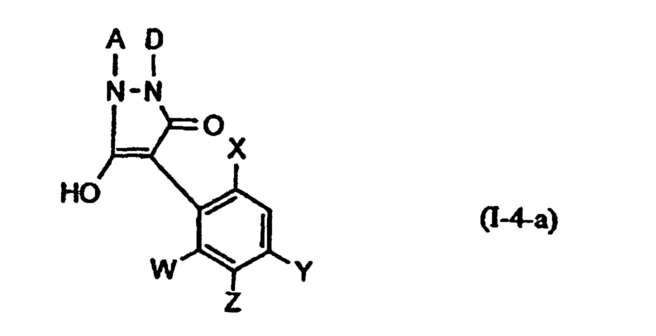 dans laquelle
A, D, W, X, Y et Z ont les définitions indiquées ci-dessus, en faisant réagir
(α) des halogénocarbonylcétènes de formule (V) 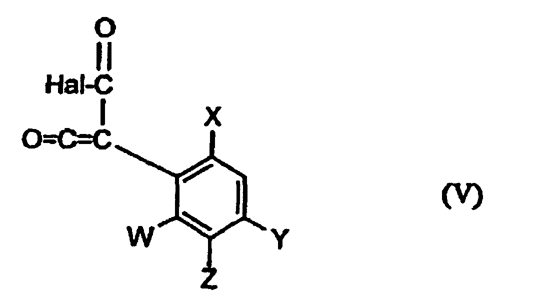 dans laquelle
W, X, Y et Z ont les définitions indiquées ci-dessus et
Hal représente un halogène,
ou bien
(β) des dérivés d'acide malonique de formule (VI) 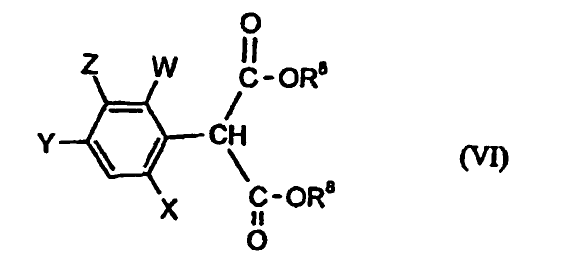 dans laquelle
R⁸, W, X, Y et Z ont les définitions indiquées ci-dessus,
avec des hydrazines de formule (VII)
**A-NH-NH-D** (VCII)
dans laquelle
A et D ont les définitions indiquées ci-dessus, éventuellement en présence d'un diluant et, le cas échéant, en présence d'une base,
E) ont obtient des composés de formule (I-5-a) 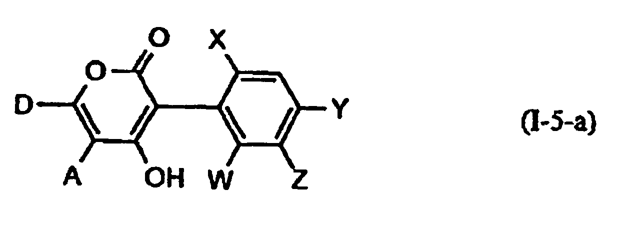 dans laquelle
A, D, W, X, Y et Z ont les définitions indiquées ci-dessus,
en faisant réagir des composés carbonyliques de formule (VIII) 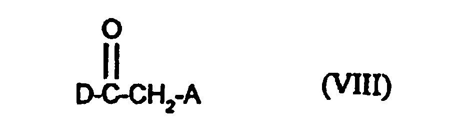 dans laquelle
A et D ont les définitions indiquées ci-dessus,
ou leurs éthers de silylénol de formule (VIIIa) 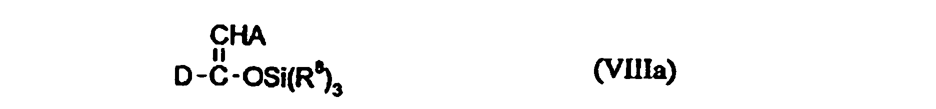 dans laquelle
A, D et R⁸ ont la définition indiquée ci-dessus,
avec des halogénures d'acides céténoïques de formule (V) 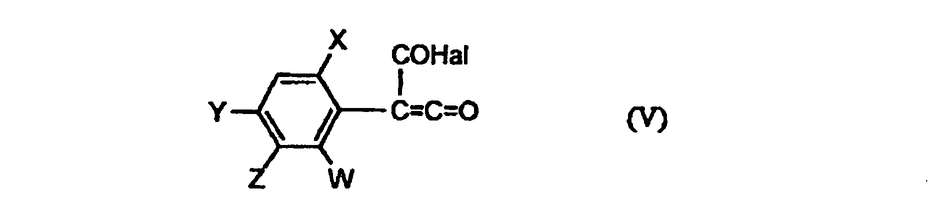 dans laquelle
W, X, Y et Z ont les définitions indiquées ci-dessus et
Hal représente un halogène,
éventuellement en présence d'un diluant et, le cas échéant, en présence d'un accepteur d'acide,
(F) on obtient des composés de formule (I-6-a) 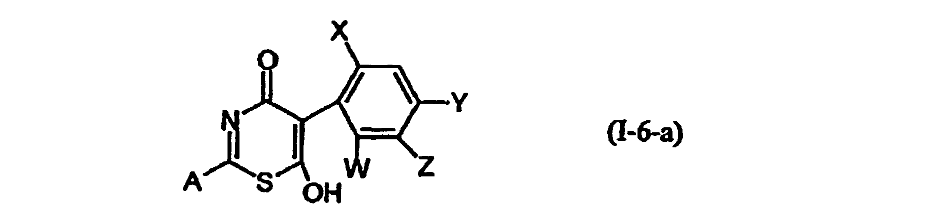 dans laquelle
A, W, X, Y et Z ont la définition indiquée ci-dessus,
en faisant réagir des thioamides de formule (IX) 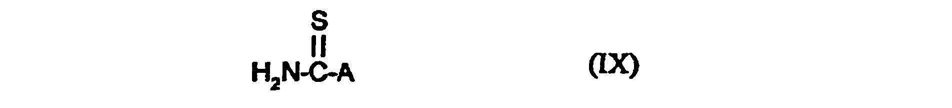 dans laquelle
A a la définition indiquée ci-dessus,
avec des halogénures d'acides céténoïques de formule (V) 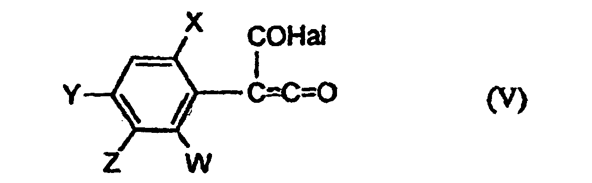 dans laquelle
Hal, W, X, Y et Z ont les définitions indiquées ci-dessus,
éventuellement en présence d'un diluant et, le cas échéant, en présence d'un accepteur d'acide,
(G) on obtient des composés de formule (I-7-a) 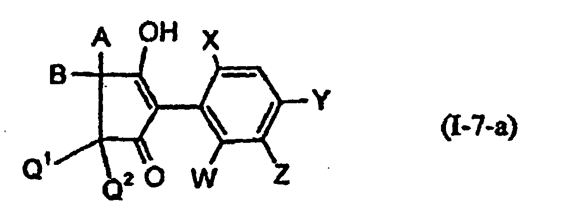 dans laquelle
A, B, Q¹, Q², W, X, Y et Z ont la définition indiquée dans la revendication 1,
en effectuant la cyclisation intramoléculaire d'esters d'acides cétocarboxyliques de formule (X) 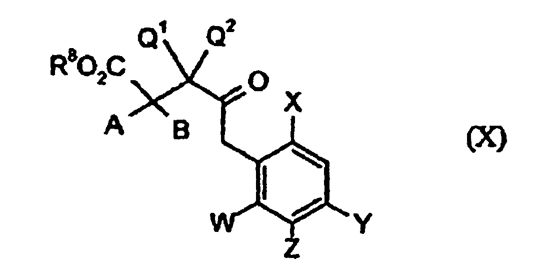 dans laquelle
A, B, Q¹, Q², W, X, Y et Z ont la définition indiquée ci-dessus, et
R⁸ est un reste alkyle,
éventuellement en présence d'un diluant et, le cas échéant, en présence d'une base,
(H) on obtient des composés de formule (I-8-a) 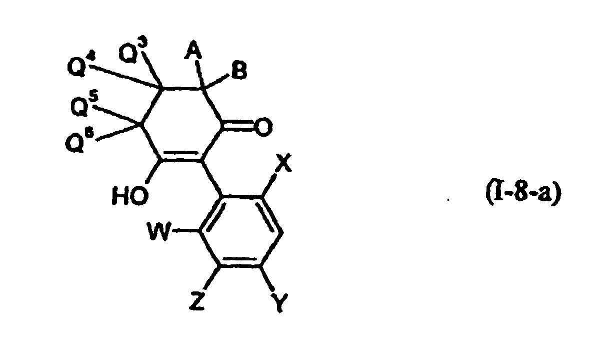 dans laquelle
A, B, Q³, Q⁴, Q⁵, Q⁶, W, X, Y et Z ont la définition indiquée dans la revendication 1,
en effectuant la condensation intramoléculaire d'esters d'acides 6-aryl-5-céto-hexanoiques de formule (XI) 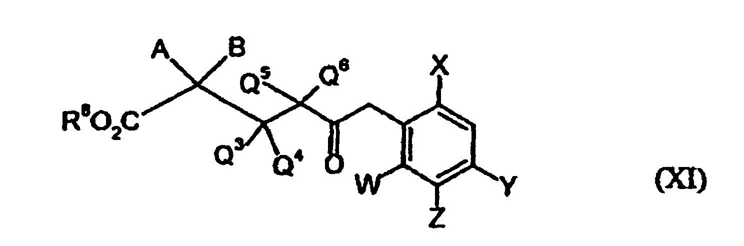 dans laquelle
A, B, Q³, Q⁴, Q⁵, Q⁶, W, X, Y et Z ont la définition indiquée ci-dessus,
et
R⁸ est un reste alkyle,
en présence d'un diluant et, le cas échéant, en présence d'une base,
(I) on obtient des composés de formules (I-8-a) indiquées ci-dessus, dans lesquelles A, B, D, Q¹, Q², Q³, Q⁴, Q⁵, Q⁶, X, Y et Z ont la définition indiquée ci-dessus, en faisant réagir des composés de formules (I-1'-a) à (I-8'-a), 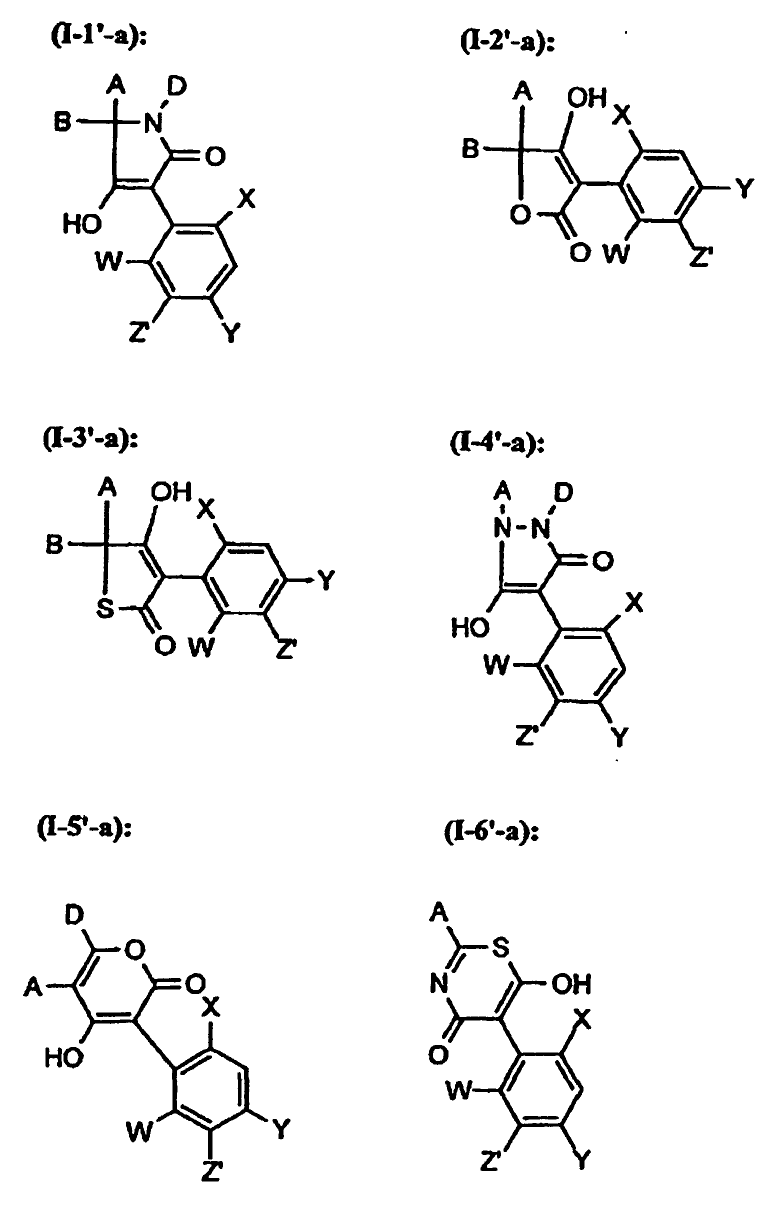 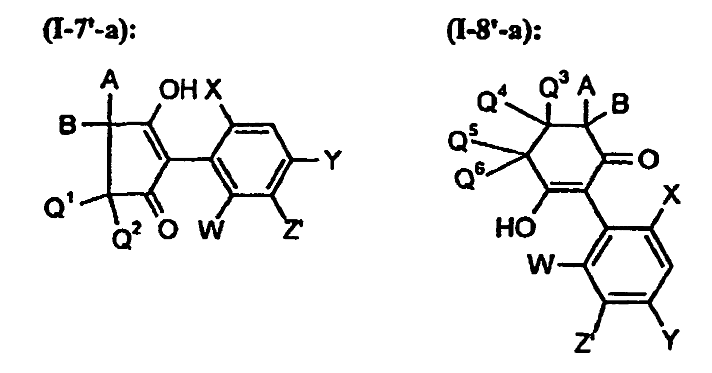 dans lesquelles
A, B, D, Q¹, Q², Q³, Q⁴, Q⁵, Q⁶, W, X et Y ont la définition indiquée ci-dessus, et
Z' représente le chlore, le brome ou l'iode,
avec des acides boroniques de formule (XII) 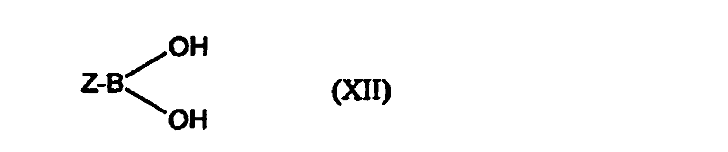 dans laquelle
Z a la définition indiquée ci-dessus,
en présence d'un solvant, d'une base ou d'un catalyseur, puis en faisant éventuellement réagir dans chaque cas les composés ainsi obtenus de formules (I-1-a) à (I-8-a)
(jα) avec des halogénures d'acides de formules (XIII) 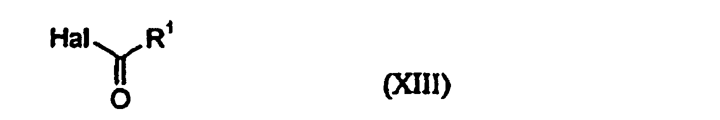 dans laquelle
R¹ a la définition indiquée dans la revendication 1 et
Hal représente un halogène,
ou bien
(β) avec des anhydrides d'acides carboxyliques de formule (XIV)
**R**^{**1**}**-CO-O-CO-R**^{**1**} (XIV)
dans laquelle
R¹ a la définition indiquée ci-dessus,
éventuellement en présence d'un diluant et, le cas échéant, en présence d'un accepteur d'acide, ou dans chaque cas
(K) avec des esters d'acide chloroformique ou des thioesters d'acide chloroformique de formule (XV)
**R**^{**2**}**-M-CO-Cl** (XV)
dans laquelle
R² et M ont les définitions indiquées dans la revendication 1,
éventuellement en présence d'un diluant et, le cas échéant, en présence d'un accepteur d'acide, ou bien dans chaque cas
(L) avec des esters d'acide chloromonothioformique ou des esters d'acide chlorodithioformique de formule (XVI) 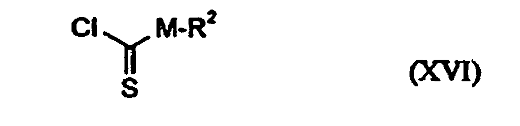 dans laquelle
M et R² ont les définitions indiquées ci-dessus,
éventuellement en présence d'un diluant et, le cas échéant, en présence d'un accepteur d'acide, ou bien dans chaque cas
(M) avec des chlorures d'acides sulfoniques de formule (XVII)
**R**^{**3**}**-SO**_{**2**}**-Cl** (XVII)
dans laquelle
R³ a la définition indiquée dans la revendication 1,
éventuellement en présence d'un diluant et, le cas échéant, en présence d'un accepteur d'acide, ou bien dans chaque cas
(N) avec des composés phosphorés de formule (XVIII) 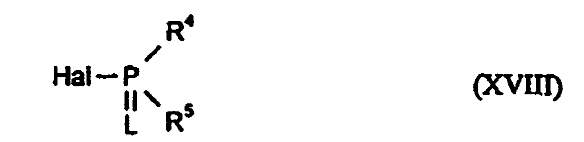 dans laquelle
L, R⁴ et R⁵ ont les définitions indiquées dans la revendication 1, et
Hal représente un halogène,
éventuellement en présence d'un diluant et, le cas échéant, en présence d'un accepteur d'acide, ou bien dans chaque cas
(L) avec des composés métalliques ou des amines de formules (XIX) ou (XX)
**Me(OR**^{**10**}**)**_{**t**} (XIX) 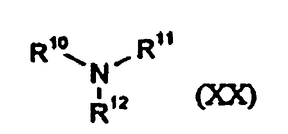
dans lesquelles
Me est un métal monovalent ou divalent,
t représente le nombre 1 ou 2, et
R¹⁰, R¹¹, R¹² représentent indépendamment les uns des autres l'hydrogène ou un reste alkyle,
éventuellement en présence d'un diluant, ou dans chaque cas,
(Pα) avec des isocyanates ou des isothiocyanates de formule (XXI)
**R**^{**6**}**-N=C=L** (XXi)
dans laquelle
R⁶ et L ont les définitions indiquées dans la revendication 1,
éventuellement en présence d'un diluant et, le cas échéant, en présence d'un catalyseur, ou dans chaque cas,
(β) avec des chlorures d'acides carbamiques ou des chlorures d'acides thiocarbamiques de formule (XXII) 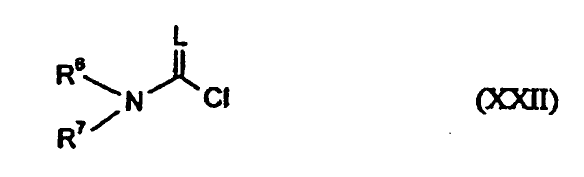 dans laquelle
L, R⁶ et R⁷ ont les définitions indiquées dans la revendication 1,
éventuellement en présence d'un diluant et, le cas échéant, en présence d'un accepteur d'acide.

5. Composés de formule (II) 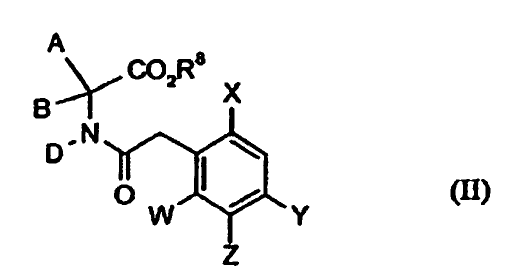 dans laquelle
A, B, D, W, X, Y et Z ont les définitions indiquées dans la revendication 1, et
R⁸ est un reste alkyle.

6. Composés de formule (XXIV) 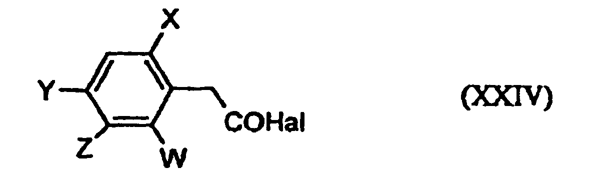 dans laquelle
W, X, Y et Z ont les définitions indiquées dans la revendication 1, et
Hal représente le chlore ou le brome.

7. Composés de formule (XXV) 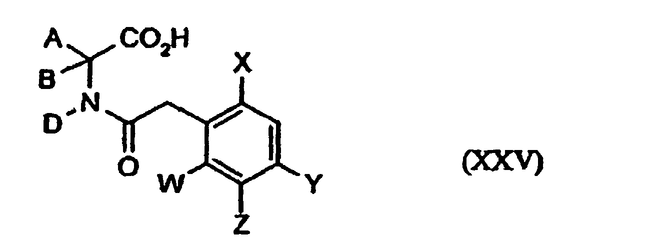 dans laquelle
A, B, D, W, X, Y et Z ont les définitions indiquées dans la revendication 1.

8. Composés de formule (XXIX) 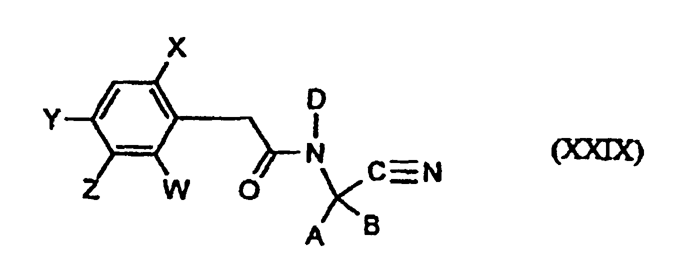 dans laquelle
A, B, D, W, X, Y et Z ont les définitions indiquées dans la revendication 1.

9. Composés de formule (III) 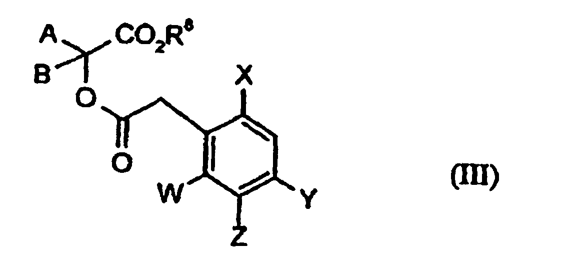 dans laquelle
A, B, W, X, Y et Z ont les définitions indiquées ci-dessus et
R⁸ est un reste alkyle.

10. Composés de formule (XXVII) 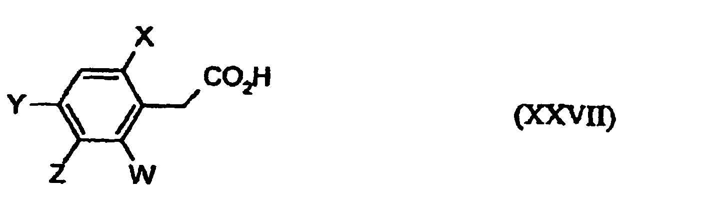 dans laquelle
W, X, Y et Z ont les définitions indiquées dans la revendication 1.

11. Composés de formule (XXXII) 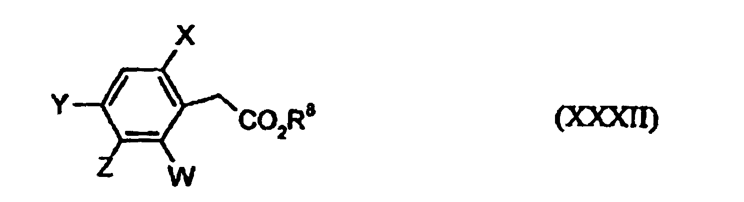 dans laquelle
W, X, Y et Z ont la définition indiquée dans la revendication 1, et
R⁸ est un reste alkyle.

12. Composés de formule (XXVII-b) 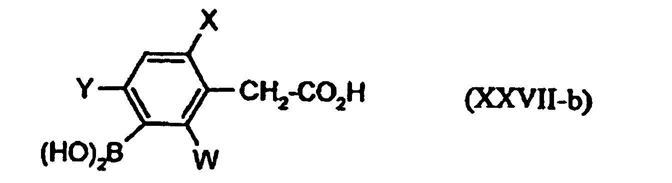 dans laquelle
W, X et Y ont la définition indiquée dans la revendication 1.

13. Composés de formule (IV) 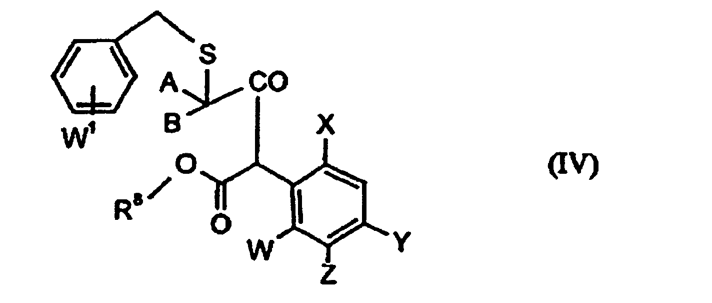 dans laquelle
A, B, W, W¹, X, Y et Z ont les définitions indiquées dans la revendication 1, et
R⁸ est un reste alkyle.

14. Composés de formule (V) 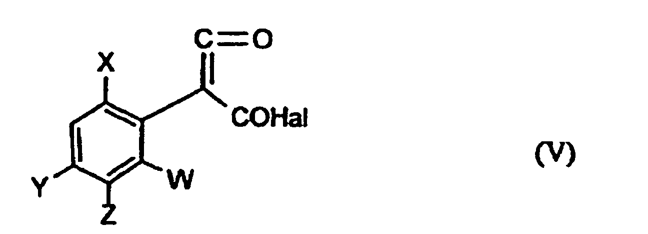 dans laquelle
W, X, Y et Z ont les définitions indiquées dans la revendication 1, et
Hal représente le chlore ou le brome.

15. Composés de formule (XXXVII) 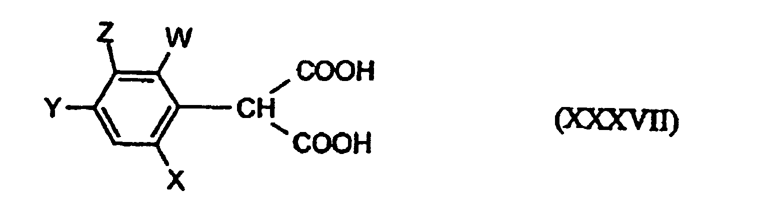 dans laquelle
W, X, Y et Z ont les définitions indiquées dans la revendication 1.

16. Composés de formule (VI) 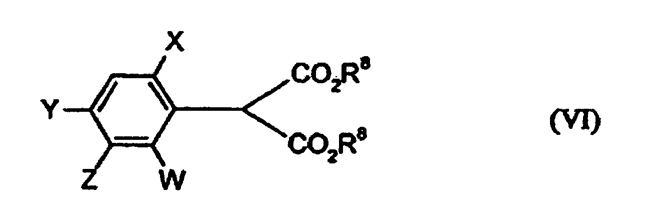 dans laquelle
W, X, Y et Z ont la définition indiquée dans la revendication 1, et
R⁸ est un reste alkyle.

17. Composés de formule (X) 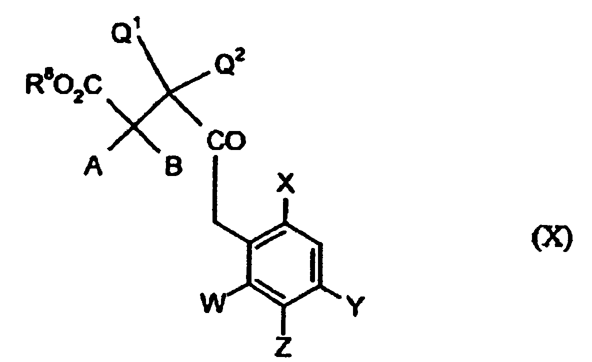 dans laquelle
A, B, Q¹, Q², W, X, Y et Z ont la définition indiquée dans la revendication 1, et
R⁸ est un reste alkyle.

18. Composés de formule (XXXVIII) 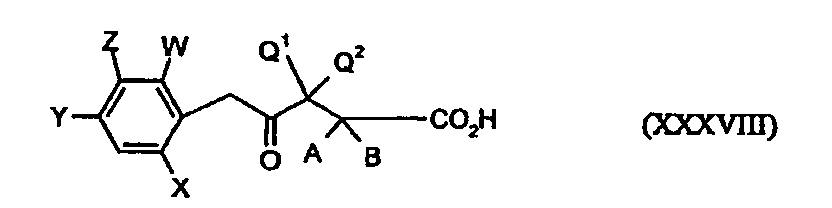 dans laquelle
W, X, Y, Z, A, B, Q¹ et Q² ont la définition indiquée dans la revendication 1.

19. Composés de formule (XXXIX) 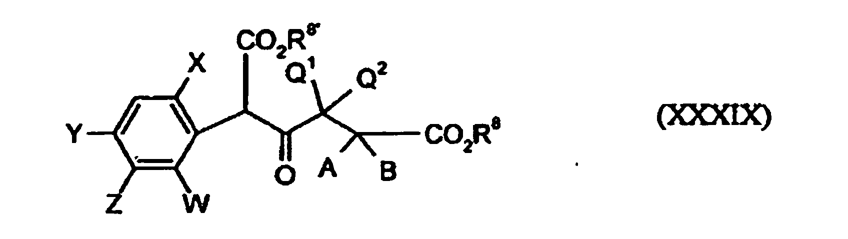 dans laquelle
A, B, D¹, D², W, X, Y et Z ont la définition indiquée dans la revendication 1, et
R⁸ et R^{8'} sont des restes alkyle.

20. Composés de formule (XI) 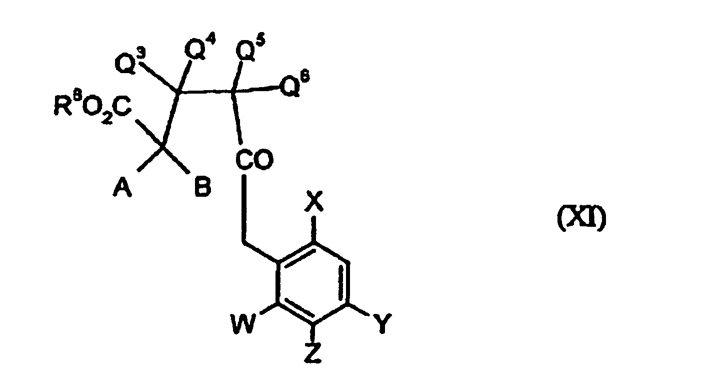 dans laquelle
A, B, Q³, Q⁴, Q⁵, Q⁶, W, X, Y et Z ont la définition indiquée dans la revendication 1, et
R⁸ est un reste alkyle.

21. Composés de formule (XLII) 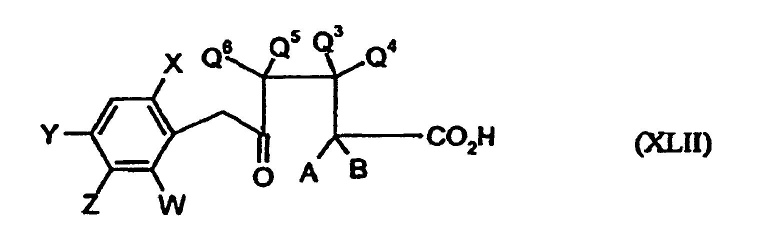 dans laquelle
A, B, Q³, Q⁴, Q⁵, Q⁶, W, X, Y et Z ont la définition indiquée dans la revendication 1.

22. Composés de formule (XLIII) 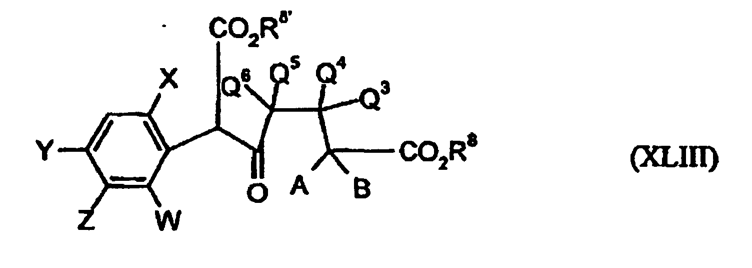 dans laquelle
A, B, Q³, Q⁴, Q⁵, Q⁶, W, X, Y et Z ont la définition indiquée dans la revendication 1, et
R⁸ et R^{8'} sont des restes alkyle.

23. Composition pesticide et/ou composition herbicide, **caractérisée par** une teneur en au moins un composé de formule (I) suivant la revendication 1.

24. Utilisation de composés de formule (I) suivant la revendication 1 pour la lutte contre des parasites dans la protection des plantes, le domaine domestique, le secteur de l'hygiène et la protection des denrées emmagasinées, excepté sur le corps humain ou animal.

25. Procédé de lutte contre des parasites dans la protection des plantes, le domaine domestique, le secteur de l'hygiène et la protection des denrées emmagasinées, excepté sur le corps humain ou animal, **caractérisé en ce qu'**on fait agir des composés de formule (I) suivant la revendication 1 sur les parasites et/ou sur leur milieu.

26. Procédé de préparation de compositions pesticides et/ou herbicides, **caractérisé en ce qu'**on mélange des composés de formule (I) suivant la revendication 1 avec des diluants et/ou des agents tensioactifs.
